# FASCICULE DE BREVET EUROPEEN

(11) **EP 0 511 917 B1**
(45) Date de publication et mention de la délivrance du brevet: **23.10.1996**
(21) Numéro de dépôt: 92401218.0
(22) Date de dépôt: 29.04.1992
(51) Int. Cl.: C07H 15/252, C07F 7/18, C07H 15/203, A61K 31/70, C07H 15/26

(54) **Prodrogues glycosylées, leur procédé de préparation et leurs utilisations**
Glykosylierte Arzneimittel-Vorstufen, Verfahren zu ihrer Herstellung und ihre Verwendungen
Glycosylated prodrugs, process for their preparation and uses thereof

(30) Priorité: 30.04.1991 FR 9105326
(43) Date de publication de la demande: 04.11.1992
(73) Titulaire: LABORATOIRES HOECHST S.A., 92800 Puteaux (FR); BEHRINGWERKE Aktiengesellschaft, 35001 Marburg (DE)
(72) Inventeur: Jacquesy, Jean-Claude, F-86180 Buxerolles (FR); Gesson, Jean-Pierre, F-86360 Chasseneuil du Poitou (FR); Monneret, Claude, F-75012 Paris (FR); Mondon, Martine, F-86000 Poitiers (FR); Renoux, Brigitte, F-86800 Saint Julien L'Ars (FR); Florent, Jean-Claude, F-91940 Les Ulis (FR); Koch, Michel, F-78170 La Celle Saint Cloud (FR); Tillequin, François, F-75014 Paris (FR); Sedlacek, Hans Harald, W-3550 Marburg (DE); Gerken, Manfred, W-3550 Marburg (DE); Kolar, Cenek, W-3550 Marburg (DE); Gaudel, Gilbert, F-75013 Paris (FR); Bosslet, Klaus, W-3550 Marburg (DE); Czech, Jörg, W-3550 Marburg (DE); Hoffman, Dieter, W-3550 Marburg-Elnhausen (DE); Seeman, Gerhard, W-3550 Marburg-Elnhausen (DE); Schorlemmer Hans-Ulrich, W-3550 Marburg-Dagobertshausen (DE); Dickneite, Gerhard, W-3550 Marburg-Cappel (DE)
(74) Mandataire: Orès, Bernard

(56) Documents cités:
- EP-A- 0 410 366

## Description

La présente invention est relative à des prodrogues glycosylées, à un procédé pour leur préparation et à leurs utilisations seules ou avec des conjugués immuno-enzymatiques spécifiques de tumeurs, dans le traitement des cancers.

De manière plus spécifique, la présente invention est relative à des prodrogues comprenant des anthracyclines modifiées qui peuvent être scindées notamment par l'action desdits conjugués immuno-enzymatiques spécifiques de tumeurs, pour donner des substances cytotoxiques actives vis-à-vis des cellules tumorales.

La combinaison d'une prodrogue et de conjugués enzyme-anticorps monoclonaux, comme agents thérapeutiques, a été décrite dans la littérature. De manière générale, les anticorps impliqués, qui sont dirigés contre un tissu spécifique et qui sont liés de manière covalente à une enzyme ayant la capacité de scinder la prodrogue, sont d'abord injectés à un animal approprié, notamment l'homme, puis on administre ensuite une prodrogue qui peut être activée par l'enzyme. La prodrogue est convertie, par l'action du conjugué enzyme-anticorps, qui est ancré au niveau du tissu spécifique en cytotoxine, qui exerce un effet cytotoxique sur ledit tissu.

La Demande Internationale WO 81/01145, au nom de UNIVERSITY OF ILLINOIS FOUNDATION, décrit des prodrogues activables par des enzymes hydrolytiques et définit cinq critères pour une efficacité optimale d'une prodrogue : (1) il doit y avoir suffisamment d'enzyme activante au niveau de la tumeur, de façon à ce que des taux cytotoxiques d'agent antitumoral soient libérés au niveau de la tumeur, (2) la prodrogue ne doit pas être activée dans d'autres zones que celle de la tumeur, (3) la prodrogue doit être un substrat approprié de l'enzyme associée à la tumeur, dans des conditions physiologiques, (4) la prodrogue ne doit pas être toxique ou beaucoup moins toxique que l'agent antitumoral activé et (5) la substance activée doit avoir une demi-vie biologique courte de telle sorte que les effets toxiques soient limités à la tumeur.

Plus spécifiquement, il est précisé dans cette Demande, que des agents antitumoraux peuvent être rendus spécifiques vis-à-vis d'une tumeur par adjonction d'un peptide qui convertit ledit agent en une prodrogue pharmacologiquement inactive, mais sélectivement activable uniquement au niveau du site tumoral par une enzyme présente en quantités importantes au niveau de la tumeur (plasmine et activateur du plasminogène, notamment). La partie peptidique de la prodrogue présente une séquence en amino-acides telle qu'elle sera scindée enzymatiquement de la partie agent antitumoral, par des protéases telles que la plasmine ou l'activateur du plasminogène, de manière à libérer l'agent antitumoral, sous sa forme active dans la zone tumorale.

Les prodrogues activables par des enzymes hydrolytiques peuvent avoir une structure dans laquelle le peptide et la partie antitumorale sont liées de manière covalente par l'intermédiaire d'un connecteur auto-immolable ayant une structure moléculaire telle que le clivage enzymatique peptide/connecteur auto-immolable entraînera spontanément le clivage de sa liaison avec la partie antitumorale.

Cependant, les prodrogues décrites dans cette Demande internationale ne peuvent être utilisées que dans les cancers qui entraînent une production accrue d'enzymes et plus particulièrement de protéases, au niveau du site tumoral ; or, ces enzymes d'activation, aptes à cliver les prodrogues décrites dans cette Demande, ne se retrouvent pas en quantité suffisante dans les cancers humains, de sorte que ces prodrogues ne permettent pas d'obtenir la toxicité sélective recherchée (K.D. BAGSHAWE, Br. J. Cancer, 1987, 56, 531).

La Demande Internationale WO 88/07378, au nom de CANCER RESEARCH CAMPAIGN TECHNOLOGY LTD décrit un système thérapeutique qui contient d'une part un conjugué enzyme-anticorps et d'autre part une prodrogue qui peut être activée par l'enzyme. L'anticorps du conjugué enzyme anticorps reconnait un antigène spécifique d'une tumeur et l'enzyme est apte à convertir la prodrogue en agent cytotoxique.

Cette Demande précise qu'il est préférable d'utiliser des enzymes autres que des enzymes de mammifères, de manière à prévenir une libération prématurée d'agent cytotoxique par des enzymes endogènes.

De manière plus spécifique, cette Demande décrit des moutardes à l'azote modifiées (telles que l'acide p-bis-N-(2-chloroéthyl)amino-benzylglutamique et ses dérivés), convertibles en moutardes à l'azote en présence de carboxypeptidases et des anthracyclines dont le groupe amino terminal est transformé en amide en présence d'un amino-acide.

Toutefois ces prodrogues présentent l'inconvénient majeur de conserver une cytotoxicité intrinsèque non négligeable.

La Demande européenne 302 473 décrit également un système thérapeutique qui contient deux composants et dans lequel le conjugué enzyme-anticorps situé sur le tissu tumoral, scinde une prodrogue en un composé actif cytotoxique. De manière plus spécifique, les conjugués enzyme-anticorps contiennent de la phosphatase alcaline (PA), de la pénicilline V amidase (PVA) ou de la cytosine désaminase (CD) et sont utilisés en association respectivement avec du 4′-phosphate étoposide et ses dérivés (ou de la 7-(2-aminoéthyl phosphate) mitomycine), avec de la N-(p-hydroxyphénoxyacétyl)adriamycine ou de la 5-fluorocytosine comme prodrogue.

Le système décrit dans cette Demande présente toutefois l'inconvénient d'utiliser soit une enzyme circulante, la phosphatase alcaline, susceptible d'activer précocément la prodrogue au niveau circulatoire, soit une enzyme exogène (PVA ou CD), susceptible d'entraîner des phénomènes d'intolérance ou de sensibilisation.

La Demande Internationale WO 90/07929, au nom de AKZO NV décrit une méthode d'activation *in vivo*, spécifique de site, d'une prodrogue, chez un animal, en utilisant un conjugué activateur-substance spécifique vis-à-vis de la cible, dont la partie activateur permet la conversion de la prodrogue en substance pharmacologiquement active. L'activateur est notamment une enzyme, tel que le lysozyme, d'origine humaine, qui n'est pas présente dans la circulation ou alors en très petites quantités et dont les substrats naturels sont également absents de la circulation ou de la surface des cellules non-cibles. La substance spécifique vis-à-vis de la cible est notamment un anticorps dirigé contre un antigène spécifique de tumeur. La prodrogue peut notamment comprendre une anthracycline (doxorubicine, par exemple) modifiée par un oligomère de chitine lié à l'anthracycline par un groupe amino au niveau du carbonyle C₁₃ sur l'anthracycline ou sur la partie glycosylée.

Toutefois, le système proposé dans cette Demande internationale présente notamment l'inconvénient majeur de ne pas libérer la doxorubicine elle-même, mais un dérivé de celle-ci, Dox-(GlcNAc)₁ ou Dox-(GlcNAc)₅ ; il ne s'agit donc pas, d'une part, d'une prodrogue au sens strict, et d'autre part, on ne dispose, en ce qui concerne ces dérivés, de recul suffisant tant du point de vue pharmacologique que du point de vue toxicologique.

Il ressort de ce qui précède que les principaux inconvénients des systèmes antérieurs sont :
1) en ce qui concerne le choix de l'enzyme :
   - le clivage non désiré de la prodrogue (enzyme circulante) ;
   - le clivage de la prodrogue lié à la production d'une enzyme en quantité importante au niveau du site tumoral ;
   - l'utilisation d'enzymes exogènes susceptibles d'entraîner des phénomènes d'intolérance ou de sensibilisation ;
2) en ce qui concerne le choix de la prodrogue :
   - la cytotoxicité intrinsèque de la prodrogue ;
   - la production d'un dérivé d'anthracycline dont les effets pharmacologiques et toxiques ne sont pas suffisamment connus ;
   - l'utilisation d'une prodrogue à deux compartiments (substrat de l'enzyme + agent cytotoxique), qui a l'inconvénient d'entraîner des interférences stériques ou électroniques avec la réaction de clivage enzymatique.

La Demanderesse s'est en conséquence donné pour but de fournir des prodrogues aptes à être transformées en substances pharmacologiquement actives notamment en présence d'un conjugué enzymatique approprié, qui répondent mieux aux besoins de la pratique que les prodrogues de l'Art antérieur, notamment en ce qu'elles sont stables, en ce qu'elles n'entraînent pas d'interférences stériques ou électroniques, lors de la réaction de clivage enzymatique et en ce qu'elles fournissent au site tumoral uniquement, la substance cytotoxique active.

La présente invention a pour objet des prodrogues d'anthracyclines, caractérisées en ce qu'elles répondent à la formule I ci-après : 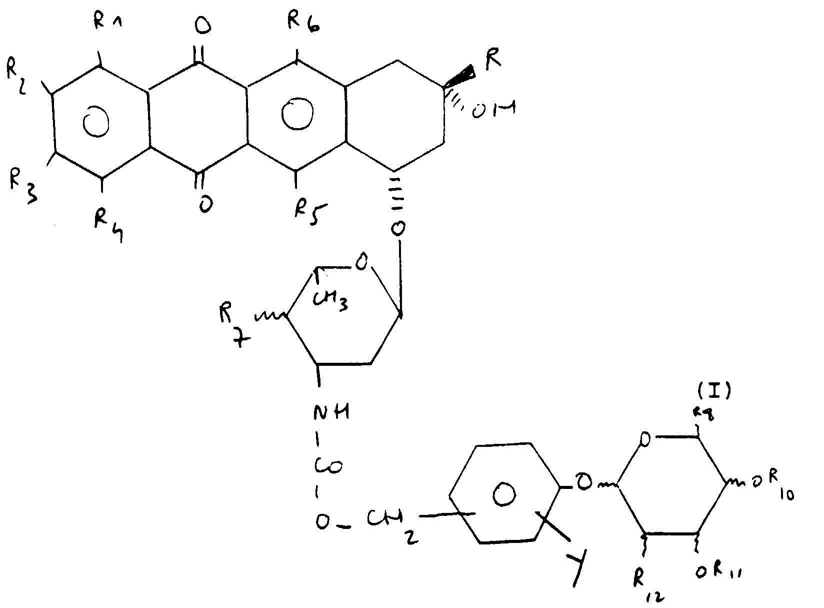 dans laquelle,
R₁, R₂, R₃, qui peuvent être identiques ou différents, représentent un atome d'hydrogène ou un groupe hydroxyle ;
R₄ représente un atome d'hydrogène, un groupe hydroxyle ou un groupe méthoxy ;
R représente un groupe CO-CH₂-R˝, dans lequel R˝ est un atome d'hydrogène, un groupe alkyle en C₁-C₆, un groupe hydroxyle, un groupe alkoxyle, un groupe O-acyle ou un groupe aryle ;
R₅ et R₆, qui peuvent être identiques ou différents, représentent un atome d'hydrogène ou un groupe hydroxyle ;
R₇ représente un atome d'hydrogène ou un groupe hydroxyle ;
R₈ représente un groupe -CH₂-OR₉ ou un groupe COOR₉ avec
R₉ étant un alkyle en C₁-C₃ ou un atome d'hydrogène ;
R₁₀ et R₁₁ représentent un atome d'hydrogène, un groupement acyle protecteur ou un groupe alkyle ;
R₁₂ représente un groupe hydroxyle, un groupement amine, un groupement amide ou un groupement O-acyle protecteur ;
le -CH₂ benzylique est de préférence en position para ou ortho de l'oxygène glycosylé ;
Y représente un atome d'hydrogène ou au moins un groupement électro-attracteur notamment choisi dans le groupe qui comprend le groupement NO₂, un atome d'halogène, un groupement SO₂X, (avec X = -CH₃, C₆H₄-CH₃, NH₂, N-(alkyle_{C1-C4})₂, NH-(alkyle_{C1-C4}), -CN, acyle ou COO-alkyle, et/ou au moins un groupement électrodonneur choisi dans le groupe qui comprend les groupements de type O-alkyle, NHCO-alkyle, N(alkyle)CO-alkyle, S-alkyle, alkyle.

On entend, au sens de la présente invention, par groupements acyle et alkyle des groupements comprenant de 1 à 6 atomes de carbone.

Selon un mode de réalisation avantageux de l'invention, lorsque Y représente un/des groupements électroattracteurs, ceux-ci sont de préférence en position ortho et/ou para de l'oxygène glycosylé et lorsque Y représente un/des groupements électrodonneurs, ceux-ci sont de préférence en position méta.

Conformément à l'invention :
. lorsque le CH₂ benzylique est en position ortho par rapport audit oxygène glycosylé, Y est en position para et représente un atome d'hydrogène ou un groupement électroattracteur et/ou en position méta et représente un atome d'hydrogène ou un groupement électrodonneur,
. lorsque le CH₂ benzylique est en position para, Y est en position ortho et représente un atome d'hydrogène ou un groupement électroattracteur et/ou en position méta et représente un atome d'hydrogène ou un groupement électrodonneur.

On obtient ainsi des dérivés para ou ortho-hydroxy benzyl carbamates d'anthracyclines, dont la fonction phénol est masquée et qui, de manière surprenante, peuvent être scindés en anthracycline cytotoxique active pharmacologiquement et en ose, aussi bien par une glycosidase que par un conjugué glycosidase-ligand spécifique d'une tumeur.

Les composés de formule I conformes à l'invention englobent leurs différents isomères.

Les prodrogues d'anthracyclines conformes à l'invention, à 3 compartiments, c'est-à-dire comprenant une anthracycline, un bras autoimmolable (para ou ortho hydroxybenzylcarbamates) et un substrat enzymatique (ose) présentent les avantages suivants :
- elles préviennent les risques de non reconnaissance enzyme/substrat,
- elles évitent les problèmes d'interférences stériques ou électroniques, lors de la réaction de clivage enzymatique,
- la connection groupement amine du sucre-liaison O-hétérosidique, permet à la fois l'attaque enzymatique et fournit un encombrement suffisant de la molécule pour diminuer significativement la cytotoxicité de la prodrogue.

De manière avantageuse, le bras intermédiaire est de préférence, comme précisé ci-dessus, un para ou ortho hydroxybenzylcarbamate et la libération de l'anthracycline active à partir de la prodrogue à 3 compartiments est déterminée par deux processus :
(1) la vitesse de l'hydrolyse enzymatique,
(2) la vitesse de coupure du bras auto-immolable, qui sont en fait liés, dans la mesure où le bras auto-immolable joue un rôle important à la fois sur la vitesse de l'hydrolyse enzymatique et sur sa vitesse de coupure,
   conformément au schéma A ci-après.

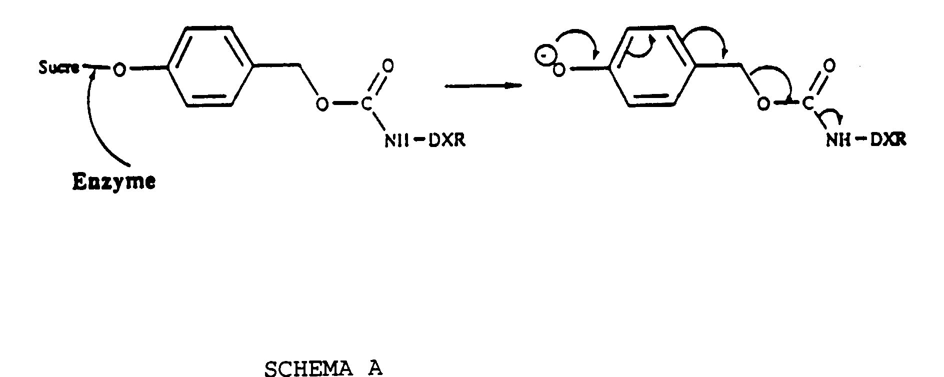

Selon un autre mode de réalisation avantageux de l'invention, les composés préférés de formule I comprennent notamment les radicaux suivants :
R₁, R₂, R₃ sont des atomes d'hydrogène,
R₄ est un groupe méthoxy,
R₅ et R₆ sont des groupes hydroxyles,
R est un groupe -CO-CH₃ ou un groupe -CO-CH₂OH,
R₇ est un atome d'hydrogène, ou un groupe hydroxyle,
R₈ est un groupe -CH₂-OAc, -CH₂OH, -COOMe, ou -COOH,
R₁₀ et R₁₁ qui peuvent être identiques ou différents représentent un atome d'hydrogène ou un groupement Ac,
R₁₂ représente un groupe hydroxyle ou un groupe OAc, lesquels radicaux R₈, R₁₀, R₁₁ et R₁₂ ont de préférence les positions suivantes : 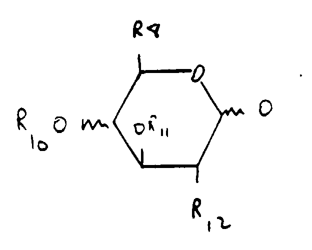
Y est un atome d'hydrogène, un groupe NO₂ ou un atome de chlore, en para ou en ortho de l'oxygène glycosylé, et/ou un groupe OCH₃ en méta de l'oxygène glycosylé.

La présente invention a également pour objet un procédé de préparation d'un composé de formule I qui peut notamment être dégradé par une glycosidase, caractérisé en ce qu'il comprend :
(1) le couplage, en présence, éventuellement, d'un promoteur approprié, d'un dérivé de formule A : 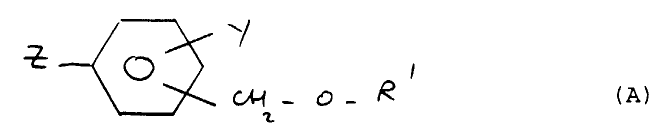 dans laquelle
   Z représente un groupe hydroxyle, un groupe O-trialkylsilyle ou un groupe 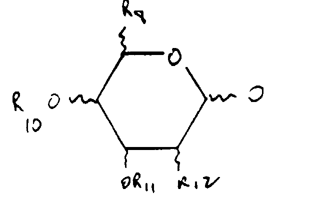 dans lequel R₈, R₁₀, R₁₁ et R₁₂ ont la même signification que ci-dessus ;
   R′ représente un atome d'hydrogène ou l'un des groupes suivants : 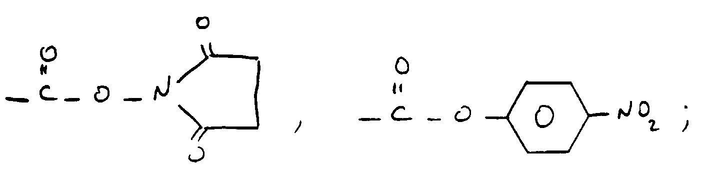 le -CH₂ benzylique est de préférence en position para ou ortho de la fonction phénol, éventuellement modifiée (glycosylée ou silylée) ;
   Y représente un atome d'hydrogène, ou au moins un groupement électro-attracteur notamment choisi dans le groupe qui comprend le groupement NO₂, un atome d'halogène, un groupement SO₂X, (avec X = -CH₃, C₆H₄-CH₃, NH₂, N-(alkyle_{C1-C4})₂, NH-alkyle_{C1-C4}), -CN, acyle ou COO-alkyle, et/ou au moins un groupement électrodonneur choisi dans le groupe qui comprend les groupements de type O-alkyle, NH-CO-alkyle, N(alkyl)CO-alkyle, S-alkyle, alkyle avec une anthracycline de formule B 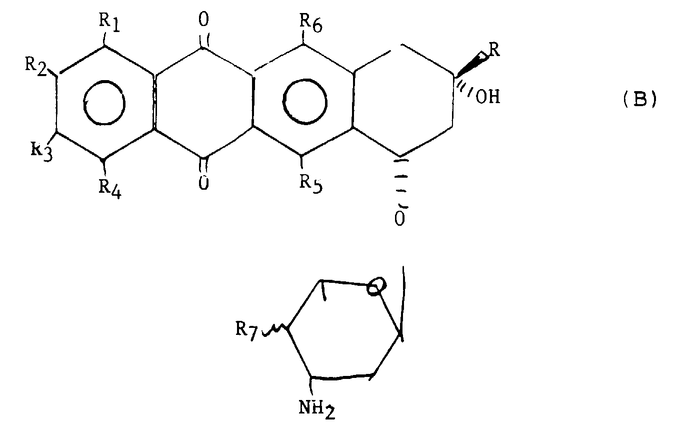 dans laquelle R₁, R₂, R₃, R₄, R₅, R₆, R₇ et R ont la même signification que ci-dessus,
(2) l'élimination des groupes protecteurs présents dans les composés obtenus, notamment par hydrolyse, transestérification ou saponification, et
(3) éventuellement la condensation appropriée avec un ose de formule suivante : 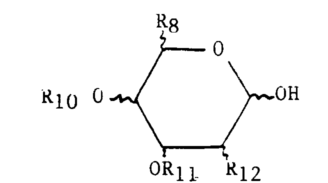 dans le cas où Z représente un groupement hydroxyle ou un groupe O-trialkylsilyle,
pour donner une prodrogue d'anthracycline de formule I, dans laquelle tous les radicaux R₁ à R₁₂ et R ont la même signification que précédemment.

Lorsque Z est un groupe O-trialkylesilyle, il est, préalablement à la condensation avec un ose, avantageusement désilylé par du fluorure de tétrabutyl ammonium, par exemple.

Selon un mode de réalisation avantageux dudit procédé, le composé de formule A est un dérivé hydroxy benzyle glycosylé de formule A₁ : 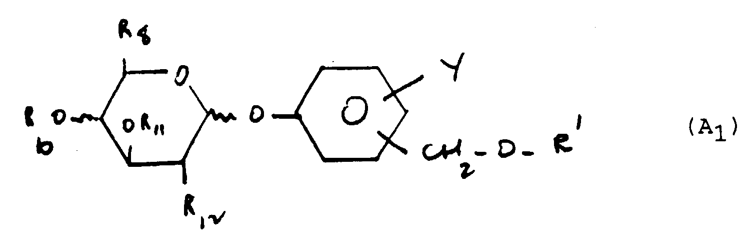 dans laquelle R₈, R₁₀, R₁₁, R₁₂, R′ et Y ont la même signification que ci-dessus,
pour fournir, après couplage avec une anthracycline de formule B, une prodrogue d'anthracycline de formule I, telle que définie ci-dessus,
conformément aux schémas I, II, III, IV, VI, X, XI, XIV, XV et XVI ci-après.

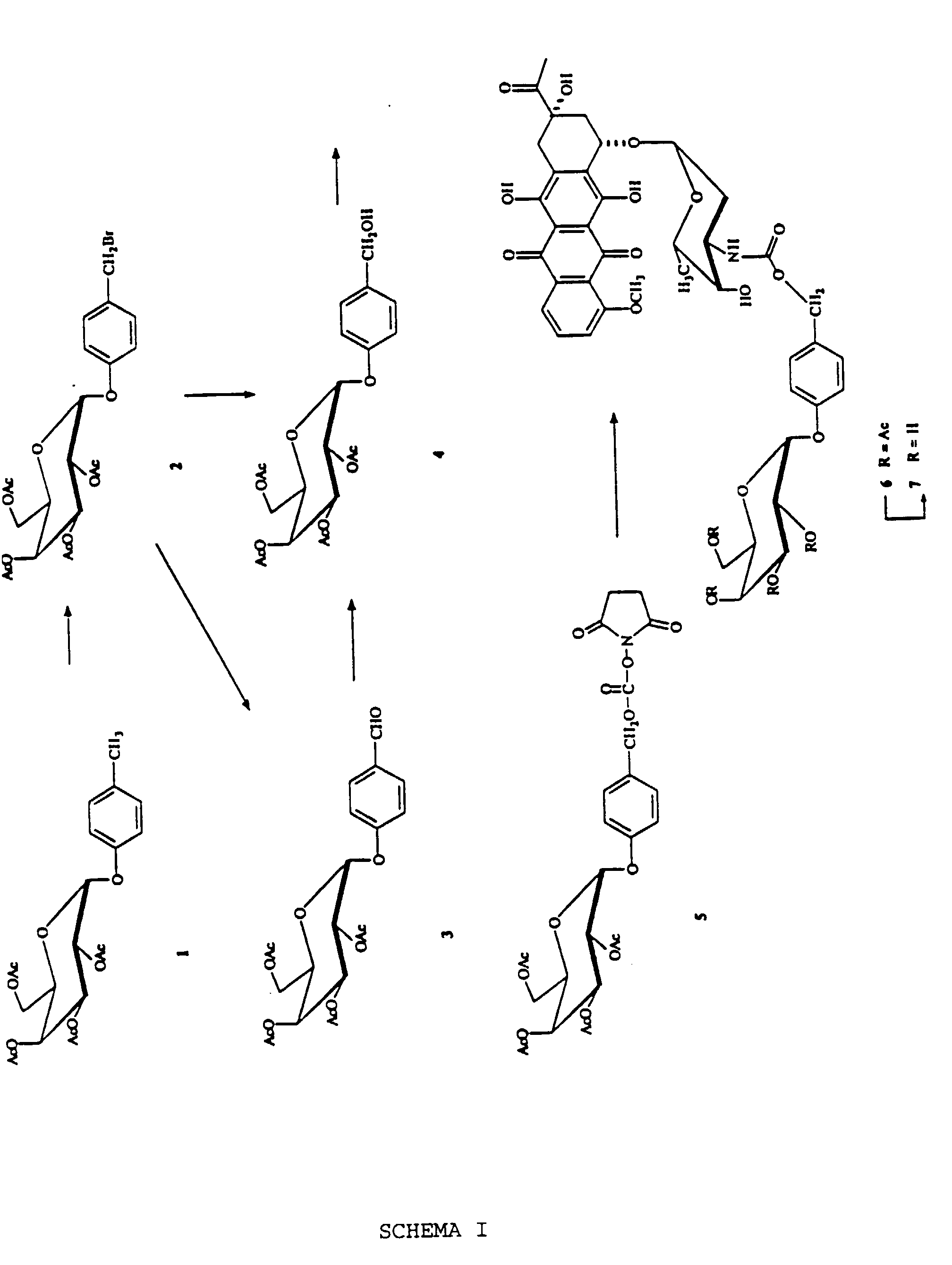

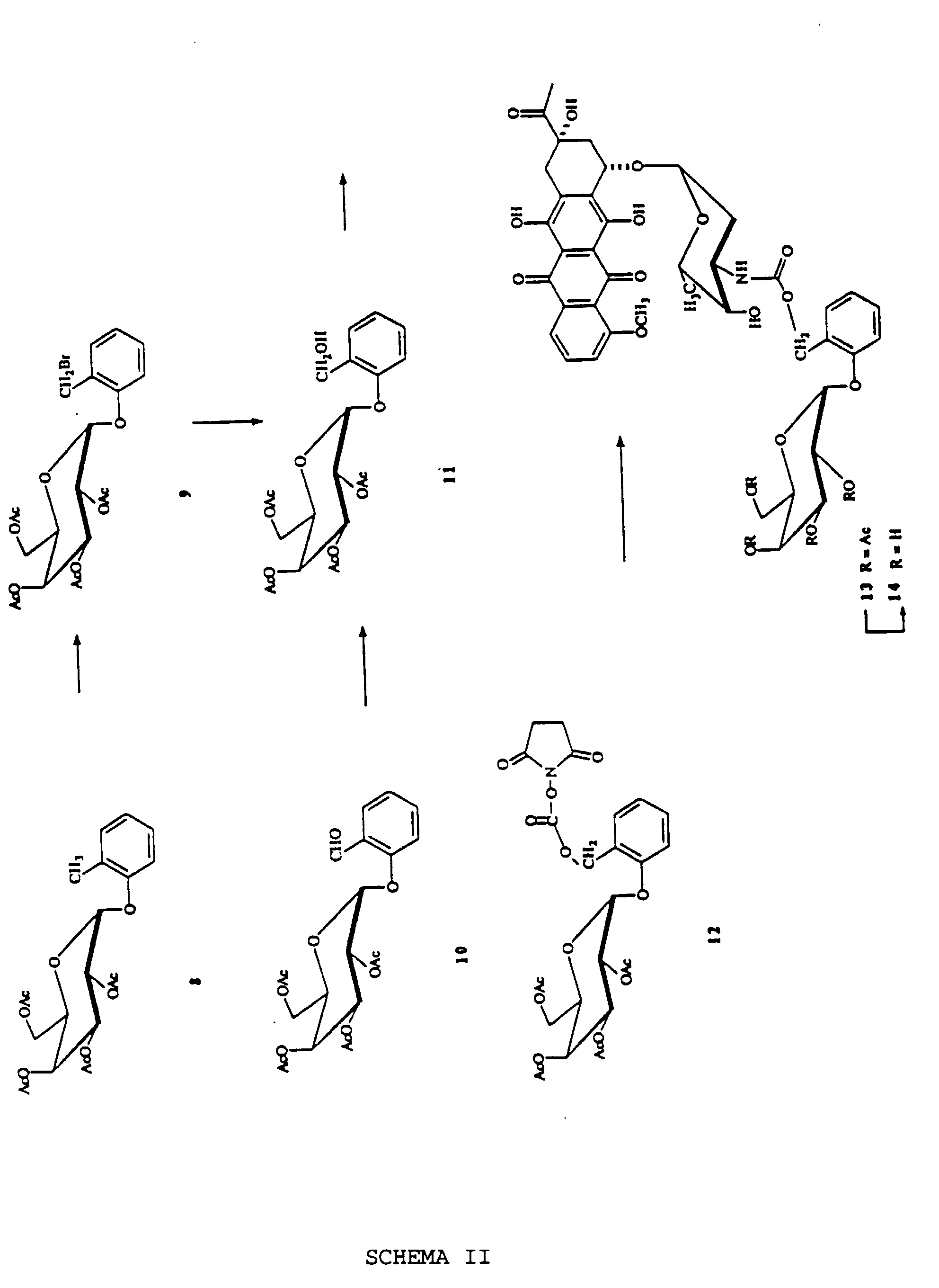

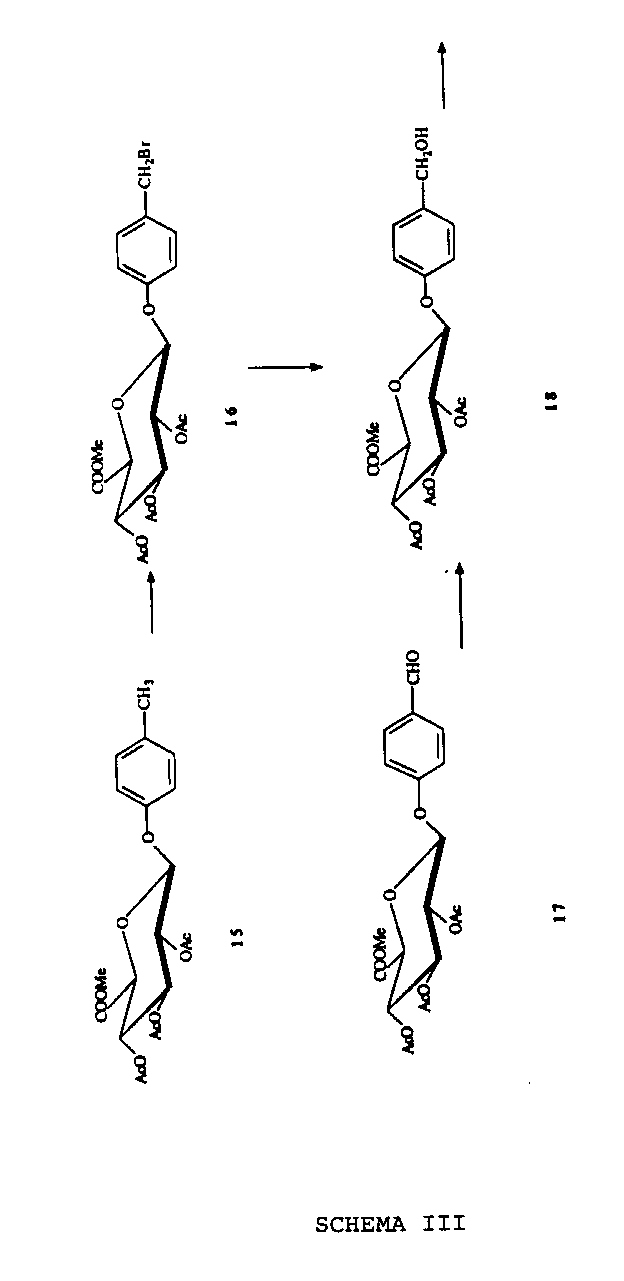

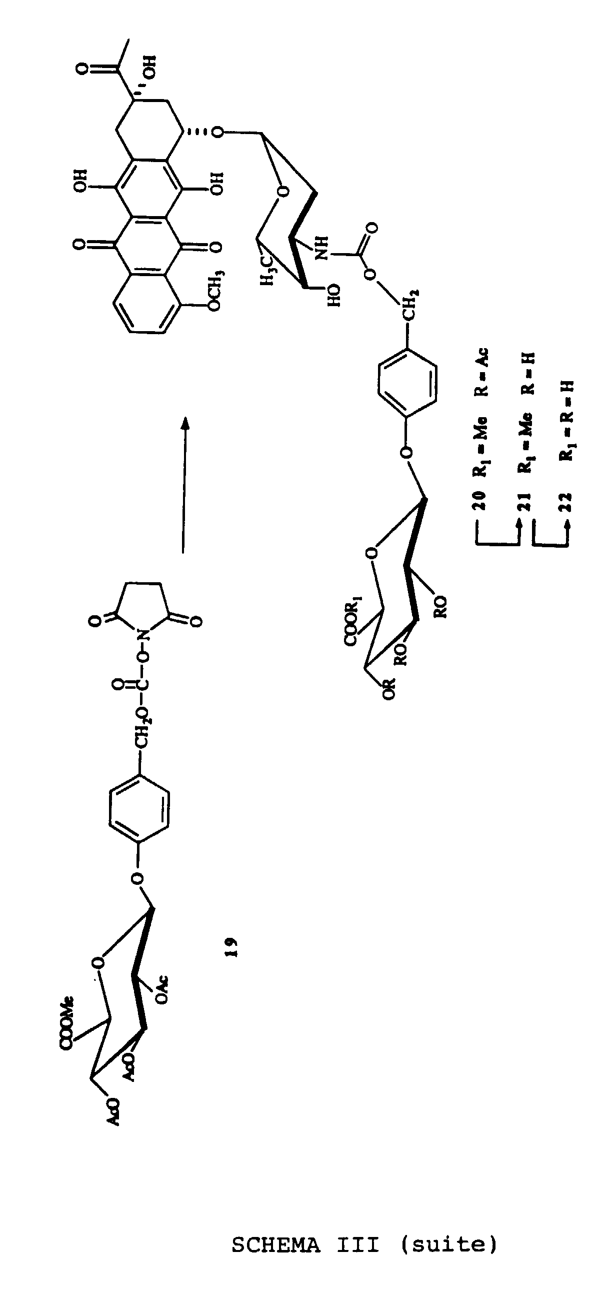

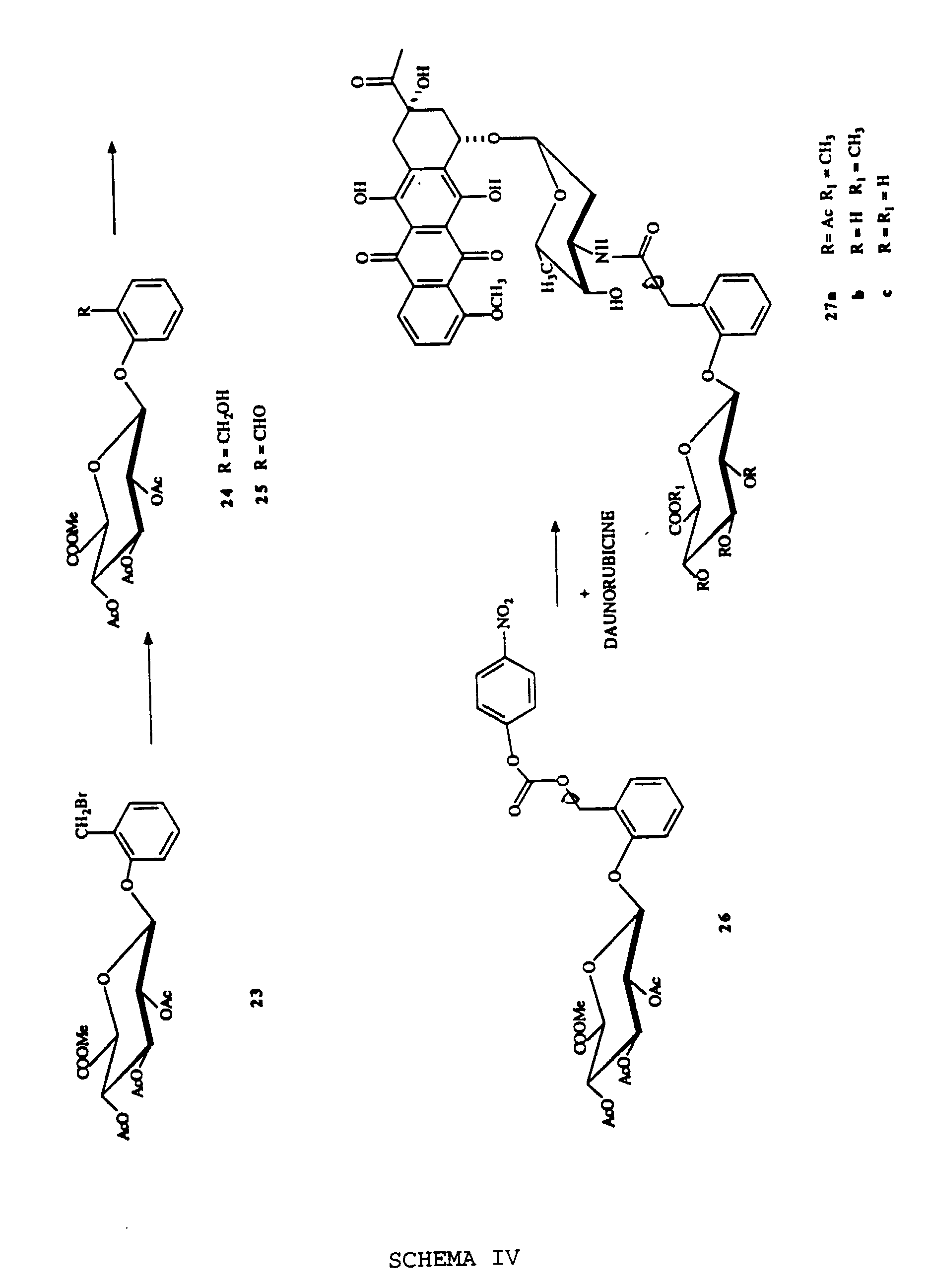

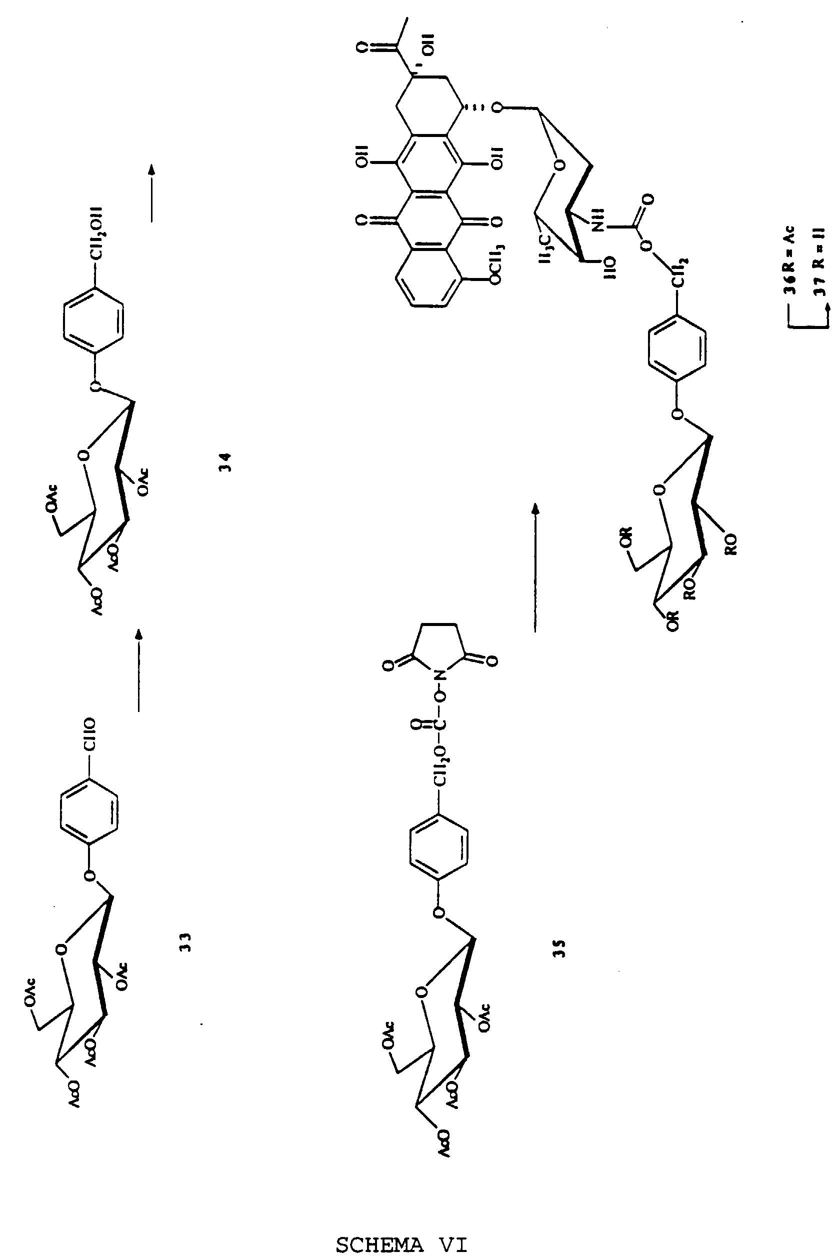

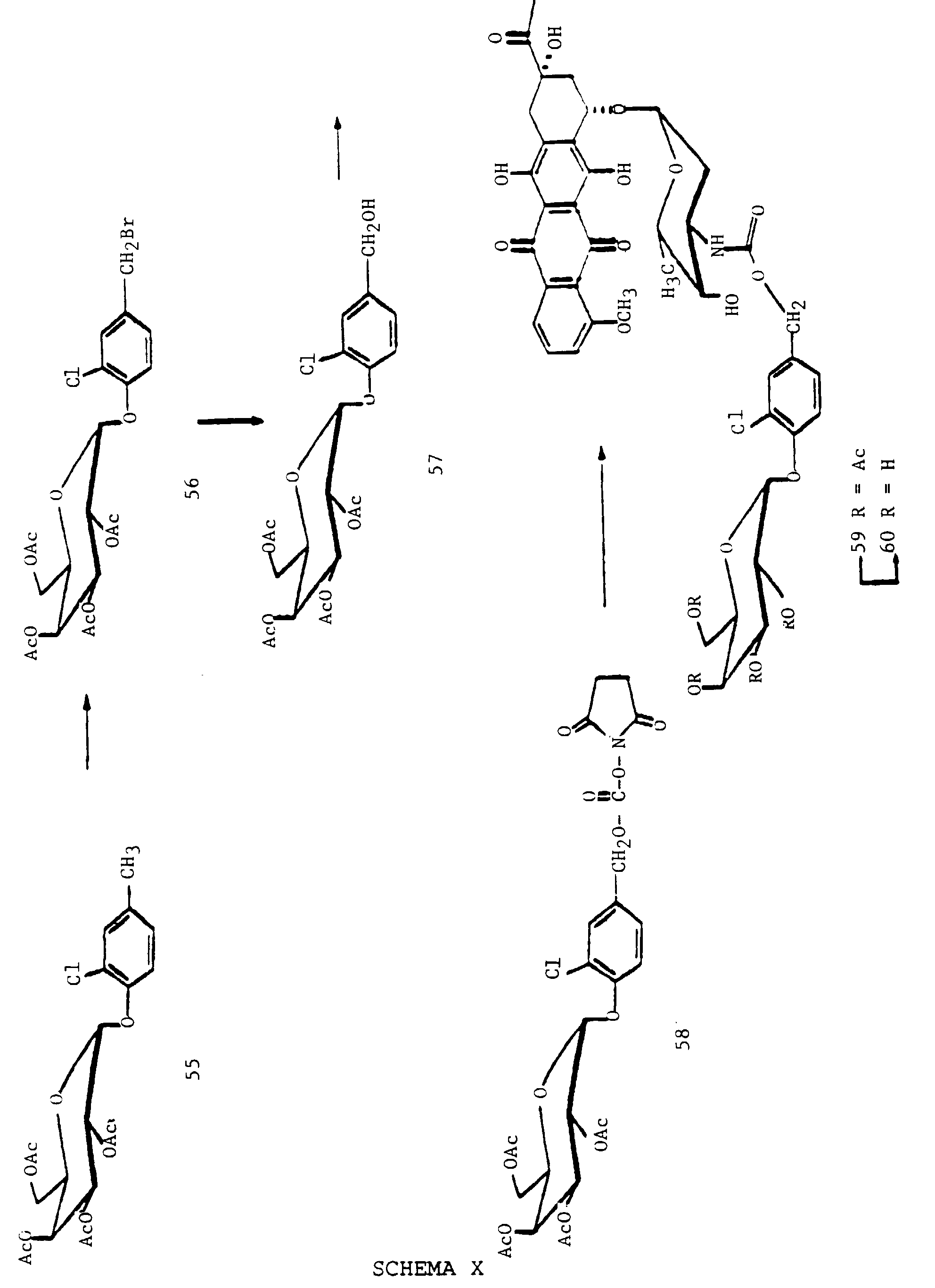

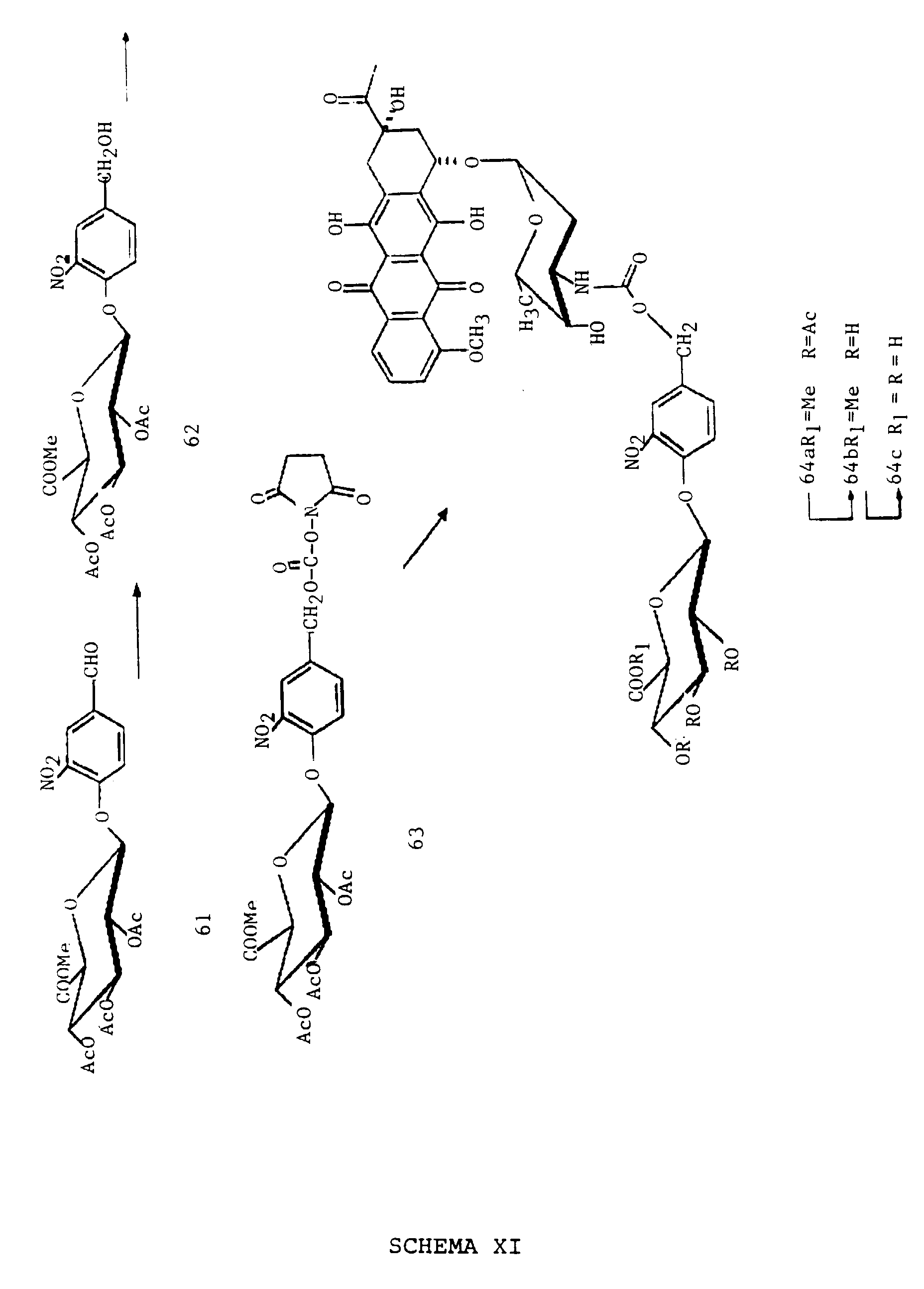

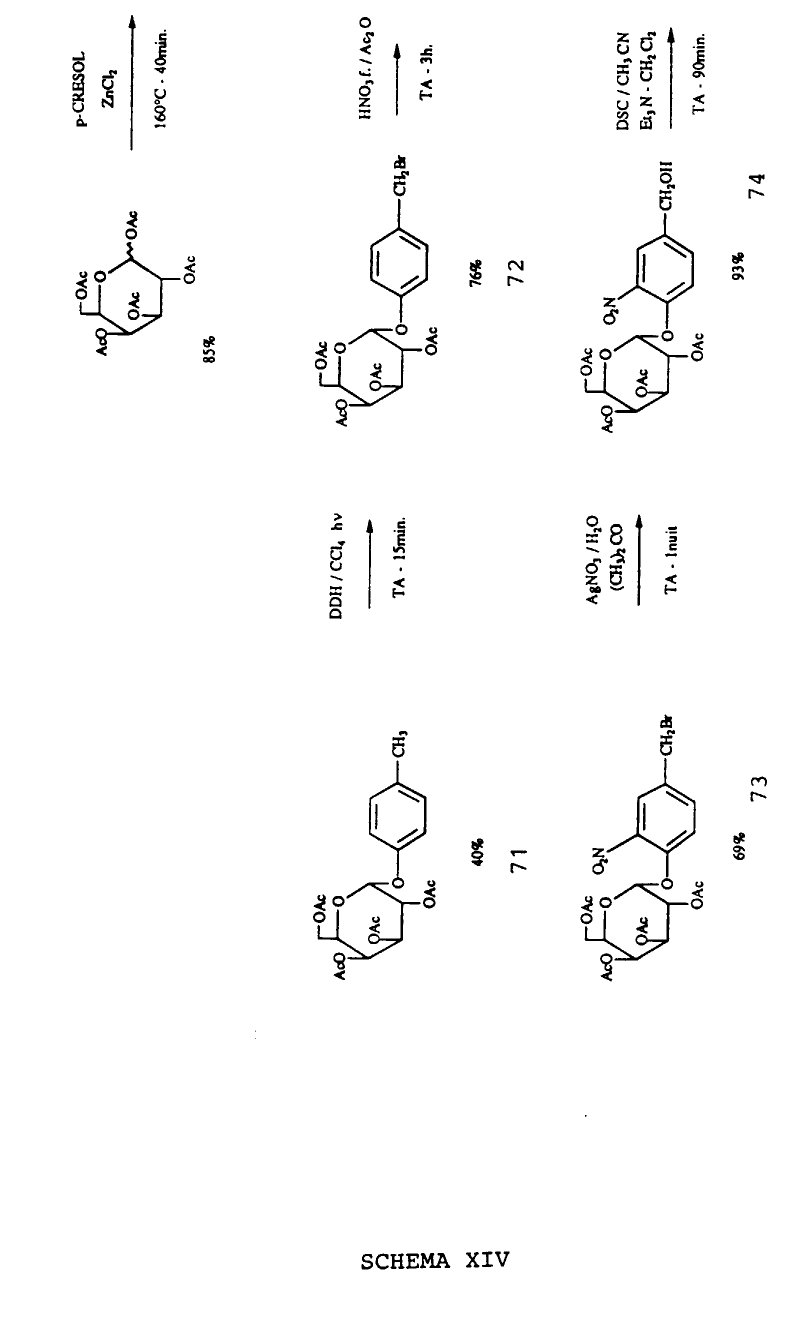

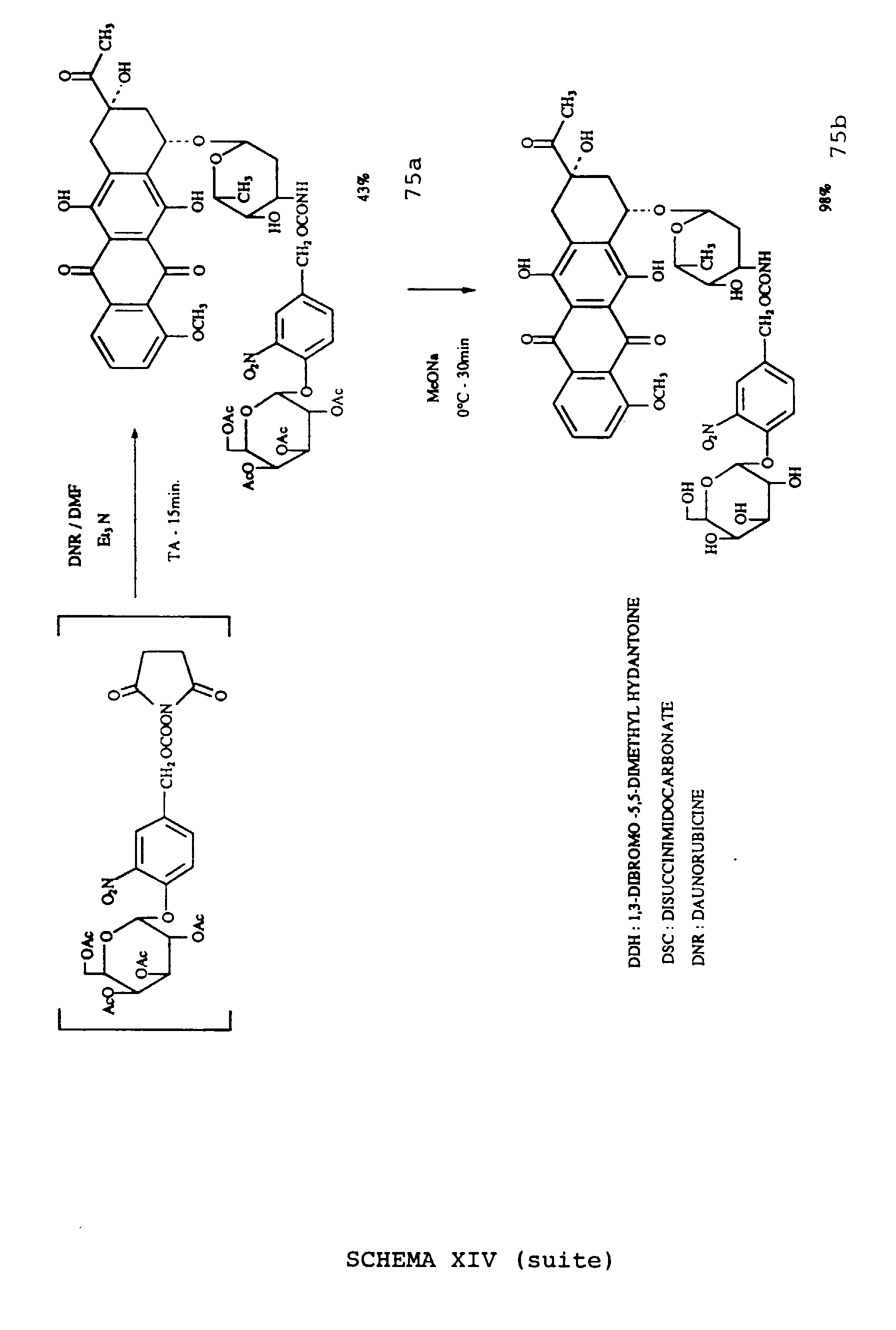

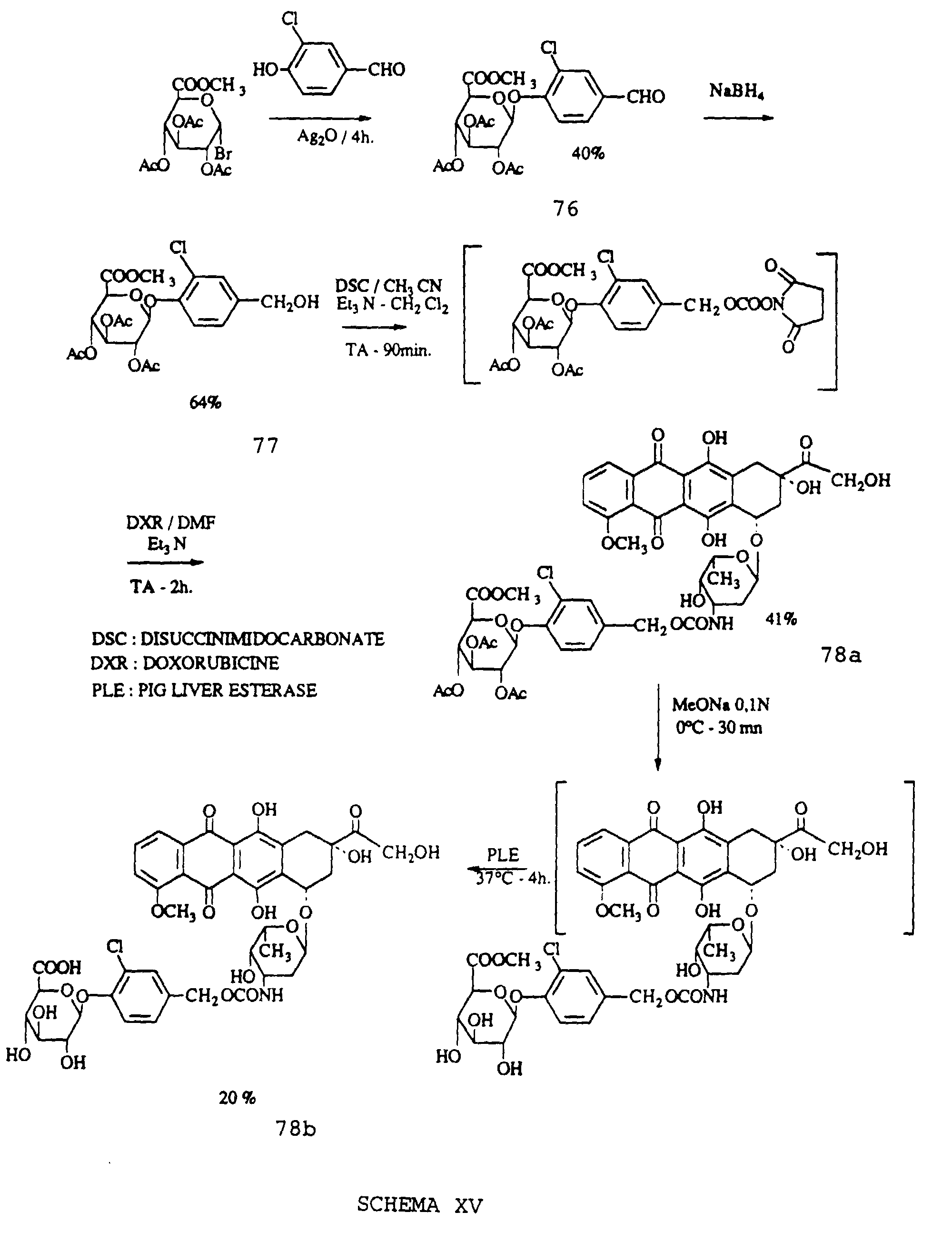

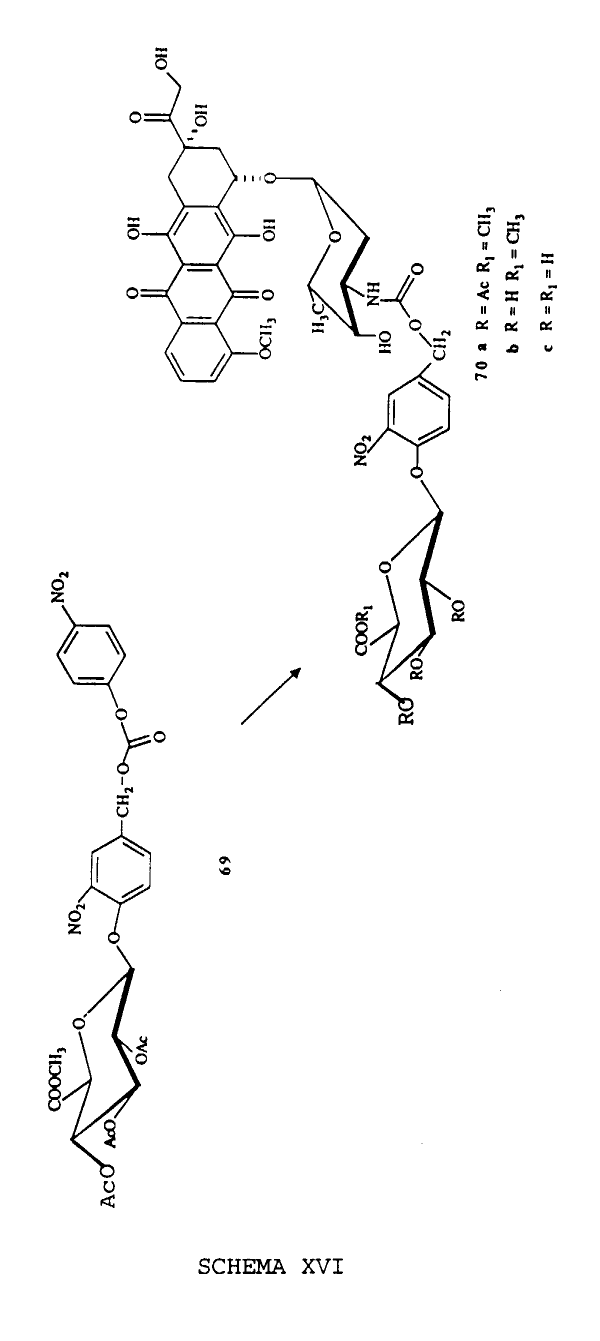

Selon une disposition avantageuse de ce mode de réalisation, préalablement à l'étape (1), le dérivé p-hydroxybenzyle glycosylé est obtenu par :
(a) fusion d'un crésol avec un ose ou un glucuronate de méthyle peracétylé,
(b) bromation benzylique du produit obtenu,
(c) solvolyse du dérivé bromé, et
(d) activation du groupe hydroxyle par un dérivé hydroxysuccinimidyle ou paranitrophénoxy-carbonyle.

Selon un autre mode de réalisation dudit procédé, le composé de formule A est un dérivé silylé de formule A₂ : 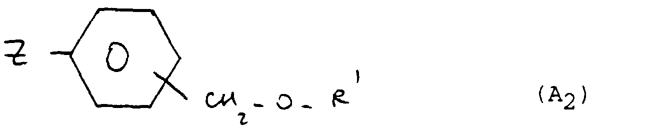 dans laquelle R' a la même signification que ci-dessus et Z représente un groupe O-diméthylthexylsilyle ou un groupe O-tertiobutyldiméthylsilyle pour fournir, après couplage avec une anthracycline de formule B, et condensation avec un ose convenable, une prodrogue d'anthracycline de formule I, telle que définie ci-dessus,
conformément aux schémas V et VII, qui décrivent le procédé d'obtention des dérivés silylés, avant leur condensation avec un ose convenable.

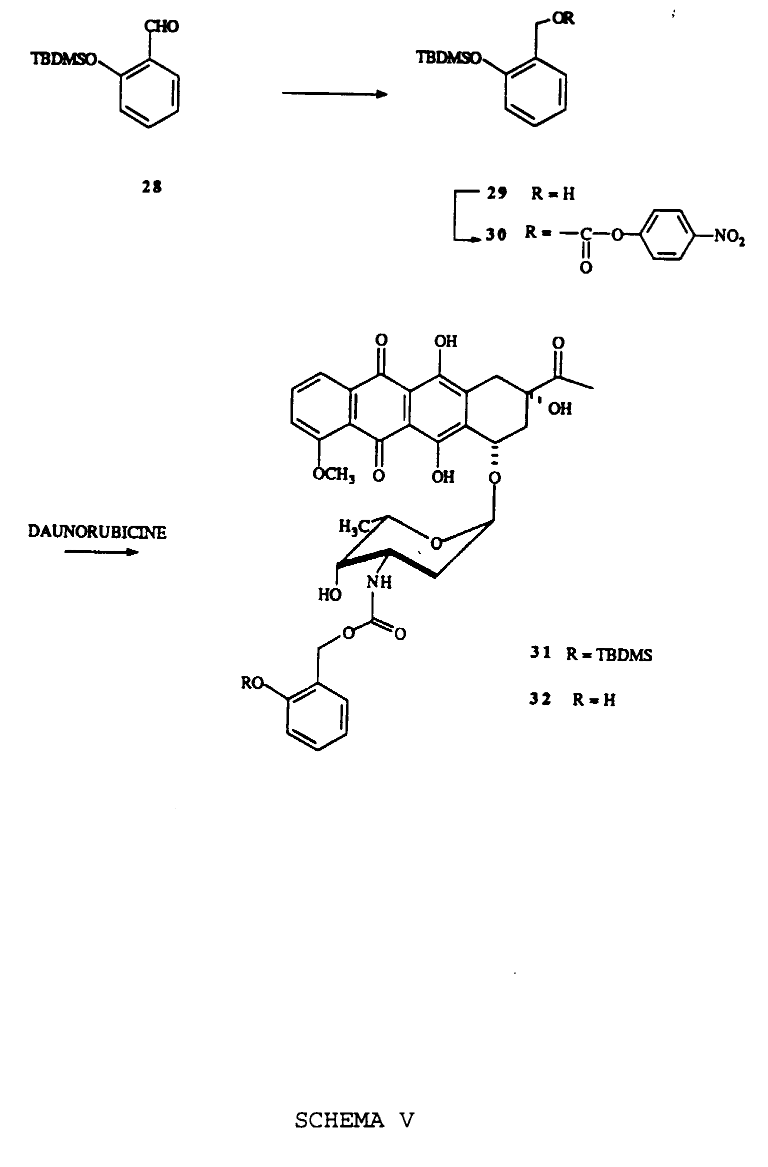

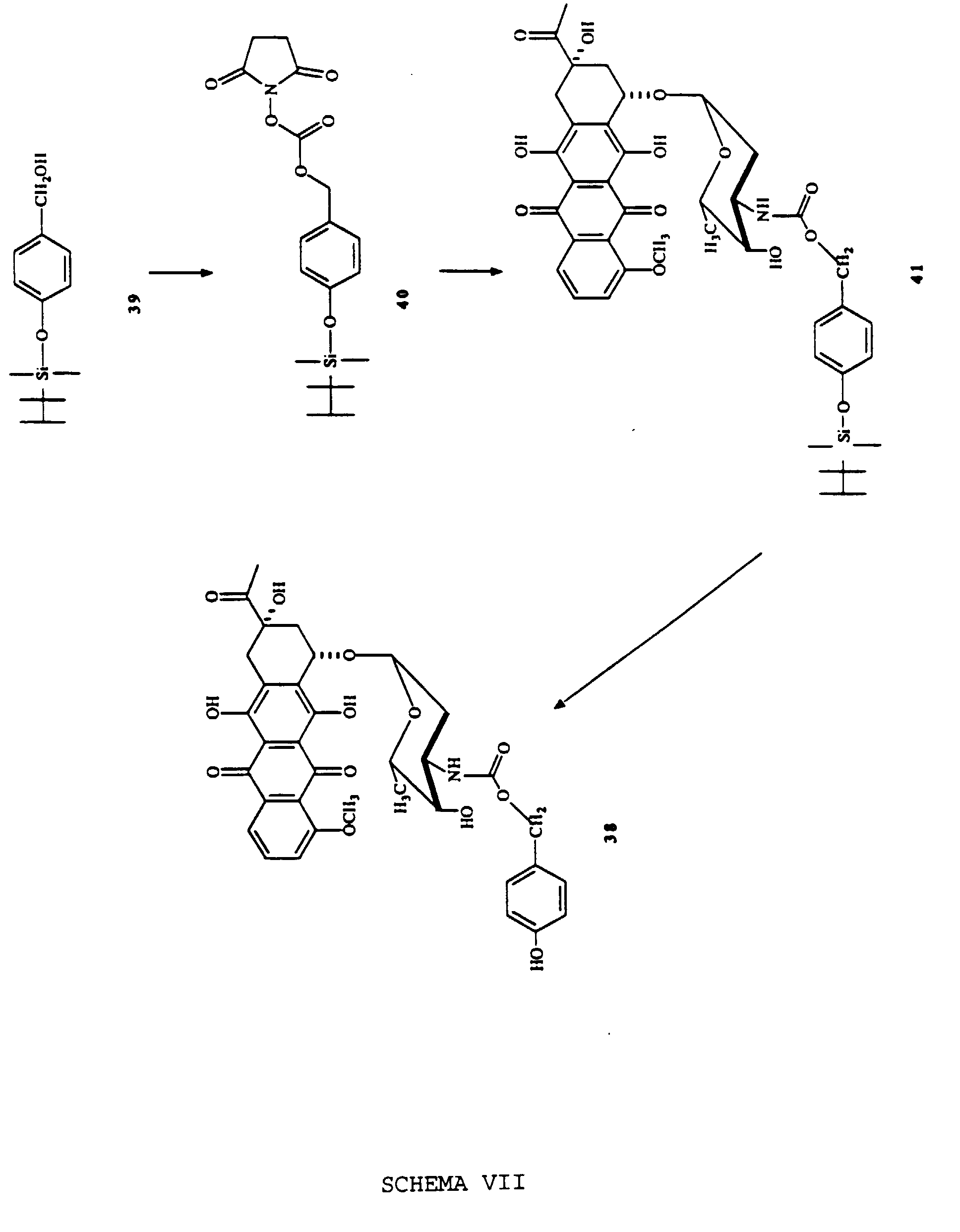

Selon encore un autre mode de mise en oeuvre du procédé conforme à l'invention, le composé de formule A est un dérivé de orthohydroxybenzyle glycosylé de formule A₃ : 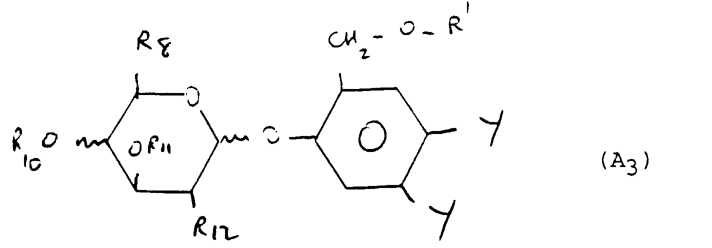 dans laquelle
R₈, R₁₀, R₁₁, R₁₂ et R' ont la même signification que ci-dessus,
et Y est en position para et représente un groupe NO₂ ou un atome d'halogène et en position méta et représente un atome d'hydrogène ou un groupe méthoxy ;
pour fournir, après couplage avec une anthracycline de formule B, une prodrogue d'anthracycline de formule I, telle que définie ci-dessus, dans laquelle :
le CH₂ benzylique est en position ortho par rapport à l'oxygène glycosylé ;
conformément aux schémas VIII, IX, XII ou XIII ci-après.

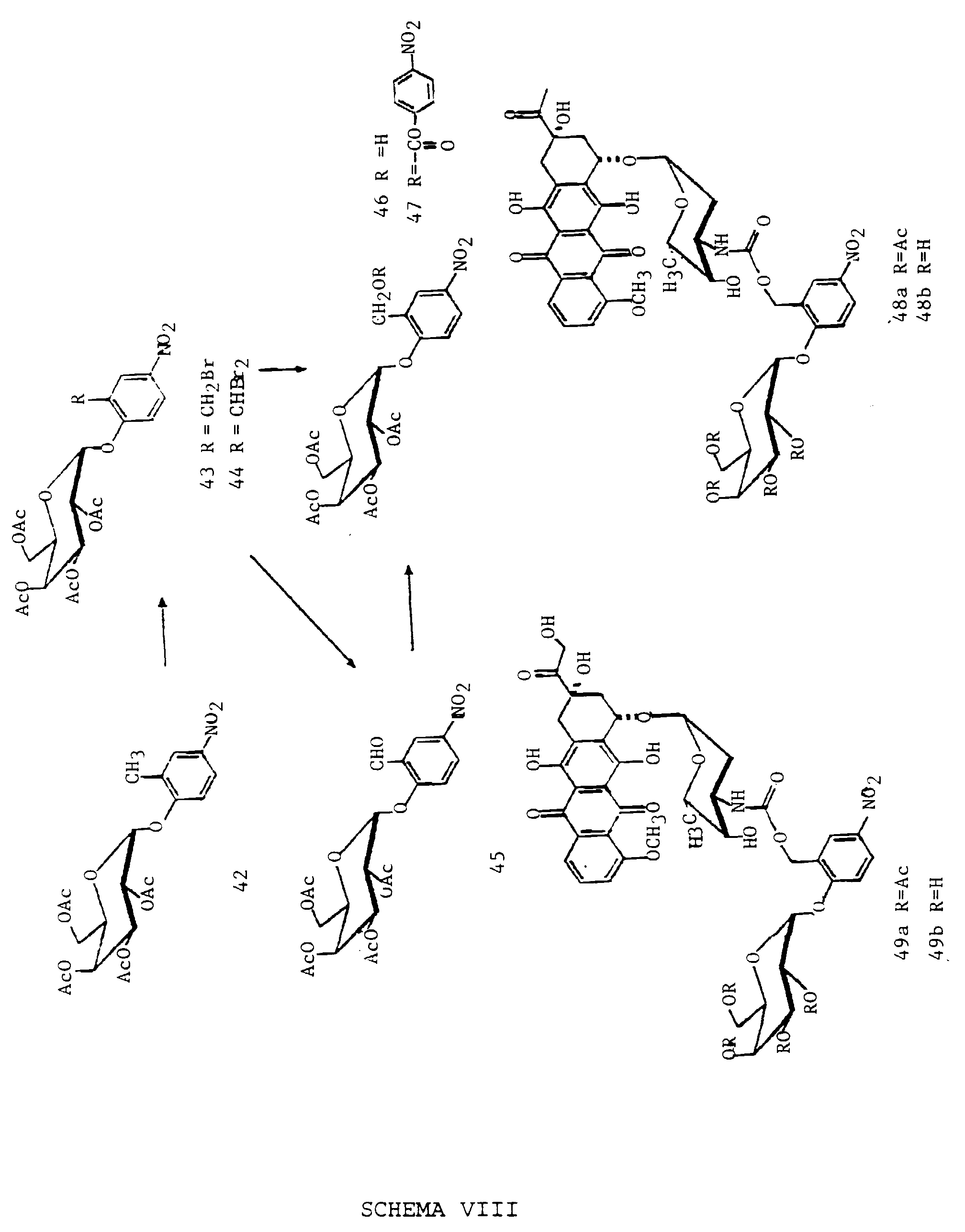

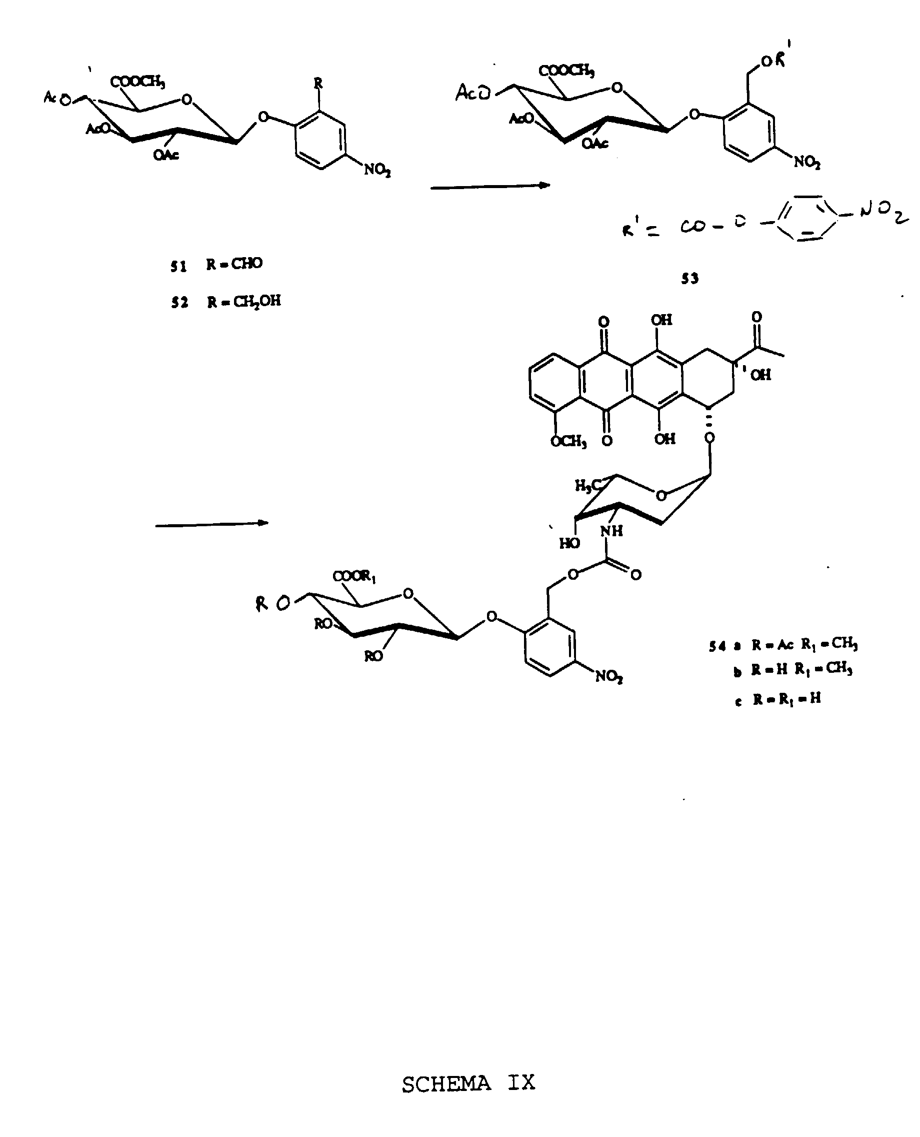

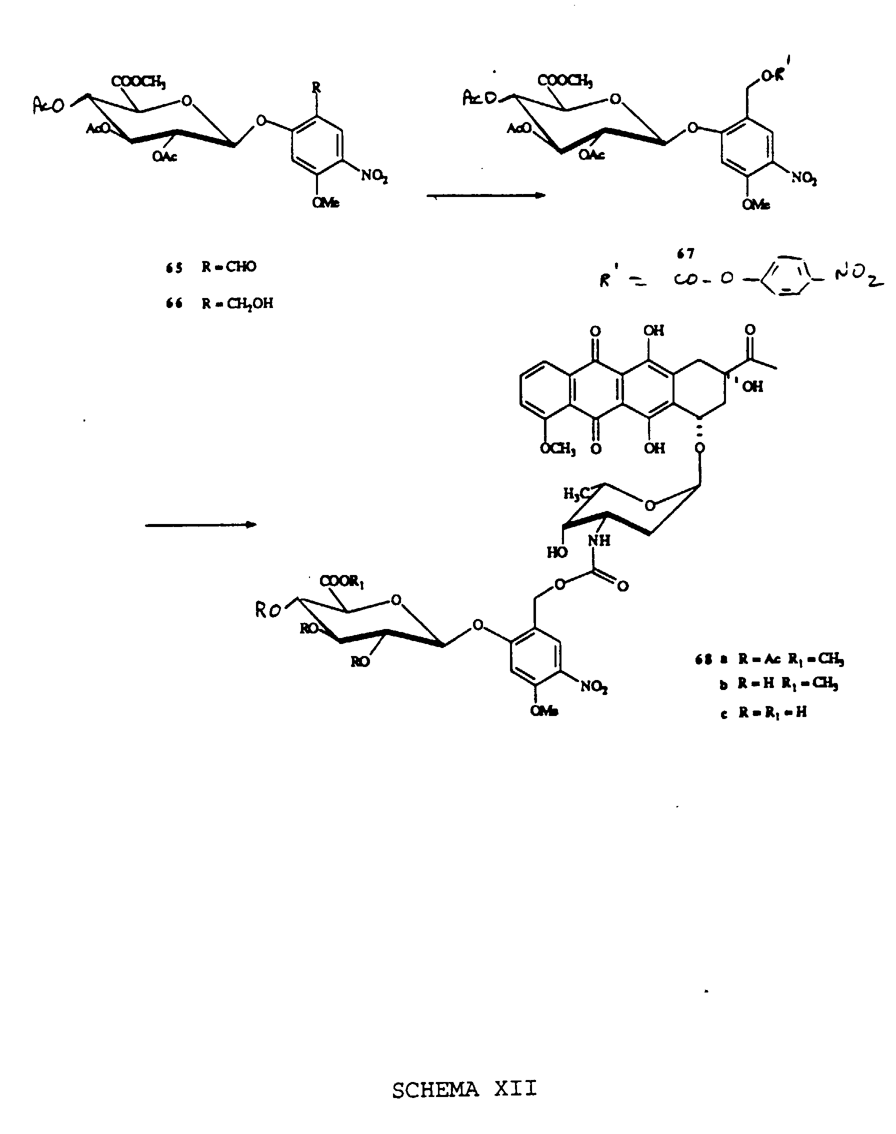

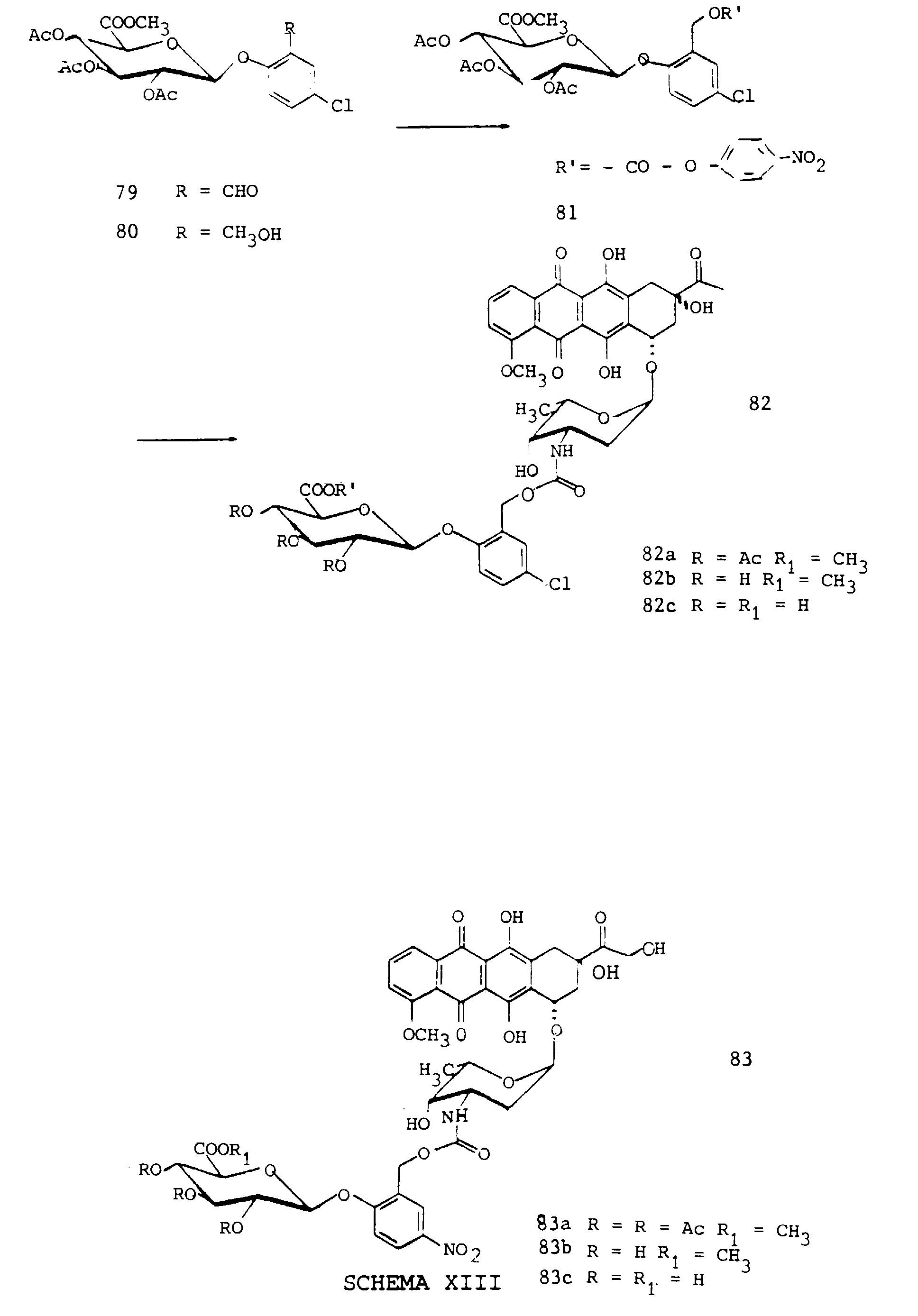

La présente invention a également pour objet des produits comprenant une prodrogue d'anthracycline conforme à l'invention et un conjugué enzyme-anticorps spécifique d'une tumeur, de formule II Ac-Sp-E, dans laquelle :
- Ac: est un anticorps ou l'un de ses fragments, qui présente une spécificité vis-à-vis d'un antigène associé à une tumeur, ou est une biomolécule qui a tendance à s'accumuler dans une tumeur, tel que l'EGF (facteur de croissance épidermique), l'α-TGF (facteur de croissance transformant α), le PDGF (facteur de croissance dérivé des plaquettes), l'IGF I+II (facteur de croissance de l'insuline I+II) ou le FGF a+b (facteur de croissance fibroblastiques a+b),
- E: représente une glycosidase qui n'est pas immunogène ou présente une immunogénicité très faible, de préférence une glycosidase de mammifère, telle que l'α ou la β-glucosidase, l'α-galactosidase, l'α ou la β-mannosidase, l'α-fucosidase, la N-acétyl-α-galactosaminidase, les N-acétyl-β- et N-acétyl-α-glucosaminidase ou la β-glucuronidase,
- Sp: (Bras) représente un groupe contenant un sulfure ou un disulfure de formule III ou IV

X′(S)ₙ Y' (III)

X′(S)ₙ (IV),

ou un bras polypeptidique, dans lequel
X′ ou Y′ représente -CO-R₁₃-(N-succinimido)- ou -C(=R₁₄)-CH₂-CH₂-
avec R₁₃ étant un groupement -CH₂-CH₂, 1,4-cyclohexylidène, 1,3 ou 1,4 phénylène ou méthoxycarbonyle ou chloro-1,4-phénylène
et R₁₄ étant un atome d'oxygène ou un groupe NH, et de plus,
Y′ représente -C(=R₁₄)-CH₂-CH₂, lorsque R₁₄ a la signification précisée ci-dessus et
n représente 1 ou 2,
pour une utilisation simultanée, séparée ou étalée dans le temps, en thérapie cytostatique.

Conformément à l'invention, aussi bien la prodrogue d'anthracycline que le conjugué enzyme-anticorps peuvent être associés à au moins un véhicule pharmaceutiquement acceptable.

Un tel conjugué enzyme-anticorps est plus particulièrement décrit dans la Demande de Brevet DE 39 35016.9, au nom de BEHRING.

Le couplage entre l'enzyme et l'anticorps, ou un fragment de celui-ci ou une biomolécule, est réalisé à l'aide d'un procédé décrit dans la littérature (A.H. BLAIR et T.I. GHOSE, Immunology Methods, 1983, 59, 129-143 ; T.I. GHOSE et al., Methods in Enzymol., 1983, 19, 280-333).

Cela implique la fonctionalisation initiale de l'enzyme via son groupe amino, en utilisant un carboxylate de succinimidyl N-maléimidoalkylidène-, cycloalkylidène ou arylène-1-, dans lequel la double liaison du groupe maléimido entre en réaction avec le groupe HS de l'anticorps fonctionnalisé, de son fragment ou des biomolécules, avec la formation d'une fonctionnalité thioéther.

Il est possible d'utiliser, pour la préparation des conjugués anticorps-enzyme, les anticorps monoclonaux décrits dans la Demande européenne 141 079, de préférence les anticorps 431/26, 250/183, 704/152 et 494/32.

La spécificité de ces anticorps pour des antigènes associés à des tumeurs a déjà été démontrée sur des animaux et chez l'Homme par des méthodes d'immunoscintigraphie et d'immunochimie.

Pour préparer les conjugués enzymatiques spécifiques d'une tumeur, il est possible, pour les enzymes qui sont mentionnées ci-après et à partir de la source identifiée, d'être purifiées par la procédure indiquée dans la littérature :
- α-galactosidase de foie humain, DEAN, K.G. and SWEELEY, C.C. (1979), J. Biol. Chem. 254, 994-1000 ;
- β-glucuronidase de foie humain, HO, K.J. (1985), Biochim. Biophys. Acta 827, 197-206 ;
- α-L-fucosidase de foie humain, DAWSON, G., TSAY, G (1977) Arch. Biochem. Biophys. 184, 12-23 ;
- α-mannosidase de foie humain, GRABOWSKI, G.A., IKONNE, J.U., DESNICK, R.J. (1980) Enzyme 25, 13-25 ;
- β-mannosidase de placenta humain, NOESKE, C., MERSMANN, G. (1983) Hoppe Seylers Z Physiol. Chem. 364, 1645-1651 ;
- α-glucosidase de muqueuse gastrointestinale humaine, ASP. N.G., GUDMAND-HOEYER, E., CHRISTIANSEN, P.M., DAHLQUIST, A. (1974) Scand. J. Clin. Lab. Invest. 33, 239-245 ;
- β-glucosidase de foie humain, DANIELS, L.B., COYLE, P.J., CHIA, Y.B., GLEW, R.H. (1981) J. Biol. Chem. 256, 13004-13013 ;
- β-glucocerebrosidase de placenta humain, FURBISH, F.S., BLAIR, H.E., SHILOACH, J., PENTCHEU, P.G., BRADY, R.O. (1977) Proc. Natl. Acad. Sci. USA 74, 3560-3563 ;
- α-N-acetylglucosaminidase de placenta humain, ROEHRBORN, W., VON FIGURA, K. (1978) Hoppe Seylers Z Physiol. Chem. 359, 1353-1362 ;
- β-N-acetylglucosaminidase de membrane amniotique humaine, ORLACCHIO, A., EMILIANI, C., DI RENZO, G.C., COSMI, E.V. (1986) Clin. Chim. Acta 159, 279-289 ;
- α-N-acetylgalactosaminidase selon SALVAYRE, R., NEGRE, A., MARET, A., DOUSTE-BLAZY, L. (1984) Pathol. Biol. (PARIS) 32, 269-284.

L'activité glycolytique des conjugués enzymes-anticorps, spécifiques de tumeur, a été déterminée, comparativement avec des glycosides de p-nitrophényle, à pH optimum.

Pour tester l'efficacité d'une utilisation séquentielle et combinée, du conjugué enzyme-anticorps et de la prodrogue, le conjugué est administré à des souris transplantées, puis après avoir attendu que le taux plasmatique d'enzyme soit revenu quasiment à zéro, l'anthracycline modifiée (prodrogue) est administrée ; on observe s'il y a arrêt de la croissance de la tumeur et si une régression s'opère.

Les prodrogues 7, 14, 48b, 49b, 60 et 75b ainsi que les acétates 6, 13, 48a, 49a, 59 et 75a, qui s'hydrolysent *in vivo* sous l'effet d'enzymes et conduisent à l'une des prodrogues précitées, sont des α-galactosides ; les prodrogues 22, 27c, 54c, 64c, 70c, 78b et 83c sont des β-glucuronides ; la prodrogue 37 et son acétate 36 sont des β-glucosides. Ces prodrogues sont avantageusement clivées en daunorubicine ou doxorubicine, selon le cas, en présence du conjugué approprié, tel que défini ci-dessus.

De manière inattendue, les compositions conformes à l'invention combinant une prodrogue à trois compartiments et un conjugué dont l'enzyme est une enzyme d'origine humaine non circulante permettent de résoudre à la fois le problème de la tolérance immunologique, de la spécificité d'action au niveau du site tumoral et comme précisé ci-dessus, évitent les interférences stériques ou électroniques, lors du clivage enzymatique.

Egalement de manière inattendue, les prodrogues à trois compartiments conformes à l'invention peuvent être clivées par des macrophages, des granulocytes, des thrombocytes ou des cellules tumorales humaines activés.

En effet, ces cellules activées libèrent de la β-glucuronidase apte à cliver efficacement (hydrolyse) les composés glucuronyle-bras auto-immolable-drogues.

De telles prodrogues sont alors directement utilisables comme médicament pour le traitement des maladies où sont impliqués des macrophages, des granulocytes, des thrombocytes ou des cellules tumorales humaines activés.

Outre les dispositions qui précèdent, l'invention comprend encore d'autres dispositions, qui ressortiront de la description qui va suivre, qui se réfère à des exemples de mise en oeuvre du procédé objet de la présente invention.

Il doit être bien entendu, toutefois, que ces exemples sont donnés uniquement à titre d'illustration de l'objet de l'invention, dont ils ne constituent en aucune manière une limitation.

### EXEMPLE 1 : synthèse de la α-D-galactopyranoside de N-(4-hydroxy-benzyloxycarbonyl) daunorubicine (7).

Conformément au schéma I, le couplage de penta-O-acétyl-D-galactopyranose avec du para-crésol est réalisé par fusion en présence de ZnCl₂ ou de ZnCl₂ dans un mélange de AcOH-Ac₂O.

La bromation benzylique du composé (1) obtenu est réalisée soit en présence de N-bromosuccinimide (NBS) et activation photochimique et fournit uniquement le composé 2, soit en présence de NBS et de peroxyde de benzoyle et fournit principalement le composé 2 et de petites quantités du dérivé aldéhyde 3.

Le déplacement du brome du dérivé 2 est réalisé dans l'acétone ou dans l'éther-acétone avec (ou sans) (Bu₃Sn)₂O et fournit le dérivé 4, alors que la réduction du dérivé aldéhydique 3 par du borohydrure de sodium fournit des quantités supplémentaires du composé 4.

L'activation du groupe OH du dérivé 4 est réalisée en utilisant le chloroformiate de N-succinimidyle ou le carbonate de disuccinimidyle (DSC).

Dans une étape suivante, le couplage du dérivé 5 avec la daunorubicine est réalisé dans du DMF en présence d'Et₃N et le dérivé 6 N-hydroxybenzylcarbonyl est déprotégé par transestérification et permet l'obtention du dérivé 7 recherché.
1) **2,3,4,6-Tétra-O-acétyl-α-D-galactopyranoside de 4-méthyl phényle (1).**

   - Préparations :
      Procédé I : 8,9 g de p. crésol, 8,9 g de penta-O-acétyl-D-galactopyranose et 0,56 g de ZnCl₂ sont mélangés et portés à 160°C et maintenus à cette température durant 30 minutes, selon la méthode d'HELFERICH. Après refroidissement jusqu'à 60°C, le milieu réactionnel est repris par 400 ml de CH₂Cl₂, lavé par deux fois 400 ml d'eau, puis par une solution d'hydroxyde de sodium (≈ 1N), jusqu'à ce que la phase aqueuse soit à peine colorée. La phase organique est enfin lavée par deux fois 400 ml d'eau, séchée sur sulfate de sodium anhydre, puis évaporée à sec au bain-marie (température 35-40°C) pour fournir un résidu de 7,08 g (Rdt = 70 %). Une chromatographie sur colonne de silice 60 H (solvant : hexane-acétate d'éthyle : 90/10 v/v) permet d'obtenir 2,9 g du composé 1 (Rdt = 29 %) et 1,17 g de son anomère β (Rdt = 12 %).
      Procédé II : 11 g de penta-O-acétyl-D-galactopyranose, 11 g de p. crésol et 2,4 g de ZnCl₂ sont dissous dans 8 ml d'un mélange d'acide acétique et d'anhydride acétique (95/5 v/v) et portés à 120°C. Le reflux est maintenu pendant 2 heures.

      Après évaporation du solvant sous pression réduite, le résidu est repris par 120 ml de CH₂Cl₂. La solution organique est lavée à l'eau, puis par une solution de soude (≈ 1N) et enfin lavée à l'eau avant d'être séchée sur sulfate de sodium anhydre et évaporée à sec sous pression réduite. Un résidu brut de 7 g est ainsi obtenu (Rdt = 64 %). Après purification sur colonne de silice 60 H (solvant : hexane-acétate d'éthyle : 70/30 v/v) le produit 1 est obtenu avec un rendement de 13 % et son anomère β avec un rendement de 6,5 %.
   - Composé 1 : C₂₁H₂₆O₁₀. M = 438. F = 163-165°C. [α]_{D}²⁰ = +164° (c 1, CHCl₃). RMN 1H (270 MHz, CDCl₃) : δ ppm 1,90 (s, 3H), 2,02 (s, 3H), 2,08 (s, 3H), 2,19 (s, 3H), 2,28 (s, 3H), 4,00 (dd, 1H, J = 12, J′ = 7 Hz), 4,09 (dd, 1H, J = 12, J′ = 6 Hz), 4,40 (ddd, 1H, J = 7, J′ = 5, J˝ = 1 Hz), 5,27 (dd, 1H, J = 10, J′ = 4 Hz), 5,54 (dd, 1H, J = 4, J′ = 1 Hz), 5,57 (dd, 1H, J = 10, J′ = 4 Hz), 5,73 (d, 1H, J = 4 Hz), 6,90 (d, 2H, J = 8 Hz), 7,10 (d, 2H, J = 8 Hz). SM. (DIC/NH₃) : m/z 456 (M+NH₄)⁺, 331.
2) **2,3,4,6-tétra-O-acétyl-α-D-galactopyranoside de 4-bromométhyl phényle (2).**

   - Préparation :
      1) avec activation photochimique :
         A une solution de 1 (2,5 g) dans 100 ml de tétrachlorure de carbone, on ajoute 1,02 g de N-bromosuccinimide, et on chauffe au reflux à 80°C avec irradiation lumineuse (1000 W) pendant 15 min. Après refroidissement, le milieu réactionnel est filtré pour éliminer le succinimide qui a précipité, puis le filtrat est évaporé sous pression réduite, ce qui permet d'obtenir 3,23 g de résidu sec. On isole 2,45 g de 2 après purification sur colonne de silice 60 H (solvant : hexane-acétate d'éthyle : 70/30 v/v) (Rdt = 83 %).
      2) avec activation par le peroxyde de benzoyle :
         A une solution de 1 g du produit 1 dans 40 ml de tétrachlorure de carbone, on ajoute 0,8 g de N-bromosuccinimide et 0,08 g de peroxyde de benzoyle. Le milieu réactionnel est chauffé au reflux et pendant 3 h. Après refroidissement, le milieu réactionnel est filtré pour éliminer le succinimide qui a précipité, puis le filtrat évaporé à sec pour obtenir 1,64 g de produit. Une "flash chromatographie" (solvant : hexane-acétate d'éthyle : 80/20 v/v) permet d'obtenir 1,74 g d'un mélange du dérivé monobromé 2 et du dérivé dibromé correspondant et d'isoler 0,08 g du composé 3 pur.
   - Composé 2 : C₂₁H₂₅O₁₀Br. M = 517. F = 187 °C (CCl₄). [α]_{D}²⁰ = +26° (c 1, CHCl₃). RMN ¹H (270 MHz, CDCl₃) : δ ppm : 1,94 (s, 3H), 2,03 (s, 3H), 2,07 (s, 3H), 2,17 (s, 3H), 4,06 (dd, 1H, J = 12, J′ = 7 Hz), 4,12 (dd, 1H, J = 12, J′= 6 Hz), 4,32 (ddd, 1H, J = 7, J′= 6, J˝ = 1 Hz), 4,49 (s, 2H), 5,29 (dd, 1H, J = 10, J′ = 4 Hz), 5,55 (dd, 1H, J = 4, J′ = 1 Hz), 5,60 (dd, 1H, J = 10, J′= 4 Hz), 5,78 (d, 1H, J = 4 Hz), 7,02 (d, 2H, J = 8 Hz), 7,33 (d, 2H, J = 8 Hz). SM (DIC/NH₃) : m/z 536 (M+NH₄)⁺, 534 (M+NH₄)⁺, 456, 331.
3) **2,3,4,6-tétra-O-acétyl-α-D-galactopyranoside de 4-formyl phényle (3).**
   C₂₁H₂₄O₁₁. M = 452. RMN ¹H (270 MHz, CDCl₃) : δ ppm : 1,91 (s, 3H), 2,03 (s, 3H), 2,06 (s, 3H), 2,20 (s, 3H), 4,09 (m, 2H), 4,28 (ddd, 1H, J = 7, J′= 6, J˝ = 1 Hz), 5,30 (dd, 1H, J = 10, J′= 4 Hz), 5,52 (m, 2H), 5,88 (d, 1H, J = 4 Hz), 7,17 (d, 2H, J = 8 Hz), 7,83 (d, 2H, J = 8 Hz), 9,89 (s, 1H). SM (DIC/NH₃) : m/z 470 (M+NH₄)⁺, 331.
4) **2,3,4,6-tétra-O-acétyl-α-D-galactopyranoside de 4-hydroxyméthyl phényle (4).**
   - Préparation :
      Une solution de 1,89 g du composé 2 dans 80 ml d'acétone est mélangée à un volume égal d'une solution aqueuse de nitrate d'argent 0,1 N. Le mélange est agité à 25°C pendant 2 h puis l'acétone est évaporée sous pression réduite et la phase aqueuse restante est extraite par CH₂Cl₂, lavée à l'eau, puis séchée sur sulfate de sodium anhydre, filtrée et évaporée à sec sous pression réduite. Le résidu sec obtenu (1,52 g) est ensuite purifié sur colonne de silice "flash" (solvant : hexane-acétate d'éthyle : 60/40 v/v). Le produit 4 (0,94 g) est obtenu avec un rendement de 56 %.
      C₂₁H₂₆O₁₁. M = 454. F = 153°C (CH₂Cl₂) . [α]_{D}²⁰ = +144,5° (c 1, CHCl₃). RMN ¹H (270 MHz, CDCl₃) δ ppm : 1,73 (1H, échang. D₂O), 1,96 (s, 3H), 2,03 (s, 3H), 2,08 (s, 3H), 2,17 (s, 3H), 4,07 (dd, 1H, J = 12, J' = 7 Hz), 4,09 (dd, 1H, J = 12, J' = 6 Hz), 4,35 (ddd, 1H, J = 7, J' = 6, J" = 1 Hz), 4,64 (s, 2H), 5,28 (dd, 1H, J = 10, J' = 4 Hz), 5,51 (dd, 1H, J = 4, J' = 1 Hz), 5,54 (dd, 1H, J = 10, J' = 4 Hz), 5,60 (d, 1H, J = 4 Hz), 7,04 (d, 2H, J = 8 Hz), 7,30 (d, 2H, J = 8 Hz). SM (DIC/NH₃) : m/z 472 (M+NH₄)⁺, 331.
5) **Carbonate de 2,5-dioxopyrrolidin-1-yle et de 4-(2,3,4,6-tétra-O-acétyl-α-D-galactopyranosyl)-benzyle (5).**
   Le composé 4 est transformé en succinimidocarbonate par couplage avec le succinimido-chloroformiate.
   a) Préparation du succinimido-chloroformiate:
      Une solution de 2 g de N-hydroxysuccinimide dans 11 ml d'éthanol est mélangée à une solution de 1 g de potasse dans 30 ml d'éthanol. Le produit formé est filtré, lavé à l'éther et séché une nuit à 40°C sous vide. On obtient ainsi 2,40 g du sel potassique de l'hydroxysuccinimide (Rdt : 94 %).
      200 mg du sel potassique du N-hydroxysuccinimide sont ajoutés, sous agitation, à 3 ml d'une solution à 20 % de COCl₂ dans le toluène à 5°C. Après 2 h sous agitation, le chlorure de potassium formé est éliminé par filtration et le filtrat évaporé sous pression réduite. Le résidu est dissous dans l'éther et un solide blanc, constitué de disuccinimido-carbonate, se dépose et est éliminé par filtration. Le filtrat est évaporé à sec sous pression réduite pour donner 150 mg de succinimido-chloroformiate (Rdt = 54 %).
   b) Préparation du succinimido-carbonate :
      150 mg du composé 4 sont additionnés à 117 mg de succinimido-chloroformiate et à 0,05 ml de pyridine dans 8 ml d'acétate d'éthyle. Le mélange est agité 48 h à température ambiante, filtré et le filtrat est évaporé sous pression réduite pour fournir 189 mg du composé 5 (Rdt = 96 %) : C₂₆H₂₉O₁₅N. M = 595. Laque. [α]_{D}²⁰ = +142° (c 0,3, CHCl₃). RMN ¹H (270 MHz, CDCl₃) δ ppm : 1,90 (s, 3H), 2,03 (s, 3H), 2,07 (s, 3H), 2,16 (s, 3H), 2,83 (s, 4H), 4,07 (dd, 1H, J = 12, J′ = 7 Hz), 4,11 (dd, 1H, J = 12, J′ = 6 Hz), 4,30 (ddd, 1H, J = 7, J′ = 6, J˝ = 1 Hz), 5,26 (s, 2H), 5,29 (dd, 1H, J = 10, J′ = 4 Hz), 5,51 (dd, 1H, J = 4, J′ = 1 Hz), 5,55 (dd, 1H, J = 10, J′ = 4 Hz), 5,77 (d, 1H, J = 4 Hz), 7,07 (d, 2H, J = 8 Hz), 7,33 (d, 2H, J = 8 Hz). SM (DIC/NH₃) : m/z 613 (M+NH₄)⁺, 437, 348, 331.
6) **N-[4-(2,3,4,6-tétra-O-acétyl-α-D-galactopyranosyl)-benzyloxycarbonyl] daunorubicine (6).**
   - Préparation :
      20 mg de daunorubicine sont dissous dans 0,8 ml de diméthylformamide. 23 mg de 5 et une goutte de triéthylamine sont ajoutés à cette solution, maintenue sous agitation sous argon durant 10 minutes à température ambiante.
      Le milieu réactionnel est repris par l'acétate d'éthyle, puis extrait par une solution saturée de NaCl.
      La phase organique est séchée sur sulfate de sodium anhydre, filtrée et évaporée à sec sous pression réduite. Les 50 mg de résidu sec obtenu sont purifiés sur colonne de silice 60 H (solvant : acétone-cyclohexane : 45/55 v/v) et permettent d'obtenir 13 mg du produit attendu 6 (Rdt = 32 %).
   - Composé 6 : C₄₉H₅₃NO₂₂. M = 1007. Amorphe. [α]_{D}²⁰ = +278° (c 0,7, CHCl₃). RMN ¹H (270 MHz, DMSO) δ ppm : 1,08 (d, 3H, J = 7 Hz), 1,40-2,20 (m, 4H), 1,76 (s, 3H), 1,91 (s, 3H), 1,96 (s, 3H), 2,08 (s, 3H), 2,20 (s, 3H), 2,91 (s, 2H), 3,31 (m, 1H), 3,66 (ddt, 1H, J = 12, J′ = 8, J˝ = 4 Hz), 3,93-4,00 (m, 5H), 4,13 (dd, 1H, J = 7, J′= 4 Hz), 4,30 (ddd, J = 7, J′ = 6, J˝ = 1 Hz), 4,70 (d, 1H, J = 6 Hz), 4,88 (m, 3H), 5,10 (dd, 1H, J = 10, J′ = 4 Hz), 5,17 (m, 1H), 5,37 (m, 2H), 5,53 (s, 1H), 5,74 (d, 1H, J = 4 Hz), 6,84 (d, 1H, J = 8 Hz), 7,03 (d, 2H, J = 8 Hz), 7,26 (d, 2H, J = 8 Hz), 7,64 (m, 1H), 7,88 (m, 2H). SM (DIC/NH₃) : m/z 1025 (M+NH₄)⁺, 376.
7) **N-[4-(α-D-galactopyranosyl)-benzyloxycarbonyl] daunorubicine (7).**
   - Préparation :
      Une solution de 9 mg de 6 dans 1 ml de méthanolate de sodium 0,1 N est agitée et maintenue à 0°C pendant 15 minutes. Le milieu est neutralisé par addition de résine Amberlite IRC 120H⁺, puis filtré. Le filtrat, évaporé à sec sous pression réduite, fournit 6,7 mg du composé 7 pur (Rdt = 89 %).
   - Composé 7 : C₄₁H₄₅NO₁₈. M = 839. Amorphe. [α]_{D}²⁰ = +243° (c 0,01, MeOH). RMN ¹H (270 MHz, CD₃OD) δ ppm : 1,34 (d, 3H), 1,50-4,00 (m, 11H), 4,50-5,50 (m, 9H), 7,06 (d, 2H, J = 8Hz), 7,23 (d, 2H, J = 8Hz), 7,30-7,60 (m, 3H). SM (DIC/NH₃) : m/z 571, 554, 459, 383, 286, 164.

### EXEMPLE 2 : synthèse de la N-[2-(α-D-galactopyranosyl)-benzyloxycarbonyl] daunorubicine (14).

Conformément au schéma II, en utilisant comme produit de départ le glycoside 8 et en utilisant les mêmes séquences de réactions, à savoir (i) bromation benzylique ; (ii) solvolyse du dérivé bromé ; (iii) activation du groupe OH par du chloroformiate de succinimidyl ; (iv) couplage de 12 avec la daunorubicine et déprotection des groupes OH du résidu sucré, que celles décrites à l'exemple 1, on obtient le produit recherché (14).
1) **2,3,4,6-tétra-O-acétyl-α-D-galactopyranoside de 2-bromométhyl phényle (9).**
   - Préparation :
      A partir du 2,3,4,6-tétra-O-acétyl-α-D-galactopyranoside de 2-méthyl phényle 8 (Réf : P.M. DEY, Chemistry and Industry (London), 39, 1637, 1967) en utilisant NBS-CCl₄ (Rdt : 80 %).
   - Composé 9 : C₂₁H₂₅BrO₁₀. M = 517. RMN ¹H (200 MHz, CDCl₃) δ ppm : 1,96 (s, 3H), 2,04 (s, 3H), 2,11 (s, 3H), 2,19 (s, 3H), 4,14 (m, 2H), 4,40 (t, 1H, J = 6 Hz), 4,60 (ABq, 2H, J = 9 Hz), 5,38 (dd, 1H, J = 11 et 4 Hz), 5,50-5,75 (m, 2H), 5,85 (d, 1H, J = 4 Hz), 7,00-7,40 (m, 4H).
2) **2,3,4,6-tétra-O-acétyl-α-D-galactopyranoside de 2-hydroxyméthyl phényle (11).**
   - Préparations :
      a) à partir de 9 en utilisant AgNO₃ (Rdt : 21 %) ;
      b) à partir de 10 en utilisant NaBH₄-THF-MeOH (Rdt : 80 %).
   - Composé 11 : C₂₁H₂₆O₁₁. M = 454. F = 96 °C. RMN ¹H (200 MHz, CDCl₃) δ ppm : 1,98 (s, 3H), 2,03 (s, 3H), 2,09 (s, 3H), 2,19 (s, 3H), 4,15 (m, 2H), 4,43 (t, 1H, J = 6 Hz), 4,57 (d, 1H, J = 12,5 Hz), 4,89 (d, 1H, J = 12,5 Hz), 5,30-5,60 (m, 3H), 5,72 (d, 1H, J = 4 Hz), 7,00-7,40 (m, 4 H).
3) **2,3,4,6-tétra-O-acétyl-α-D-galactopyranoside de 2-formyl phényle (10).**
   - Préparation :
      à partir de 8 par bromation, puis en utilisant AgNO₃ (Rdt : 52 %).
   - Composé 10 : C₂₁H₂₄O₁₁. M = 452. RMN ¹H (200 MHz, CDCl₃) δ ppm : 2,00 (s, 3H), 2,06 (s, 3H), 2,08 (s, 3H), 2,19 (s, 3H), 4,18 (m, 2H), 4,43 (t, 1H, J = 6 Hz), 5,38 (d, 1H, J = 11 Hz), 5,40-5,60 (m, 2H), 5,84 (d, 1H, J = 4 Hz), 7,19 (t, 1H, J = 8 Hz), 7,30 (d, 1H, J = 8 Hz), 7,58 (t, 1H, J = 8 Hz), 7,91 (d, 1H, J = 8 Hz), 10,56 (s, 1H).
4) **Carbonate de 2,5-dioxopyrrolidin-1-yle et de 2-(2,3,4,6-tétra-O-acétyl-α-D-galactopyranosyl)benzyle (12).**
   - Préparation :
      à partir de 11, en utilisant le succinimido-chloroformiate (cf. préparation de 5, Rdt : 72 %).
   - Composé 12 : C₂₆H₂₉NO₁₅. M = 595. RMN ¹H (200 MHz, CDCl₃) δ ppm 1,98 (s, 3H), 1,99 (s, 3H), 2,06 (s, 3H), 2,17 (s, 3H), 2,87 (sl, 4H), 4,15 (m, 2H), 4,43 (t, 1H, J = 6 Hz), 5,16 (d, 1H, J = 12 Hz), 5,33 (dd, 1H, J = 12 et 4 Hz), 5,40-5,90 (m, 5H), 7,10 (t, 1H, J = 8 Hz), 7,20-7,50 (m, 3H).
5) **N- [2-(2,3,4,6-tétra-O-acétyl-**α**-D-galactopyranosyl)benzyloxycarbonyl] daunorubicine (13).**
   - Préparation :
      à partir de 12 et de la daunorubicine (Rdt : 85 %), selon le protocole décrit pour 6.
   - Composé 13 : F = 130°C. [α]_{D}²⁰ = +216° (c 0,25, CHCl₃). C₄₉H₅₃NO₂₂, M=1007. RMN ¹H (200 MHz, CDCl₃) δ ppm : 1,87 (s, 3H), 2,00 (s, 3H), 2,08 (s, 3H), 2,12 (s, 3H), 2,41 (s, 3H), 2,94 (d, 1H, J = 18 Hz), 3,23 (d, 1H, J = 18 Hz), 3,66 (s, 1H), 3,70-4,20 (m, 6H), 4,08 (s, 3H), 5,10 (ABq, 2H, J = 9 Hz), 5,20-5,70 (m, 7H), 5,85 (d, 1H, J = 4 Hz), 7,00-7,50 (m, 4H), 7,78 (t, 1H, J = 8 Hz), 8,03 (d, 1H, J = 8 Hz), 13,28 (s, 1H), 13,98 (s, 1H).
6) **N-[2-(α-D-galactopyranosyl)-benzyloxycarbonyl] daunorubicine (14).**
   - Préparation :
      à partir de 13, en utilisant MeONa (cat)-MeOH (cf. Préparation de 7, Rdt : 83 %).
      C₄₁H₄₅NO₁₈, M = 839. F = 130°C.

### EXEMPLE 3 : synthèse de la β-D-glucuronide de N-(4-hydroxy-benzyloxycarbonyl) daunorubicine (22).

Conformément au schéma III, le couplage de per-O-acétyl-D-glucuronate de méthyle avec du para crésol est réalisé dans une solution de CH₂Cl₂ en présence de TMSOTf comme catalyseur.

La préparation du dérivé 22 est ensuite réalisée à l'aide des mêmes séquences de réactions que celles des exemples 1 et 2, à l'exception de la déprotection additionnelle du groupe carbométhoxy, qui est réalisée par mélange dans du MeOH en présence de BaO.
1) **(2,3,4-tri-O-acétyl-β-D-glucopyranoside de 4-bromométhyl phényle) uronate de méthyle (16).**
   Il est obtenu (Rdt 79 %) selon le procédé indiqué pour 2 (voir exemple 1), à partir de (2,3,4-tri-O-acétyl-β-D-glucopyranoside de 4-méthyl phényle) uronate de méthyle (15), obtenu comme décrit dans G.N. BOLLENBACK, J. Am. Chem. Soc., 1955, 77, 3310-3315.
   C₂₀H₂₃O₁₀Br, M = 503. F = 144-146°C (éthanol). [α]_{D}²⁰ = +3° (c 0,85, CHCl₃). I.R. (CDCl₃) : 1758 (CO) cm⁻¹. RMN ¹H (90 MHz, CDCl₃) δ ppm : 2,00 (s, 9H), 3,66 (s, 3H), 4,16 (m, 1H), 4,43 (s, 2H), 5,03-5,36 (m, 4H), 6,89-7,33 (AB, 4H). SM (DIC/NH₃) : m/z 521 (M+NH₄)⁺.
2) **(2,3,4-tri-O-acétyl-β-D-glucopyranoside de 4-hydroxyméthyl phényle) uronate de méthyle (18) et (2,3,4-tri-O-acétyl-β-D-glucopyranoside de 4-formyl-phényle) uronate de méthyle (17).**
   Ils sont obtenus à partir de 16 (1,2 g, 2,3 mmol) selon le procédé indiqué précédemment pour l'obtention de 4 (exemple 1). Une chromatographie du résidu sur gel de silice, avec élution par un mélange hexane/acétate d'éthyle (2:1, v/v) permet d'isoler du produit de départ 16 (100 mg) ; 18 (650 mg, 38 %) puis 17 (200 mg, 12 %).
   - Composé 18 : C₂₀H₂₄O₁₁, M = 440. F = 134-136°C ; [α]_{D}²⁰ = -45° (c 0,55, CHCl₃) ; I.R. (KBr) : 3556 (OH), 1758 (CO) cm⁻¹ ; RMN ¹H (90 MHz, CDCl₃) δ ppm : 1,96 (s, 9H), 3,63 (s, 3H), 3,96-4,20 (m, 2H) ; 4,53 (s, 2H) ; 4,96-5,33 (m, 4H) ; 7,20 et 6,86 (AB, 4H). SM (DIC/NH₃) : m/z : 458 (M+NH₄)⁺.
   - Composé 17 : C₂₀H₂₂O₁₁, M = 438. F = 170-172°C ; [α]_{D}²⁰ = -31° (c 0,95, CHCl₃) ; I.R. (CDCl₃) : 1758 (C=O, ester), 1698 (C=O, PhCHO) cm⁻¹ ; RMN ¹H (90 MHz, CDCl₃) δ ppm : 2,12 (s, 9H), 3,72 (s, 3H), 4,21-4,39 (m, 1H), 5,30-5,56 (m, 4H), 7,07-8,03 (dd, 4H), 9,91 (s, 1H). SM (DIC/NH₃) m/z : 456 (M+NH₄)⁺.
3) **Carbonate de 2,5-dioxopyrrolidin-1-yle et de 4-((2,3,4-tri-O-acétyl-β-D-glucopyranosyl) uronate de méthyle)-benzyle (19).**
   - Préparation : Une solution de 18 (170 mg, 0,39 mmol) et de triéthylamine (54 µl, 0,39 mmol) dans du dichlorométhane anhydre (4 ml) est ajoutée sous argon goutte à goutte à une solution de DSC (197 mg, 0,77 mmol) dans 8 ml d'acétonitrile. Après 24 h d'agitation à température ambiante, le milieu est filtré et le filtrat concentré jusqu'à siccité. Le produit 19 est obtenu (117 mg, 52 %) sous la forme d'un sirop incolore.
   - Composé 19 : C₂₅H₂₇NO₁₅, M = 581. [α]_{D}²⁰ = +3° (c 0,85, CHCl₃). I.R. (CDCl₃) : 1747 (C=0) cm⁻¹. RMN ¹H (90 MHz, CDCl₃) δ ppm : 2,08 (s, 9H), 2,82 (s, 4H), 3,73 (s, 3H), 4,24 (d, 1H, J = 9), 5,13-5,44 (m, 6H), 6,96-7,44 (m, 4H). SM (DIC/NH₃) m/z : 599 (M+NH₄)⁺.
4) **N-[4-((2,3,4-tri-O-acétyl-β-D-glucopyranosyl)**-**benzyloxycarbonyl) uronate de méthyle] daunorubicine (20).**
   - Préparation :
      Selon le procédé indiqué pour obtenir 6 et 13 (Rdt 70 %) (voir exemples 1 et 2).
   - Composé 20 : Produit amorphe. C₄₈H₅₁NO₂₂, M = 993. [α]_{D}²⁰ = +112° (c 0,06, CHCl₃). RMN ¹H (250 MHz, CDCl₃) δ ppm : 1,32 (d, 3H, J = 6 Hz), 1,74 (s, 1H), 1,77 et 1,95 (AB, 2H, J = 5, J′ = 12), 2,07 (3s, 9H), 2,12-2,38 (AB, 2H), 2,44 (s, 3H), 2,97 et 3,26 (AB, 1H, J = 20), 3,70 (m, 1H), 3,73 (s, 3H), 3,92 (large s, 1H), 4,12 (s, 3H), 4,19 (m, 1H), 5,00 (s, 2H), 5,14 (d, 1H, J = 7), 5,68 (dd, J = 3, J′ = 7, 1H), 5,53 (d, 1H, J = 3,5), 6,98 et 7,28 (AB, 4H, J = 8), 7,43 (d, 1H, J = 8), 7,83 (t, 1H, J = J' = 8), 8,07 (d, 1H, J = 8), 13,33 et 14,00 (2s, 2H). SM(FAB) : m/z : 1016 (M+Na)⁺.
5) **N-[4-((β-D-glucopyranosyl)-uronate de méthylebenzyloxycarbonyl)] daunorubicine (21).**
   Par traitement MeONa-MeOH de 20. (cf. préparation de 7 et 14, exemples 1 et 2, Rdt 78 %). Sirop rouge. C₄₂H₄₅NO₁₉, M = 867. [α]_{D}²⁰ = +140° (c 0,02, CH₃OH).SM (252C FPD) : m/z : 890 (M+Na)⁺.
6) **β-D-glucuronide de N-(4-hydroxy-benzyloxycarbonyl) daunorubicine (22)**.
   Le composé 21 (2,7 mg) est dissous dans 0,2 ml de MeOH et l'on agite durant 3 h à température ambiante en présence d'oxyde de baryum. Le milieu est ensuite neutralisé par addition de résine Amberlite IR 50 H+ et après filtration on concentre sous pression réduite. On isole 1,8 mg de 22 pur par précipitation dans un mélange MeOH/Et₂O/hexane. C₄₁H₄₃NO₁₉, M = 853. [α]_{D}²⁰ = +2° (c 0,06, MeOH).

### EXEMPLE 4 : synthèse de la N-[2-((β-D-glucopyranosyl) acide uronique)-benzyloxycarbonyl] daunorubicine (27c).

Conformément au schéma IV, le méthyl 2-bromométhylphényl-(2,3,4-tri-O-acétyl-β-D-glucopyranosyl) uronate 23, obtenu à partir du méthyl 2-méthyl-phényl-(2,3,4-tri-O-acétyl-β-D-glucopyranosyl) uronate, comme décrit dans G.N. BOLLENBACK et al. (J. Am. Chem. Soc., 1955, 77, 3310), est converti en produit 24 (et/ou en produit 25) puis le produit 24 est transformé en produit 26.

Dans ce cas, pour lier le bras à l'anthracycline, le carbonate 26 est préparé à l'aide de chloroformiate de 4-nitrophényle disponible dans le commerce.

Le composé 27c (prodrogue recherchée) est ensuite obtenu comme décrit ci-dessus aux exemples 1, 2 et 3.
1) **(2,3,4-tri-O-acétyl-β-D-glucopyranoside de 2-bromométhyl phényl) uronate de méthyle (23).**
   - Préparation :
      Une solution de méthyl 2-méthyl-phényl-(2,3,4-tri-O-acétyl-β-D-glucopyranosyl) uronate de méthyle (940 mg, 2,2 mmol) (réf. : G.N. BOLLENBACK, J. Am. Chem. Soc., 77, 3310-3315, 1955), de NBS (509 mg, 2,86 mmol) et de 52 mg de peroxyde de benzoyle dans 25 ml de CCl₄ est portée au reflux durant 12 h. Après extraction classique et purification sur colonne de silice, on isole 880 mg de 23 (80 %).
   - Composé 23 : C₂₀H₂₃BrO₁₀, M = 503. RMN ¹H (200 MHz, CDCl₃) δ ppm : 2,05 (s, 3H), 2,07 (s, 3H), 2,11 (s, 3H), 3,74 (s, 3H), 4,19 (d, 1H, J = 9 Hz), 4,35 et 4,65 (2d, CH₂, J = 12 Hz), 5,23 (m, 1H), 5,38 (m, 3H), 7, 05 (m, 2H), 7, 32 (m, 2H).
2) **(2,3,4-tri-O-acétyl-β-D-glucopyranoside de 2-hydroxy méthyl phényl) uronate de méthyle (24) et de 2-formyl phényl) uronate de méthyle (25).**
   - Préparation :
      Une solution de dérivé bromé 23 (690 mg, 1,37 mmol) dans 14 ml d'une solution acétone/eau (50/50) est agitée durant 2 h en présence de 631 mg (3,7 mmol) de AgNO₃. Après filtration et extraction classique, on isole par chromatographie sur gel de silice, 80 mg d'aldéhyde 25 (13 %) et 340 mg d'alcool 24 (56 %).
      Caractéristiques de 24 : C₂₀H₂₄O₁₁, M = 440. F = 143-147°C. [α]_{D}^{20°} -26° (c 0,9, CHCl₃). RMN (200 MHz, CDCl₃) : 2,05 (s, 3H) ; 2,07 (s, 3H) ; 2,11 (s, 3H) ; 3,72 (s, 3H) ; 4,13 (d, J = 9 Hz, H-5) ; 4,48 et 4,77 (2d, J = 12,5 Hz, 2H) ; 5,15 (d, J = 7,2 Hz, 1H) ; 5,35 (m, 3H) ; 7,02 (d, J = 8 Hz, 1H) ; 7,11 (t, J = 7,2 Hz, 1H) ; 7,29 (d, J = 6,8 Hz, 1H) ; 7,34 (t, J = 7,4 Hz, 1H) ppm. I.R. (CH₂Cl₂) : 3540, 3030, 2960, 1760, 1605, 1590, 1490, 1455, 1440, 1375, 1225, 1140, 1090, 1075, 1040 cm⁻¹. SM (FAB⁺) m/z : 463 (M+Na)⁺.
   - Composé 25 : C₂₀H₂₂O₁₁, M = 438. RMN ¹H (200 MHz, CDCl₃) δ ppm : 2,08 (s, 9H), 3,75 (s, 3H), 4,25 (d, 1H, J = 9 Hz), 5,34 (m, 4H), 7,15 (t, 1H, J = 8,6 Hz), 7,25 (d, 1H, J = 7,3 Hz), 7,58 (t, 1H, J = 7 Hz), 7,26 (d, 1H, J = 7,6 Hz), 10,34 (s, 1H).
3) **Carbonate de 4-nitro-phényle et de 2-((2,3,4-tri-O-acétyl-β-D-glucopyranosyl) uronate de méthyle)-benzyle (26).**
   - Préparation :
      Une solution de 24 (33 mg, 0,075 mmol) dans 0,021 ml de pyridine et 0,2 ml d'acétate d'éthyle est maintenue sous agitation pendant 12 h à température ambiante en présence de 46 mg (0,22 mmol) de chloroformiate de paranitrophényle. Après filtration et évaporation des solvants, une purification sur plaque de silice donne 45 mg de 26 (rendement quantitatif).
   - Composé 26 : C₂₇H₂₇NO₁₅, M = 605. F = 67-70°C. [α]_{D}²⁰ = +2° (c 1, CDCl₃). RMN ¹H (200 MHz, CHCl₃) δ ppm : 2,07 (s, 9H) ; 3,74 (s, 3H), 4,24 (d large, J = 8,5 Hz, H-5), 5,3 (m, 6H), 7,10 (m, 2H), 7,39 (m, 4H), 8,27 (d, J = 9 Hz, 2H).
4) **N-[2-((2,3,4,tri-O-acétyl-β-D-glucopyranosyl) uronate de méthyle)-benzyloxycarbonyl] daunorubicine (27a).**
   - Préparation :
      Une solution de daunorubicine (15,8 mg, 0,03 mmol) et de glycoside 26 (1,1 éq., 19,8 mg) et de Et₃N (3,6 mg, 1,2 éq.) dans 0,1 ml de DMF est maintenue sous agitation pendant 12 heures. Après extraction classique, une purification sur plaque de silice permet d'obtenir 20 mg (67 %) de 27.
   - Composé 27a : C₄₈H₅₁NO₂₂, M = 993. F = 153-155°C. [α]_{D}²⁰ = +122° (c 0,4, CHCl₃). RMN ¹H (200 MHz, CDCl₃), δ ppm : 1,31 (d, 3H, J = 6,4 Hz), 2,06 (s, 6H), 2,09 (s, 3H), 2,42 (s, 3H), 2,91 et 3,24 (2d, J = 19 Hz, 2H), 3,60 (s, 3H), 4,08 (s, 3H), 4,20 (m, 2H), 5,00-5,32 (m, 8H), 7,05 (m, 2H), 7,26 (m, 2H), 7,38 (d, J = 7,4 Hz, 1H), 7,77 (t, J = 7,4 Hz, 1H), 8,03 (d, J = 7,4 Hz, 1H), 13,24 (s, 1H), 12,57 (s, 1H). SM : [M+K]⁺ = 1032.
5) **N-[2-((β-D-glucopyranosyl) uronate de méthyle)-benzyloxycarbonyl] daunorubicine (27b).**
   Par traitement MeONa-MeOH de 27a (cf. préparation de 7 et 14, exemples 1 et 2). Solide rouge. C₄₂H₄₅O₁₉N, M = 867.
6) **N-[2-((β-D-glucopyranosyl) acide uronique)**-**benzyloxycarbonyl] daunorubicine (27c).**
   Par traitement de 27b avec BaO ou K₂CO₃ (cf préparation de 22 dans l'exemple 3). Solide rouge C₄₁H₄₃NO₁₉, M = 853.

### EXEMPLE 5 : synthèse de la N-[2-hydroxybenzyloxycarbonyl] daunorubicine (32).

Conformément au schéma V, le dérivé 32 est préparé soit par synthèse à partir du dérivé 28 (2-tertiobutyldiméthylsilyloxy-benzaldéhyde), qui est réduit en présence de NaBH₄ pour donner le composé 29, l'activation par le chloroformiate de 4-nitrophényle conduit au dérivé 30 qui est condensé avec la daunorubicine, le produit 32 est ensuite obtenu par déprotection au KF, soit par hydrolyse enzymatique du produit 14 (voir exemple 2) par l'α-galactosidase.
1) **N-[2-hydroxy-benzyloxycarbonyl] daunorubicine (32).**

   a) Hydrolyse enzymatique de 14 :
      Le composé 14 (1 mg) est dissous dans 0,02 ml de DMF. On ajoute alors 1 ml d'une solution 100 mM d'HEPES dans l'eau distillée. Après addition d'α-galactosidase (2U, EC 3.2.1.22, Sigma, n° G-8507), le mélange réactionnel est agité pendant 2 heures à 35°C. Après hydrolyse et extraction on obtient quantitativement le composé 32.
   b) Hydrolyse de 31 :
      Le dérivé silylé 31 (2 mg, 0,0025 mmol) est dissous dans le THF (0,1 ml). On ajoute alors 0,2 ml d'une solution aqueuse de KF (à 0,1 g par mL). Après 12 heures à température ambiante une extraction usuelle fournit 32.
      - Caractéristiques de 32 : C₃₅H₃₅NO₁₃, M = 677. RMN ¹H (200 MHz, CDCl₃) δ ppm : 1,26 (d, J = 6 Hz, 3H), 2,42 (s, 3H), 2,89 (d, J = 20 Hz, 1H), 3,24 (d, J = 20 Hz, 1H), 3,61 (s, 1H), 4,07 (s, 3H), 4,43 (s, 1H), 5,00 (s, 2H), 5,28 (s large, 1H), 5,47 (d, J = 2 Hz, 1H), 6,7-7,3 (m, 4H), 7,40 (d, J = 8 Hz, 1H), 7,80 (t, J = 8 Hz, 1H), 8,00 (d, J = 8 Hz, 1H), 8,78 (s, 1H), 13,27 (s, 1H), 13,96 (s, 1H).

   Le produit 32 peut être avantageusement condensé avec un sucre pour fournir les produits 27a, 27b ou 27c.
2) **2-tertiobutyldiméthylsilyloxy-benzaldéhyde (28).**
   - Préparation :
      Une solution de 2-hydroxy benzaldéhyde (500 mg, 0,44 ml, 4,09 mmol), d'imidazole (685 mg) et de chlorure de tertiobutyldiméthylsilyle (680 mg) dans le DMF est agitée pendant 24 heures à température ambiante. Après hydrolyse et extraction usuelle, une chromatographie permet d'obtenir 28 (890 mg, 93 %).
   - Composé 28 : C₁₃H₂₀O₂Si, M = 236. RMN ¹H (200 MHz, CDCl₃) δ ppm : 0,28 (s, 6H), 1,00 (s, 9H), 0,91 (d, J = 8Hz, 1H), 7,02 (t, J = 8Hz, 1H), 7,46 (t, J = 8 Hz, 1H), 7,81 (d, J = 8 Hz, 1H), 10,47 (s, 1H).
3) **alcool 2-tertiobutyldiméthylsilyloxy benzylique (29).**
   - Préparation :
      Une solution de 28 (200 mg) dans le méthanol (5,5 ml) contenant NaBH₄ (28 mg) est agitée pendant 1 heure à température ambiante. Après hydrolyse et extraction usuelle, on obtient 29 (140 mg, 68 %).
   - Composé 29 : C₁₃H₂₂O₂Si, M = 238. RMN ¹H (200 MHz, CDCl₃) δ ppm : 0,30 (s, 6H), 1,06 (s, 9H), 2,67 (s, 1H), 4,70 (s, 2H), 6,86 (d, J = 8 Hz, 1H), 6,97 (t, J = 8 Hz, 1H), 7,20 (t, J = 8 Hz, 1H), 7,33 (d, J = 8 Hz, 1H).
4) **Carbonate de 4-nitro-phényle et de 2-(tertiobutyldiméthylsilyloxy)-benzyle (30).**
   - Préparation :
      L'alcool 29 (135 mg, 0,56 mmol) est dissous dans l'acétate d'éthyle (0,82 ml). On ajoute alors de la pyridine (0,081 ml) puis le chloroformiate de 4-nitro phényle (275 mg, 2,4 éq.). Après une nuit à température ambiante, le solvant est évaporé. Une chromatographie sur gel de silice fournit le carbonate 30 (206 mg, 91 %).
   - Caractéristiques de 30 : C₂₀H₂₅NO₆Si, M = 403. RMN ¹H (200 MHz, CDCl₃) δ ppm : 0,27 (s, 6H), 1,03 (s, 9H), 5,32 (s, 2H), 6,86 (d, J = 8 Hz, 1H), 6,96 (t, J = 8 Hz, 1H), 7,1-7,5 (m, 4H), 8,21 (d, J = 8 Hz, 2H).
5) **N-[2-(tertiobutyldiméthylsilyloxy)-benzyloxycarbonyl] daunorubicine (31).**
   - Préparation :
      Une solution de 30 (8,3 mg, 0,022 mmol), de daunorubicine (9,6 mg, 0,018 mmol) et de triéthylamine (0,003 ml) dans le DMF (0,1 ml) est agitée pendant 4 heures à température ambiante. Après extraction usuelle, une chromatographie permet de séparer 31 (6 mg, 41 %) et 32 (4,5 mg, 36 %).
   - Composé 31 : C₄₁H₄₉NO₁₃Si, M = 791. NMR ¹H (200 MHz, CDCl₃) δ ppm : 0,20 (s, 6H), 0,96 (s, 9H), 1,30 (d, J = 6 Hz, 1H), 2,42 (s, 3H), 2,9 (m, 2H), 3,24 (d, J = 20 Hz, 1H), 3,65 (s large, 1H), 3,90 (s large, 1H), 4,09 (s, 3H), 4,48 (s, 1H), 5,06 (s, 2H), 5,28 (s, 1H), 5,52 (d, J = 2 Hz, 1H), 6,8-7,0 (m, 4H), 7,42 (d, J = 8 Hz, 1H), 7,80 (t, J = 8 Hz, 1H), 8,03 (d, J = 8 Hz, 1H), 13,31 (s, 1H), 14,0 (s, 1H).

### EXEMPLE 6 : synthèse de la N-[4-(β-D-glucopyranosyl)-benzyloxycarbonyl] daunorubicine (37).

Conformément au schéma VI, une catalyse par transfert de phase est utilisée pour la condensation du peracétyl-α-D-glucose avec le p. hydroxybenzaldéhyde et le glycoside 33 est converti en composé 37, conformément aux exemples 1, 2, 3, 4 ou 5 ci-dessus.
1) **2,3,4,6-tétra-O-acétyl-β-D-glucopyranoside de 4-formyl phényle (33).**
   - Préparation :
      On dissout le 2,3,4,6-tétra-O-acétyl-α-D-bromoglucose (2 g, 4,8 mmol) dans 20 ml de chloroforme. On ajoute dans la solution précédente un mélange de p-hydroxy-benzaldéhyde (585 mg, 4,8 mmol) et de bromure de benzyltriéthylammonium (1,1 g), préalablement dissous dans une solution de soude 1,25 N (10 ml), et l'on chauffe à reflux pendant 24 h. On effectue une extraction avec 50 ml d'eau. La phase organique obtenue est lavée successivement avec une solution de NaOH 1N (2 x 50 ml), avec une solution de HCl(1N), puis avec de l'eau, séchée et concentrée jusqu'à siccité. Le produit brut obtenu est ensuite recristallisé dans de l'éthanol pour donner 33 (0,5 g, 23 %).
   - Composé 33 : C₂₁H₂₄O₁₁, M = 452. F = 150-152°C ; [α]_{D}²⁰ = -28° (c 0,5, CHCl₃). I.R. (CDCl₃) : 1757 (C=O, ester), 1698 (C=O, aldéhyde) cm⁻¹. RMN ¹H (90 MHz, CDCl₃) δ ppm : 2,09 (s, 12H), 3,99 (m, 1H), 4,22 (AB, 1H), 4,33 (AB, 1H), 5,13-5,48 (m, 4H), 7,15 et 7,89 (AB, 4H, J = 12 Hz), 9,96 (s, 1H). SM (DIC) : m/z : 470 (M+NH₄)⁺.
2) **2,3,4,6-tétra-O-acétyl-β-D-glucopyranoside de 4-hydroxyméthyl phényle (34).**
   - Préparation :
      Il est obtenu à partir de 33 selon le procédé utilisé pour préparer 11 à partir de 10 (voir exemple 2) et cristallise de l'éthanol (Rdt 92 %).
   - Composé 34 : C₂₁H₂₆O₁₁, M = 454. F = 102-103°C ; [α]_{D}²⁰ = -18° (c 0,5, CHCl₃). I.R. (CDCl₃) : 1757 (C=O, ester) cm⁻¹. RMN ¹H (200 MHz, CDCl₃) δ ppm : 1,98-2,03 (4s, 12H, 4 OAc), 3,75-3,84 (m, 1H, H-5), 4,11 (AB, Jgem = 6 Hz, H-6a), 4,57 (s, 1H, OH), 4,99-5,25 (m, 4H, H-1, H-2, H-3 et H-4), 6,92 et 7,24 (AB, Jgem = 8 Hz, 4H, Ph). SM (DIC) : m/z : 472 (M+NH₄)⁺.
3) **Carbonate de 2,5-dioxopyrrolidin-1-yle et de 4**-(**2,3,4,6-tétra-O-acétyl-β**-**D-glucopyranosyl)-benzyle (35).**
   - Préparations :
      Méthode A : Par activation par le succinimido-chloroformiate (cf. : préparation de 5, exemple 1) du composé 34 (617 mg, 1,36 mmol). On obtient le produit pur 35 (780 mg, 95 %).
      Méthode B : Par activation par le DSC (cf. : préparation de 19, exemple 3) du composé 34 (Rdt 80 %).
   - Composé 35 : C₂₆H₂₉O₁₅N, M = 595. F = 164°C ; [α]_{D}²⁰ = -18° (c 0,09, CHCl₃) I.R. (KBr) : :1791 (C=O) cm⁻¹. RMN ¹H (200 MHz, CDCl₃) δ ppm : 2,02 (4s, 12H), 2,77 (s, 4H), 3,81 (m, 1H), 4,14 (AB, J = 12 Hz, 1H), 4,24 (AB, J = 5 Hz, 1H), 5,02-5,31 (m, 6H), 6,95-7,31 (AB, J = 12 Hz, 4H). SM (DIC/NH₃) m/z : 613 (M+NH₄)⁺.
4) **N-[4-(2,3,4,6-tétra-O-acétyl-β-D-glucopyranosyl)benzyloxycarbonyl] daunorubicine (36).**
   - Préparation :
      Par condensation du composé 35 (26 mg, 0,04 mmol) sur la daunorubicine (20 mg, 0,04 mmol) (cf. préparation de 6 et de 13, exemples 1 et 2). On isole le dérivé 36 pur (25 mg, 90 %) sous forme d'une laque rouge.
   - Composé 36 : C₄₉H₅₃O₂₂N, M = 1007. [α]_{D}²⁰ = 135° (c 0,04, CHCl₃). I.R. (CDCl₃) : 3435 (OH) ; 1758 (C=O) cm⁻¹. RMN ¹H (400 MHz, CDCl₃) δ ppm : 1,29 (d, 3H, J = 5 Hz), 1,79 (AB, 1H, J = 3,5 Hz), 1,91 (AB, 1H, Jgem = 14 Hz), 2,07 (4s, 12H), 2,15 (AB, 1H, J = 3,5 Hz), 2,35 (AB, 1H, J = 18 Hz), 2,50 (s, 3H), 2,95 (AB, 1H, J = 18 Hz), 3,25 (AB, 1H, J = 18 Hz), 3,70 (m, 1H), 3,84-3,90 (m, 1H), 3,92 (1H, OH), 4,11 (s, 3H, OMe), 4,17 (AB, 1H, J = 2 Hz), 4,24 (m, 1H), 4,30 (AB, 1H, J = 12 Hz), 5,00 (s, 2H), 5,08 (d, 1H, J = 7 Hz), 5,14 (d, 1H, J = 8,5 Hz), 5,17 (t, 1H, J = J′ = 10 Hz), 5,24-5,34 (m, 3H), 5,23 (dd, 1H, H-1', J < 0,5 Hz, J′ = 3,5 Hz), 6,96 et 7,28, (AB, 4H), 7,42 (d, 1H, J = 8 Hz), 7,82 (t, J = J′ = 8 Hz), 8,07 (d, 1H, J = 8 Hz), 13,25 (s, 1H), 14,00 (s, 1H). SM (DIC/NH₃) : m/z : 1026 (M+NH₄)⁺.
5) **N-[4-(β-D-glucopyranosyl)-benzyloxycarbonyl] daunorubicine (37).**
   Par MeONa cat.-MeOH sur le composé 36 (91 %), 37 est obtenu sous la forme d'un sirop rouge.
   C₄₁H₄₅O₁₈N, M = 839. [α]_{D}²⁰ = +200° (c 0,001, CH₃OH). I.R. (KBr) : 3422 (OH) ; 1617 (C=O) cm⁻¹. SM (FAB) : m/z : 838 (M+1)⁺.

### EXEMPLE 7 : préparation de la N-[4-hydroxy-benzyloxycarbonyl] daunorubicine (38).

Selon le schéma VII, le dérivé 38 est préparé soit par synthèse, soit par hydrolyse enzymatique, conformément à l'exemple 5 (dérivé 32).
1) **N-[4-hydroxy-benzyloxycarbonyl] daunorubicine (38).**
   Le composé 37 (3 mg, 3,68 µMol) est dissous dans une solution tampon d'acétate de sodium, pH 5,5 (0,46 ml), puis une suspension d'enzyme (20 µl, 5 U de β-D-glucosidase (20 mg de β-D-glucosidase dans 400 µl d'eau plus 80 µl d'éthanol) est ajoutée à 37°C pendant 15 min. La réaction enzymatique est complète. On filtre, évapore les solvants, puis on chromatographie le résidu sur gel de silice à l'aide du mélange CH₂Cl₂-CH₃OH (9:1, v/v). On isole le dérivé 38 pur (1,5 mg, 60 %), qui se présente sous la forme d'un liquide rouge sirupeux.

   C₃₅H₃₅O₁₃N, M = 676.

   Le composé 38 peut avantageusement être condensé avec un autre sucre et notamment un dérivé de l'acide glucuronique pour fournir les produits 20, 21 ou 22.
2) **alcool 4-diméthylthexylsilyloxy benzylique (39).**
   2,8 g (41,2 mmol) d'imidazole sont ajoutés à une solution d'alcool 4-hydroxybenzylique (2,55 g, 20,6 mmol) dans 25 ml de DMF anhydre. Le mélange est agité à température ambiante jusqu'à obtention d'une solution limpide, puis est refroidi à 0°C sous argon. On ajoute alors 4,4 ml (22,6 mmol) de chlorure de thexyldiméthylsilyle. Après 48 h d'agitation à 0°C, la phase organique est lavée à l'eau, séchée sur Na₂SO₄ et évaporée sous pression réduite. On obtient 39 (3,7 g, 68 %) sous forme de gomme.
   C₁₅H₂₆O₂Si, M = 266,44. I.R. (CDCl₃) : 3304 (OH), 2960 (CH) cm⁻¹. RMN ¹H (90 MHz, CDCl₃) δ ppm : 0,00 (s, 6H, CH₃Si), 0,83 (d, 6H, CH₃CCSi, J = 5 Hz), 0,83 (d, 6H, CH₃CSi), 1,50 (m, 1H, CHCSi), 4,50 (s, 2H, CH₂Ph), 6,67-7,03 (AB, Jgem = 6 Hz, 4H, Ph). SM (DIC) : m/z : 284 (M+NH₄)⁺.
3) **Carbonate de 2,5-dioxopyrrolidin-1-yle et de 4-diméthylthexyl-silyloxybenzyle (40).**
   A une solution de DSC (1,39 g, 5 mmol) dans l'acétate d'éthyle anhydre (50 ml), on ajoute 1,85 g (10 mmol) de composé 35 et 0,8 ml de pyridine. Après 24 h d'agitation à la température ambiante, le mélange est filtré, et le filtrat concentré sous pression réduite, fournissant ainsi 40 (1,84 g, 91 %) sous forme d'une gomme.
   C₂₀H₂₉O₆SiN, M = 407,52. I.R. (CDCl₃) : 2961 (CH), 1296 (CO) cm⁻¹.
   RMN ¹H (90 MHz, CDCl₃) : δ ppm : 0,00 (s, 6H, CH₃Si), 0,80 (d, 6H, CH₃CCSi, J = 5 Hz), 0,80 (s, 6H, CH₃CSi), 1,50 (m, 1H, CHCSi), 2,73 (s, 4H, CH₂CO), 4,57 (s, 2H, CH₂Ph), 7,16 (AB, 4H, Ph, Jgem = 6 Hz). SM(DIC) : m/z : 425 (M+NH₄)⁺.
4) **N-[4-diméthylthexylsilyloxy-benzyloxycarbonyl] daunorubicine (41).**
   A un mélange de 40 (29 mg, 0,073 mmol) et de daunomycinone (20 mg, 0,043 mmol) dissous dans 0,4 ml de DMF anhydre, on ajoute 76 µl (0,73 mmol) de Et₃N et, après 5 min d'agitation à la température ambiante, on effectue une extraction par de l'acétate d'éthyle. La solution obtenue est lavée avec une solution saturée de chlorure de sodium, puis séchée sur Na₂SO₄ et concentrée sous pression réduite. On isole 41 (24 mg, 70 %) sous forme d'un sirop rouge.
   C₄₃H₅₂SiN, M = 819. [α]_{D}²⁰ = +53° (c 0,03, CHCl₃). I.R. (CDCl₃) : 3597-3439 (OH), 2960 (CH), 1762 (CO) cm⁻¹. RMN ¹H (250 MHz, CDCl₃) δ ppm : 0,00 (m, 6H, CH₃Si), 0,82 (d, 6H, J = 5 Hz), 0,82 (s, 6H), 1,27 (d, 3H, J = 6 Hz), 1,62 (m, 3H), 2,36 (s, 3H), 2,89 et 3,20 (AB, 2H, J = 6 Hz), 3,93 (s, 1H), 4,04 (s, 3H), 4,20 (m, 1H), 4,40 (m, 1H), 4,62 (s, 2H), 5,52 (s, 1H), 5,33 (d, 1H, J = 3 Hz), 5,49 (dd, 1H, J = 2, J′ < 0,5 Hz), 6,95 et 7,19 (AB, 4H, J = 3 Hz), 7,34 (d, 1H, J = 9 Hz), 7,78 (t, 1H, J = J′ = 9 Hz), 8,00 (d, 1H, J = 9 Hz). SM (DIC) : m/z : 837 (M+NH₄)⁺.

### EXEMPLE 8 : synthèse de la N-[2-(α-D-galactopyranosyl)-5-nitro-benzyloxycarbonyl] daunorubicine (48b) et de la N-[2-(α-D-galactopyranosyl)-5-nitro-benzyloxycarbonyl] doxorubicine (49b).

Selon le schéma VIII, la glycosidation catalysée par SnCl₄ du 5-nitro-o-crésol avec le peracétyl-D-galactose permet d'obtenir le dérivé 42.

La bromation benzylique, utilisant le NBS dans CCl₄ permet d'obtenir un mélange de 43 et 44.

L'hydrolyse du dérivé dibromé 44 fournit l'aldéhyde 45 qui peut être réduit par le NaBH₄ en dérivé 46, tandis que l'hydrolyse du dérivé 43 conduit à un mélange de 45 et 46. Le formiate de 4-nitrophényle est utilisé pour activer le dérivé 46 et le couplage de 47 avec la daunorubicine et la doxorubicine conduit respectivement aux dérivés 48a et 49a.
1) **2,3,4,6-tétra-O-acétyl-α-D-galactopyranoside de 2-méthyl 4-nitro phényle (42).**
   On additionne lentement SnCl₄ (0,6 ml) à une solution maintenue à température ambiante de 2-méthyl 4-nitro phénol (Réf. : J.S. ANDERSON et K.C. BROWN, Synthetic Commun., 13, 233-236, 1983) et de 1,2,3,4,6-penta-O-acétyl-β-D-galactose (1 g, 2,56 mmol). Le mélange réactionnel est alors agité sous azote pendant 12 heures à 60°C. Après hydrolyse dans l'eau glacée, une extraction avec CH₂Cl₂, suivie d'un lavage avec une solution saturée d'hydrogénocarbonate de sodium puis à l'eau, fournit 42 qui est alors purifié par chromatographie sur gel de silice (Rdt : 45 %).
   C₂₁H₂₅NO₁₂, M = 483. F = 166°C. [α]_{D}²⁰ = +184° (c 0,56, CHCl₃). I.R. (CHCl₃) : 3030, 2960, 2925, 2860, 1745, 1580, 1520, 1490, 1370, 1345, 1220 cm⁻¹. RMN ¹H (200 MHz, CDCl₃) δ ppm : 1,96 (s, 3H), 2,05 (s, 3H), 2,09 (s, 3H), 2,19 (s, 3H), 2,36 (s, 3H), 4,13 (m, 2H), 4,28 (m, 1H), 5,85 (d, J = 4 Hz, 1H), 7,21 (d, J = 9 Hz, 1H), 8,10 (s, 1H et d, J = 9 Hz, 1H).
2) **2,3,4,6-tétra-O-acétyl-α-D-galactopyranoside de 2-bromométhyl 4-nitro phényle (43).**
   - Préparation :
      à partir de 42, en utilisant NBS (1,5 éq.) -CCl₄ (cf. préparation des composés 2 et 9, exemple 1, Rdt : 51 % ) .
   - Composé 43 : C₂₁H₂₄BrNO₁₂, M = 562. RMN ¹H (200 MHz, CDCl₃) δ ppm : 1,96 (s, 3H), 2,06 (s, 3H), 2,12 (s, 3H), 2,20 (s, 3H), 4,13 (m, 2H), 4,32 (t, J = 6 Hz, 1H), 4,59 (AB q, J = 9 Hz, 2H), 5,38 (dd, J = 12 et 4 Hz, 1H), 5,5-5,8 (m, 2H), 5,99 (d, J = 4 Hz, 1H), 7,28 (d, J = 9 Hz, 1H), 8,20 (d, J = 9 Hz, 1H), 8,29 (s, 1H).
3) **2,3,4,6-tétra-O-acétyl-α-D-galactopyranoside de 2-dibromométhyl 4-nitro phényle (44).**
   - Préparations :
      a) à partir de 42 en utilisant NBS (1,5 éq.) -CCl₄ (Rdt : 27 %) ;
      b) à partir de 42 en utilisant NBS (4 éq.) -CCl₄ (Rdt : 60-90 %).
   - Composé 44 : C₂₁H₂₃Br₂NO₁₂, M = 641. F = 70°C. [α]_{D}²⁰ = +143° (c 0,47, CHCl₃). I.R. (CHCl₃) : 3030, 3020, 2960, 2930, 2860, 1745, 1620, 1590, 1525, 1480, 1425, 1370, 1345, 1220, 1130, 1110, 1075, 1040 cm⁻¹. RMN ¹H (200 MHz, CDCl₃) δ ppm : 1,97 (s, 3H), 2,06 (s, 3H), 2,13 (s, 3H), 2,20 (s, 3H), 4,13 (m, 2H), 4,31 (t, J = 6 Hz, 1H), 5,3-5,7 (m, 3H), 5,93 (d, J = 14 Hz, 1H), 7,01 (s, 1H), 7,29 (d, J = 9 Hz, 1H), 8,20 (d, J = 9 Hz), 8,75 (s, 1H).
4) **2,3,4,6-tétra-O-acétyl-α-D-galactopyranoside de 2-formyl 4-nitro phényle (45).**
   - Préparations :
      a) à partir de 43, en utilisant AgNO₃ (Rdt : 15 %) ;
      b) à partir de 44, en utilisant AgNO₃ (Rdt : 83 %).
   - Composé 45 : C₂₁H₂₃NO₁₃, M = 497. F = 180°C. [α]_{D}²⁰ = +97° (c 2, CHCl₃). I.R. (CHCl₃) : 3030, 2960, 2930, 2880, 2860, 1745, 1695, 1645, 1610, 1590, 1530, 1480, 1430, 1370, 1345, 1220, 1180, 1125, 1110, 1075, 1045 cm⁻¹. RMN ¹H (200 MHz, CDCl₃) δ ppm : 1,98 (s, 3H), 2,05 (s, 3H), 2,08 (s, 3H), 2,20 (s, 3H), 4,13 (m, 2H), 4,35 (t, J = 6 Hz, 1H), 5,3-5,6 (m, 3H), 5,99 (d, J = 4 Hz, 1H), 7,46 (d, J = 9 Hz, 1H), 8,41 (dd, J = 9 et 2 Hz, 1H), 10,52 (s, 1H).
5) **2,3,4,6-tétra-O-acétyl-α-galactopyranoside de (2-hydroxyméthyl-4-nitro) phényle (46).**
   - Préparations :
      a) à partir de 43, en utilisant AgNO₃ (Rdt : 19 %) (cf. préparation de 11 et 18) ;
      b) à partir de 45, en utilisant NaBH₄-THF-MeOH (Rdt : 73 %).
   - Composé 46 : C₂₁H₂₅NO₁₃, M = 499. F = 140°C. [α]_{D}²⁰ = +152° (c 0,42, CDCl₃). I.R. (CDCl₃) : 3700, 3600, 3030, 2970, 2940, 2860, 1750, 1630, 1625, 1595, 1585, 1370, 1350, 1210, 1130, 1110, 1075, 1035 cm⁻¹. RMN (200 MHz, CHCl₃) δ ppm : 1,97 (s, 3H), 2,05 (s, 3H), 2,10 (s, 3H), 2,19 (s, 3H), 4,12 (m, 3H), 4,33 (t, J = 6 Hz, 1H), 4,71 (d, J=14 Hz, 1H), 4,89 (d, J = 14 Hz, 1H), 5,35-5,6 (m, 3H), 5,87 (d, J = 2 Hz, 1H), 7,28 (d, J = 9 Hz, 1H), 8,19 (dd, J = 9 et 2 Hz, 1H), 8,32 (d, J = 2Hz, 1H) .
6) **Carbonate de 4-nitro-phényle et de 2-(2,3,4,6-tétra-O-acétyl-α-D-galactopyranosyl)-5-nitro-benzyle (47).**
   - Préparation :
      à partir de 46 et du chloroformiate de p-nitrophényle (Rdt : 52 %).
   - Composé 47 : C₂₈H₂₈N₂O₁₇, M = 664. RMN ¹H (200 MHz, CDCl₃) δ ppm : 1,93 (s, 3H), 2,04 (s, 3H), 2,09 (s, 3H), 2,19 (s, 3H), 4,12 (m, 2H), 4,30 (t, J = 6 Hz, 1H), 5,3-5,6 (m, 5H), 5,93 (d, J = 4 Hz, 1H), 7,34 (d, J = 9 Hz, 1H), 7,44 (d, J = 9 Hz, 1H), 8,2-8,4 (m, 4H).
7) **N-[2-(2,3,4,6-tétra-O-acétyl-α-D-galactopyranosyl)-5-nitro-benzyloxycarbonyl] daunorubicine (48a).**
   - Préparation :
      à partir de 47 et de la daunorubicine (cf préparation de 6, exemple 1, Rdt : 31 %).
   - Composé 48 : C₄₉H₅₂N₂O₂₄, M = 1052. RMN ¹H (200 MHz, CDCl₃) δ ppm : 1,86 (s, 3H), 2,02 (s, 3H), 2,08 (s, 3H), 2,14 (s, 3H), 2,39 (s, 3H), 2,94 (d, J = 20 Hz, 1H), 3,23 (d, J = 20 Hz, 1H), 3,60 (s, 1H), 4,08 (s, 3H), 5,02 (d, J = 12 Hz, 1H), 5,21 (d, J = 12 Hz, 1H), 5,3-5,8 (m, H), 5,93 (d, J = 4 Hz, 1H), 7,18 (d, J = 9 Hz, 1H), 7,40 (d, J = 8 Hz, 1H), 7,80 (t, J = 8 Hz, 1H), 8,02 (d, J = 8 Hz, 1H), 8,17 (dd, J = 9 et 2 Hz, 1H), 8,26 (s large, 1H), 13,30 (s, 1H) et 13,99 (s, 1H).
8) **N-[2-(α-D-galactopyranosyl)-5-nitro-benzyloxycarbonyl] daunorubicine (48b).**
   - Préparation :
      à partir de 48a, en utilisant MeONa (cat.)-MeOH (Rdt : 83 %).
   - Composé 48b : Amorphe. C₄₁H₄₄N₂O₂₀, M = 884. F = 140°C (dec.).
9) **N-[2-(2,3,4,6-tétra-O-acétyl-α-D-galactopyranosyl)-5-nitro-benzyloxycarbonyl] doxorubicine (49a).**
   - Préparation :
      à partir de 47 et de la doxorubicine. Rdt : 13 %
   - Composé 49a : C₄₉H₅₂N₂O₂₅, M = 1068. RMN ¹H (200 MHz, CDCl₃) δ ppm : 1,86 (s, 3H), 2,01 (s, 3H), 2,09 (s, 3H), 2,13 (s, 3H), 2,99 (s, 1H), 3,03 (d, J = 20 Hz, 1H), 3,29 (d, J = 20 Hz, 1H), 4,10 (s, 3H), 4,59 (s, 1H), 4,74 (s, 1H), 5,07 (d, J = 12 Hz, 1H), 5,22 (d, J = 12 Hz, 1H), 5,3-5,8 (m, H), 5,95 (d, J = 4 Hz, 1H), 7,17 (d, J = 9 Hz, 1H), 7,40 (d, J = 8 Hz, 1H), 7,80 (t, J = 8 Hz, 1H), 8,03 (d, J = 8 Hz, 1H), 8,18 (dd, J = 9 et 2 Hz, 1H), 8,27 (s large, 1H), 13,28 (s, 1H), 14,0 (s, 1H).
10) **N-[2-(α-D-galactopyranosyl)-5-nitro**-**benzyloxycarbonyl] doxorubicine (49b).**
   Par traitement de 49a avec MeONa-MeOH (cf préparation de 7 et 14, exemples 1 et 2). Solide rouge C₄₁H₄₄N₂O₂₁, M = 900.

### EXEMPLE 9 : synthèse de la N-[2-((β-D-glucopyranosyl) acide uronique)-5-nitro-benzyloxycarbonyl] daunorubicine (54c).

Selon le schéma IX, le bromure de peracétyl-β-D-glucopyranoside uronate du méthyle est condensé avec le 2-hydroxy-5-nitrobenzaléhyde en présence d'Ag₂O à température ambiante. Le glycoside 51 (Rdt = 46 %) est converti en dérivé 52 (NaBH₄-THF, MeOH, rdt = 70 %) et le dérivé activé de 52, i.e. 53 (rdt = 73 %) réagit avec la daunorubicine pour fournir le dérivé 54a (rdt = 62 %).
1) **(2,3,4-tri-O-acétyl-β-D-glucopyranoside de 2-formyl 4-nitro phényle) uronate-de méthyle (51).**
   - Préparation :
      Une solution de bromure de 2,3,4-tri-O-acétyl-β-D-glucopyranoside uronate de méthyle (1,45 g, 3,64 mmol) et de 2-hydroxy-5-nitrobenzaldéhyde (609 mg = 3,64 mmol) dans 22 ml d'acétonitrile contenant de l'oxyde d'argent (1,3 g, 5, 6 mmol) est agitée 4 heures à température ambiante. Après filtration du milieu réactionnel et évaporation du filtrat, une chromatographie "flash" permet d'obtenir 800 mg de 51 (Rdt : 46 %).
   - Composé 51 : C₂₀H₂₁NO₁₃, M = 483. F = 173-174°C. [α]_{D}^{20°} = -52° (c 1, CHCl₃) ; I.R. (CH₂Cl₂) : 3060, 2960, 1755, 1690, 1590, 1530 cm⁻¹ ; RMN ¹H (200 MHz, CDCl₃) δ ppm : 2,09 (s, 9H), 3,73 (s, 3H), 4,40 (d, H-5, J = 8,5 Hz), 5,5 (m, 4H), 7,32 (d, 1H, J = 9,1 Hz), 8,42 (dd, 1H, J = 9,1 et 3 Hz), 8,67 (d, 1H, J = 3 Hz), 10,31 (s, 1H).
2) **(2,3,4-tri-O-acétyl-β-D-glucopyranoside de 2-hydroxy méthyl 4-nitro-phényl) uronate de méthyle (52).**
   - Préparation :
      A partir de 51, en utilisant NaBH₄-THF-MeOH (Rdt = 70 %).
   - Composé 52 : C₂₀H₂₃NO₁₃, M = 485. F = 137-145°C. [α]_{D}^{20°} = -34° (c 0,7, CHCl₃) ; I.R. (CH₂Cl₂) : 3600, 3570, 3060, 2960, 1755, 1620, 1590 1525, 1485, 1435, 1370, 1340, 1230, 1075, 1040, 900 cm⁻¹ ; RMN ¹H (200 MHz, CDCl₃) δ ppm : 2,07 (s, 3H), 2,08 (s, 3H), 2,10 (s, 3H), 2,95 (OH), 3,72 (s, 3H), 4,30 (d, H-5, J = 8,7 Hz), 4,61 et 4,72 (ABq, 2H, J = 13,8 Hz), 5,32 (m, 4H), 7,08 (d, 1H, J = 9 Hz), 8,12 (dd, 1H, J = 9 et J = 2,7 Hz), 8,27 (d, 1H, J = 2,7 Hz). SM (FAB⁺) m/z : 508 (M+Na)⁺.
3) **Carbonate de 4-nitro-phényle et de 2-((2,3,4-tri-O-acétyl-β-D-glucopyranosyl) uronate de méthyle)-5-nitro-benzyle (53).**
   - Préparation :
      A partir de 52 et de chloroformiate de paranitrophényle (Rdt : 73 %).
   - Composé 53 : C₂₇H₂₆N₂O₁₆, M = 634. F = 154-155°C. [α]_{D}^{20°} = -34,5° (c 1, CHCl₃), I.R. (CH₂Cl₂) : 2960, 2880, 1760, 1620, 1595, 1525, 1490, 1370, 1345, 1230, 1215, 1080, 1040, 860 cm⁻¹ ; RMN ¹H (200 MHz, CDCl₃) δ ppm : 2,08 et 2,09 (2s, 9H), 3,74 (s, 3H), 4,36 (d large, 1H, J = 8,7 Hz), 5,36 (m, 6H), 7,23 (d, 1H, J = 9,1 Hz), 7,43 (d, 2H, J = 8,3 Hz), 8,26 (m, 4H).
4) **N-[2-((2,3,4-tri-O-acétyl-β-D-glucopyranosyl) uronate de méthyle)-5-nitro-benzyloxycarbonyl] daunorubicine (54a).**
   - Préparation :
      A partir de 53 et de daunorubicine (Rdt : 62 %).
   - Composé 54a : C₄₈H₅₀O₂₄N₂, M = 1038. F = 142-145°C. [α]_{D}^{20°} = +115° (c 0,6, CHCl₃) ; I.R. (CH₂Cl₂) : 2950, 1755, 1720, 1710, 1620, 1580, 1525, 1345, 1230, 1210, 1110, 1080, 1035 cm⁻¹ ; RMN ¹H (200 MHz, CDCl₃) δ ppm : 1,32 (d, 3H, J = 7,3 Hz), 2,09 et 2,11 (2s, 9H), 2,43 (s, 3H), 2,93 et 3,24 (ABq, 2H, J = 19 Hz), 3,59 (s, 3H), 4,30 (m, 2H), 4,51 (s, 1H), 4,96 et 5,11 (ABq, 2H, J = 13 Hz), 5,20 et 5,60 (m, 6H), 7,14 (d, 1H, J = 8,5 Hz), 7,41 (d, 1H, J = 8 Hz), 7,79 (t, 1H, J = 8 Hz), 8,05 (d, 1H, J = 8 Hz), 8,21 (2H), 12,15 (s, 1H), 12,91 (s, 1H).
5) **N-[2-((β-D-glucopyranosyl) uronate de méthyle)-5-nitro-benzyloxycarbonyl] daunorubicine (54b).**
   A partir de 54a avec MeONa-MeOH (cf. préparation de 7 et 14, exemples 1 et 2). Solide rouge. C₄₂H₄₄N₂O₂₁, M = 912. RMN (CD₃COCD₃) : δ ppm : 2,37 (s, 3H), 3,70 (s, 3H), 4,04 (s, 3H), 6,50 (d, J = 7,5 Hz, 1H), 7,32 (d, J = 7,4 Hz, 1H), 7,60 (d, J = 7,4 Hz, 1H), 7,89 (m, 2H), 8,19 (m, 2H), 13,30 (s, 1H), 14,15 (s, 1H).
6) **N-[2-((β-D-glucopyranosyl) acide uronique)-5-nitro**-**benzyloxycarbonyl] daunorubicine (54c).**
   A partir de 54b et de BaO (cf. préparation de 22 dans l'exemple 3). Solide rouge. C₄₁H₄₂N₂O₂₁, M = 898.

### EXEMPLE 10 : Synthèse de N-[2-((β-D-glucopyranosyl) acide uronique)-5-nitro-benzyloxycarbonyl] doxorubicine (80c).

1) **Synthèse de N-[2-((2,3,4-tri-O-acétyl-β-D-glucopyranosyl) uronate de méthyle)-5-nitro-benzyloxycarbonyl] doxorubicine (83a).**
   - Préparation :
      A partir de 53 et de doxorubicine (65 %).
   - Composé 83a : C₄₈H₅₀N₂O₂₅ ; M = 1054 ; F = 162-167°C (dec) ; [α]_{D}²⁰ 122° (c 0,5, CHCl₃) ; IR (CH₂Cl₂) : 1757, 1720, 1618, 1580, 1525, 1410, 1370, 1344, 1230 cm⁻¹ ; RMN (200 MHz, CDCl₃) : δ ppm : 1,30 (d, 3H, J = 7,3 Hz), 2,06 et 2,08 (2s, 9H), 2,94 et 3,23 (ABq, 2H, J = 19 Hz), 3,60 (s, 3H), 4,06 (s, 3H), 4,28 (d, 1H, J = 9 Hz), 5,03 (ABq, 2H, J = 13 Hz), 5,27-5,51 (m, 6H), 7,11 (d, 1H, J = 8 Hz), 7,99 (d, 1H, J = 8 Hz), 8,15 (m, 2H), 12,62 (s, 1H), 13,15 (s, 1H).
2) **Synthèse de N-[2-((β-D-glucopyranosyl) uronate de méthyle)-5-nitro-benzyloxycarbonyl] doxorubicine (83b).**
   - Préparation :
      A partir de 83a avec MeONa-MeOH (cf. préparation de 7 et 14, exemples 1 et 2).
   - Composé 83b : C₄₂H₄₄N₂O₂₂ ; M = 928 ; F = 180°C (dec.) ; [α]_{D}²⁰ 0° (c 0,5, CHCl₃) ; RMN (200 MHz, CD₃COCD₃) : δ ppm : 1,29 (d, 3H, J = 7,3 Hz), 3,79 (s, 3H), 4,07 (s, 3H), 6,09 (d, NH), 7,12 (d, 1H, J = 8,6 Hz), 7,41 (d, 1H, J = 8 Hz), 7,79 (t, 1H, J = 8 Hz), 8,00 (d, 1H, J = 8 Hz), 8,16 (m, 2H), 12,75 (s, 1H), 13,25 (s, 1H).
3) **Synthèse de N-[2-((β-D-glucopyranosyl) acide uronique)-5-nitro-benzyloxycarbonyl] doxorubicine (83c).**
   - Préparation :
      A partir de 83b avec NaOH ou l'estérase de foie de porc (EC 3.1.1.1.).
   - Composé 83c : solide rouge ; C₄₁H₄₂N₂O₂₂ ; M = 914.

### EXEMPLE 11

1) **2,3,4,6-tétra-o-acétyl-α-D-galactopyranoside de 2-chloro-4-méthyl phényle (55).**
   - Préparation :
      A partir du 2,3,4,6-tétra-O-acétyl-α-D-galactopyranose et du 2-chloro-4-méthylphénol (cf. préparation de 1, Rdt = 40 %).
   - Composé 55 : C₂₁H₂₅O₁₀Cl. M = 472,5. RMN ¹H (270 MHz, CDCl₃) : δ ppm : 2,02 (s, 3H), 2,06 (s, 3H), 2,14 (s, 3H), 2,20 (s, 3H), 2,32 (s, 3H), 4,12 (dd, 1H, J = 12, J' = 7 Hz), 4,17 (dd, 1H, J = 12, J' = 6 Hz), 4,54 (ddd, 1H, J = 7, J' = 6, J" = 1 Hz), 5,29 (dd, 1H, J = 10, J' = 4 Hz), 5,61 (m, 1H), 5,63 (dd, 1H, J = 10, J' = 4 Hz), 5,76 (d, 1H, J = 4 Hz), 7,02 (dd, 1H, J = 8, J' = 2 Hz), 7,07 (d, 1H, J = 8 Hz), 7,23 (d, 1H, J = 2 Hz). SM (DIC/NH₃): m/z 490 (M+NH₄)⁺, 456, 331.
2) **2,3,4,6-tétra-**O**-acétyl-α-D-galactopyranoside de 2-chloro-4-bromométhyl phényle (56).**
   - Préparation :
      Monobromation à partir de 55 avec activation photochimique (cf. préparation de 2, Rdt = 80 %).
   - Composé 56 : C₂₁H₂₄O₁₀ClBr. M = 551,5. RMN ¹H (270 MHz, CDCl₃) : δ ppm : 2,00 (s, 3H), 2,03 (s, 3H), 2,12 (s, 3H), 2,18 (s, 3H), 4,09 (dd, 1H, J = 12, J' = 7 Hz), 4,14 (dd, 1H, J = 12, J' = 6 Hz), 4,42 (s, 2H), 4,50 (ddd, 1H, J = 7, J' = 6, J" = 1 Hz), 5,27 (dd, 1H, J = 10, J' = 4 Hz), 5,58 (m, 1H), 5,60 (dd, 1H, J = 10, J' = 4 Hz), 5,80 (d, 1H, J = 4 Hz), 7,13 (d, 1H, J = 8 Hz), 7,22 (dd, 1H, J = 8, J' = 2 Hz), 7,42 (d, 1H, J = 2 Hz). SM (DIC/NH₃): m/z 571 (M+NH₄)⁺, 569 (M+NH₄)⁺, 490, 331.
3) **2,3,4,6-tétra-O-acétyl-α-D-galactopyranoside de 2-chloro-4-hydroxyméthyl phényle (57).**
   - Préparation :
      A partir de 56 (cf. préparation de 4, Rdt = 34 %).
   - Composé 57 : C₂₁H₂₅O₁₁Cl. M = 488,5. RMN ¹H (270 MHz, CDCl₃) : δ ppm : 1,98 (s, 3H), 2,04 (s, 3H), 2,10 (s, 3H), 2,18 (s, 3H), 4,09 (dd, 1H, J = 12, J' = 7 Hz), 4,14 (dd, 1H, J = 12, J' = 6 Hz), 4,49 (ddd, 1H, J = 7, J' = 6, J" = 1 Hz), 4,64 (s, 2H), 5,26 (dd, 1H, J = 10, J' = 4 Hz), 5,58 (m, 1H), 5,60 (dd, 1H, J = 10, J' = 4 Hz), 5,77 (d, 1H, J = 4 Hz), 7,15 (d, 1H, J = 8 Hz), 7,20 (dd, 1H, J = 8, J' = 2 Hz), 7,42 (d, 1H, J = 2 Hz). SM (DIC/NH₃): m/z 506 (M+NH₄)⁺, 472, 331.
4) **Carbonate de 2,5-dioxopyrrolidin-1-yle et de 3-chloro-4-(2,3,4,6-tétra-O-acétyl-α-D-galactopyranosyl)benzyle (58) .**
   - Préparation :
      A partir de 57 et de disuccinimidocarbonate (DSC) (cf. préparation de 19, exemple 3).
      Remarquons que du fait de son instabilité, le composé 58 n'a pu être purifié et isolé sur colonne de silice.
   - Composé 58 : C₂₆H₂₈ClNO₁₅. M = 629,5.
5) **N-[3-chloro-4-(2,3,4,6-tétra-O-acétyl-α-D-galactopyranosyl)-benzyloxycarbonyl] daunorubicine (59).**
   - Préparation :
      Couplage de 58 avec la daunorubicine (cf. préparation de 6, Rdt = 20 %).
   - Composé 59 : C₄₉H₅₂ClNO₂₂. M = 1 041,5. RMN ¹H (270 MHz, DMSO) : δ ppm : 1,12 (d, 3H, J = 7 Hz), 1,48 (m, 1H), 1,88 (m, 2H), 1,98 (s, 3H), 2,02 (s, 3H), 2,04 (s, 3H), 2,15 (s, 3H), 2,23 (m, 1H), 2,25 (s, 3H), 2,76 (m, 1H), 2,96 (m, 1H), 3,50-4,20 (m, 5H), 4,00 (s, 3H), 4,44 (m, 1H), 4,72 (m, 1H), 4,90 (m, 3H), 5,20 (m, 1H), 5,32 (m, 1H), 5,43 (m, 2H), 5,53 (s, 1H), 5,85 (d, 1H, J = 4 Hz), 6,94 (m, 1H), 7,23 (m, 2H), 7,47 (d, 1H, J = 2 Hz), 7,66 (m, 1H), 7,92 (m, 2H). SM (DIC/NH₃) : m/z 1060 (M+NH₄)⁺, 506, 331.
6) **N-[3-chloro-4-(α-D-galactopyranosyl)-benzyloxycarbonyl] daunorubicine (60).**
   - Préparation :
      A partir de 59 (cf. préparation de 7, Rdt = 91 %).

### EXEMPLE 12 : Synthèse de N-[3-nitro-4-((β-D-glucopyranosyl) acide uronique)-benzyloxycarbonyl] daunorubicine (64c).

1) **(2,3,4-tri-O-acétyl-β-D-glucopyranoside de 4-formyl 2-nitro-phényle) uronate de méthyle (61).**
   - Préparation :
      A partir d'une solution de bromure de (2,3,4-tri-O-acétyl-D-glucopyranoside) uronate de méthyle (5,3 g) et de 3-nitro-p-hydroxybenzaldéhyde (3,55 g) et d'oxyde d'argent (15,45 g) dans les conditions indiquées pour préparer 51. Après chromatographie flash et cristallisation on obtient 5,35 g (80 %) de 61 pur.
   - Composé 61 : C₂₀H₂₁NO₁₃, M = 483. F = 172-173°C ; [α]_{D}²⁰ +10° (c 1, CHCl₃) ; IR (laque) : 1760, 1230 cm⁻¹ ; RMN ¹H (250 MHz, CDCl₃) δ ppm : 2,16 (s, 3H), 2,12 (s, 3H), 2,07 (s, 3H), 3,71 (s, 3H, OMe), 4,33 (d, 1H, J = 8,2 Hz), 5,45-5,25 (m, 4H), 7,49 (d, 1H, J = 7,5 Hz), 8,08 (dd, 1H, J = 7,5, J′= 1,8 Hz), 8,31 (dd, 1H, J = 1,8 Hz), 9,97 (s, 1H) ; SM (DIC/NH₃) ; m/z 501 (M+NH₄) ⁺.
2) **(2,3,4-tri-O-acétyl-β-D-glucopyranoside de 4-hydroxyméthyl-2-nitro phényle) uronate de méthyle (62).**
   - Préparation :
      A partir de 61 en utilisant NaBH₄-THF-MeOH (Rdt 72 %).
   - Composé 62 : C₂₀H₂₃NO₁₃. M = 485. F = 173-174°C ; [α]_{D}²⁰ : +10° (c 1, CHCl₃) ; RMN ¹H : δ ppm 2,08 (s, 6H), 2,01 (s, 3H), 3,63 (s, 3H) 4,12 (d, 1H, J = 8 Hz), 4,62 (s, 2H), 5,35-5,02 (m, 4H), 7,21 (d, 1H, J = 7 Hz), 7,39 (dd, 1H, J = 7 Hz, J′ = 1,8 Hz), 7,65 (d, 1H, J = 1,8 Hz). SM (DIC/NH₃) : m/z 503 (M+NH₄)⁺.
3) **N-[3-nitro-4-((2,3,4-tri-O-acétyl-β-D-glucopyranosyl) uronate de méthyle)-benzyloxycarbonyl] daunorubicine (64a).**
   - Préparation :
      A partir de 62 et de DSC on obtient intermédiairement le carbonate 63 qui n'est pas purifié mais immédiatement remis en réaction avec la daunorubicine selon les conditions décrites pour préparer 54.
      On isole 64a avec 50 % de rendement.
   - Composé 64a : C₄₈H₅₀N₂O₂₄. M = 1038. F = 137-138°C ; [α]_{D}²⁰ +88° (c 0,5, chloroforme) ; IR (CDCl₃) : 1760, 1720, 1220 cm⁻¹ ; RMN ¹H δ ppm 1,30 (d, 3H, J = 6Hz), 1,75 (m, 2H), 2,12 (s, 3H), 2,10 (s, 3H), 2,13 (s, 3H), 2,45 (s, 3H), 3,00 (d, 1H, J = 20 Hz), 3,30 (d, 1H, J = 20 Hz), 3,75 (s, 3H), 4,14 (s, 3H), 4,26 (m, 2H), 4,47 (s, 1H), 5,08 (s, 2H), 5,10-5,47 (m, 4H), 5,58 (d, 1H), 7,50 (1H), 7,89 (1H), 8,05 (1H), 14,00 (s, 1H), 13,30 (s, 1H).
4) **β-D-glucopyranoside de N-(4-hydroxy-3-nitro-benzyloxycarbonyl) daunorubicine (64b).**
   A partir de 64a avec MeOH-MeONa (cf. préparation de 7 et 14, exemples 1 et 2). Solide rouge. C₄₂H₄₄N₂O₂₁. M = 912.
5) **β-D-glucuronide de N-(4-hydroxy-3-nitro-benzyloxycarbonyl) daunorubicine (64c).**
   A partir de 64b et de BaO ou K₂CO₃ (cf. préparation de 22 dans l'exemple 3). Solide rouge. C₄₁H₄₂N₂O₂₁. M = 898.

### EXEMPLE 13 : Synthèse du N-[4-méthoxy-5-nitro-2((β-D-glucopyranosyl) acide uronique)-benzyloxycarbonyl] daunorubicine (68c).

Conformément au schéma XII, la glycosidation catalysée par Ag₂O du 2-hydroxy-4-méthoxy-5-nitrobenzaldéhyde avec le bromure de peracétyl-β-D-glucopyranoside uronate de méthyle permet d'obtenir le composé 65 qui est ensuite converti, selon le procédé décrit dans l'exemple 9, en dérivé 68c.
1) **(2,3,4-tri-O-acétyl-β-D-glucopyranoside de 2-formyl-5-méthoxy-4-nitro-phényl) uronate de méthyle (65).**
   Il est obtenu avec un rendement de 53 % selon le procédé utilisé pour 51 (voir exemple 9).
   - Composé 65 : C₂₁H₂₃NO₁₄. M = 513. F : 159°C. [α]_{D} -83° (c 0,95, CHCl₃). IR (CHCl₃) : 3080, 3010, 1765, 1695, 1620, 1580, 1540, 1450, 1375, 1300, 1220, 1080, 1045 cm⁻¹. RMN ¹H (200 MHz, CDCl₃) : δ ppm : 2,08 (s, 6H), 2,10 (s, 3H), 3,74 (s, 3H), 4,04 (s, 3H), 4,39 (d, 1H, J = 9 Hz), 5,39 (m, 4H), 6,92 (s, 1H), 8,43 (s, 1H), 10,16 (s, 1H).
2) **(2,3,4-tri-O-acétyl-β-D-glucopyranoside de 2-hydroxyméthyl-5-méthoxy-4-nitro-phényl) uronate de méthyle (66).**
   Préparation à partir de 65 en utilisant NaBH₄-THF-MeOH (Rdt. 30 %).
   - Composé 66 : C₂₁H₂₅NO₁₄. M = 514. F : 176°C. [α]_{D} -61° (c 1,05, CHCl₃). IR (CHCl₃) : 3560, 3040, 2960, 1760, 1625, 1590, 1530, 1445, 1375, 1350, 1290, 1220, 1075, 1040 cm⁻¹. RMN ¹H (200 MHz, CDCl₃) : δ ppm : 2,07 (s, 3H), 2,09 (s, 3H), 2,13 (s, 3H), 3,72 (s, 3H), 3,96 (s, 3H), 4,27 (1H), 4,50 et 4,63 (qAB, 2H, J = 12,9 Hz), 5,23 (m, 4H), 6,99 (s, 1H), 7,99 (s, 1H).
3) **Carbonate de 4-nitro-phényle et de 4-méthoxy-5-nitro-2-((2,3,4-tri-O-acétyl-β-D-glucopyranosyl) uronate de méthyle)-benzyle (67).**
   Préparation à partir de 66 et de chloroformiate de 4-nitro-phényle (Rdt. 74 %).
   - Composé 67 : C₂₈H₂₈N₂O₁₈. M = 680. F : 90°C. [α]_{D} -47° (c 1,05, CHCl₃). IR (CHCl₃) : 3020, 2960, 1745, 1615, 1575, 1435, 1340, 1280, 1205, 1068, 1030, 895, 852 cm⁻¹. RMN ¹H (200 MHz, CDCl₃) : δ ppm : 2,06 (s, 3H), 2,07 (s, 3H), 2,09 (s, 3H), 3,75 (s, 3H), 3,98 (s, 3H), 4,35 (1H), 5,14 et 5,24 (qAB, 2H, J = 12 Hz), 5,39 (m, 4H), 6,92 (s, 1H), 7,43 (d, 2H, J = 9,1 Hz), 8,09 (s, 1H), 8,30 (d, 2H, J = 9,1 Hz).
4) **N-[4-méthoxy-5-nitro-2-((2,3,4-tri-O-acétyl-β-D-glucopyranosyl) uronate de méthyle)-benzyloxycarbonyl] daunorubicine (68a).**
   Préparation à partir de 67 et de daunorubicine (Rdt. 83 %).
   - Composé 68a : C₄₉H₅₂N₂O₂₅. M = 1068. F : 153°C (dec.). [α]_{D} +105° (c 0,24, CHCl₃). RMN ¹H (200 MHz, CDCl₃) : δ ppm : 1,31 (d, 3H, J = 7 Hz), 2,07 et 2,12 (2s, 9H), 2,42 (s, 3H), 2,91 et 3,24 (qAB, 2H, J = 18,7 Hz), 3,62 (s, 3H), 3,94 (s, 3H), 4,08 (s, 3H), 4,93 (qAB, 2H), 6,88 (s, 1H), 7,39 (d, 1H, J = 8 Hz), 7,77 (t, 1H, J = 8 Hz), 7,94 (s, 1H), 8,03 (d, 1H, J = 8 Hz), 9,59 (s, 1H), 10, 29 (s, 1H).
5) **N-[4-méthoxy-5-nitro-2-((β-D-glucopyranosyl) uronate de méthyle)-benzyloxycarbonyl] daunorubicine (68b).**
   Préparation à partir de 68a (Rdt. 91 %) en utilisant MeONa-MeOH (-20°C, 12 heures).
   - Composé 68b : C₄₃H₄₆N₂O₂₂. M = 942. F : 176-177°C. [α]_{D} +1° (c, 0,19, CHCl₃). IR (CHCl₃) : 3000, 1750, 1720, 1710, 1620, 1580, 1520, 1440, 1410, 1365, 1345, 1280. RMN ¹H (200 MHz, CD₃OD) : δ ppm : 2,43 (s, 3H), 3,10 (qAB, 2H), 3,79 (s, 3H), 3,95 (s, 3H), 4,08 (s, 3H), 6,28 (d, 1H), 6,87 (s, 1H), 7,46 (d, 1H, J = 8 Hz), 7,83 (d, 1H, J = 8 Hz), 8,02 (m, 2H). SM (FAB⁺) m/z : 1092.
6) **N-[4-méthoxy-5-nitro-2-((β-D-glucopyranosyl) acide uronique)-benzyloxycarbonyl] daunorubicine (68c).**
   Préparation à partir de 68b en utilisant Na₂CO₃-MeOH-H₂O.
   - Composé 68c : solide rouge. C₄₂H₄₄N₂O₂₂. M = 928.

### EXEMPLE 14 : Synthèse du N-[2-((β-D-glucopyranosyl) acide uronique)-5-chloro-benzyloxycarbonyl] daunorubicine (82c).

1) **(2,3,4-tri-O-acétyl-β-D-glucopyranoside de 2-formyl-4-chlorophényle) uronate de méthyle (79).**
   - Ce composé est obtenu avec un rendement de 41 % selon le procédé utilisé pour 51 (voir exemple 9).
   - Composé 79 : C₂₀H₂₁ClO₁₁ ; M = 472,5 ; F = 156-157°C ; [α]_{D}²⁰ -39° (c 1, CHCl₃) ; IR (CH₂Cl₂ : 2940, 2870, 1755, 1680, 1585, 1465 cm⁻¹ ; RMN (200 MHz, CDCl₃) : δ 2,09 (s, 9H), 3,74 (s, 3H), 4,25 (d, 1H, J = 8,5 Hz), 5,30 (m, 4H), 7,10 (d, 1H, J = 8,5 Hz), 7,53 (dd, 1H, J = 8,5 et 2,5 Hz), 7,80 (d, 1H, J = 2,5 Hz), 10,27 (s, 1H) ppm.
2) **(2,3,4-tri-O-acétyl-β-D-glucopyranoside de 2-hydroxyméthyl-4-chlorophényle) uronate de méthyle (80).**
   - Préparation à partir de 79, en utilisant NaBH₄-THF-MeOH (70 %).
   - Composé 80 : C₂₀H₂₃ClO₁₁ ; M = 474,5 ; F = 120°C ; [α]_{D}²⁰ -20° (c 0,9, CHCl₃) ; IR (CH₂Cl₂) : 3050, 2980, 1750, 1470, 1415, 1250, 1220 cm⁻¹ ; RMN (200 MHz, CDCl₃) : δ 2,06 (s, 3H), 2,07 (s, 3H), 2,11 (s, 3H), 3,71 (s, 3H), 4,14 (d, 1H, J = 9 Hz), 4,3 et 4,72 (ABq, 2H, J = 13 Hz), 5,11 (d, 1H, J = 6,5 Hz), 5,34 (m, 3H), 6,94 (d, 1H, J = 8,7 Hz), 7,23 (dd, 1H, J = 8,7 et 2,5 Hz), 7,36 (d, 1H, J = 2,5 Hz) ppm.
3) **Carbonate de 4-chlorophényle et de 2-((2,3,4-tri-O-acétyl-β-D-glucopyranosyl) uronate de méthyle)-5-nitrobenzyle (81).**
   - Préparation à partir de 80 et de chloroformiate de 4-nitrophényle (50 %).
   - Composé 81 : C₂₇H₂₆ClNO₁₄ ; M = 623,5 ; F = 136°C ; RMN (200 MHz, CDCl₃) : δ 2,08 (s, 9H), 3,74 (s, 3H), 4,22 (d large, 1H, J = 8,7 Hz), 5,30 (m, 6H), 7,03 (d, 1H, J = 8,2 Hz), 7,30 (m, 4H), 8,30 (d, 2H, J = 8,3 Hz) ppm.
4) **N-[2-((2,3,4-tri-O-acétyl-β-D-glucopyranosyl) uronate de méthyle)-5-chloro-benzyloxycarbonyl] daunorubicine (82a).**
   - Préparation à partir de 81 et de daunorubicine (73 %).
   - Composé 82a : C₄₈H₅₀ClNO₂₂ ; M = 1027,5 ; F = 155°C (dec.) ; [α]_{D}²⁰ 125° (c 0,24, CHCl₃) ; IR (CH₂Cl₂) : 1755, 1715, 1610, 1575 cm⁻¹ ; RMN (200 MHz, CDCl₃) : δ 1,31 (d, 3H, J = 7 Hz), 2,05 (s, 6H), 2,08 (s, 3H), 2,42 (s, 3H), 2,94 et 3,25 (ABq, 2H, J = 19 Hz), 3,62 (s, 3H), 4,06 (s, 3H), 4,20 (m, 2H), 4,53 (s large, 1H), 4,96 (s large, 2H), 5,15-5,52 (m, 6H), 7,00 (d, 1H, J = 8 Hz), 7,20 (m, 2H), 7,40 (d, 1H, J = 8 Hz), 7,79 (t, 1H, J = 8 Hz), 8,05 (d, 1H, J = 8 Hz) ppm.
5) **N-[2-((β-D-glucopyranosyl) uronate de méthyle)-5-chloro-benzyloxycarbonyl] daunorubicine (82b).**
   - Préparation à partir de 82a avec MeONa-MeOH (voir préparation de 7 et 14, exemples 1 et 2), avec un rendement de 47 %.
   - Composé 82b : solide rouge ; C₄₂H₄₄ClNO₁₉ ; M = 901,5 ; F = 167-170°C ; [α]_{D}²⁰ 0° (c 0,03, MeOH) ; RMN (200 MHz, CDCl₃) δ 2,42 (s, 3H), 2,96 et 3,23 (ABq, 2H, J = 18 Hz), 3,79 (s, 3H), 4,07 (s, 3H), 5,89 (d, NH), 6,99 (d large, 1H, J = 8 Hz), 7,20 (d large, 1H, J = 8 Hz), 7,33 (s, 1H), 7,41 (d, 1H, J = 8 Hz), 7,80 (t, 1H, J = 8 Hz), 8,03 (d, 1H, J = 8 Hz) ppm.
6) **N-[2-((β-D-glucopyranosyl) acide uronique)-5-chloro-benzyloxycarbonyl] daunorubicine (82c).**
   - Préparation à partir de 82b et de Na₂CO₃ (voir préparation de 22, exemple 3).
   - Composé 82c : solide rouge ; C₄₁H₄₂ClNO₁₉ ; M = 887,5.

### EXEMPLE 15 : Synthèse du β-D-glucuronide de N-[4-hydroxy-3-nitro-benzyloxycarbonyl) doxorubicine (70c).

1) **Carbonate de 4-nitro-phényle et de 4-(2,3,4-tri-O-acétyl-β-glucopyranosyl) uronate de méthyle-5-nitrobenzyle (69).**
   - Préparation à partir de 62 et de chloroformiate de paranitrophényle (Rdt : 47 %).
   - Composé 69 : C₂₇H₂₆N₂O₁₇ ; M = 650 ; F = 126°C ; [α]_{D}²⁰ = +12° (c 0,1, CHCl₃), IR (KBr)_{νmax} 1730 (CO, ester), 1210 ; RMN ¹H (90 MHz, CDCl₃) : δ ppm 7,81 (d, 1H, J = 5 Hz), 7,54 (dd, 1H, J = 8,4 Hz), 7,30 (d, 1H, J = 8,4 Hz), 5,36-5,29 (m, 3H), 5,18 (d, 1H, J = 6,7 Hz), 4,72 (s, 2H), 4,20 (d, 1H, J = 8,4 Hz), 3,74 (s, 3H), 2,12 (s, 3H), 2,06 (s, 3H), 2,05 (s, 3H). SM (DCl/NH₃) m/z : 668 (M+18)⁺, 608, 503, 443.
2) **N-[3-nitro-4-(2,3,4-tri-O-acétyl-β-D-glucopyranosyl) uronate de méthyle)-benzyloxycarbonyl] doxorubicine (70a).**
   - Préparation à partir de 69 et de la doxorubicine (Rdt :86 %).
   - Composé 70a : C₄₈H₅₀O₂₅N₂, M = 1054 ; F = 114°C ; [α]_{D}²⁰ = +121° (c 0,05, CHCl₃). IR (KBr)_{νmax} 1760, 1730, 1220. RMN ¹H (250 MHz, CDCl₃) : δ ppm 13,90 (s, 1H, OH phénol), 13,15 (s, 1H, OH phénol), 8,00-7,20 (m, 6H arom.), 5,47 (s, 1H), 5,29-5,16 (m, 5H), 4,97 (s, 2H), 4,73 (s, 2H), 4,20-4,15 (m, 2H), 4,21 (s, 3H), 3,80 (l s, 1H), 3,72 (s, 3H), 3,68 (s, 1H), 3,19 (d, 1H, J = 19 Hz) et 2,91 (d, 1H, J = 19 Hz), 2,30 (d, 1H, J = 13,6 Hz), 2,15 (s, 3H), 2,10 (d, 1H, J = 13,6 Hz), 2,07 (s, 6H), 1,83 (br s, 2H), 1,27 (d, 1H, J = 5,4 Hz).
3) **N-[3-nitro-4-(β-D-glucopyranosyl) uronate de méthyle)-benzyloxycarbonyl] doxorubicine (70b).**
   - Préparation à partir de 70a (1,48 g) dissous dans du DMF anhydre (20 mL) auquel on ajoute 200 mL de MeOH anhydre puis, après refroidissement à 0°C, 18 mL de MeONa 1M. On laisse agiter durant 1 h à 0°C et neutralise par une solution méthanolique d'AcOH (10 %) en laissant à 0°C. Après évaporation à sec et chromatographie flash (solvant CH₂Cl₂/MeOH 90:10), on isole 500 mg de 70b pur.
   - Composé 70b : C₄₂H₄₄O₂₂N₂ ; M = 928 ; F = 150°C ; [α]_{D}²⁰ = +85° (c 0,05, THF) ; RMN ¹H (250 MHz, CDCl₃) : δ ppm 13, 92 (s, 1H), 13, 22 (s, 1H), 7,92-7,18 (m, 6H), 5,23 (s, 1H), 5,07 (m, 2H), 4,95 (s, 2H), 3,88 (s, 1H), 4,57 (s, 2H), 4,07 (m, 2H), 3,96 (s, 3H), 3,64 (s, 3H), 3,01 (d, 1H) et 2,85 (d, 1H, J = 18 Hz) (AB syst), 2,22 (d, 1H, J = 13,6 Hz), 2,03 (dd, 1H, J = 13,6, J′ = 5 Hz), 1,92-1,37 (m, 4H), 1,15 (d, 1H, J = 6,8 Hz). SM (FAB) m/e : 951 (M+ 23).
4) **β-D-glucuronide de N-[4-hydroxy-3-nitro-benzyloxycarbonyl) doxorubicine (70c).**
   - Préparation : le composé 70b (780 mg) est dissous dans le THF (75 mL), puis l'on ajoute de l'eau (**≈** 30 mL) et de la soude 2N (goutte à goutte) (750 µL), après refroidissement à 0°C. Après agitation à 0°C durant 1 h 30, on neutralise par addition de résine IR 50H⁺. Le filtrat est alors évaporé jusqu'à un volume d'environ 35 mL, puis lyophilisé. On obtient 750 mg de 70c pur.
   - Composé 70c : C₄₁H₄₁O₂₂N₂ ; M = 914 ; F = 210°C ; [α]_{D}²⁰ = -78° (c 0,05, H₂O). RMN ¹H (250 MHz, DMSO) : δ ppm 14, 00 (l s, 2H), 8, 00-6, 90 (m, 6H), 5, 48 (s, 1H), 5,23 (s, 1H), 5,07 (d, J = 6,4 Hz), 4,98 (m, 2H), 4,59 (s, 2H), 3,99 (s, 3H), 3,30-3,10 (m, 2H), 2,96 (d, 1H, J = 14 Hz), 2,91 (d, 1H, J = 14 Hz), 2,20 (d, 1H, J = 11,5 Hz), 2,13 (d, 1H, J = 11,5 Hz), 1,87 (d, 1H, J = 13 Hz,), 1,48 (d, 1H, J = 13 Hz), 1,13 (d, 3H, J = 6,4 Hz).

### EXEMPLE 16 : Synthèse de α-D-galactopyranoside de N-4-hydroxy-3-nitro-(benzyloxycarbonyl) daunorubicine (75b).

1) **2,3,4,6-tétra-O-acétyl-α-D-galactopyranoside de 4-méthylphényle (71).**
   Un mélange de penta-O-acétyl-D-galactopyranose (36 g, 92 mmoles), de p-crésol (30 g, 280 mmoles) et de chlorure de zinc anhydre (1,8 g) est porté à 160°C pendant 30 minutes, selon la technique d'Helferich. Après refroidissement, le mélange, directement chromatographié sur gel de silice 60H (hexane-acétate d'éthyle : 90/10-v/v) fournit le 2,3,4,6-tétra-O-acétyl-α-D-galactopyranoside de 4-méthylphényle (15 g, 40 %), qui cristallise dans l'éthanol. Ses caractéristiques analytiques sont identiques à celles décrites précédemment.
2) **2,3,4,6-tétra-O-acétyl-α-D-galactopyranoside de 4-bromométhylphényle (72).**
   - Préparation : une solution de 2,3,4,6-tétra-O-acétyl-α-D-galactopyranoside de 4-méthylphényle (71) (1,4 g, 3,2 mmoles) et de 1,3-dibromo 5,5-diméthylhydantoïne (0,46 g, 1,6 mmole) dans 100 ml de tétrachlorure de carbone, est portée à reflux et irradiée (1 000 W) pendant 15 minutes. Après refroidissement, filtration et évaporation à sec, le 2,3,4,6-tétra-O-acétyl-α-D-galactopyranoside de 4-bromométhylphényle (72) est isolé par cristallisation dans le méthanol (3,5 g, 76 %).
   - Composé 72 : C₂₁H₂₅O₁₀Br, M = 517 ; F = 105°C (MeOH) ; [α]_{D}²⁰ = +168° (c 1, CHCl₃) ; IR (KBr) cm⁻¹ : 1747 (νC = O ester), 1222 (νCH₂Br) ; RMN ¹H (270 MHz, CDCl₃) δ ppm : 1,97 (s, 3H), 2,08 (s, 3H), 2,10 (s, 3H), 2,21 (s, 3H), 4,05 (dd, J = 11 et 7 Hz, 1H), 4,13 (dd, J = 11 et 6 Hz, 1H), 4,18 (t, J = 7 Hz, 1H), 4,52 (s, 2H), 5,28 (dd, J = 11 et 4 Hz, 1H), 5,52 (d, J = 3 Hz, 1H), 5,58 (dd, J = 11 et 3 Hz, 1H), 5,79 (d, J = 4 Hz, 1H), 7,04 (d, J = 9 Hz, 2H), 7,35 (d, J = 9 Hz, 2H). SM (DIC/NH₃) m/z : 534/536 (M+NH₄)⁺.
3) **2,3,4,6-tétra-O-acétyl-α-D-galactopyranoside de (4-bromométhyl 2-nitro)-phényle (73).**
   - Préparation : à une solution de 2,3,4,6-tétra-O-acétyl-α-D-galactopyranoside de 4-bromométhylphényle (72) (1,5 g, 2,9 mmoles) dans 10 ml d'anhydride acétique, sont ajoutés goutte à goutte, en 2 heures, à 20°C, 5 ml d'acide nitrique fumant. Après une heure d'agitation, le milieu est neutralisé par une solution d'hydrogénocarbonate de sodium. Après extraction usuelle, une chromatographie sur gel de silice 60H permet d'isoler le 2,3,4,6-tétra-O-acétyl-α-D-galactopyranoside de (4-bromométhyl-2-nitro)-phényle (73) (1,12 g, 69 %).
   - Composé 73 : C₂₁H₂₄NO₁₂Br ; M = 562 ; F = 168°C (MeOH) ; [α]_{D}²⁰ = +165° (c 1,01, CHCl₃), IR (KBr) cm⁻¹ : 1747 (νCO ester), 1534 (νasNO), 1222 (ν CH₂Br) ; RMN ¹H (270 MHz, CDCl₃) δ ppm : 1,84 (s, 3H), 1,88 (s, 3H), 2,08 (s, 3H), 2,15 (s, 3H), 4,11 (d, J = 7 Hz, 2H), 4,39 (t, J = 7 Hz, 1H), 4,47 (s, 2H), 5,24 (dd, J = 11 et 4 Hz, 1H), 5,48 (dd, J = 11 et 3 Hz, 1H), 5,57 (d, J = 3 Hz, 1H), 5,89 (d, J = 4 Hz, 1H), 7,32 (d, J = 9 Hz, 1H), 7,56 (dd, J = 9 et 2 Hz, 1H), 7,84 (d, J = 2 Hz, 1H). SM (DIC/NH₃) m/z : 579/581 (M+NH₄)⁺.
4) **2,3,4,6-tétra-O-acétyl-α-D-galactopyranoside de (4-hydroxyméthyl 2-nitro)-phényle (74).**
   - Préparation : une solution de 2,3,4,6-tétra-O-acétyl-α-D-galactopyranoside de (4-bromométhyl 2-nitro)-phényle (73) (0,3 g, 0,5 mmole) dans 15 ml d'acétone, additionnée de 15 ml d'une solution aqueuse de nitrate d'argent 0,1 N est agitée à 20°C, durant une nuit. Après filtration puis évaporation de l'acétone, le 2,3,4,6-tétra-O-acétyl-α-D-galactopyranoside de (4-hydroxyméthyl 2-nitro)-phényle (74) précipite dans l'eau. Ce dernier est recueilli puis séché (0,25 g, 93 %).
   - Composé 74 : C₂₁H₂₅NO₁₃ ; M = 499 ; F = 170°C (MeOH) ; [α]_{D}²⁰ = +174° (c 1,02, CHCl₃) ; IR (KBr) cm⁻¹ : 3500 (νOH), 1746 (νC = O ester), 1234 (νC-OH) ; RMN ¹H (270 MHz, CDCl₃) δ ppm : 2,00 (s, 3H), 2,02 (s, 3H), 2,11 (s, 3H), 2,18 (s, 3H), 4,13 (d, J = 6 Hz, 2H), 4,43 (t, J = 6 Hz, 1H), 4,73 (d, J = 5 Hz, 2H), 5,27 (dd, J = 11 et 4 Hz, 1H), 5,50 (dd, J = 11 et 3 Hz, 1H), 5,59 (d, J = 3 Hz, 1H), 5,88 (d, J = 4 Hz, 1H), 7,32 (d, J = 9 Hz, 1H), 7,53 (dd, J = 9 et 2 Hz, 1H), 7,84 (d, J = 2 Hz, 1H). SM (DIC/NH₃) m/z : 517 (M+NH₄)⁺.
5) **2,3,4,6-tétra-O-acétyl-α-D-galactopyranoside de N-4-hydroxy-3-nitro-(benzyloxycarbonyl)-daunorubicine (75a).**
   - Préparation : une solution de 74 (0,05 g-0,1 mmole) et de triéthylamine (28 µl, 0,2 mmole) dans 5 ml de dichlorométhane anhydre est ajoutée, à 20°C, sous argon, goutte à goutte, à une solution de disuccinimidocarbonate (0,05 g-0,2 mmole) dans 2 ml d'acétonitrile. Le mélange est agité durant 90 minutes. Après filtration et évaporation à sec, le succinimidocarbonate est obtenu quantitativement sous forme d'un précipité.
      Au succinimidocarbonate brut précédemment préparé, est ajoutée à 20°C, sous argon, une solution de daunorubicine (0,03 g-0,06 mmole) et de triéthylamine (50 µl, 0,35 mmole) dans 3 ml de diméthylformamide. Après 15 minutes de réaction et évaporation du solvant, le 2,3,4,6-tétra-O-acétyl-α-D-galactopyranoside de N-4-hydroxy-3-nitro-(benzyloxycarbonyl)-daunorubicine est purifié par chromatographie sur gel de silice 60H (cyclohexane-acétone : 50/50-v/v) puis lavage par de l'eau (45,2 mg, 43 % global).
   - Composé 75a : C₄₉H₅₂N₂O₂₄ ; M = 1052 ; amorphe ; [α]_{D}²⁰ = +217° (c 0,10, CHCl₃) ; RMN ¹H (270 MHz, CDCl₃) δ ppm : 1,28 (d, J = 7 Hz, 3H), 1,97 (s, 3H), 2,02 (s, 3H), 2,09 (s, 3H), 2,16 (s, 3H), 2,41 (s, 3H), 2,69 (s, 1H éch/D₂O), 2,88 (d, J = 12 Hz, 1H), 2,93 (d, J = 19 Hz, 1H), 3,24 (d, J = 12 Hz, 1H), 3,24 (d, J = 19 Hz, 1H), 3,69 (s large, 1H), 3,78 (m, 1H éch/D₂O), 4,09 (s, 3H), 4,13 (d, J = 6 Hz, 2H), 4,23 (d, J = 7 Hz, 1H), 4,39 (t, J = 6 Hz, 1H), 4,72 (s, 1H éch/D₂O), 5,01 (s, 2H), 5,24 (dd, J = 10 et 4 Hz, 1H), 5,27 (m, 2H), 5,48 (dd, J = 10 et 3 Hz, 1H), 5,50 (m, 1H), 5,58 (d, J = 3 Hz, 1H), 5,87 (d, J = 4 Hz, 1H), 7,29 (d, J = 8 Hz, 1H), 7,40 (d, J = 8 Hz, 1H), 7,48 (dd, J = 8 et 2 Hz, 1H), 7,76 (d, J = 2 Hz, 1H), 7,79 (t, J = 8 Hz, 1H), 8,02 (d, J = 8 Hz, 1H). SM (DIC/NH₃) m/z : 813 (M-239)⁺, 672 (M-380)⁺.
6) **α-D-galactopyranoside de N-4-hydroxy-3-nitro-(benzyloxycarbonyl)-daunorubicine (75b).**
   - Préparation : une solution de 2,3,4,6-tétra-O-acétyl-α-D-galactopyranoside de N-4-hydroxy-3-nitro**-**(benzyloxycarbonyl)-daunorubicine (17 mg, 0,016 mmole) dans 2 ml de méthanolate de sodium 0,1 N est agitée et maintenue à 0°C pendant 30 minutes. Le milieu est neutralisé par addition de résine Amberlite IRC 120 H⁺, puis filtré. Le filtrat, évaporé à sec, fournit l'α-D-galactopyranoside de N-4-hydroxy-3-nitro-(benzyloxycarbonyl)**-**daunorubicine (14 mg-98 %).
   - Composé 75b : C₄₁H₄₄N₂O₂₀ ; M = 884 ; amorphe ; [α]_{D}²⁰ = +246° (c 0,10, EtOH) ; RMN ¹H (270 MHz, CDCl₃) δ ppm : 1,22 (d, J = 7 Hz, 3H), 1,95 (m, 2H), 2,28 (m, 1H), 2,38 (m, 1H), 2,41 (s, 3H), 2,81 (d, J = 19 Hz, 1H), 3,01 (d, J = 19 Hz, 1H), 4,04 (s, 3H), 5,38 (m, 1H), 5,82 (m, 1H), 7,50-7,70 (m, 4H), 7,90 (m, 2H). SM (FAB, matrice : thioglycérol) m/z : 885 (M+H)⁺.

### EXEMPLE 17 : Synthèse du β-D-glucuronide de N-[4-hydroxy-3-chloro-(benzyloxycarbonyl)]-doxorubicine (78b).

1) **2,3,4-tri-O-acétyl-β-D-méthylglucuronide de 4-hydroxy-3-chlorobenzaldéhyde (76).**
   - Préparation : à une solution de 4-hydroxy-3-chlorobenzaldéhyde (2 g ; 12,8 mmoles) et de bromure de 2,3,4-tri-O-acétyl-α-D-méthylglucuronyle (3,4 g ; 8,5 mmoles) dans l'acétonitrile anhydre (150 ml), on ajoute de l'oxyde d'argent (10 g). Le milieu réactionnel est agité 4 heures à 20°C puis filtré sur célite et évaporé à sec. Après chromatographie sur gel de silice 60H (cyclohexane-acétone ; 80/20 v/v) du résidu, on obtient le 2,3,4-tri-O-acétyl-β-D-méthylglucuronide de 4-hydroxy-3-chlorobenzaldéhyde (76) (1,6 g ; 40 %).
   - Composé 76 : C₂₀H₂₁ClO₁₁ ; M = 472,5 ; F = 125°C ; [α]_{D}²⁰ = -57° (c 0,5, CHCl₃) ; IR (KBr) ν cm⁻¹ : 3030, 2975, 2875, 1760, 1680, 1595, 1375, 1235, 1040 ; RMN ¹H (270 MHz, CDCl₃) δ ppm : 2,09 (3H, s), 2,11 (3H, s), 2,20 (3H, s), 3,73 (3H, s), 4,28 (1H, d, J = 9 Hz), 5,25 (1H, d, J = 7 Hz), 5,32-5,42 (3H, m), 7,30 (1H, d, J = 8 Hz), 7,77 (1H, dd, J = 8 et 2 Hz), 7,93 (1H, d, J = 2 Hz), 9,90 (1H, s). SM (DIC/NH₃) m/z : 490/492 (M+NH₄)⁺ 352, 317.
2) **2,3,4-tri-O-acétyl-β-D-méthylglucuronide de (4-hydroxyméthyl-2-chloro)-phényle (77).**
   - Préparation : une solution de 2,3,4-tri-O-acétyl-β-D-méthylglucuronide de 4-hydroxy-3-chlorobenzaldéhyde (76) (1,56 g ; 3,3 mmoles) dans un mélange de chloroforme (35 ml) et d'isopropanol (7,5 ml) est additionnée à 0°C de silice (3 g) puis de borohydrure de sodium (0,3 g, 7,8 mmoles). Après 2 heures d'agitation à température ambiante, le milieu est filtré, dilué par CH₂Cl₂ (30 ml), lavé à l'eau (3x30 ml), séché sur Na₂SO₄ et évaporé pour fournir le 2,3,4-tri-O-acétyl-β-D-méthylglucuronide de (4-hydroxyméthyl-2-chloro)-phényle (1,00 g ; 64 %).
   - Composé 77 : C₂₀H₂₃ClO₁₁ ; M = 474,5 ; F = 138-140°C ; [α]_{D}²⁰ = -95° (c 0,1, CHCl₃) ; IR (KBr) ν cm⁻¹ : 3510, 2955, 1765, 1740, 1500, 1375, 1235, 1100, 1050, 900, 820, 775 ; RMN ¹H (270 MHz, CDCl₃) δ ppm : 2,06 (3H, s), 2,09 (3H, s), 2,12 (3H, s), 3,74 (3H, s), 4,15 (1H, d, J = 8 Hz), 4,63 (2H, s), 5,04 (1H, d, J = 7 Hz), 5,35 (3H, m), 7,20 (2H, m), 7,38 (1H, d, J = 1,5 Hz). SM (DIC/NH₃) m/z : 492/494 (M+NH₄)⁺, 317.
3) **2,3,4-tri-O-acétyl-β-D-méthylglucuronide de N-(4-hydroxy-3-chloro-benzyloxycarbonyl)-doxorubicine (78a).**
   - Préparation : une solution de 2,3,4-tri-O-acétyl-β-D-méthylglucuronide de (4-hydroxyméthyl-2-chloro) phényle (77) (1,00 g ; 2 mmoles) et de triéthylamine (300 µl ; 2,1 mmoles) dans 80 ml de dichlorométhane anhydre est ajoutée goutte à goutte, à 20°C et sous argon, à une solution de disuccinimidocarbonate (1,08 g ; 4,2 mmoles) dans 50 ml d'acétonitrile. Le mélange est agité pendant 90 minutes puis filtré et évaporé à sec pour fournir quantitativement le succinimidocarbonate (1,3 g), utilisé sans purification pour la condensation avec la doxorubicine.
      Au succinimidocarbonate brut précédemment préparé, on ajoute à 20°C, sous argon, une solution de doxorubicine (0,882 g ; 1,6 mmoles) et de triéthylamine (230 µl ; 1,6 mmoles) dans 40 ml de diméthylformamide. Après 2 heures de réaction, le solvant est évaporé sous pression réduite. Le résidu obtenu fournit, après chromatographie sur colonne de gel de silice 60H (dichlorométhane-méthanol, 95/5 v/v), le 2,3,4-tri-O-acétyl-β-D-méthylglucuronide de N-(4-hydroxy-3-chlorobenzyloxycarbonyl)-doxorubicine (78a) (0,691 g ; 41 %).
   - Composé 78a : C₄₈H₅₀ClNO₂₃ ; M = 1043,5 ; amorphe ; [α]_{D}²⁰ = +260° (c 0,01, CH₃OH) ; IR (KBR) ν cm⁻¹ : 3430, 2960, 2950, 1725, 1580, 1460, 1380, 1290, 1235, 1125, 1070, 1040, 755. RMN ¹H (270 MHz, CDCl₃) δ ppm : 1,30 (3H, d, J = 6,5 Hz), 1,85 (2H, m), 2,02 (3H, s), 2,05 (3H, s), 2,09 (3H, s), 2,17 (1H, dd, J = 15 et 3,5 Hz), 2,35 (1H, dd, J = 15 et 1 Hz), 2,97 (1H, d, J = 19 Hz), 3,27 (1H, d, J = 19 Hz), 3,67 (1H, m), 3,77 (3H, s), 3,86 (1H, m), 4,08 (3H, s), 4,15 (2H, m), 4,75 (2H, s), 4,97 (2H, s), 5,02 (1H, d, J = 7 Hz), 5,18 (3H, m), 5,50 (1H, dd, J = 3,5 et 1 Hz), 7,18 (2H, m), 7,30 (1H, d, J = 1 Hz), 7,38 (1H, d, J = 8 Hz), 7,79 (1H, t, J = 8Hz), 8,03 (1H, d, J = 8 Hz), 13,15 (1H, s, éch. D₂O), 13,85 (1H, s, éch. D₂O).
4) **β-D-glucuronide de N-(4-hydroxy-3-chloro-benzyloxycarbonyl)-doxorubicine (78b).**
   - Préparation : une solution de 2,3,4-tri-O-acétyl-β-D-méthylglucuronide de N-(4-hydroxy-3-chloro-benzyloxycarbonyl) doxorubicine (78a) (0,280 g ; 0,27 mmoles) dans 60 ml de méthanolate de sodium 0,05 N est agitée à 0°C pendant 45 min. Après neutralisation par addition de résine Amberlite IRC 120H⁺, le milieu est filtré puis évaporé à sec. Le β-D-méthylglucuronide de N-(4-hydroxy-3-chloro-benzyloxycarbonyl)-doxorubicine ainsi obtenu (240 mg) est utilisé sans purification pour la déprotection du groupement carboxyle.
      Le β-D-méthylglucuronide de N-(4-hydroxy-3-chlorobenzyloxycarbonyl)-doxorubicine brut précédemment préparé est mis en solution dans 48 ml de tampon phosphate (pH = 8), additionné de 1,2 ml de solution d'estérase de foie de porc (Sigma, réf. E-3128) et de 24 ml d'acétone puis maintenu 4 heures à 37°C. Après évaporation à sec et chromatographie du résidu sur colonne de gel de silice 60H (acétonitrile-eau ; 95/5 v/v), on obtient le β-D-glucuronide de N-(4-hydroxy-3-chloro-benzyloxycarbonyl)-doxorubicine (78b) (0,049 g ; 20 %).
   - Composé 78b : C₄₁H₄₂ClNO₂₀ ; M = 903,5 ; amorphe ; [α]_{D}²⁰ = +140° (c 0,01, CH₃OH) ; IR (KBr) ν cm⁻¹ : 3400, 2950, 1580, 1510, 1470, 1415, 1240, 1215, 1100, 830, 760 ; RMN ¹H (300 MHz, DMSO-d₆) δ ppm : 1,11 (3H, d, J = 6,5Hz), 1,90 (2H, m), 2,15 (2H, m), 3,98 (3H, s), 4,54 (2H, s), 4,87 (2H, s), 4,94 (1H, d, J = 7 Hz), 5,20 (1H, dd, J = 3,5 et 1 Hz), 7,18 (2H, m), 7,36 (1H, d, J = 1 Hz), 7,70 (2H, m), 7,94 (1H, d, J = 8 Hz). SM (FAB, matrice : alcool nitrobenzylique) m/z : 904/906 (M+H) ⁺.

### COMPTE RENDU PHARMACOLOGIQUE CONCERNANT DES PRODROGUES SELON L'INVENTION.

### TEST PHARMACOLOGIQUE :

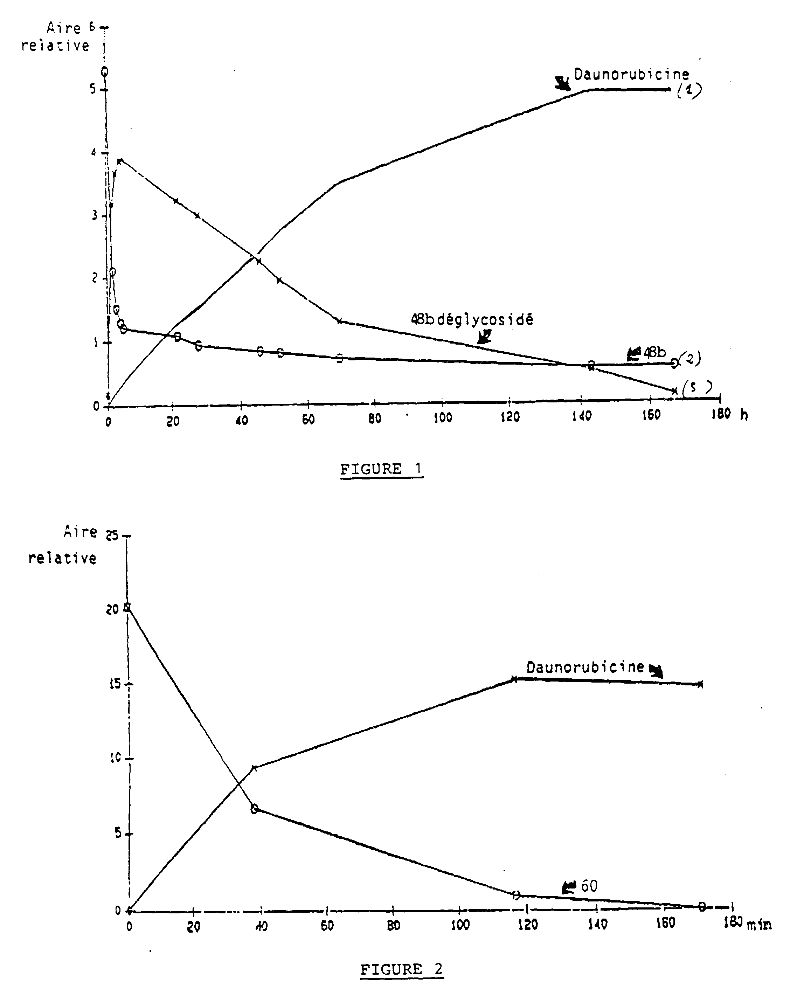

- Exemple A :
   . Les essais biologiques et biochimiques ont consisté en :
      - une évaluation de la cytotoxicité de la prodrogue glycosylée,
      - une étude de la clivabilité du glycoside par l'enzyme correspondante,
      - une détermination *in vitro* de la cinétique de disparition du glycoside et de la formation des deux produits (anthracycline + bras auto-immolable et anthracycline) résultant d'abord de la coupure du sucre sur le phénol glycosylé, et ensuite de la coupure du bras autoimmolable (figures 1, 2, 3 et 4).

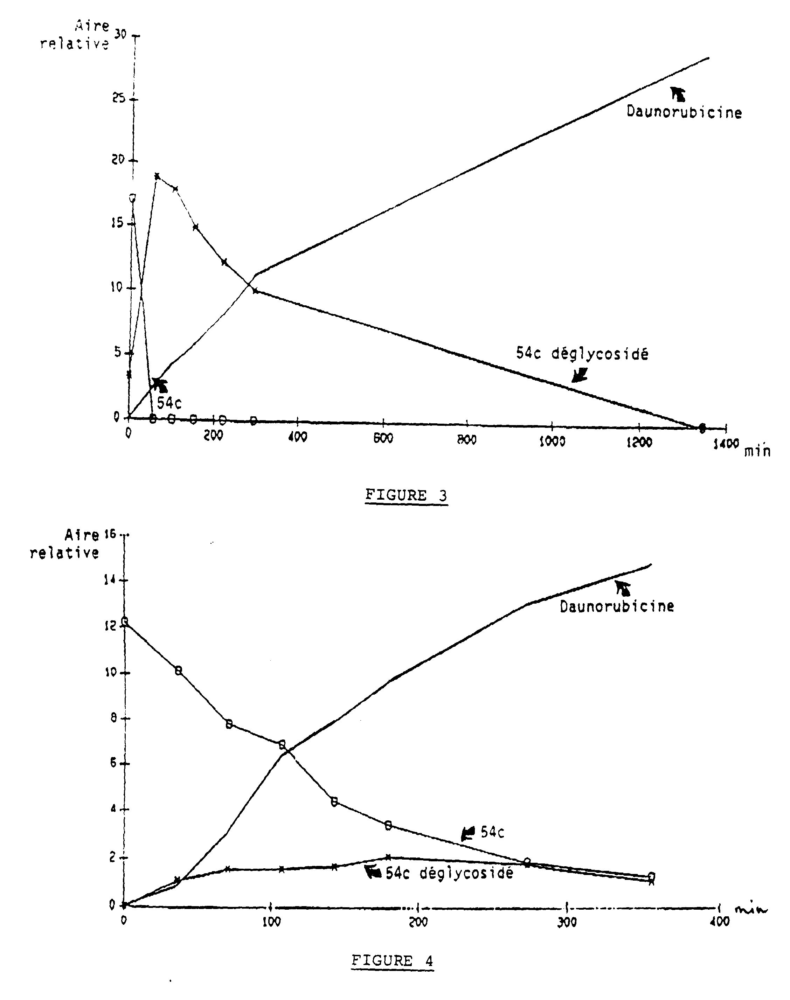

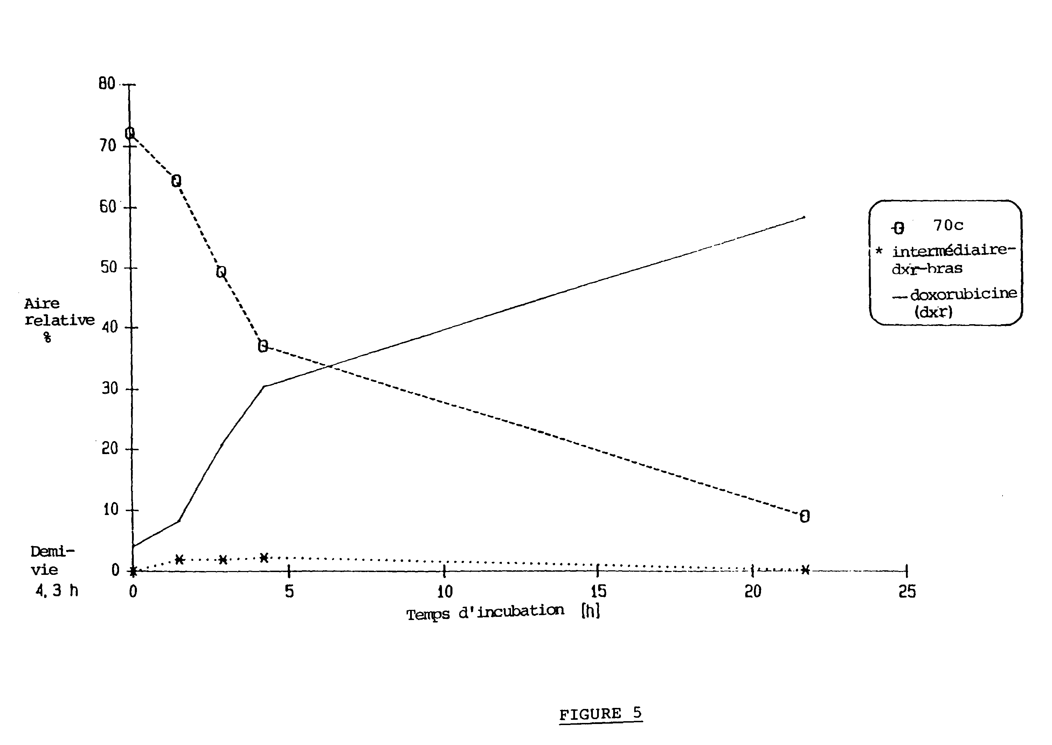

I. demi-vie : Les figures 1, 2, 3, 4 et 5 illustrent les résultats obtenus avec les dérivés 48b (figure 1), 60 (figure 2), 54c (figures 3 et 4) et 70c (figure 5). Ces figures comportent en abscisse le temps et en ordonnées, l'aire relative et permettent d'évaluer les concentrations de prodrogue et d'anthracycline en fonction du temps. Il faut noter que suivant le type de bras auto-immolable, la vitesse d'apparition de la daunorubicine est variable.
. Résultats obtenus avec le dérivé 48b :
   Celui-ci est incubé avec l'α-galactosidase (d'origine placentaire humaine) et 0,075 M de N-acétyl galactosamine à 37°C et pH 5 (tampon phosphate 0,02 M). Les résultats sont déterminés par HPLC (daunorubicine : tᵣ = 8,5 min ; 48b : tᵣ = 11,5 min ; 48b déglycosidé : tᵣ = 16,7 min), le 48b est hydrolysé avec une demi-vie de 1 h.
   A pH 5, le composé DNM-bras auto-immolable (produit déglycosidé) disparaît avec une demi-vie de 45 h, celle-ci est inférieure à 16 h pour le même produit à pH 7,3 et la daunomycine apparaît dans le milieu.
   Le produit est stable dans le plasma.
   La figure 1 illustre les résultats obtenus avec le dérivé 48b.
   Cette figure 1 représente les concentrations en fonction du temps de la daunorubicine (1), du dérivé 48b (2) (prodrogue d'anthracycline conforme à l'invention) et de l'intermédiaire daunorubicine + bras auto-immolable (dérivé déglycosidé) (3).
. Résultats obtenus avec le dérivé 60 (figure 2). Celui-ci est incubé avec de l'α-galactosidase (grains de café vert, 0,04 U/ml) à 37°C et pH 6,8 (tampon phosphate 0,02 M).
   Ces résultats sont déterminés par HPLC (daunorubicine : tᵣ = 8,5 min ; 60 : tᵣ = 11,0 min) ; la concentration en dérivé 60 est de 660 µg/ml.
   A pH 6,8, le dérivé 60 déglycosidé n'est pas détecté, en raison de sa vitesse élevée de disparition.
. Résultats obtenus avec le dérivé 54c (figures 3 et 4).
   La figure 3 montre les résultats obtenus lorsque le dérivé 54c est incubé avec de la β-glucuronidase humaine recombinante concentrée à 37°C et pH 7,2. La concentration de 54c est de 530 µg/ml. Les résultats ont été déterminés par HPLC (daunorubicine : tᵣ = 8,8 min ; 54c : tᵣ = 9,7 min ; 54c déglycosidé : tᵣ = 16,0 min).
   La figure 4 montre les résultats obtenus en présence de β-glucuronidase diluée à 1/100 (autres conditions identiques à celles de la figure 3).
   A pH 7,2, le dérivé 54 déglycosidé a une durée de demi-vie de l'ordre de 5,3 h.
. Résultats obtenus avec le dérivé 70c. La figure 5 montre les résultats obtenus lorsque la prodrogue 70c est incubée avec de la β-glucuronidase humaine (0,45 U/ml) à 37°C et à pH 7,2.

II. test de prolifération :
A. Protocole opératoire (réduction MTT) :
Des cellules tumorales L1210, à une densité de 5.10³/ml dans un milieu RPMI sont incubées dans des plaques de microtitration contenant 96 puits pendant 72 heures (37°C, 5 % CO₂, 95 % d'humidité relative) avec différentes prodrogues conformes à l'invention.
Les témoins consistent en des cellules tumorales exposées à un milieu de culture. Quatre puits sont préparés pour chaque concentration en anthracycline et pour le témoin. Après 65 heures, 50 µl de MTT (2,5 mg/ml dans du PBS) sont ajoutés.
Le MTT sera réduit en présence de cellules vivantes en un colorant formazan rouge insoluble. Après 7 à 24 heures d'incubation supplémentaire (dépendant des cellules utilisées), le surnageant est enlevé. Le colorant formazan est solubilisé par l'addition de 100 µl de DMSO dans chaque puits, suivie d'une agitation douce.
L'extinction est mesurée pour chaque puits, à 492 nm (photomètre Multiscan 340 CC Fa. Flow).
B. Résultats :
Les résultats sont exprimés comme le rapport de l'extinction après incubation avec les prodrogues sur l'extinction obtenue avec les témoins. Le coefficient de variation est inférieur à 15 %. Les prodrogues ont une cytotoxicité considérablement réduite par rapport à la doxorubicine.

| Produits testés | L1210 CI₅₀ (µg/ml) |
|---|---|
| doxorubicine | 0,02 |
| dérivé 6 | > 1 |
| dérivé 7 | > 1 |
| dérivé 13 | > 1 |
| dérivé 14 | > 1 |
| dérivé 22 | > 10 |
| dérivé 27c | > 1 |
| dérivé 48b | > 1 |
| dérivé 48a | > 10 |

Les acétates 6, 13 et 48a sont hydrolysés *in vivo* respectivement en 7, 14 et 48b.
III. Comparaison de l'aptitude au clivage de prodrogues glycosylées conformes à l'invention avec des drogues glycosylées et influence de la structure du bras sur la vitesse de coupure du glycosyle et la vitesse d'élimination du bras auto-immolable :
On synthétise des prodrogues conformes à l'invention du type daunomycine-bras auto-immolable-β-glucuronides, doxorubicine-bras auto-immolable-β-glucuronides et doxorubicine-bras auto-immolable-α-galactosides conformément au procédé décrit ci-dessus.
On incube les substances à 37°C avec de la β-glucuronidase recombinante (ou de l'α-galactosidase de grains de café) (1 U/ml), à un pH de 7,2 à 6,8, et on détermine *in vitro* la cinétique de disparition du glycoside ainsi que la cinétique de formation de daunomycine et de doxorubicine à l'aide d'une HPLC à phase inverse. Le temps de coupure de 50 % du glycoside et le temps de transformation de 50 % du composé anthracycline-bras en anthracycline active sont indiqués dans le Tableau I ci-après, qui se lit en référence à la formule suivante :

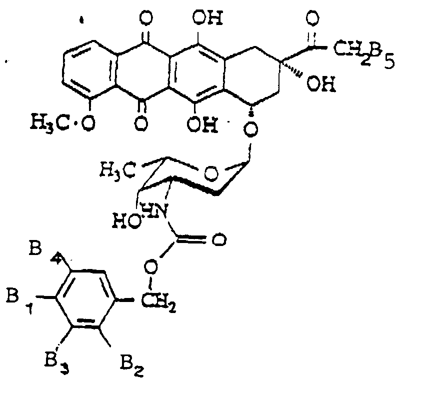

**TABLEAU I**

| Substance | B₁ | B₂ | B₃ | B₄ | t_{1/2} clivage du glycoside (h) | t_{1/2} bras (h) |
|---|---|---|---|---|---|---|
| B₅ = H (daunomycine) | | | | | | |
| 1 (68c) | OCH₃ | β-Gluc | H | NO₂ | 1,88 | 0,75 |
| 2 (54c) | H | β-Gluc | H | NO₂ | 0,69 | 5,30 |
| 3 (82c) | H | β-Gluc | H | Cl | 4,06 | 12,00 |
| 4 (64c) | β-Gluc | H | H | NO₂ | 0,42 | <0,08 |
| 5 (60) | α-Gal | H | H | Cl | 0,02 | <0,08 |
| B₅ = OH (doxorubicine | | | | | | |
| 6 (70c) | H | β-Gluc | H | NO₂ | 0,92 | n.t. |

Le β-glucuronide exempt de bras, servant de substance de comparaison, est clivé de 50 à 100 fois moins vite que les composés daunomycine-bras-glucuronides ou que les composés doxorubicine-bras-glucuronides. Ceci indique que la présence d'un bras auto-immolable adéquat pour le clivage du composé glucuronide-prodrogue est d'une importance décisive. De plus, les substituants présents sur le cycle aromatique de ce bras auto-immolable (hydroxybenzylcarbamates, par exemple) ont une influence sur la cinétique de coupure du dérivé glycosylé et sur la cinétique de décomposition du produit anthracycline + bras auto-immolable. On trouve un clivage plus rapide du glycosyle et une élimination automatique plus rapide du bras pour les prodrogues conformes à l'invention, dans lesquelles B₁ est un β-glucuronide et B₄ est un groupe NO₂ ou un atome de chlore. D'autres substituants permettent également d'obtenir les cinétiques souhaitées.
Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

## Revendications (Revendications pour l'(les) Etat(s) contractant(s) suivant(s): AT, BE, CH, LI, DE, DK, FR, GB, IT, LU, NL, PT, SE)

1. Prodrogues d'anthracyclines, caractérisées en ce qu'elles répondent à la formule I ci-après : 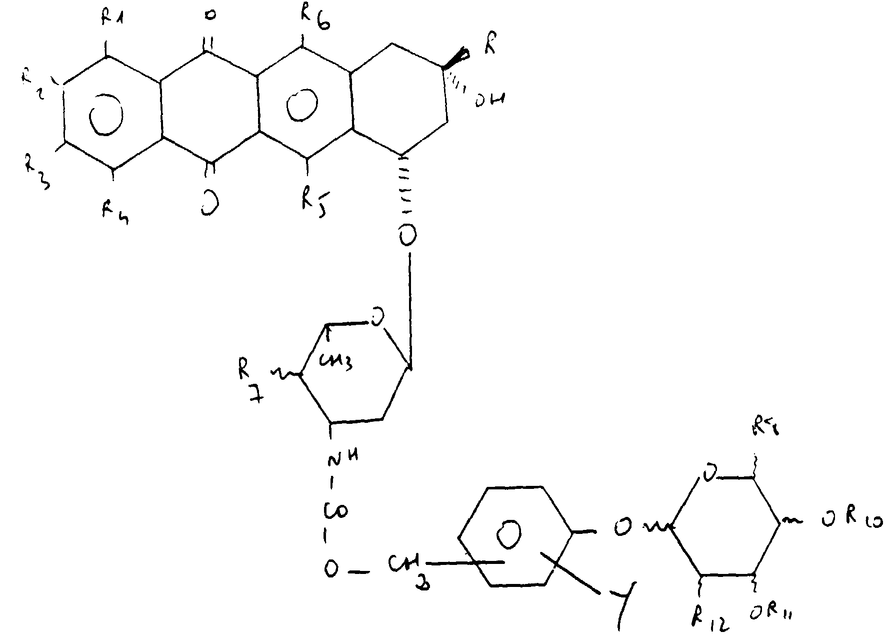 dans laquelle,
R₁, R₂, R₃, qui peuvent être identiques ou différents, représentent un atome d'hydrogène ou un groupe hydroxyle ;
R₄ représente un atome d'hydrogène, un groupe hydroxyle ou un groupe méthoxy ;
R représente un groupe CO-CH₂-R˝, dans lequel R" est un atome d'hydrogène, un groupe alkyle en C₁-C₆, un groupe hydroxyle, un groupe alkoxyle, un groupe O-acyle ou un groupe aryle ;
R₅ et R₆, qui peuvent être identiques ou différents, représentent un atome d'hydrogène ou un groupe hydroxyle ;
R₇ représente un atome d'hydrogène ou un groupe hydroxyle ;
R₈ représente un groupe -CH₂-OR₉ ou un groupe COOR₉ avec
R₉ étant un alkyle en C₁-C₃ ou un atome d'hydrogène ;
R₁₀ et R₁₁ représentent un atome d'hydrogène, un groupement acyle protecteur ou un groupe alkyle ;
R₁₂ représente un groupe hydroxyle, un groupement amine, un groupement amide ou un groupement O-acyle protecteur ;
le -CH₂ benzylique est de préférence en position para ou ortho de l'oxygène glycosylé ;
Y représente un atome d'hydrogène, ou au moins un groupement électro-attracteur notamment choisi dans le groupe qui comprend le groupement NO₂, un atome d'halogène, un groupement SO₂X, (avec X = -CH₃, C₆H₄-CH₃, NH₂, N-(alkyle_{C1-C4})₂, NH-(alkyle_{C1-C4}), -CN, acyle ou COO-alkyle, et/ou au moins un groupement électrodonneur choisi dans le groupe qui comprend les groupements de type O-alkyle, NHCO-alkyle, N(alkyl)CO-alkyle, S-alkyle, alkyle.

2. Prodrogues selon la revendication 1, caractérisées en ce que lorsque Y représente un/des groupements électroattracteurs, ceux-ci sont de préférence en position ortho et/ou para de l'oxygène glycosylé et lorsque Y représente un/des groupements électrodonneurs, ceux-ci sont de préférence en position méta.

3. Prodrogues selon la revendication 1 ou la revendication 2, caractérisées en les composés préférés de formule I comprennent notamment les radicaux suivants :
R₁, R₂, R₃ sont des atomes d'hydrogène,
R₄ est un groupe méthoxy,
R₅ et R₆ sont des groupes hydroxyles,
R est un groupe -CO-CH₃ ou un groupe -CO-CH₂OH,
R₇ est un atome d'hydrogène, ou un groupe hydroxyle,
R₈ est un groupe -CH₂-OAc, -CH₂OH, -COOMe, ou -COOH,
R₁₀ et R₁₁, qui peuvent être identiques ou différents représentent un atome d'hydrogène ou un groupement Ac,
R₁₂ représente un groupe hydroxyle ou un groupe OAc, lesquels radicaux R₈, R₁₀, R₁₁ et R₁₂ ont de préférence les positions suivantes : 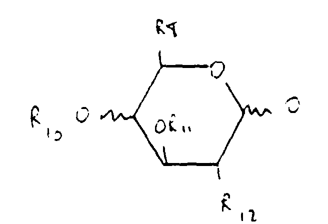 Y est un atome d'hydrogène, un groupe NO₂ ou un atome de chlore, en para ou en ortho de l'oxygène glycosylé ou un groupe OCH₃ en méta de l'oxygène glycosylé.

4. Prodrogue selon l'une quelconque des revendications 1 à 3, caractérisée en ce qu'elle présente la formule 6 ci-après :

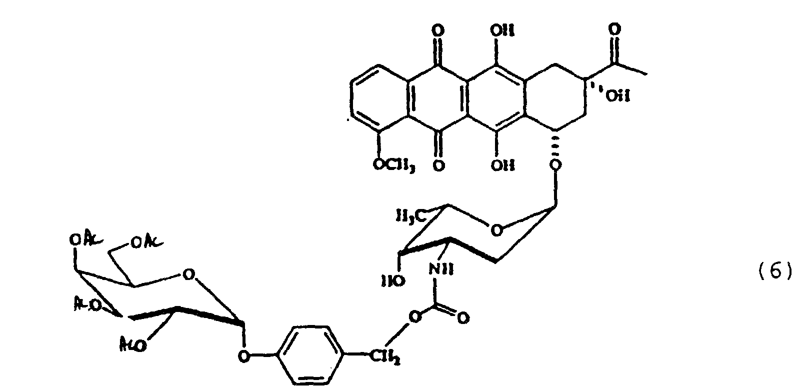

5. Prodrogue selon l'une quelconque des revendications 1 à 3, caractérisée en ce qu'elle présente la formule 7 ci-après :

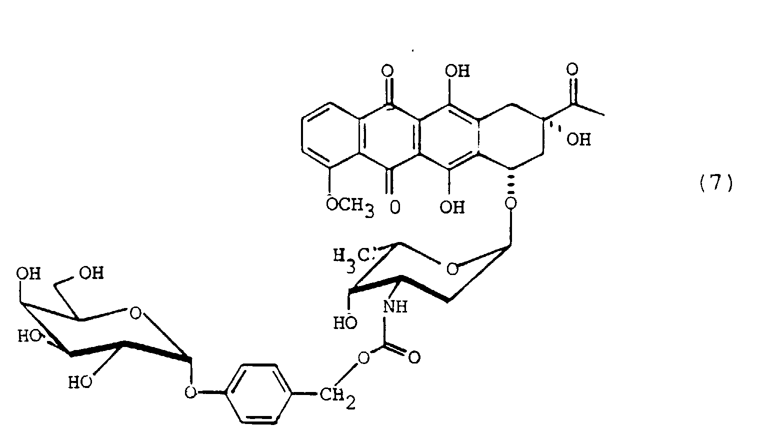

6. Prodrogue selon l'une quelconque des revendications 1 à 3, caractérisée en ce qu'elle présente la formule 13 ci-après :

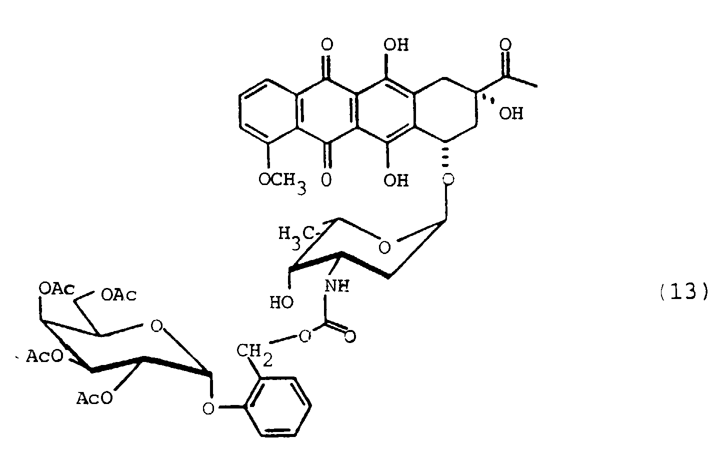

7. Prodrogue selon l'une quelconque des revendications 1 à 3, caractérisée en ce qu'elle présente la formule 14 ci-après :

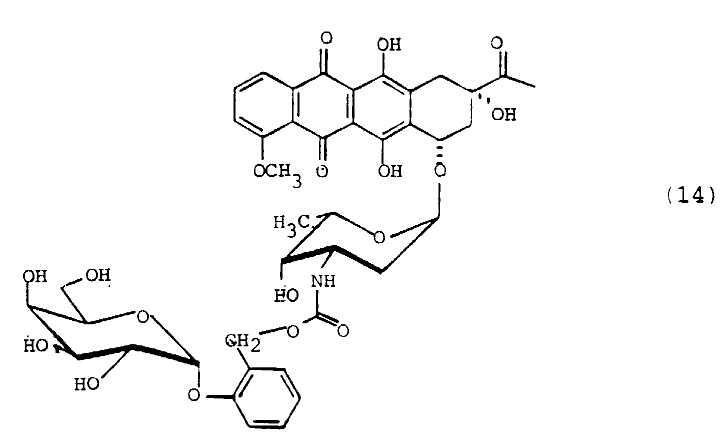

8. Prodrogue selon l'une quelconque des revendications 1 à 3, caractérisée en ce qu'elle présente la formule 22 ci-après :

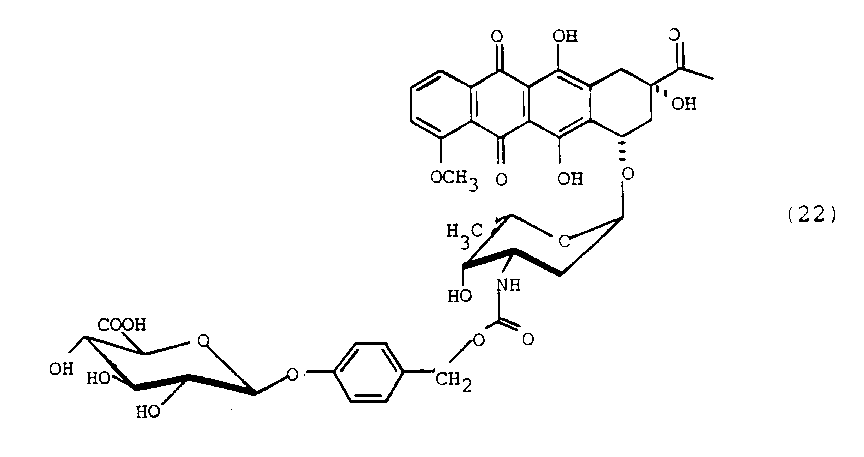

9. Prodrogue selon l'une quelconque des revendications 1 à 3, caractérisée en ce qu'elle présente la formule 27c ci-après :

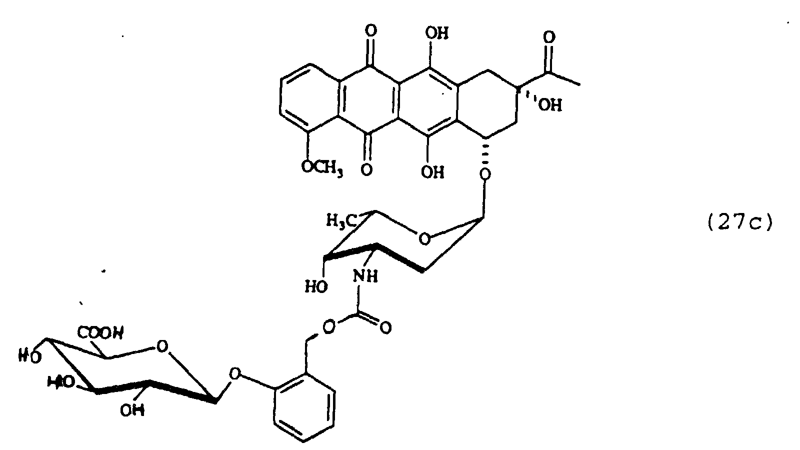

10. Prodrogue selon l'une quelconque des revendications 1 à 3, caractérisée en ce qu'elle présente la formule 36 ci-après :

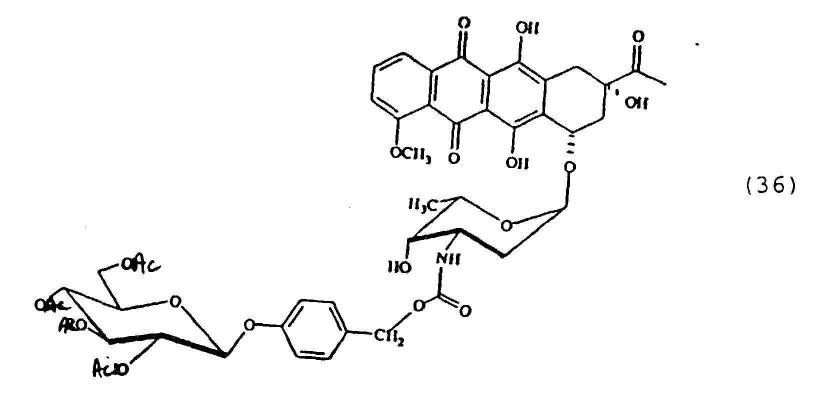

11. Prodrogue selon l'une quelconque des revendications 1 à 3, caractérisée en ce qu'elle présente la formule 37 ci-après :

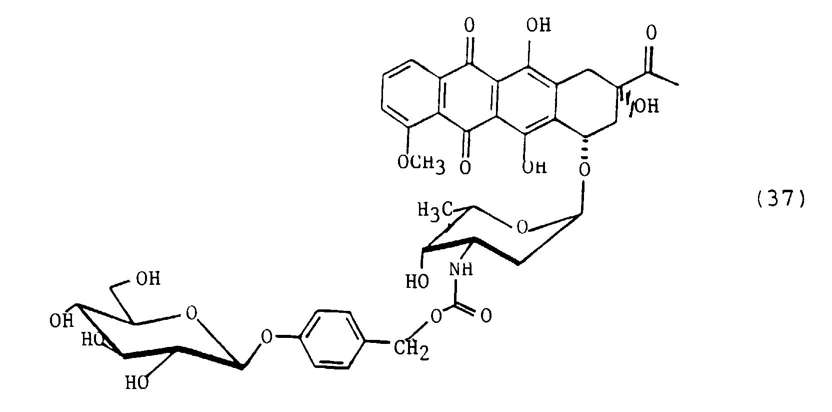

12. Prodrogue selon l'une quelconque des revendications 1 à 3, caractérisée en ce qu'elle présente la formule 48a ci-après :

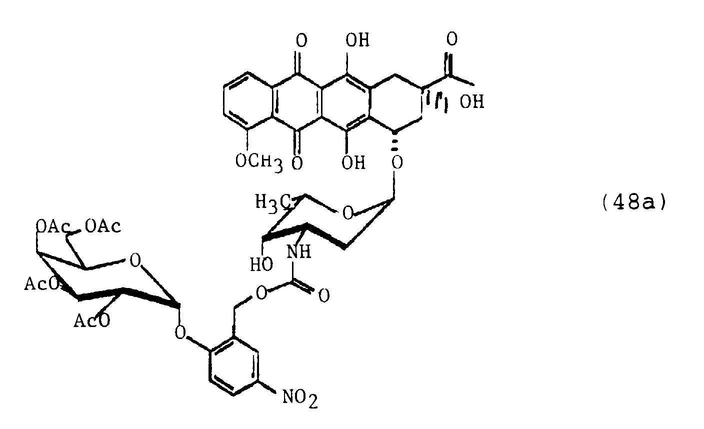

13. Prodrogue selon l'une quelconque des revendications 1 à 3, caractérisée en ce qu'elle présente la formule 48b ci-après :

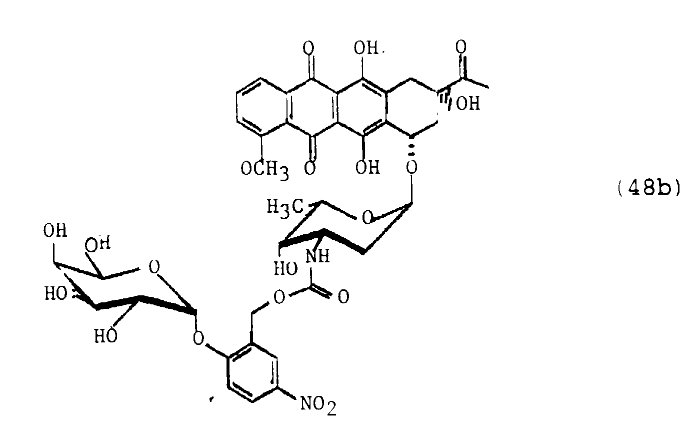

14. Prodrogue selon l'une quelconque des revendications 1 à 3, caractérisée en ce qu'elle présente la formule 49a ci-après :

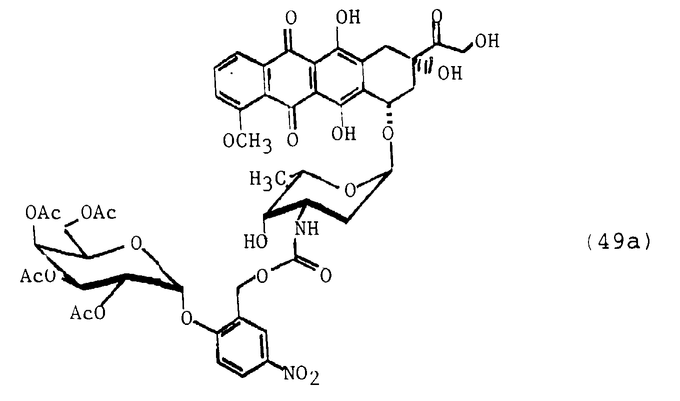

15. Prodrogue selon l'une quelconque des revendications 1 à 3, caractérisée en ce qu'elle présente la formule 49b ci-après :

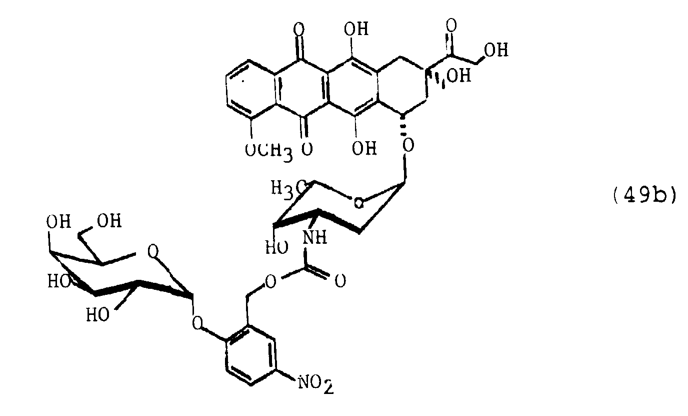

16. Prodrogue selon l'une quelconque des revendications 1 à 3, caractérisée en ce qu'elle présente la formule 54c ci-après :

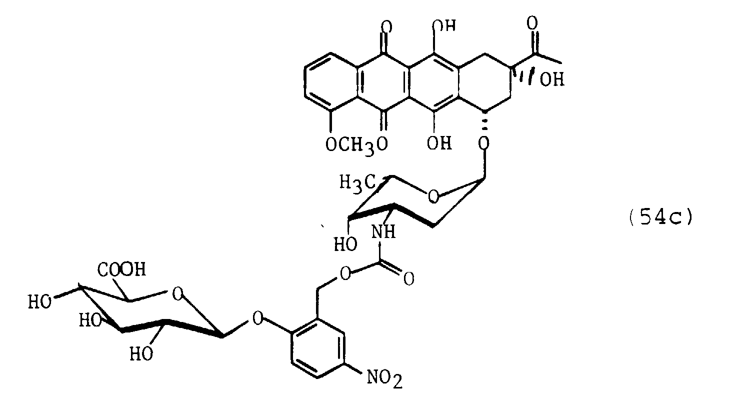

17. Prodrogue selon l'une quelconque des revendications 1 à 3, caractérisée en ce qu'elle présente la formule 59 ci-après :

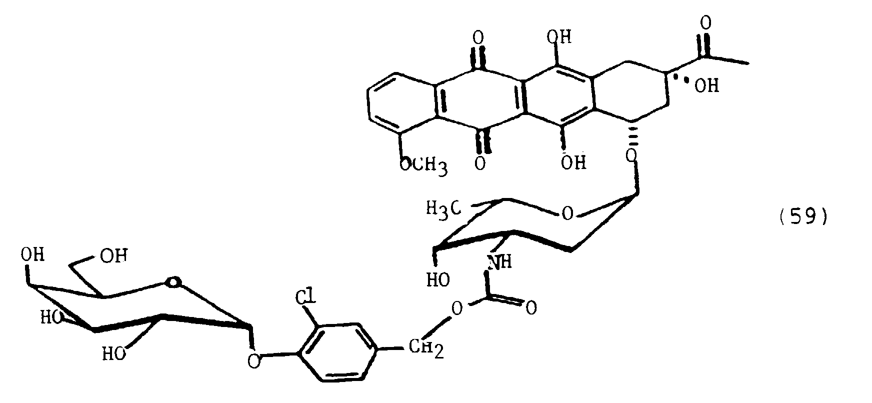

18. Prodrogue selon l'une quelconque des revendications 1 à 3, caractérisée en ce qu'elle présente la formule 60 ci-après :

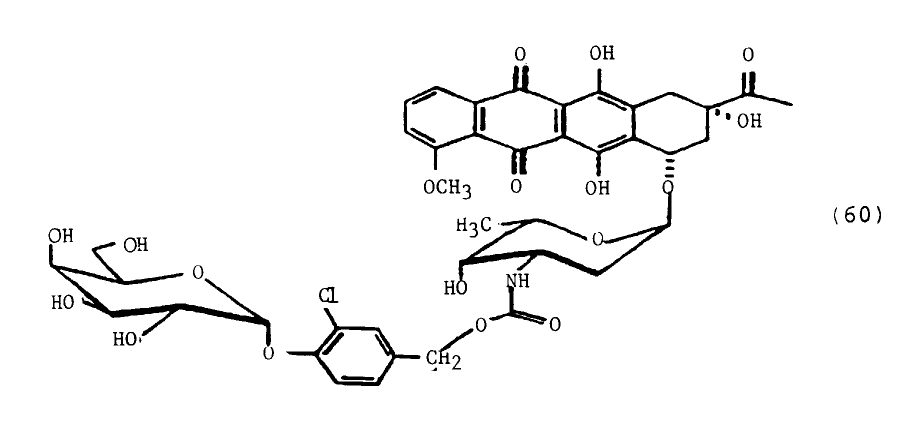

19. Prodrogue selon l'une quelconque des revendications 1 à 3, caractérisé en ce qu'elle présente la formule 64c ci-après :

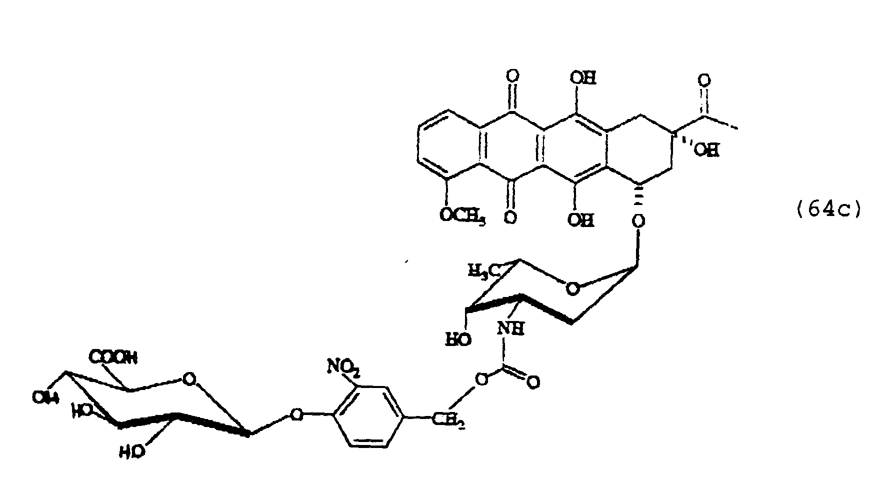

20. Prodrogue selon l'une quelconque des revendications 1 à 3, caractérisée en ce qu'elle présente la formule 68c ci-après :

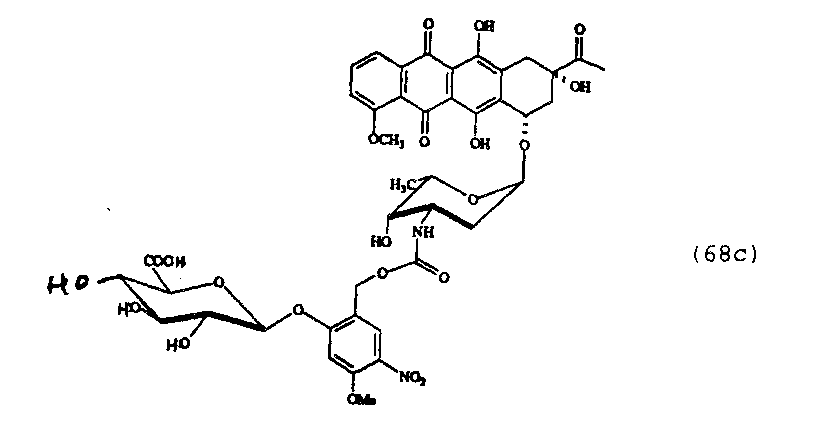

21. Prodrogue selon l'une quelconque des revendications 1 à 3, caractérisée en ce qu'elle présente la formule 75a ci-après :

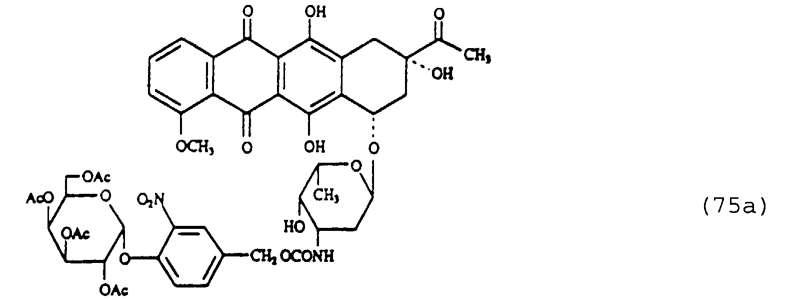

22. Prodrogue selon l'une quelconque des revendications 1 à 3, caractérisée en ce qu'elle présente la formule 75b ci-après :

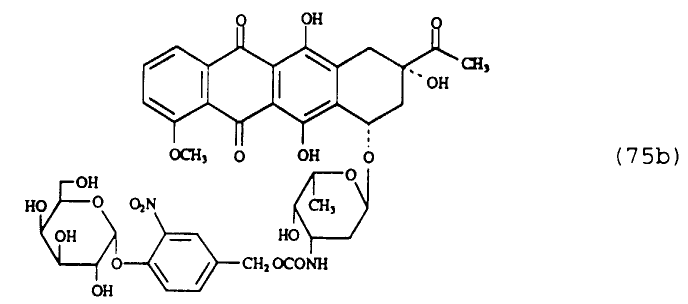

23. Prodrogue selon l'une quelconque des revendications 1 à 3, caractérisée en ce qu'elle présente la formule 78a ci-après :

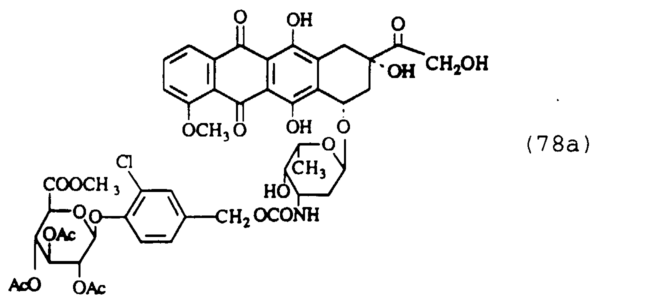

24. Prodrogue selon l'une quelconque des revendications 1 à 3, caractérisée en ce qu'elle présente la formule 78b ci-après :

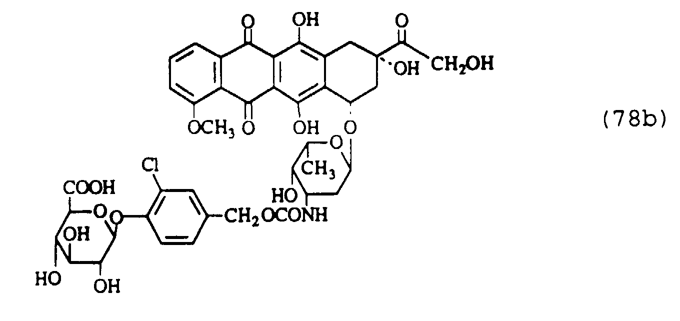

25. Prodrogue selon l'une quelconque des revendications 1 à 3, caractérisé en ce que ladite prodrogue présente la formule 70 ci-après : 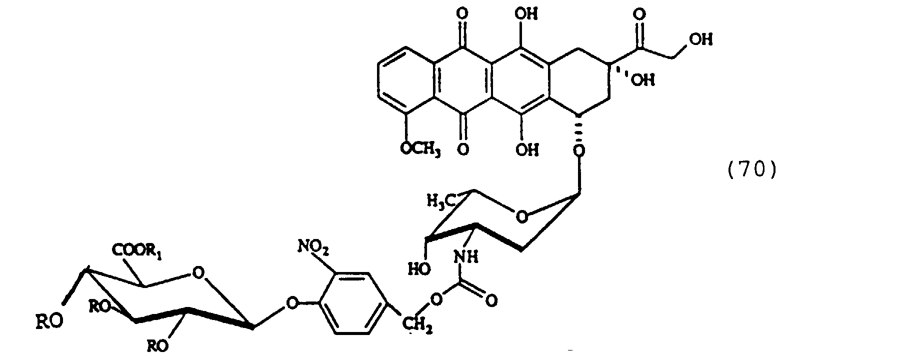 avec
70a : R=Ac, R₁=CH₃
70b : R=H, R₁=CH₃
70c : R=R₁=H

26. Prodrogue selon l'une quelconque des revendications 1 à 3, caractérisée en ce qu'elle présente la formule 82 ci-après : 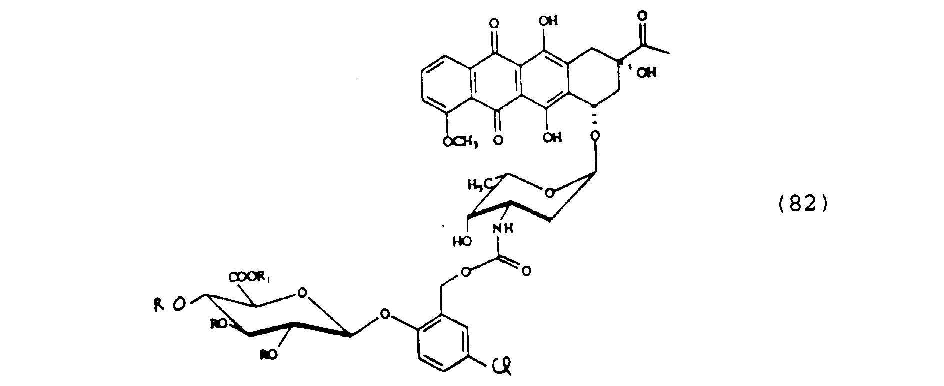 avec :
82a : R = Ac , R₁ = CH₃
82b : R = H , R₁ = CH₃
82c : R = R-₁ = H

27. Prodrogue selon l'une quelconque des revendications 1 à 3, caractérisée en ce qu'elle présente la formule 83 ci-après : 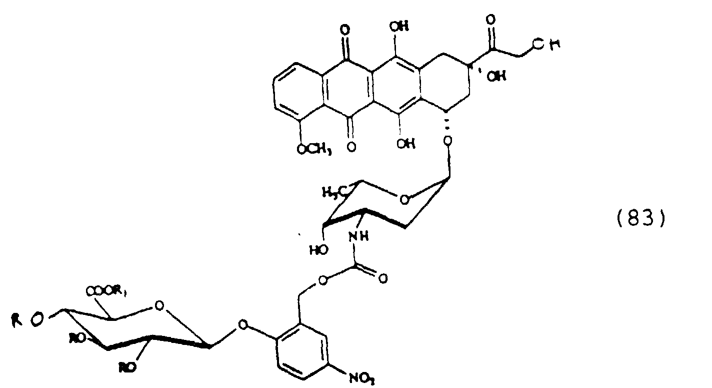 avec
83a : R = Ac , R₁ = CH₃
83b : R = H , R₁ = CH₃
83c : R = R₁ = H

28. Procédé de préparation d'un composé de formule I qui peut notamment être dégradé par une glycosidase, caractérisé en ce qu'il comprend :
(1) le couplage, en présence, éventuellement, d'un promoteur approprié, d'un dérivé de formule A : 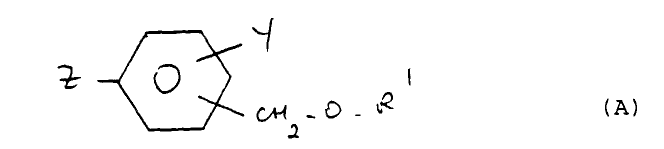 dans laquelle
Z représente un groupe hydroxyle, un groupe O-trialkylsilyle ou un groupe 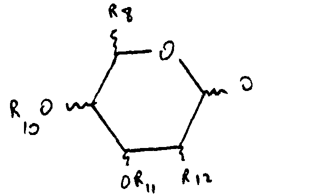 dans lequel R₈, R₁₀, R₁₁ et R₁₂ ont la même signification que ci-dessus ;
R' représente un atome d'hydrogène ou l'un des groupes suivants : 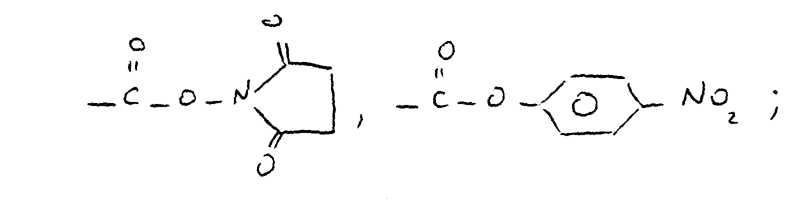 le -CH₂ benzylique est de préférence en position para ou ortho de la fonction phénol, éventuellement modifiée (glycosylée ou silylée) ;
Y représente un atome d'hydrogène, ou au moins un groupement électro-attracteur notamment choisi dans le groupe qui comprend le groupement NO₂, un atome d'halogène, un groupement SO₂X, (avec X = -CH₃, C₆H₄-CH₃, NH₂, N-(alkyle_{C1-C4})₂, NH-alkyle_{C1-C4}), -CN, acyle ou COO-alkyle, et/ou au moins un groupement électrodonneur choisi dans le groupe qui comprend les groupements de type O-alkyle, NH-CO-alkyle, N(alkyl)CO-alkyle, S-alkyle, alkyle avec une anthracycline de formule B 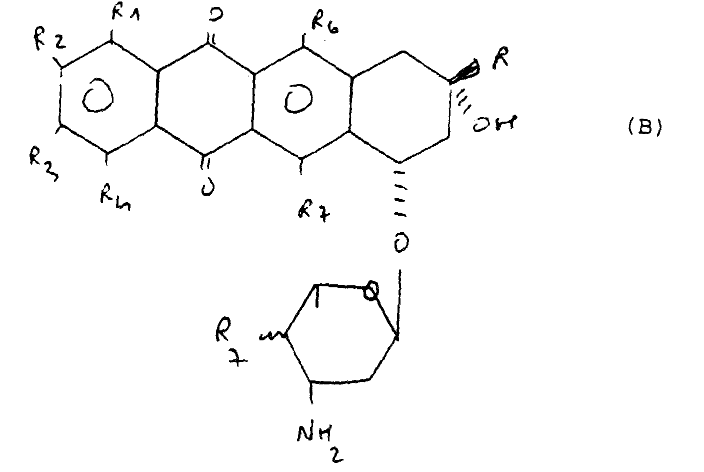 dans laquelle R₁, R₂, R₃, R₄, R₅, R₆, R₇ et R ont la même signification que ci-dessus,
(2) l'élimination des groupes protecteurs présents dans les composés obtenus, notamment par hydrolyse, transestérification ou saponification, et
(3) éventuellement, la condensation appropriée avec un ose de formule suivante : 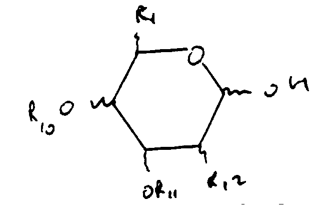 dans le cas où Z représente un groupement hydroxyle ou un groupe O-trialkylsilyle,
pour donner une prodrogue d'anthracycline de formule I, dans laquelle tous les radicaux R₁ à R₁₂ et R ont la même signification que précédemment.

29. Procédé selon la revendication 28, caractérisé en ce que préalablement à l'étape (1), le dérivé p-hydroxybenzyle glycosylé est obtenu par :
(a) fusion d'un crésol avec un ose ou un glucuronate de méthyle peracétylé,
(b) bromation benzylique du produit obtenu,
(c) solvolyse du dérivé bromé, et
(d) activation du groupe hydroxyle par un dérivé hydroxysuccinimidyle ou paranitrophénoxy-carbonyle.

30. Produits comprenant une prodrogue d'anthracycline modifiée selon l'une quelconque des revendications 1 à 27 et un conjugué enzyme-anticorps spécifique vis-à-vis d'une tumeur, de formule II
Ac-Sp-E, (II)
dans laquelle :
Ac est un anticorps ou l'un de ses fragments, qui présente une spécificité vis-à-vis d'un antigène associé à une tumeur, ou est une biomolécule qui a tendance à s'accumuler dans une tumeur, tel que l'EGF (facteur de croissance épidermique), l'α-TGF (facteur de croissance transformant α), le PDGF (facteur de croissance dérivé des plaquettes), l'IGF I+II (facteur de croissance de l'insuline I+II) ou le FGF a+b (facteur de croissance fibroblastiques a+b),
E représente une glycosidase qui n'est pas immunogène ou présente une immunogénicité très faible, de préférence une glycosidase de mammifère, telle que l'α ou la β-glucosidase, l'α-galactosidase, l'α ou la β-mannosidase, l'α-fucosidase, la N-acétyl-α-galactosaminidase, la N-acétyl-β-/N-acétyl-α-glucosaminidase ou la β-glucuronidase,
Sp (Bras) représente un groupe contenant un sulfure ou un disulfure de formule III ou IV
X′ (S)ₙ Y′ (III)
X′ (S)ₙ (IV),
ou un bras polypeptidique, dans lequel
X′ ou Y′ représente -CO-R₁₃-(N-succinimido)- ou -C(=R₁₄)-CH₂-CH₂-
avec R₁₃ étant un groupement -CH₂-CH₂, 1,4-cyclohexylidène, 1,3 ou 1,4 phénylène ou méthoxycarbonyle ou chloro-1,4-phénylène
et R₁₄ étant un atome d'oxygène ou un groupe NH, et de plus,
Y′ représente -C(=R₁₄)-CH₂-CH₂, lorsque R₁₄ a la signification précisée ci-dessus et
n représente 1 ou 2,
pour une utilisation simultanée, séparée ou étalée dans le temps, en thérapie cytostatique.

31. 2,3,4,6-tétra-O-acétyl-α-D-galactopyranoside de 4-bromométhyl phényle de formule 2, produit intermédiaire du procédé selon la revendication 28.

32. 2,3,4,6-tétra-O-acétyl-α-D-galactopyranoside de 4-formyl phényle de formule 3, produit intermédiaire du procédé selon la revendication 28.

33. 2,3,4,6-Tétra-O-acétyl-α-D-galactopyranoside de 4-hydroxyméthyl phényle de formule 4, produit intermédiaire du procédé selon la revendication 28.

34. 2,3,4,6-tétra-O-acétyl-α-D-galactopyranoside de 2-bromométhyl phényle de formule 9, produit intermédiaire du procédé selon la revendication 28.

35. 2,3,4,6-tétra-O-acétyl-α-D-galactopyranoside de 2-formyl phényle de formule 10, produit intermédiaire du procédé selon la revendication 28.

36. 2,3,4,6-tétra-O-acétyl-α-D-galactopyranoside de 2-hydroxyméthyl phényle de formule 11, produit intermédiaire du procédé selon la revendication 28.

37. (2,3,4-tri-O-acétyl-β-D-glucopyranoside de 4-bromométhyl phényle) uronate de méthyle de formule 16, produit intermédiaire du procédé selon la revendication 28.

38. (2,3,4-tri-O-acétyl-β-D-glucopyranoside de 4-formyl-phényle) uronate de méthyle de formule 17, produit intermédiaire du procédé selon la revendication 28.

39. (2,3,4-tri-O-acétyl-β-D-glucopyranoside de 4-hydroxyméthyl phényle) uronate de méthyle de formule 18, produit intermédiaire du procédé selon la revendication 28.

40. (2,3,4-tri-O-acétyl-β-D-glucopyranoside de 2-bromométhyl phényl) uronate de méthyle de formule 23, produit intermédiaire du procédé selon la revendication 28.

41. (2,3,4-tri-O-acétyl-β-D-glucopyranoside de 2-hydroxyméthyl phényl) uronate de méthyle de formule 24, produit intermédiaire du procédé selon la revendication 28.

42. (2,3,4-tri-O-acétyl-β-D-glucopyranoside de 2-formyl phényl) uronate de méthyle de formule 25, produit intermédiaire du procédé selon la revendication 28.

43. 2-tertiobutyldiméthylsilyloxy-benzaldéhydede formule 28, produit intermédiaire du procédé selon la revendication 28.

44. alcool 2-tertiobutyldiméthylsilyloxybenzylique de formule 29, produit intermédiaire du procédé selon la revendication 28.

45. N-[2-hydroxy-benzyloxycarbonyl] daunorubicine de formule 32, produit intermédiaire du procédé selon la revendication 28.

46. 2,3,4,6-tétra-O-acétyl-β-D-glucopyranoside de 4-formyl phényle de formule 33, produit intermédiaire du procédé selon la revendication 28.

47. 2,3,4,6-tétra-O-acétyl-β-D-glucopyranoside de 4-hydroxyméthyl phényle de formule 34, produit intermédiaire du procédé selon la revendication 28.

48. N-[4-hydroxy-benzyloxycarbonyl] daunorubicine de formule 38, produit intermédiaire du procédé selon la revendication 28.

49. 2,3,4,6-tétra-O-acétyl-α-D-galactopyranoside de 2-méthyl 4-nitro phényle de formule 42, produit intermédiaire du procédé selon la revendication 28.

50. 2,3,4,6-tétra-O-acétyl-α-D-galactopyranoside de 2-bromométhyl 4-nitro phényle de formule 43, produit intermédiaire du procédé selon la revendication 28.

51. 2,3,4,6-tétra-O-acétyl-α-D-galactopyranoside de 2-dibromométhyl 4-nitro phényle de formule 44, produit intermédiaire du procédé selon la revendication 28.

52. 2,3,4,6-tétra-O-acétyl-α-D-galactopyranoside de 2-formyl 4-nitro phényle de formule 45, produit intermédiaire du procédé selon la revendication 28.

53. 2,3,4,6-tétra-O-acétyl-α-galactopyranoside de (2-hydroxyméthyl-4-nitro) phényle de formule 46, produit intermédiaire du procédé selon la revendication 28.

54. (2,3,4-tri-O-acétyl-β-D-glucopyranoside de 2-formyl 4-nitro phényle) uronate de méthyle de formule 51, produit intermédiaire du procédé selon la revendication 28.

55. (2,3,4-tri-O-acétyl-β-D-glucopyranoside de 2-hydroxyméthyl 4-nitro-phényl)uronate de méthyle de formule 52, produit intermédiaire du procédé selon la revendication 28.

56. 2,3,4,6-tétra-O-acétyl-α-D-galactopyranoside de 2-chloro-4-méthyl phényle de formule 55, produit intermédiaire du procédé selon la revendication 28.

57. 2,3,4,6-tétra-O-acétyl-α-D-galactopyranoside de 2-chloro-4-bromométhyl phényle de formule 56, produit intermédiaire du procédé selon la revendication 28.

58. 2,3,4,6-tétra-O-acétyl-α-D-galactopyranoside de 2-chloro-4-hydroxyméthyl phényle de formule 57, produit intermédiaire du procédé selon la revendication 28.

59. (2,3,4-tri-O-acétyl-β-D-glucopyranoside de 4-formyl 2-nitro-phényle) uronate de méthyle de formule 61, produit intermédiaire du procédé selon la revendication 28.

60. (2,3,4-tri-O-acétyl-β-D-glucopyranoside de 4-hydroxyméthyl-2-nitro phényle) uronate de méthyle de formule 62, produit intermédiaire du procédé selon la revendication 28.

61. 2,3,4,6-tétra-O-acétyl-α-D-galactopyranoside de (4-bromométhyl 2-nitro)-phényle de formule 73, produit intermédiaire du procédé selon la revendication 28.

62. 2,3,4,6-tétra-O-acétyl-α-D-galactopyranoside de (4-hydroxyméthyl 2-nitro)-phényle de formule 74, produit intermédiaire du procédé selon la revendication 28.

63. 2,3,4-tri-O-acétyl-β-D-méthylglucuronide de 4-hydroxy-3-chlorobenzaldéhyde de formule 76, produit intermédiaire du procédé selon la revendication 28.

64. 2,3,4-tri-O-acétyl-β-D-méthylglucuronide de (4-hydroxy-méthyl-2-chloro)-phényle de formule 77, produit intermédiaire du procédé selon la revendication 28.

65. Carbonate de 2,5-dioxopyrrolidin-1-yle et de 4-(2,3,4,6-tétra-O-acétyl-α-D-galactopyranosyl)benzyle de formule 5, produit intermédiaire du procédé selon la revendication 28.

66. Carbonate de 2,5-dioxopyrrolidin-1-yle et de 2-(2,3,4,6-tétra-O-acétyl-α-D-galactopyranosyl)benzyle de formule 12, produit intermédiaire du procédé selon la revendication 28.

67. Carbonate de 2,5-dioxopyrrolidin-1-yle et de 4-((2,3,4-tri-O-acétyl-β-D-glucopyranosyl) uronate de méthyle)-benzyle de formule 19, produit intermédiaire du procédé selon la revendication 28.

68. Carbonate de 4-nitro-phényle et de 2-((2,3,4-tri-O-acétyl-β-D-glucopyranosyl) uronate de méthyle)-benzyle de formule 26, produit intermédiaire du procédé selon la revendication 28.

69. Carbonate de 4-nitro-phényle et de 2-(tertiobutyldiméthylsilyloxy)-benzyle de formule 30, produit intermédiaire du procédé selon la revendication 28.

70. Carbonate de 2,5-dioxopyrrolidin-1-yle et de 4-(2,3,4,6-tétra-O-acétyle-β-D-glucopyranosyl)-benzyle de formule 35, produit intermédiaire du procédé selon la revendication 28.

71. Carbonate de 2,5-dioxopyrrolidin-1-yle et de 4-diméthylthexyl-silyloxybenzyle de formule 40, produit intermédiaire du procédé selon la revendication 28.

72. Carbonate de 4-nitro-phényle et de 2-(2,3,4,6-tétra-O-acétyl-α-D-galactopyranosyl)-5-nitrobenzyle de formule 47, produit intermédiaire du procédé selon la revendication 28.

73. Carbonate de 4-nitro-phényle et de 2-((2,3,4-tri-O-acétyl-β-D-glucopyranosyl) uronate de méthyle)-5-nitro-benzyle de formule 53, produit intermédiaire du procédé selon la revendication 28.

74. Carbonate de 4-nitro-phényle et de 4-méthoxy-5-nitro-2((2,3,4-tri-O-acétyl-β-D-glucopyranosyl) uronate de méthyle)-benzyle de formule 67, produit intermédiaire du procédé selon la revendication 28.

75. Carbonate de 4-nitro-phényle et de 4-(2,3,4-tri-O-acétyl-β-glucopyranosyl) uronate de méthyle-5-nitro-benzyle de formule 69, produit intermédiaire du procédé selon la revendication 28.

76. Carbonate de 4-chlorophényle et de 2-((2,3,4-tri-O-acétyl-β-D-glucopyranosyl) uronate de méthyle)-5-nitrobenzyle de formule 81, produit intermédiaire du procédé selon la revendication 28.

77. Application des prodrogues selon l'une quelconque des revendications 1 à 27, pour l'obtention d'un médicament destiné au traitement des maladies dans lesquelles des macrophages, granulocytes, thrombocytes ou cellules tumorales, activés, sont impliqués.

## Revendications (Revendications pour l'(les) Etat(s) contractant(s) suivant(s): ES, GR)

1. Procédé de préparation de prodrogues d'anthracyclines qui répondent à la formule I ci-après : 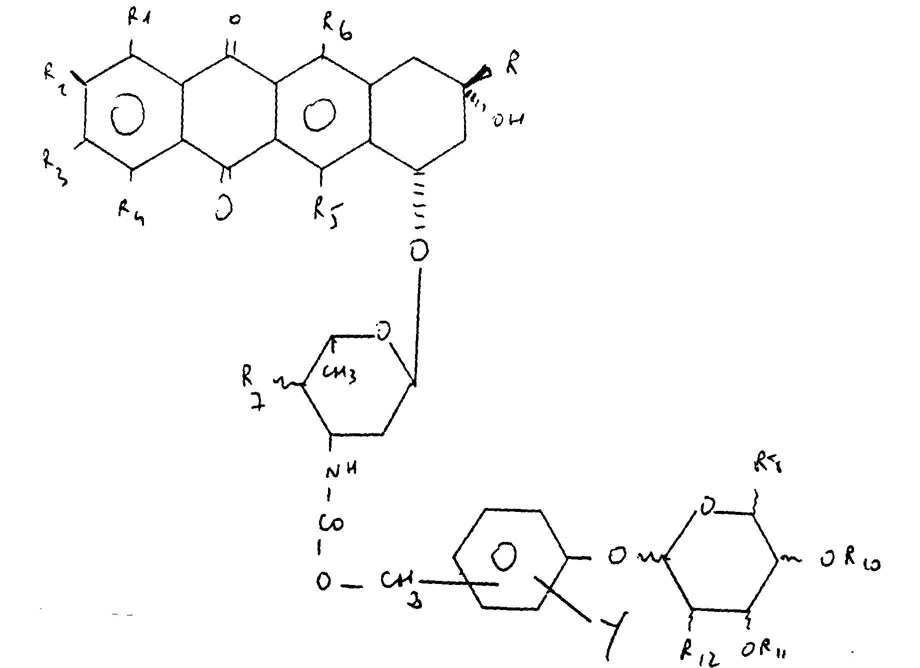 dans laquelle,
R₁, R₂, R₃, qui peuvent être identiques ou différents, représentent un atome d'hydrogène ou un groupe hydroxyle ;
R₄ représente un atome d'hydrogène, un groupe hydroxyle ou un groupe méthoxy ;
R représente un groupe CO-CH₂-R", dans lequel R" est un atome d'hydrogène, un groupe alkyle en C₁-C₆, un groupe hydroxyle, un groupe alkoxyle, un groupe O-acyle ou un groupe aryle ;
R₅ et R₆, qui peuvent être identiques ou différents, représentent un atome d'hydrogène ou un groupe hydroxyle ;
R₇ représente un atome d'hydrogène ou un groupe hydroxyle ;
R₈ représente un groupe -CH₂-OR₉ ou un groupe COOR₉ avec
R₉ étant un alkyle en C₁-C₃ ou un atome d'hydrogène ;
R₁₀ et R₁₁ représentent un atome d'hydrogène, un groupement acyle protecteur ou un groupe alkyle ;
R₁₂ représente un groupe hydroxyle, un groupement amine, un groupement amide ou un groupement O-acyle protecteur ; le -CH₂ benzylique est de préférence en position para ou ortho de l'oxygène glycosylé ;
Y représente un atome d'hydrogène, ou au moins un groupement électro-attracteur notamment choisi dans le groupe qui comprend le groupement NO₂, un atome d'halogène, un groupement SO₂X, (avec X = -CH₃, C₆H₄-CH₃, NH₂, N-(alkyle_{C1-C4})₂ NH-(alkyle_{C1-C4}), -CN, acyle ou COO-alkyle, et/ou au moins un groupement électrodonneur choisi dans le groupe qui comprend les groupements de type O-alkyle, NHCO-alkyle, N(alkyl)CO-alkyle, S-alkyle, alkyle,
qui peuvent notamment être dégradées par une glycosidase, caractérisé en ce qu'il comprend :
(1) le couplage, en présence, éventuellement, d'un promoteur approprié, d'un dérivé de formule A : 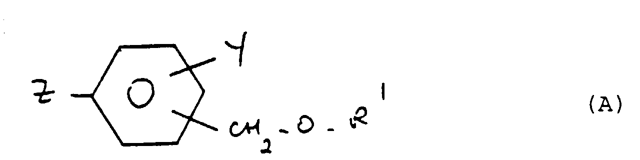 dans laquelle
Z représente un groupe hydroxyle, un groupe O-trialkylsilyle ou un groupe 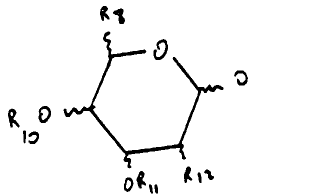 dans lequel R₈, R₁₀, R₁₁ et R₁₂ ont la même signification que ci-dessus ;
R' représente un atome d'hydrogène ou l'un des groupes suivants : 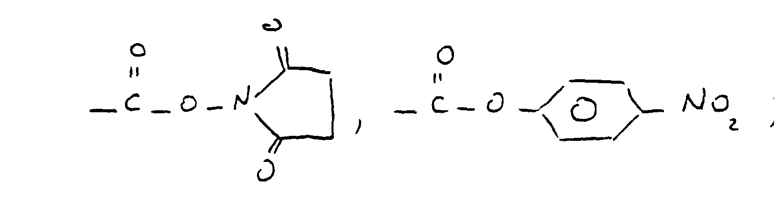 le -CH₂ benzylique est de préférence en position para ou ortho de la fonction phénol, éventuellement modifiée (glycosylée ou silylée) ;
Y représente un atome d'hydrogène, ou au moins un groupement électro-attracteur notamment choisi dans le groupe qui comprend le groupement NO₂, un atome d'halogène, un groupement SO₂x, (avec X = -CH₃, C₆H₄-CH₃, NH₂, N-(alkyle_{C1-C4})₂, NH-alkyle_{C1-C4}), -CN, acyle ou COO-alkyle, et/ou au moins un groupement électrodonneur choisi dans le groupe qui comprend les groupements de type O-alkyle, NH-CO-alkyle, N(alkyl)CO-alkyle, S-alkyle, alkyle avec une anthracycline de formule B 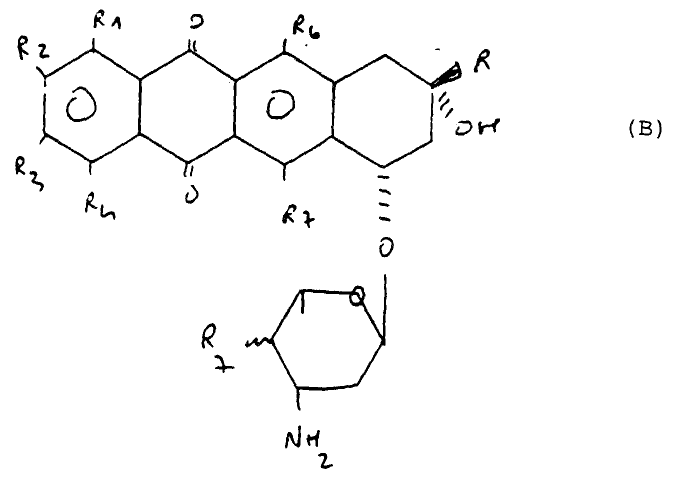 dans laquelle R₁, R₂, R₃, R₄, R₅, R₆, R₇ et R ont la même signification que ci-dessus,
(2) l'élimination des groupes protecteurs présents dans les composés obtenus, notamment par hydrolyse, transestérification ou saponification, et
(3) éventuellement, la condensation appropriée avec un ose de formule suivante : 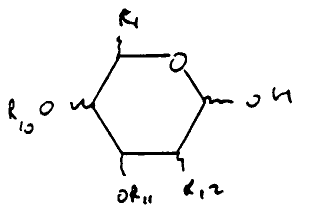 dans le cas où Z représente un groupement hydroxyle ou un groupe O-trialkylsilyle,
pour donner une prodrogue d'anthracycline de formule I, dans laquelle tous les radicaux R₁ à R₁₂ et R ont la même signification que précédemment.

2. Procédé de préparation de prodrogues selon la revendication 1, caractérisé en ce que lorsque Y représente un/des groupements électroattracteurs, ceux-ci sont de préférence en position ortho et/ou para de l'oxygène glycosylé et lorsque Y représente un/des groupements électrodonneurs, ceux-ci sont de préférence en position méta.

3. Procédé de préparation de prodrogues selon la revendication 1 ou la revendication 2, caractérisé en les composés préférés de formule I comprennent notamment les radicaux suivants :
R₁, R₂, R₃ sont des atomes d'hydrogène,
R₄ est un groupe méthoxy,
R₅ et R₆ sont des groupes hydroxyles,
R est un groupe -CO-CH₃ ou un groupe -CO-CH₂OH,
R₇ est un atome d'hydrogène, ou un groupe hydroxyle,
R₈ est un groupe -CH₂-OAc, -CH₂OH, -COOMe, ou -COOH,
R₁₀ et R₁₁, qui peuvent être identiques ou différents représentent un atome d'hydrogène ou un groupement Ac,
R₁₂ représente un groupe hydroxyle ou un groupe OAc, lesquels radicaux R₈, R₁₀, R₁₁ et R₁₂ ont de préférence les positions suivantes : 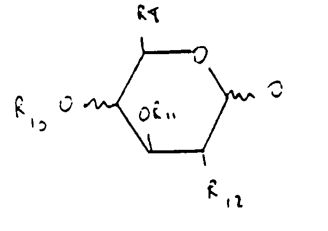
Y est un atome d'hydrogène, un groupe NO₂ ou un atome de chlore, en para ou en ortho de l'oxygène glycosylé ou un groupe OCH₃ en méta de l'oxygène glycosylé.

4. Procédé de préparation d'une prodrogue selon l'une quelconque des revendications 1 à 3, caractérisé en ce que ladite prodrogue présente la formule 6 ci-après :

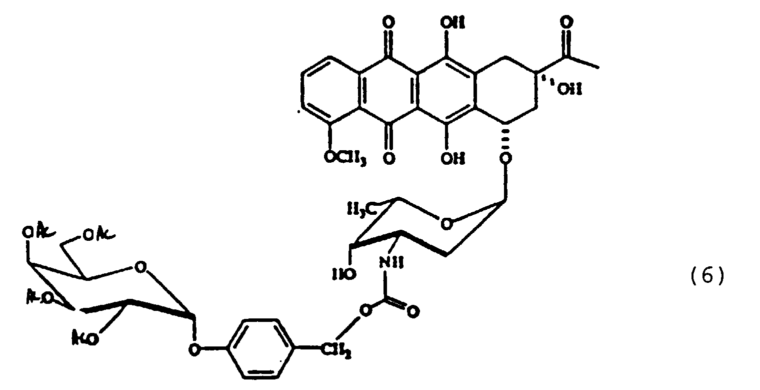

5. Procédé de préparation d'une prodrogue selon l'une quelconque des revendications 1 à 3, caractérisé en ce que ladite prodrogue présente la formule 7 ci-après :

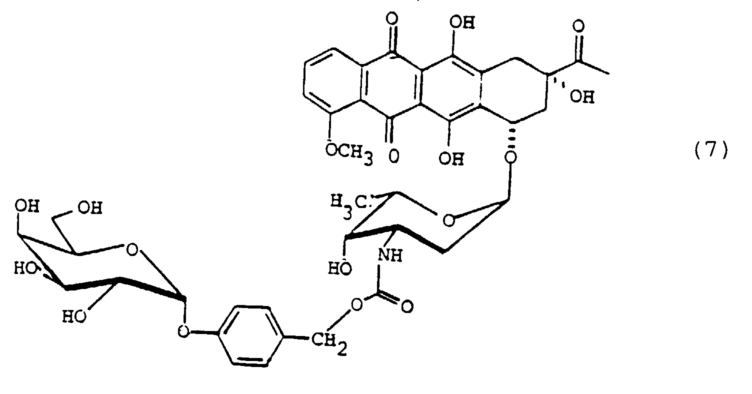

6. Procédé de préparation d'une prodrogue selon l'une quelconque des revendications 1 à 3, caractérisé en ce que ladite prodrogue présente la formule 13 ci-après :

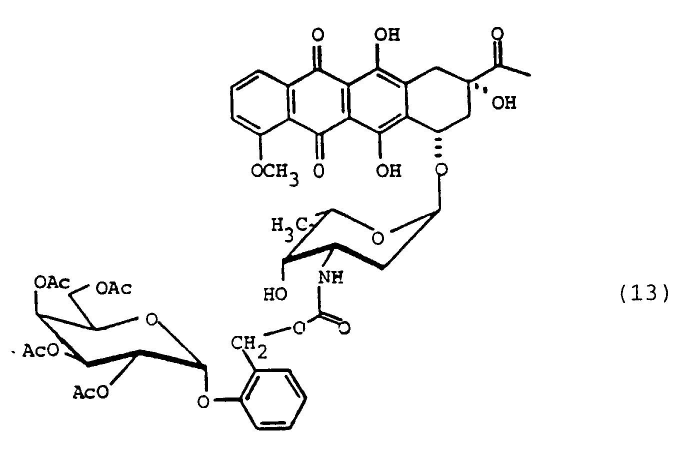

7. Procédé de préparation d'une prodrogue selon l'une quelconque des revendications 1 à 3, caractérisé en ce que ladite prodrogue présente la formule 14 ci-après :

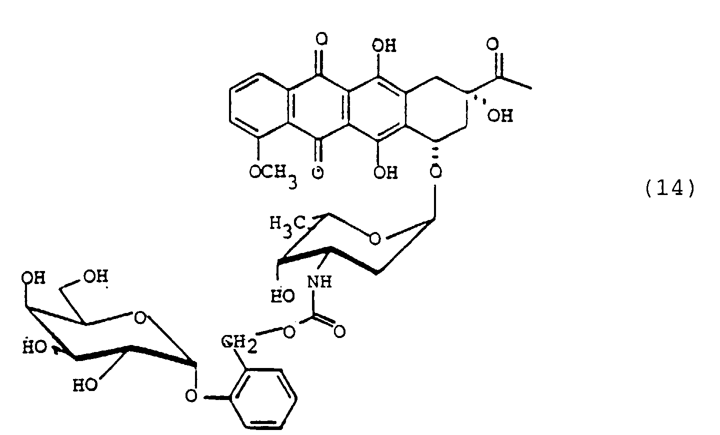

8. Procédé de préparation d'une prodrogue selon l'une quelconque des revendications 1 à 3, caractérisé en ce que ladite prodrogue présente la formule 22 ci-après :

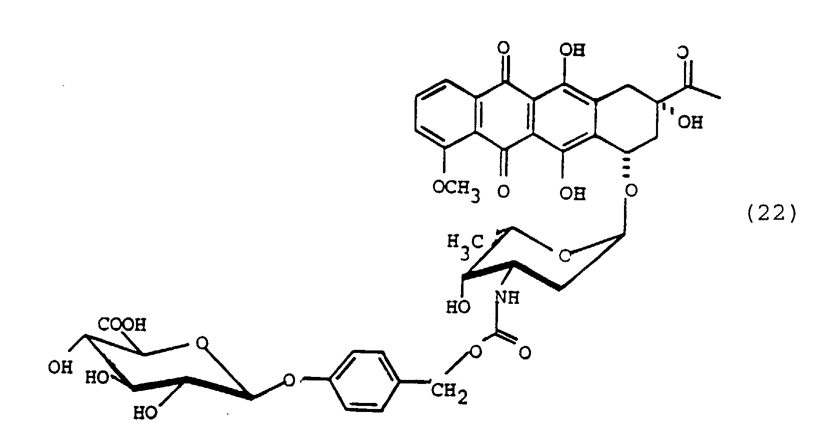

9. Procédé de préparation d'une prodrogue selon l'une quelconque des revendications 1 à 3, caractérisé en ce que ladite prodrogue présente la formule 27c ci-après :

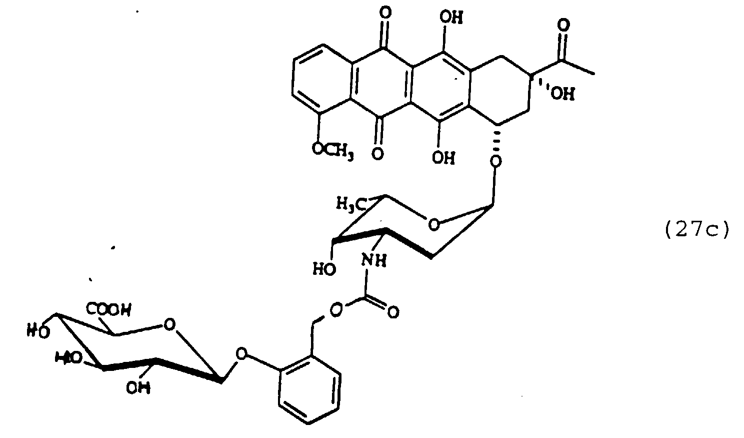

10. Procédé de préparation d'une prodrogue selon l'une quelconque des revendications 1 à 3, caractérisé en ce que ladite prodrogue présente la formule 36 ci-après :

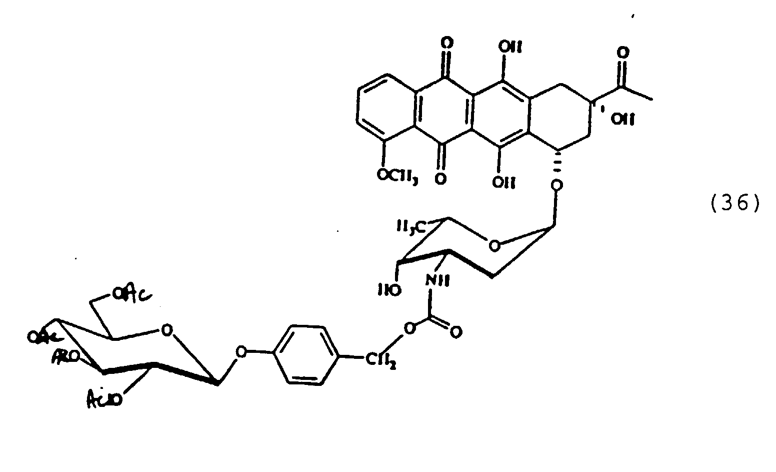

11. Procédé de préparation d'une prodrogue selon l'une quelconque des revendications 1 à 3, caractérisé en ce que ladite prodrogue présente la formule 37 ci-après :

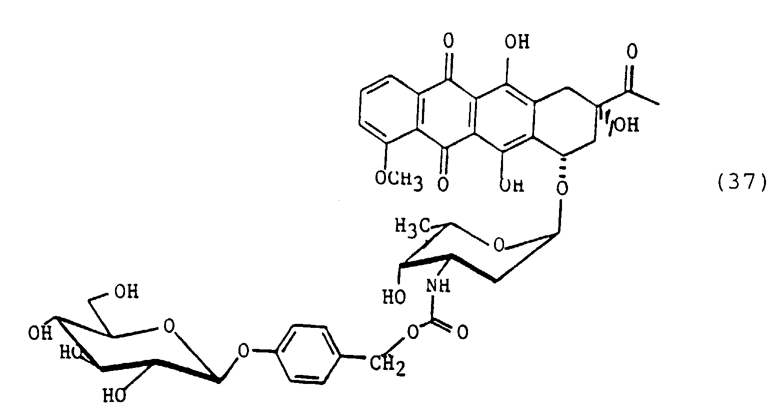

12. Procédé de préparation d'une prodrogue selon l'une quelconque des revendications 1 à 3, caractérisé en ce que ladite prodrogue présente la formule 48a ci-après :

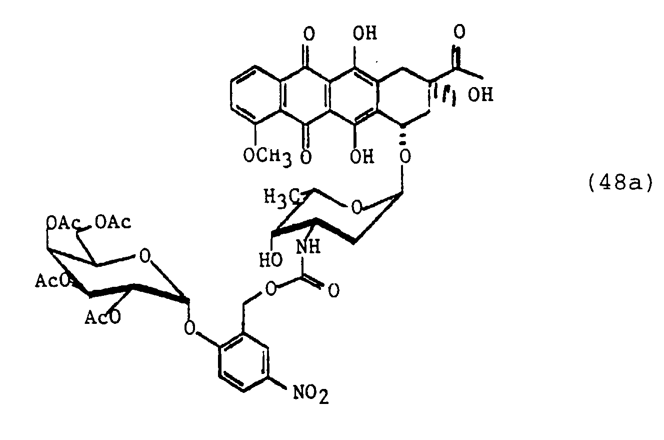

13. Procédé de préparation d'une prodrogue selon l'une quelconque des revendications 1 à 3, caractérisé en ce que ladite prodrogue présente la formule 48b ci-après :

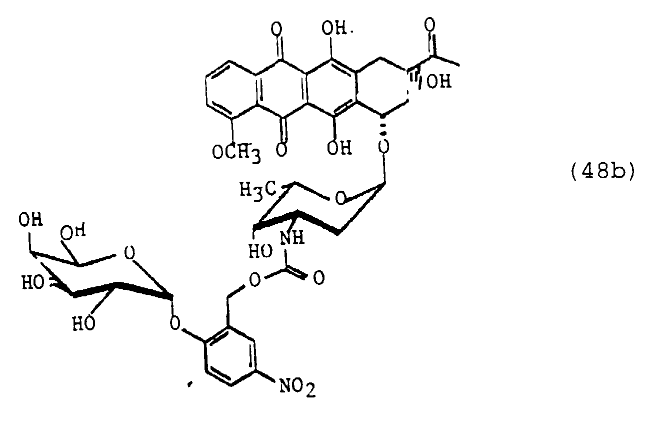

14. Procédé de préparation d'une prodrogue selon l'une quelconque des revendications 1 à 3, caractérisé en ce que ladite prodrogue présente la formule 49a ci-après :

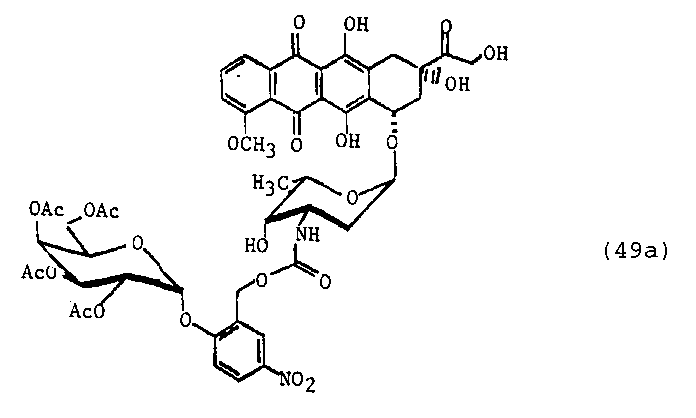

15. Procédé de préparation d'une prodrogue selon l'une quelconque des revendications 1 à 3, caractérisé en ce que ladite prodrogue présente la formule 49b ci-après :

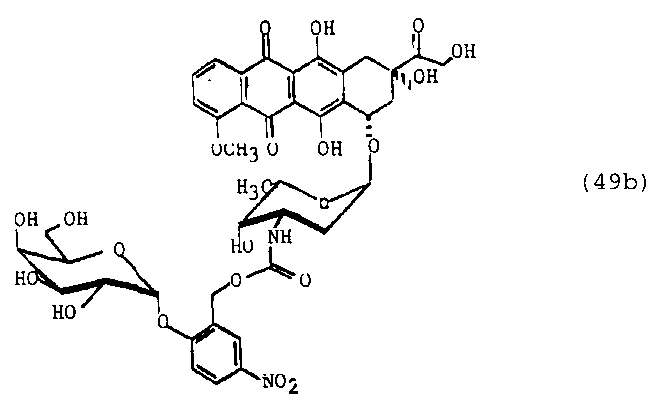

16. Procédé de préparation d'une prodrogue selon l'une quelconque des revendications 1 à 3, caractérisé en ce que ladite prodrogue présente la formule 54c ci-après :

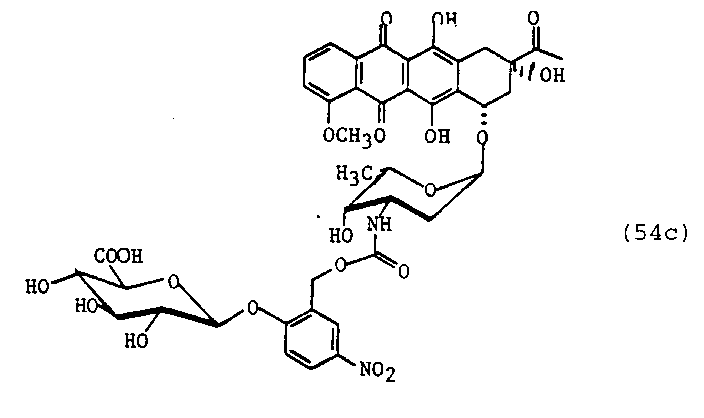

17. Procédé de préparation d'une prodrogue selon l'une quelconque des revendications 1 à 3, caractérisé en ce que ladite prodrogue présente la formule 59 ci-après :

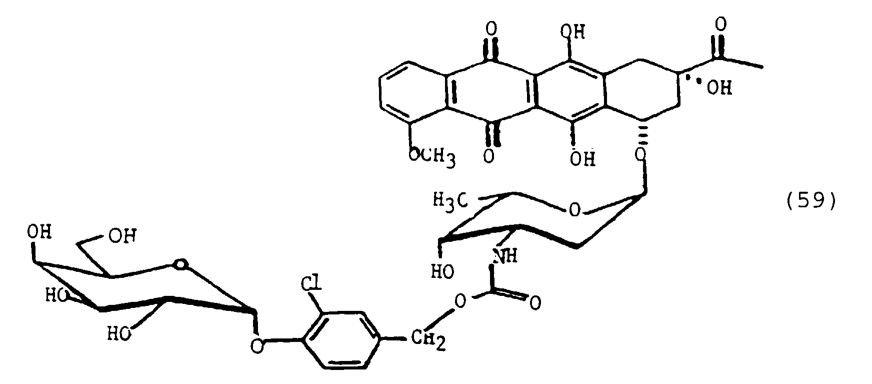

18. Procédé de préparation d'une prodrogue selon l'une quelconque des revendications 1 à 3, caractérisé en ce que ladite prodrogue présente la formule 60 ci-après :

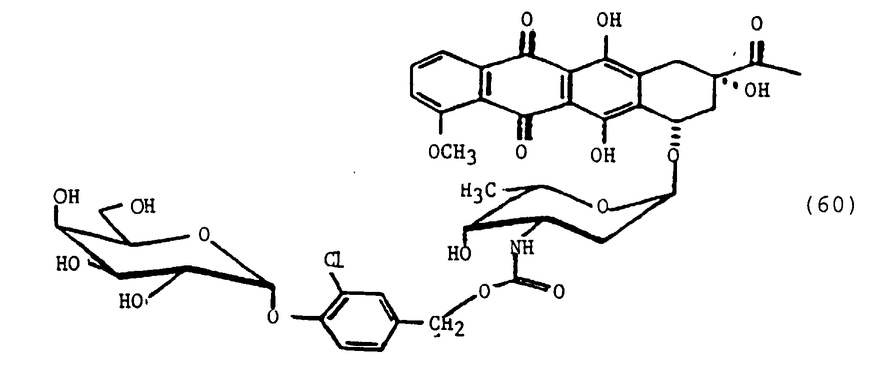

19. Procédé de préparation d'une prodrogue selon l'une quelconque des revendications 1 à 3, caractérisé en ce que ladite prodrogue présente la formule 64c ci-après :

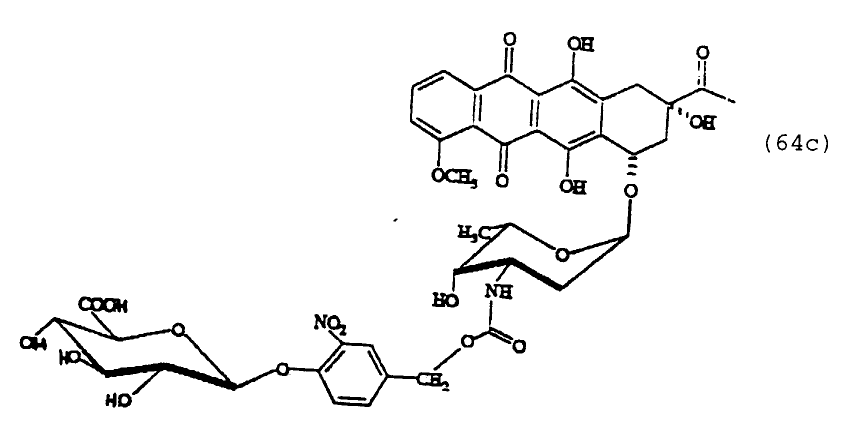

20. Procédé de préparation d'une prodrogue selon l'une quelconque des revendications 1 à 3, caractérisé en ce que ladite prodrogue présente la formule 68c ci-après :

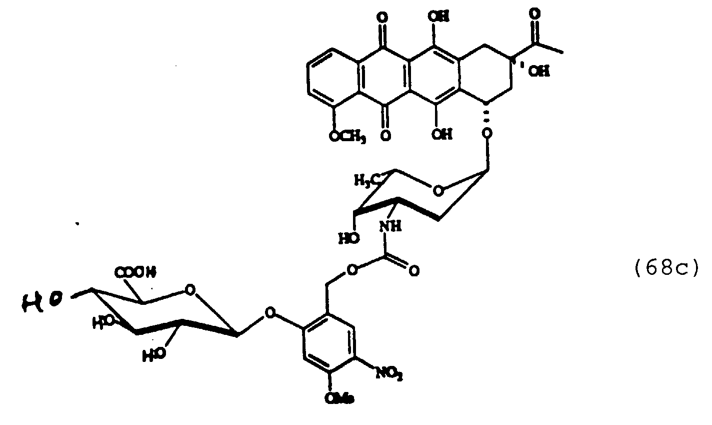

21. Procédé de préparation d'une prodrogue selon l'une quelconque des revendications 1 à 3, caractérisé en ce que ladite prodrogue présente la formule 75a ci-après :

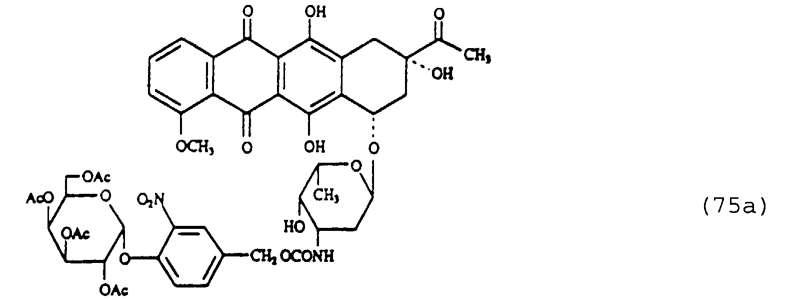

22. Procédé de préparation d'une prodrogue selon l'une quelconque des revendications 1 à 3, caractérisé en ce que ladite prodrogue présente la formule 75b ci-après :

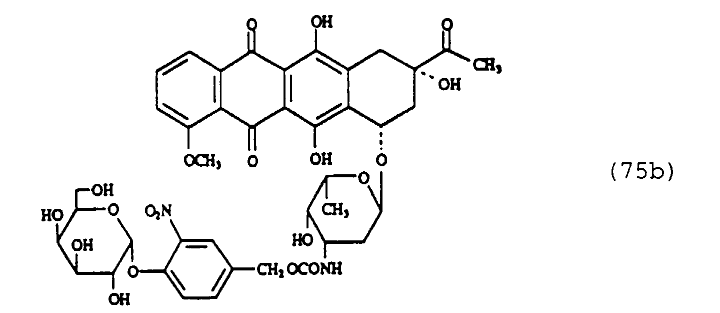

23. Procédé de préparation d'une prodrogue selon l'une quelconque des revendications 1 à 3, caractérisé en ce que ladite prodrogue présente la formule 78a ci-après :

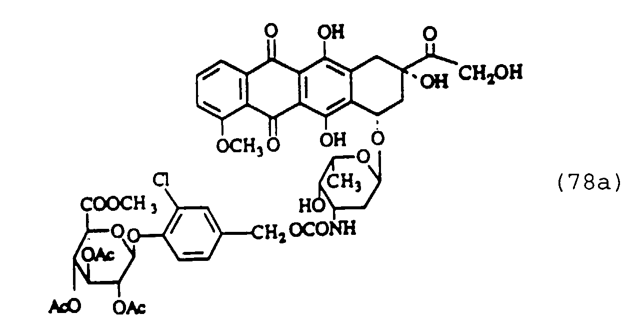

24. Procédé de préparation d'une prodrogue selon l'une quelconque des revendications 1 à 3, caractérisé en ce que ladite prodrogue présente la formule 78b ci-après :

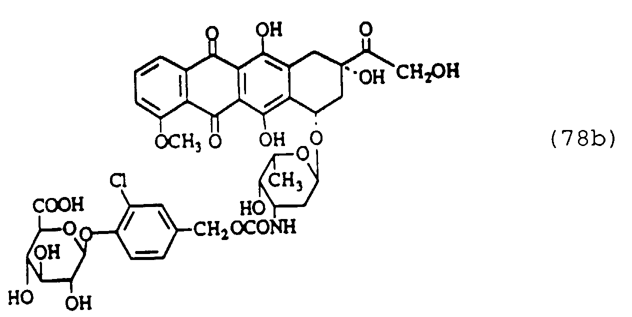

25. Procédé de préparation d'une prodrogue selon l'une quelconque des revendications 1 à 3, caractérisé en ce que ladite prodrogue présente la formule 70c ci-après :

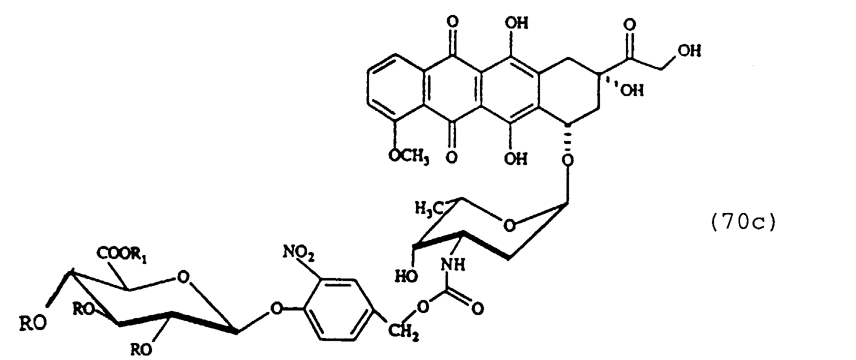

26. Procédé de préparation d'une prodrogue selon l'une quelconque des revendications 1 à 3, caractérisé en ce que ladite prodrogue présente la formule 82 ci-après : 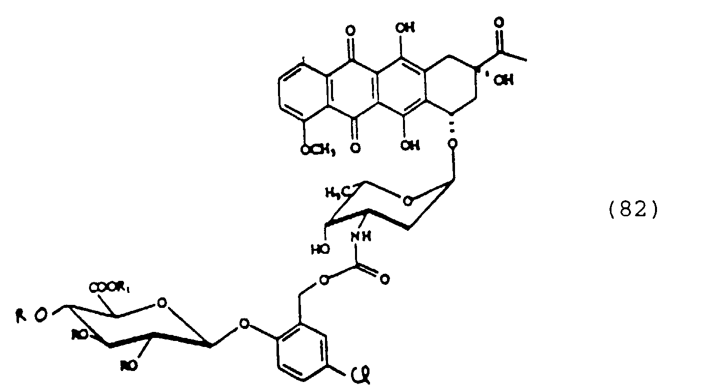 avec :
82a : R = Ac , R₁ = CH₃
82b : R = H , R₁ = CH₃
82c : R = R-₁ = H

27. Procédé de préparation d'une prodrogue selon l'une quelconque des revendications 1 à 3, caractérisé en ce que ladite prodrogue présente la formule 83 ci-après : 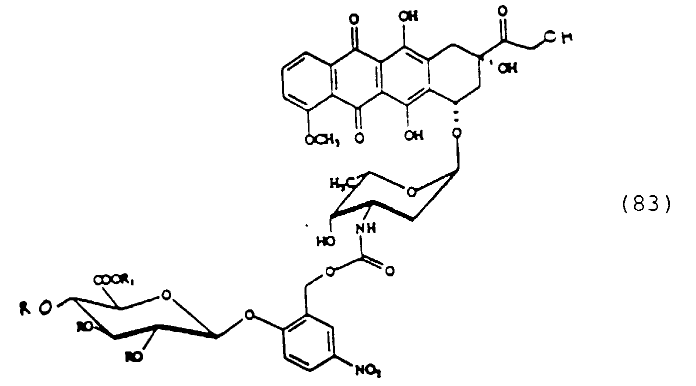 avec :
83a : R = Ac , R₁ = CH₃
83b : R = H , R₁ = CH₃
83c : R = R₁ = H

28. Procédé selon l'une quelconque des revendications 1 à 27, caractérisé en ce que préalablement à l'étape (1), le dérivé p-hydroxybenzyle glycosylé est obtenu par :
(a) fusion d'un crésol avec un ose ou un glucuronate de méthyle peracétylé,
(b) bromation benzylique du produit obtenu,
(c) solvolyse du dérivé bromé, et
(d) activation du groupe hydroxyle par un dérivé hydroxysuccinimidyle ou paranitrophénoxy-carbonyle.

29. Procédé de préparation de produits comprenant une prodrogue d'anthracycline modifiée telle que définie à l'une quelconque des revendications 1 à 27, caractérisé en ce qu'il comprend l'association d'une de ces prodrogues et d'un conjugué enzyme-anticorps spécifique vis-à-vis d'une tumeur, de formule II
Ac-Sp-E, (II)
dans laquelle :
Ac est un anticorps ou l'un de ses fragments, qui présente une spécificité vis-à-vis d'un antigène associé à une tumeur, ou est une biomolécule qui a tendance à s'accumuler dans une tumeur, tel que l'EGF (facteur de croissance épidermique), l'α-TGF (facteur de croissance transformant α), le PDGF (facteur de croissance dérivé des plaquettes), l'IGF I+II (facteur de croissance de l'insuline I+II) ou le FGF a+b (facteur de croissance fibroblastiques a+b),
E représente une glycosidase qui n'est pas immunogène ou présente une immunogénicité très faible, de préférence une glycosidase de mammifère, telle que l'α ou la β-glucosidase, l'α-galactosidase, l'α ou la β-mannosidase, l'α-fucosidase, la N-acétyl-α-galactosaminidase, la N-acétyl-β-/N-acétyl-α-glucosaminidase ou la β-glucuronidase,
Sp (Bras) représente un groupe contenant un sulfure ou un disulfure de formule III ou IV
X'(S)ₙ Y' (III)
X'(S)ₙ (IV),
ou un bras polypeptidique, dans lequel
X' ou Y' représente -CO-R₁₃-(N-succinimido)- ou -C (=R₁₄)-CH₂-CH₂-
avec R₁₃ étant un groupement -CH₂-CH₂, 1,4-cyclohexylidène, 1,3 ou 1,4 phénylène ou méthoxycarbonyle ou chloro-1,4-phénylène
et R₁₄ étant un atome d'oxygène ou un groupe NH, et de plus,
Y' représente -C(=R₁₄)-CH₂-CH₂, lorsque R₁₄ a la signification précisée ci-dessus et
n représente 1 ou 2,
pour une utilisation simultanée, séparée ou étalée dans le temps, en thérapie cytostatique.

30. Procédé de préparation selon l'une quelconque des revendications 1 à 27, caractérisé en ce qu'il met en oeuvre, en tant que produit intermédiaire, le 2,3,4,6-tétra-O-acétyl-α-D-galactopyranoside de 4-bromométhyl phényle de formule 2.

31. Procédé de préparation selon l'une quelconque des revendications 1 à 27, caractérisé en ce qu'il met en oeuvre, en tant que produit intermédiaire, le 2,3,4,6-tétra-O-acétyl-α-D-galactopyranoside de 4-formyl phényle de formule 3.

32. Procédé de préparation selon l'une quelconque des revendications 1 à 27, caractérisé en ce qu'il met en oeuvre, en tant que produit intermédiaire, le 2,3,4,6-Tétra-O-acétyl-α-D-galactopyranoside de 4-hydroxyméthyl phényle de formule 4.

33. Procédé de préparation selon l'une quelconque des revendications 1 à 27, caractérisé en ce qu'il met en oeuvre, en tant que produit intermédiaire, le 2,3,4,6-tétra-O-acétyl-α-D-galactopyranoside de 2-bromométhyl phényle de formule 9.

34. Procédé de préparation selon l'une quelconque des revendications 1 à 27, caractérisé en ce qu'il met en oeuvre, en tant que produit intermédiaire, le 2,3,4,6-tétra-O-acétyl-α-D-galactopyranoside de 2-formyl phényle de formule 10.

35. Procédé de préparation selon l'une quelconque des revendications 1 à 27, caractérisé en ce qu'il met en oeuvre, en tant que produit intermédiaire, le 2,3,4,6-tétra-O-acétyl-α-D-galactopyranoside de 2-hydroxyméthyl phényle de formule 11.

36. Procédé de préparation selon l'une quelconque des revendications 1 à 27, caractérisé en ce qu'il met en oeuvre, en tant que produit intermédiaire, le (2,3,4-tri-O-acétyl-β-D-glucopyranoside de 4-bromométhyl phényle) uronate de méthyle de formule 16.

37. Procédé de préparation selon l'une quelconque des revendications 1 à 27, caractérisé en ce qu'il met en oeuvre, en tant que produit intermédiaire, le (2,3,4-tri-O-acétyl-β-D-glucopyranoside de 4-formylphényle) uronate de méthyle de formule 17.

38. Procédé de préparation selon l'une quelconque des revendications 1 à 27, caractérisé en ce qu'il met en oeuvre, en tant que produit intermédiaire, le (2,3,4-tri-O-acétyl-β-D-glucopyranoside de 4-hydroxyméthyl phényle) uronate de méthyle de formule 18.

39. Procédé de préparation selon l'une quelconque des revendications 1 à 27, caractérisé en ce qu'il met en oeuvre, en tant que produit intermédiaire, le (2,3,4-tri-O-acétyl-β-D-glucopyranoside de 2-bromométhyl phényl) uronate de méthyle de formule 23.

40. Procédé de préparation selon l'une quelconque des revendications 1 à 27, caractérisé en ce qu'il met en oeuvre, en tant que produit intermédiaire, le (2,3,4-tri-O-acétyl-β-D-glucopyranoside de 2-hydroxyméthyl phényl) uronate de méthyle de formule 24.

41. Procédé de préparation selon l'une quelconque des revendications 1 à 27, caractérisé en ce qu'il met en oeuvre, en tant que produit intermédiaire, le (2,3,4-tri-O-acétyl-β-D-glucopyranoside de 2-formyl phényl) uronate de méthyle de formule 25.

42. Procédé de préparation selon l'une quelconque des revendications 1 à 27, caractérisé en ce qu'il met en oeuvre, en tant que produit intermédiaire, le 2-tertiobutyldiméthylsilyloxy-benzaldéhyde de formule 28.

43. Procédé de préparation selon l'une quelconque des revendications 1 à 27, caractérisé en ce qu'il met en oeuvre, en tant que produit intermédiaire, l'alcool 2-tertiobutyldiméthylsilyloxy-benzylique de formule 29.

44. Procédé de préparation selon l'une quelconque des revendications 1 à 27, caractérisé en ce qu'il met en oeuvre, en tant que produit intermédiaire, le N-[2-hydroxy-benzyloxycarbonyl] daunorubicine de formule 32.

45. Procédé de préparation selon l'une quelconque des revendications 1 à 27, caractérisé en ce qu'il met en oeuvre, en tant que produit intermédiaire, le 2,3,4,6-tétra-O-acétyl-β-D-glucopyranoside de 4-formyl phényle de formule 33.

46. Procédé de préparation selon l'une quelconque des revendications 1 à 27, caractérisé en ce qu'il met en oeuvre, en tant que produit intermédiaire, le 2,3,4,6-tétra-O-acétyl-β-D-glucopyranoside de 4-hydroxyméthyl phényle de formule 34.

47. Procédé de préparation selon l'une quelconque des revendications 1 à 27, caractérisé en ce qu'il met en oeuvre, en tant que produit intermédiaire, le N-[4-hydroxy-benzyloxycarbonyl] daunorubicine de formule 38.

48. Procédé de préparation selon l'une quelconque des revendications 1 à 27, caractérisé en ce qu'il met en oeuvre, en tant que produit intermédiaire, le 2,3,4,6-tétra-O-acétyl-α-D-galactopyranoside de 2-méthyl 4-nitro phényle de formule 42.

49. Procédé de préparation selon l'une quelconque des revendications 1 à 27, caractérisé en ce qu'il met en oeuvre, en tant que produit intermédiaire, le 2,3,4,6-tétra-O-acétyl-α-D-galactopyranoside de 2-bromométhyl 4-nitro phényle de formule 43.

50. Procédé de préparation selon l'une quelconque des revendications 1 à 27, caractérisé en ce qu'il met en oeuvre, en tant que produit intermédiaire, le 2,3,4,6-tétra-O-acétyl-α-D-galactopyranoside de 2-dibromométhyl 4-nitro phényle de formule 44.

51. Procédé de préparation selon l'une quelconque des revendications 1 à 27, caractérisé en ce qu'il met en oeuvre, en tant que produit intermédiaire, le 2,3,4,6-tétra-O-acétyl-α-D-galactopyranoside de 2-formyl 4-nitro phényle de formule 45.

52. Procédé de préparation selon l'une quelconque des revendications 1 à 27, caractérisé en ce qu'il met en oeuvre, en tant que produit intermédiaire, le 2,3,4,6-tétra-O-acétyl-α-galactopyranoside de (2-hydroxyméthyl-4-nitro) phényle de formule 46.

53. Procédé de préparation selon l'une quelconque des revendications 1 à 27, caractérisé en ce qu'il met en oeuvre, en tant que produit intermédiaire, le (2,3,4-tri-O-acétyl-β-D-glucopyranoside de 2-formyl 4-nitro phényle) uronate de méthyle de formule 51.

54. Procédé de préparation selon l'une quelconque des revendications 1 à 27, caractérisé en ce qu'il met en oeuvre, en tant que produit intermédiaire, le (2,3,4-tri-O-acétyl-β-D-glucopyranoside de 2-hydroxyméthyl 4-nitro-phényl)uronate de méthyle de formule 52.

55. Procédé de préparation selon l'une quelconque des revendications 1 à 27, caractérisé en ce qu'il met en oeuvre, en tant que produit intermédiaire, le 2,3,4,6-tétra-O-acétyl-α-D-galactopyranoside de 2-chloro-4-méthyl phényle de formule 55.

56. Procédé de préparation selon l'une quelconque des revendications 1 à 27, caractérisé en ce qu'il met en oeuvre, en tant que produit intermédiaire, le 2,3,4,6-tétra-O-acétyl-α-D-galactopyranoside de 2-chloro-4-bromométhyl phényle de formule 56.

57. Procédé de préparation selon l'une quelconque des revendications 1 à 27, caractérisé en ce qu'il met en oeuvre, en tant que produit intermédiaire, le 2,3,4,6-tétra-O-acétyl-α-D-galactopyranoside de 2-chloro-4-hydroxyméthyl phényle de formule 57.

58. Procédé de préparation selon l'une quelconque des revendications 1 à 27, caractérisé en ce qu'il met en oeuvre, en tant que produit intermédiaire, le (2,3,4-tri-O-acétyl-β-D-glucopyranoside de 4-formyl 2-nitro-phényle) uronate de méthyle de formule 61.

59. Procédé de préparation selon l'une quelconque des revendications 1 à 27, caractérisé en ce qu'il met en oeuvre, en tant que produit intermédiaire, le (2,3,4-tri-O-acétyl-β-D-glucopyranoside de 4-hydroxyméthyl-2-nitro phényle) uronate de méthyle de formule 62.

60. Procédé de préparation selon l'une quelconque des revendications 1 à 27, caractérisé en ce qu'il met en oeuvre, en tant que produit intermédiaire, le 2,3,4,6-tétra-O-acétyl-α-D-galactopyranoside de (4-bromométhyl 2-nitro)-phényle de formule 73.

61. Procédé de préparation selon l'une quelconque des revendications 1 à 27, caractérisé en ce qu'il met en oeuvre, en tant que produit intermédiaire, le 2,3,4,6-tétra-O-acétyl-α-D-galactopyranoside de (4-hydroxyméthyl 2-nitro)-phényle de formule 74.

62. Procédé de préparation selon l'une quelconque des revendications 1 à 27, caractérisé en ce qu'il met en oeuvre, en tant que produit intermédiaire, le 2,3,4-tri-O-acétyl-β-D-méthylglucuronide de 4-hydroxy-3-chlorobenzaldéhyde de formule 76.

63. Procédé de préparation selon l'une quelconque des revendications 1 à 27, caractérisé en ce qu'il met en oeuvre, en tant que produit intermédiaire, le 2,3,4-tri-O-acétyl-β-D-méthylglucuronide de (4-hydroxy-méthyl-2-chloro)-phényle de formule 77.

64. Procédé de préparation selon l'une quelconque des revendications 1 à 27, caractérisé en ce qu'il met en oeuvre, en tant que produit intermédiaire, le carbonate de 2,5-dioxopyrrolidin-1-yle et de 4-(2,3,4,6-tétra-O-acétyl-α-D-galactopyranosyl)-benzyle de formule 5.

65. Procédé de préparation selon l'une quelconque des revendications 1 à 27, caractérisé en ce qu'il met en oeuvre, en tant que produit intermédiaire, le carbonate de 2,5-dioxopyrrolidin-1-yle et de 2-(2,3,4,6-tétra-O-acétyl-α-D-galactopyranosyl)-benzyle de formule 12.

66. Procédé de préparation selon l'une quelconque des revendications 1 à 27, caractérisé en ce qu'il met en oeuvre, en tant que produit intermédiaire, le carbonate de 2,5-dioxopyrrolidin-1-yle et de 4-((2,3,4-tri-O-acétyl-β-D-glucopyranosyl) uronate de méthyle)-benzyle de formule 19.

67. Procédé de préparation selon l'une quelconque des revendications 1 à 27, caractérisé en ce qu'il met en oeuvre, en tant que produit intermédiaire, le carbonate de 4-nitro-phényle et de 2-((2,3,4-tri-O-acétyl-β-D-glucopyranosyl) uronate de méthyle)-benzyle de formule 26.

68. Procédé de préparation selon l'une quelconque des revendications 1 à 27, caractérisé en ce qu'il met en oeuvre, en tant que produit intermédiaire, le carbonate de 4-nitro-phényle et de 2-(tertiobutyldiméthylsilyloxy)-benzyle de formule 30.

69. Procédé de préparation selon l'une quelconque des revendications 1 à 27, caractérisé en ce qu'il met en oeuvre, en tant que produit intermédiaire, le carbonate de 2,5-dioxopyrrolidin-1-yle et de 4-(2,3,4,6-tétra-O-acétyle-β-D-glucopyranosyl)-benzyle de formule 35.

70. Procédé de préparation selon l'une quelconque des revendications 1 à 27, caractérisé en ce qu'il met en oeuvre, en tant que produit intermédiaire, le carbonate de 2,5-dioxopyrrolidin-1-yle et de 4-diméthylthexyl-silyloxybenzyle de formule 40.

71. Procédé de préparation selon l'une quelconque des revendications 1 à 27, caractérisé en ce qu'il met en oeuvre, en tant que produit intermédiaire, le carbonate de 4-nitro-phényle et de 2-(2,3,4,6-tétra-O-acétyl-α-D-galactopyranosyl)-5-nitro-benzyle de formule 47.

72. Procédé de préparation selon l'une quelconque des revendications 1 à 27, caractérisé en ce qu'il met en oeuvre, en tant que produit intermédiaire, le carbonate de 4-nitro-phényle et de 2-((2,3,4-tri-O-acétyl-β-D-glucopyranosyl) uronate de méthyle)-5-nitrobenzyle de formule 53.

73. Procédé de préparation selon l'une quelconque des revendications 1 à 27, caractérisé en ce qu'il met en oeuvre, en tant que produit intermédiaire, le carbonate de 4-nitro-phényle et de 4-méthoxy-5-nitro2((2,3,4-tri-O-acétyl-β-D-glucopyranosyl) uronate de méthyle)-benzyle de formule 67.

74. Procédé de préparation selon l'une quelconque des revendications 1 à 27, caractérisé en ce qu'il met en oeuvre, en tant que produit intermédiaire, le carbonate de 4-nitro-phényle et de 4-(2,3,4-tri-O-acétyl-β-glucopyranosyl) uronate de méthyle-5-nitrobenzyle de formule 69.

75. Procédé de préparation selon l'une quelconque des revendications 1 à 27, caractérisé en ce qu'il met en oeuvre, en tant que produit intermédiaire, le carbonate de 4-chlorophényle et de 2-((2,3,4-tri-O-acétyl-β-D-glucopyranosyl) uronate de méthyle)-5-nitrobenzyle de formule 81.

76. Procédé de préparation d'un médicament destiné au traitement des maladies dans lesquelles des macrophages, granulocytes, thrombocytes ou cellules tumorales, activés, sont impliqués, caractérisé en ce qu'il met en oeuvre une prodrogue telle que définie à l'une quelconque des revendications 1 à 27.

## Patentansprüche (Patentansprüche für folgende(n) Vertragsstaat(en): AT, BE, CH, LI, DE, DK, FR, GB, IT, LU, NL, PT, SE)

1. Anthracyclin-Arzneimittelvorstufen (Anthracyclin-Pro-Drugs), dadurch **gekennzeichnet**, daß sie der nachstehenden Formel I entsprechen, 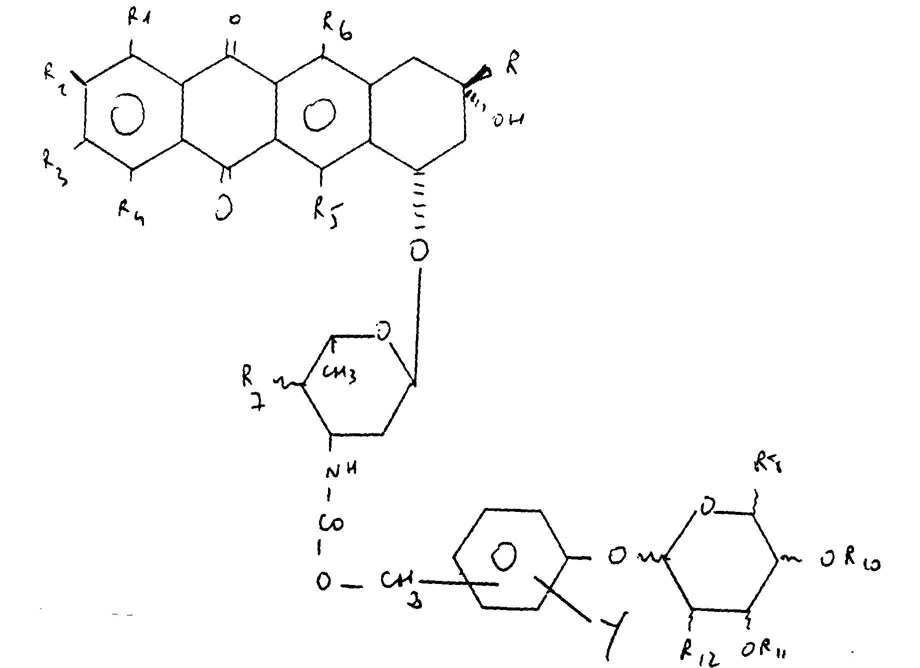 worin
R₁, R₂, R₃, die gleich oder verschieden sein können, ein Wasserstoffatom oder eine Hydroxylgruppe bedeuten,
R₄ ein Wasserstoffatom, eine Hydroxylgruppe oder eine Methoxygruppe bedeutet,
R einen Rest CO-CH₂-R" bedeutet, worin R" ein Wasserstoffatom, ein C₁-C₆-Alkylrest, eine Hydroxylgruppe, ein Alkoxylrest, ein O-Acylrest oder ein Arylrest ist,
R₅ und R₆, die gleich oder verschieden sein können, ein Wasserstoffatom oder eine Hydroxylgruppe bedeuten,
R₇ ein Wasserstoffatom oder eine Hydroxylgruppe bedeutet,
R₈ einen Rest -CH₂-OR₉ oder einen Rest COOR₉ bedeutet, worin R₉ einen C₁-C₃-Alkylrest oder ein Wasserstoffatom bedeutet,
R₁₀ und R₁₁ ein Wasserstoffatom, eine Acylschutzgruppe oder einen Alkylrest bedeuten,
R₁₂ eine Hydroxylgruppe, eine Amingruppe, eine Amidgruppe oder eine O-Acylschutzgruppe bedeutet,
das Benzyl-CH₂ bevorzugt in para- oder ortho-Position des Glycosylsauerstoffs steht,
Y ein Wasserstoffatom oder mindestens eine elektronenziehende Gruppierung, ausgewählt insbesondere aus der Gruppe, umfassend NO₂, ein Halogenatom, eine SO₂X-Gruppierung (wobei X = -CH₃, C₆H₄-CH₃, NH₂, N-(C₁-C₄-Alkyl)₂, NH-C₁-C₄-Alkyl), -CN, Acyl oder COO-Alkyl, und/oder mindestens eine elektronenspendende Gruppierung ausgewählt aus der Gruppe, umfassend Gruppierungen vom Typ O-Alkyl, NHCO-Alkyl, N(Alkyl)CO-Alkyl, S-Alkyl, Alkyl, ist.

2. Arzneimittelvorstufen nach Anspruch 1, dadurch **gekennzeichnet**, daß wenn Y eine elektronenziehende Gruppierung/Gruppierungen bedeutet, diese sich bevorzugt in ortho- und/oder para-Position des Glycosylsauerstoffs befinden, und wenn Y eine elektronenspendende Gruppierung/Gruppierungen bedeutet, sich diese bevorzugt in meta-Position befinden.

3. Arzneimittelvorstufen nach Anspruch 1 oder 2, dadurch **gekennzeichnet**, daß die bevorzugten Verbindungen der Formel I insbesondere die folgenden Reste umfassen:
R₁, R₂, R₃ sind Wasserstoffatome,
R₄ ist eine Methoxygruppe,
R₅ und R₆ sind Hydroxylgruppen,
R ist ein Rest -CO-CH₃ oder ein Rest -CO-CH₂OH,
R₇ ist ein Wasserstoffatom oder eine Hydroxylgruppe,
R₈ ist ein Rest -CH₂-OAc, -CH₂OH, -COOMe oder -COOH,
R₁₀ und R₁₁, die gleich oder verschieden sein können, sind Wasserstoffatome oder eine Ac-Gruppe,
R₁₂ ist eine Hydroxylgruppe oder eine OAc-Gruppe, wobei die Reste R₈, R₁₀, R₁₁ und R₁₂ bevorzugt die folgenden Positionen besitzen: 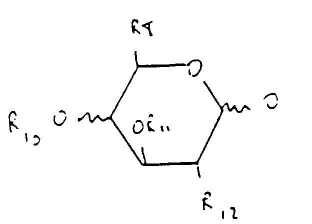
Y ist ein Wasserstoffatom, eine NO₂-Gruppe oder ein Chloratom in para- oder ortho-Position des Glycosylsauerstoffs, oder eine OCH₃-Gruppe in meta-Position des Glycosylsauerstoffs.

4. Arzneimittelvorstufe nach einem der Ansprüche 1 bis 3, dadurch **gekennzeichnet**, daß sie die nachstehende Formel 6 besitzt:

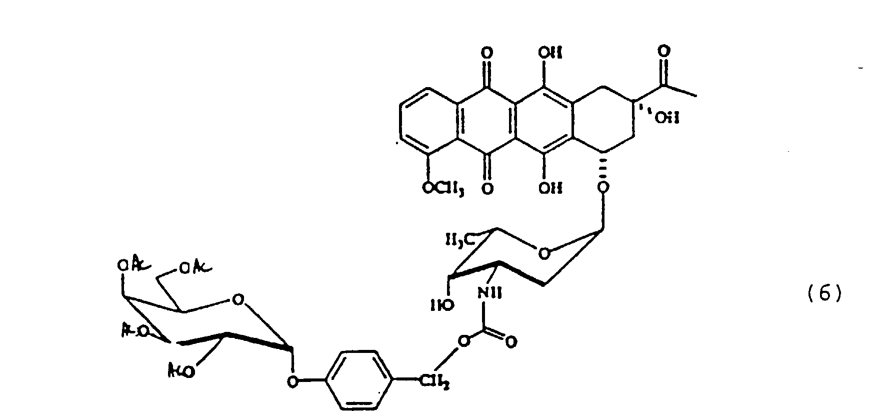

5. Arzneimittelvorstufe nach einem der Ansprüche 1 bis 3, dadurch **gekennzeichnet**, daß sie die nachstehende Formel 7 besitzt:

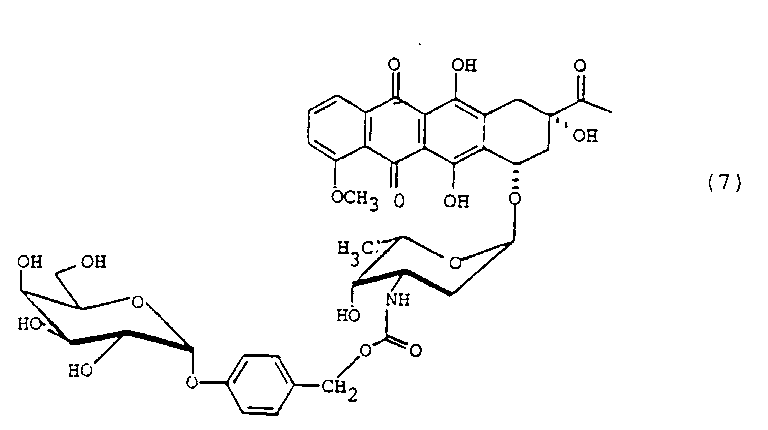

6. Arzneimittelvorstufe nach einem der Ansprüche 1 bis 3, dadurch **gekennzeichnet**, daß sie die nachstehende Formel 13 besitzt:

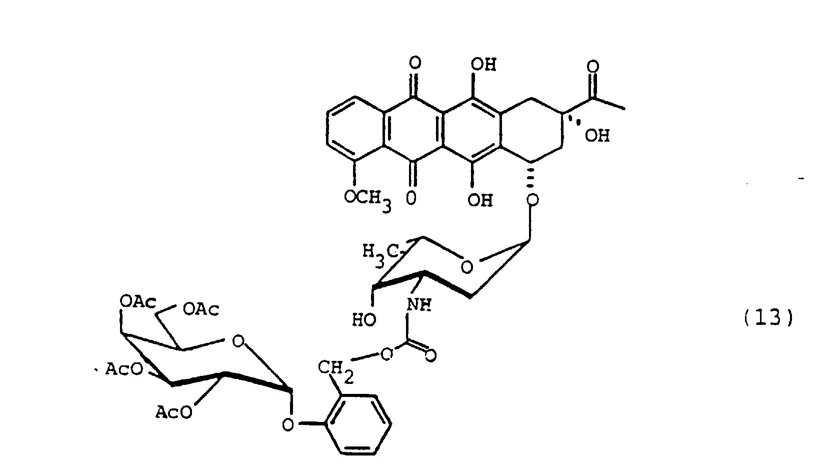

7. Arzneimittelvorstufe nach einem der Ansprüche 1 bis 3, dadurch **gekennzeichnet**, daß sie die nachstehende Formel 14 besitzt:

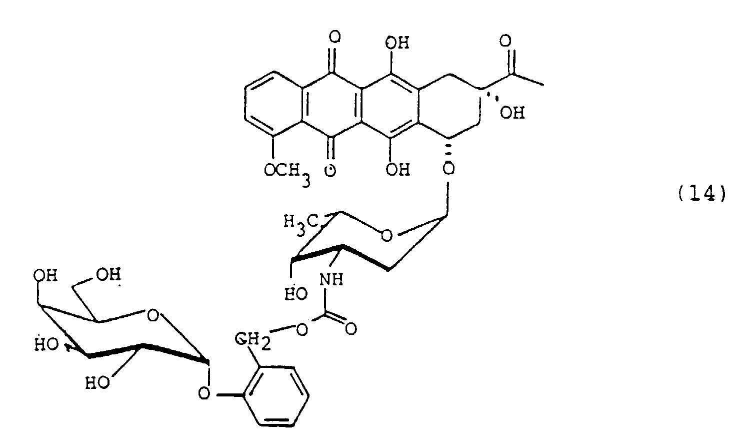

8. Arzneimittelvorstufe nach einem der Ansprüche 1 bis 3, dadurch **gekennzeichnet**, daß sie die nachstehende Formel 22 besitzt:

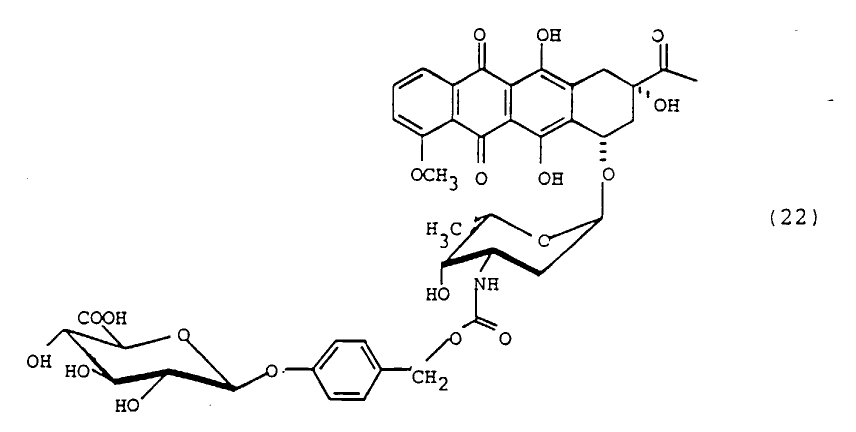

9. Arzneimittelvorstufe nach einem der Ansprüche 1 bis 3, dadurch **gekennzeichnet**, daß sie die nachstehende Formel 27c besitzt:

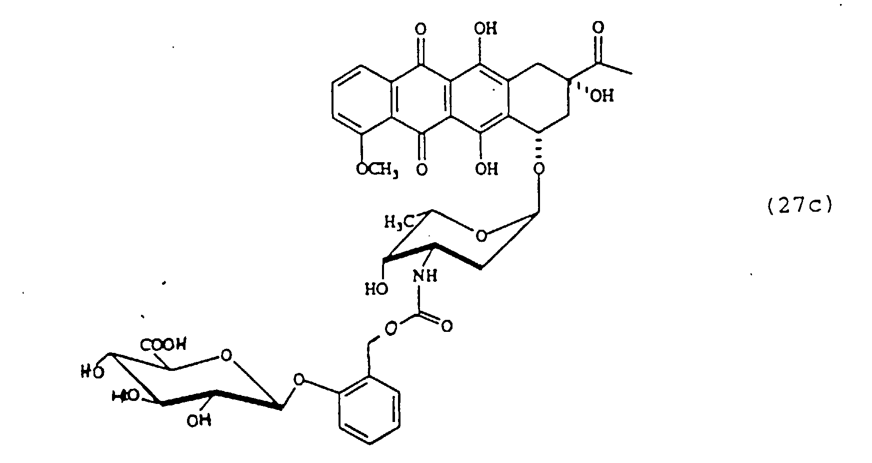

10. Arzneimittelvorstufe nach einem der Ansprüche 1 bis 3, dadurch **gekennzeichnet**, daß sie die nachstehende Formel 36 besitzt:

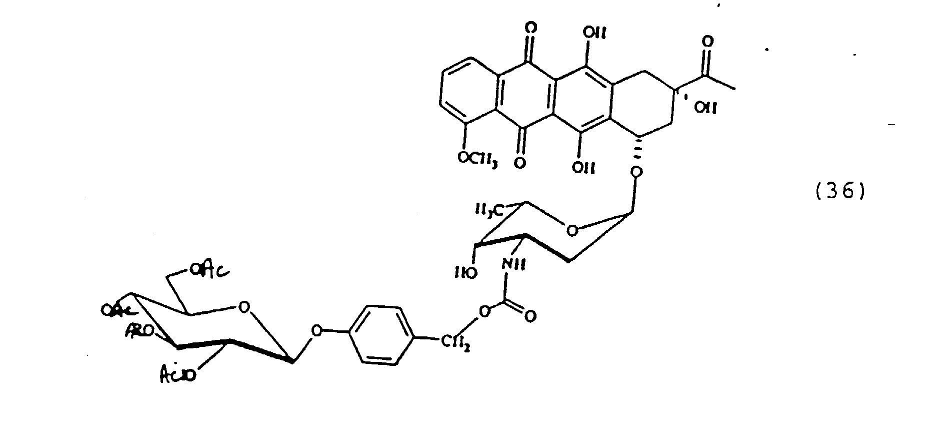

11. Arzneimittelvorstufe nach einem der Ansprüche 1 bis 3, dadurch **gekennzeichnet**, daß sie die nachstehende Formel 37 besitzt:

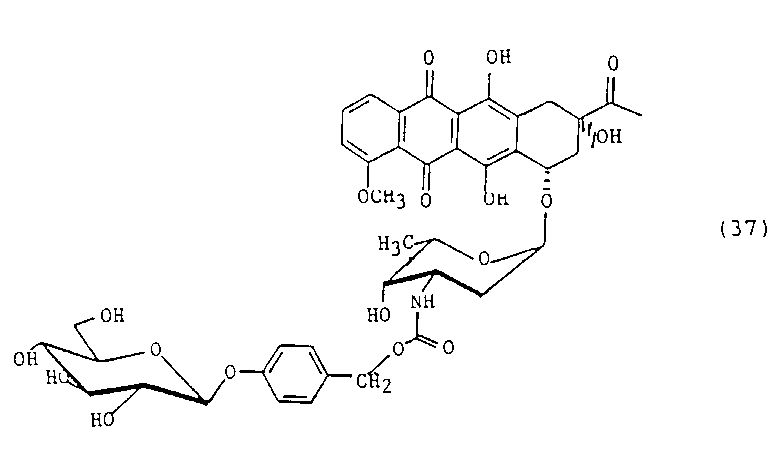

12. Arzneimittelvorstufe nach einem der Ansprüche 1 bis 3, dadurch **gekennzeichnet**, daß sie die nachstehende Formel 48a besitzt:

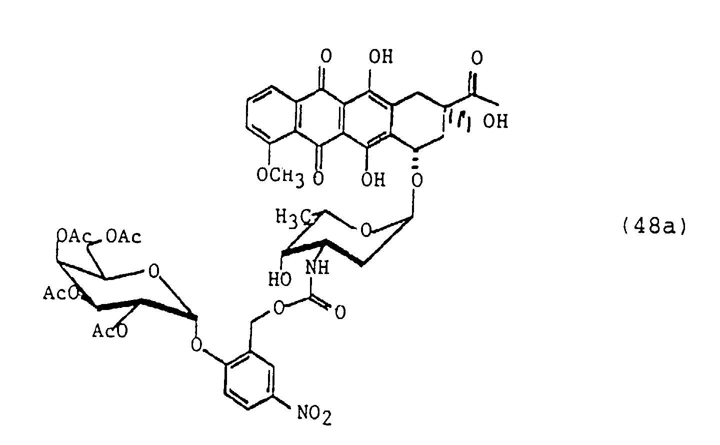

13. Arzneimittelvorstufe nach einem der Ansprüche 1 bis 3, dadurch **gekennzeichnet**, daß sie die nachstehende Formel 48b besitzt:

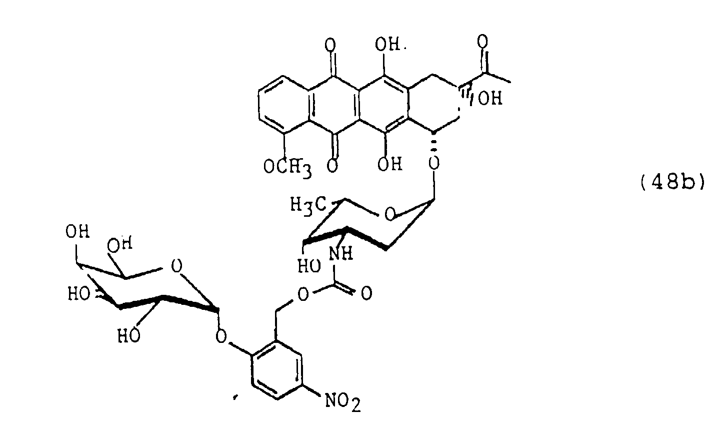

14. Arzneimittelvorstufe nach einem der Ansprüche 1 bis 3, dadurch **gekennzeichnet**, daß sie die nachstehende Formel 49a besitzt:

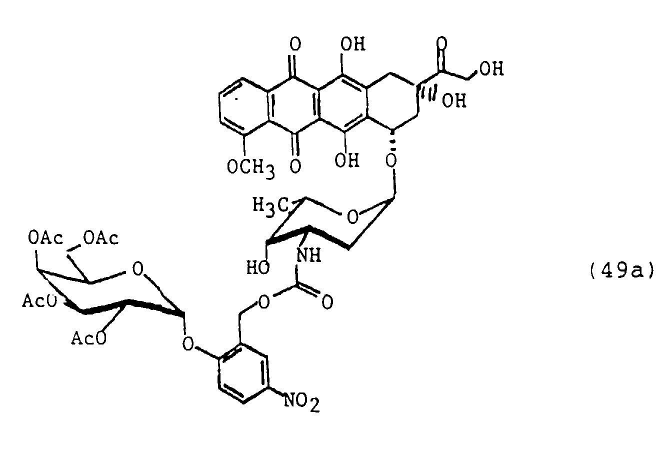

15. Arzneimittelvorstufe nach einem der Ansprüche 1 bis 3, dadurch **gekennzeichnet**, daß sie die nachstehende Formel 49b besitzt:

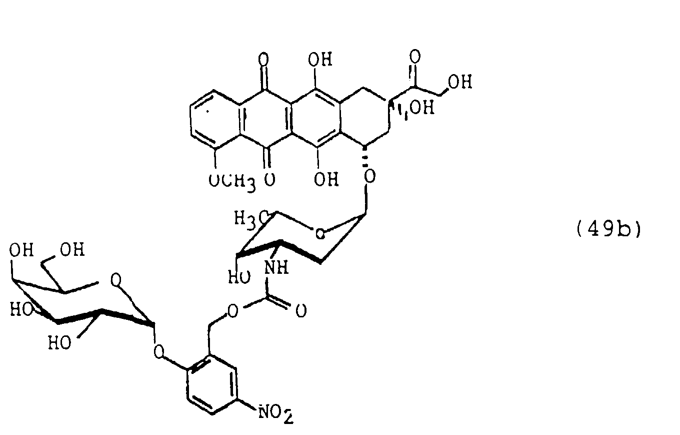

16. Arzneimittelvorstufe nach einem der Ansprüche 1 bis 3, dadurch **gekennzeichnet**, daß sie die nachstehende Formel 54c besitzt:

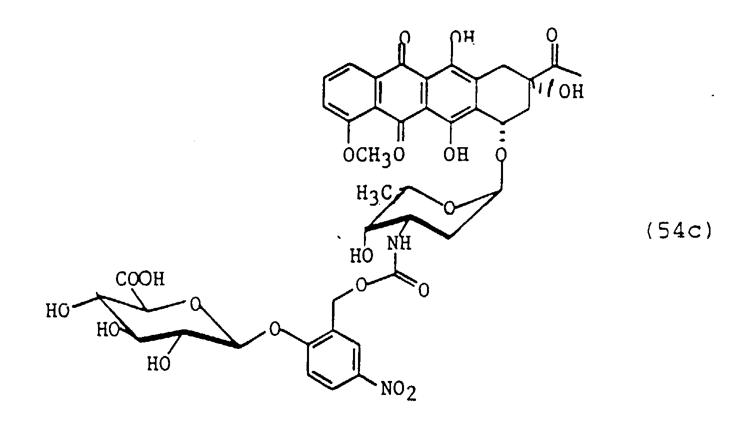

17. Arzneimittelvorstufe nach einem der Ansprüche 1 bis 3, dadurch **gekennzeichnet**, daß sie die nachstehende Formel 59 besitzt:

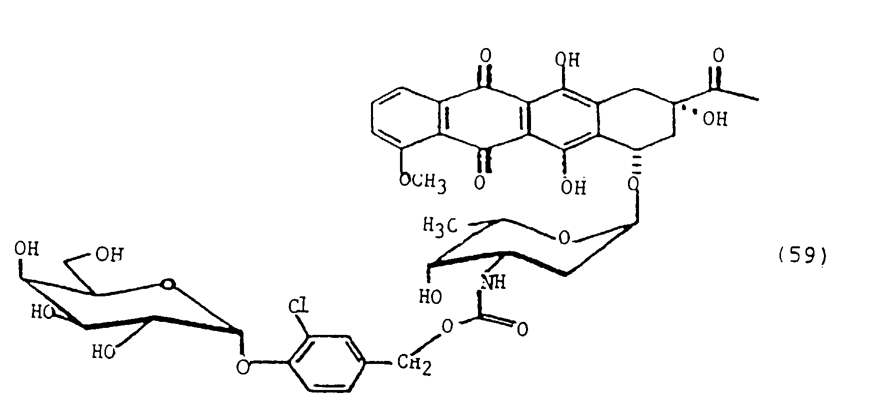

18. Arzneimittelvorstufe nach einem der Ansprüche 1 bis 3, dadurch **gekennzeichnet**, daß sie die nachstehende Formel 60 besitzt:

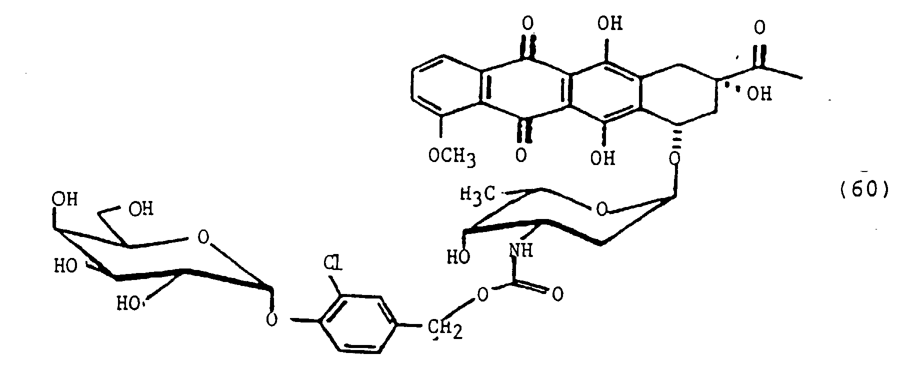

19. Arzneimittelvorstufe nach einem der Ansprüche 1 bis 3, dadurch **gekennzeichnet**, daß sie die nachstehende Formel 64c besitzt:

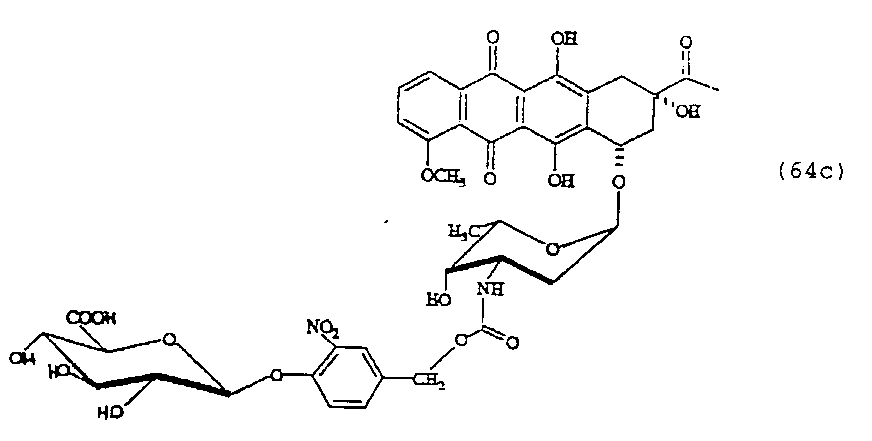

20. Arzneimittelvorstufe nach einem der Ansprüche 1 bis 3, dadurch **gekennzeichnet**, daß sie die nachstehende Formel 68c besitzt:

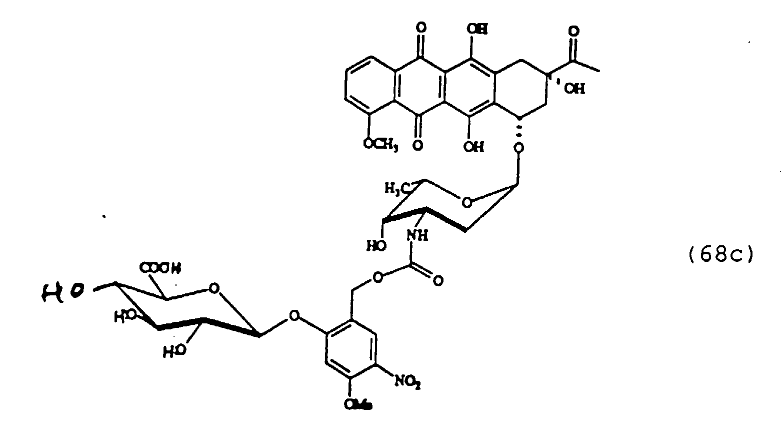

21. Arzneimittelvorstufe nach einem der Ansprüche 1 bis 3, dadurch **gekennzeichnet**, daß sie die nachstehende Formel 75a besitzt:

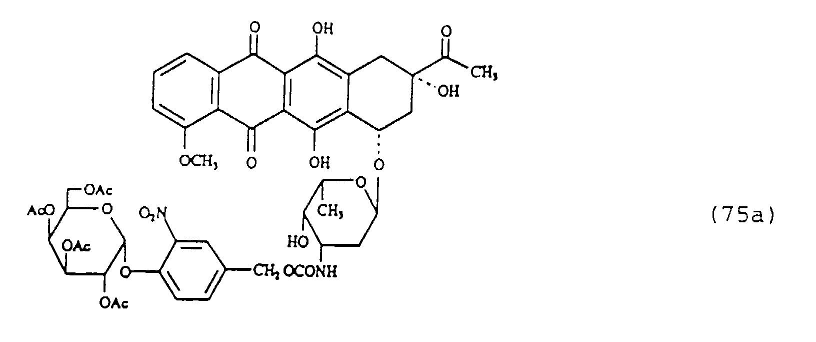

22. Arzneimittelvorstufe nach einem der Ansprüche 1 bis 3, dadurch **gekennzeichnet**, daß sie die nachstehende Formel 75b besitzt:

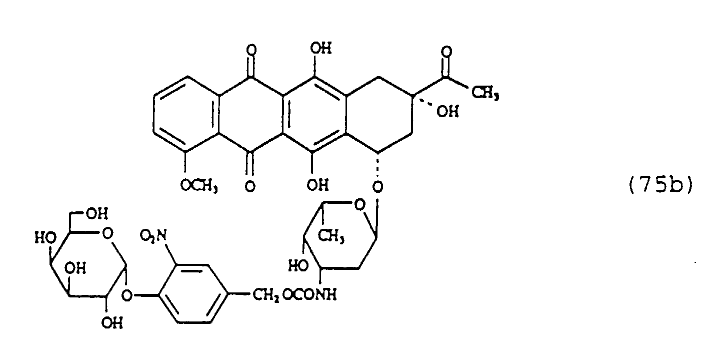

23. Arzneimittelvorstufe nach einem der Ansprüche 1 bis 3, dadurch **gekennzeichnet**, daß sie die nachstehende Formel 78a besitzt:

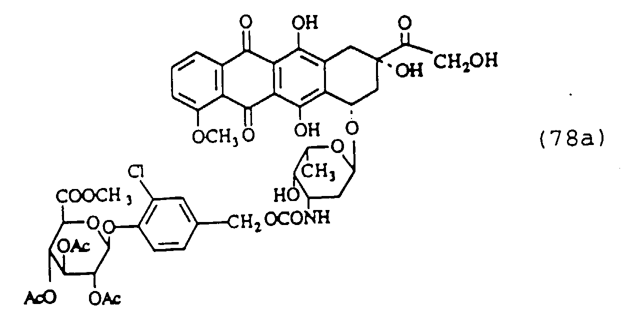

24. Arzneimittelvorstufe nach einem der Ansprüche 1 bis 3, dadurch **gekennzeichnet**, daß sie die nachstehende Formel 78b besitzt:

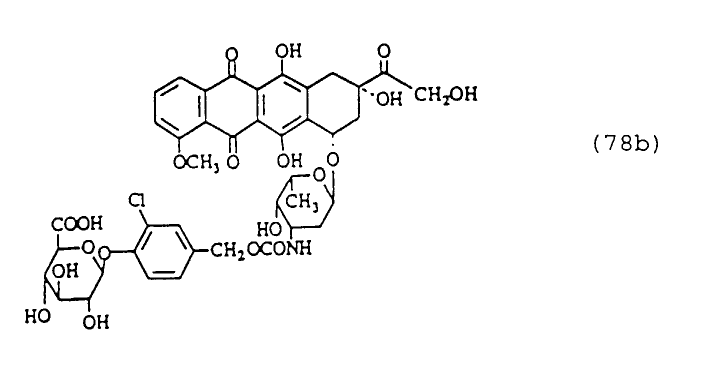

25. Arzneimittelvorstufe nach einem der Ansprüche 1 bis 3, dadurch **gekennzeichnet**, daß die Arzneimittelvorstufe die nachstehende Formel 70 besitzt: 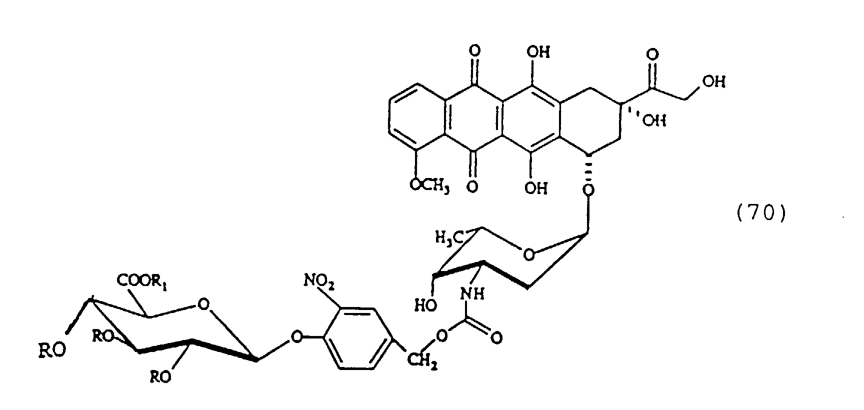 wobei
70a : R=Ac, R₁=CH₃
70b : R=H, R₁=CH₃
70c : R=R₁=H

26. Arzneimittelvorstufe nach einem der Ansprüche 1 bis 3, dadurch **gekennzeichnet**, daß die Arzneimittelvorstufe die nachstehende Formel 82 besitzt: 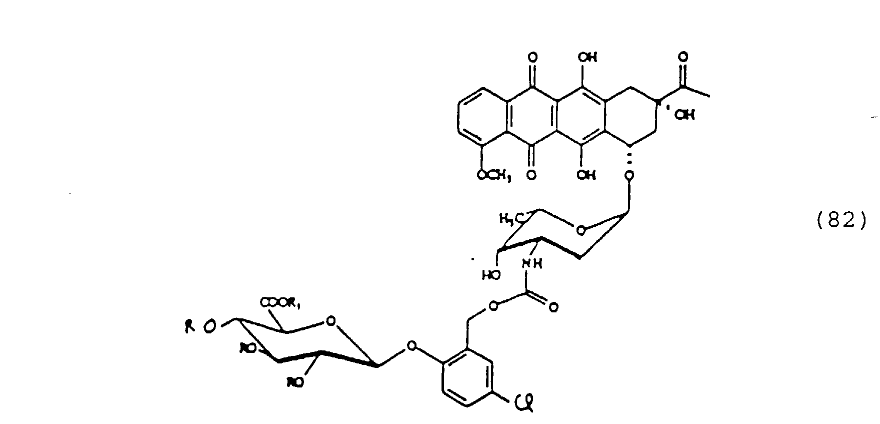 wobei
82a : R = Ac, R₁ = CH₃
82b : R = H , R₁ = CH₃
82c: R = R-₁ = H

27. Arzneimittelvorstufe nach einem der Ansprüche 1 bis 3, dadurch **gekennzeichnet**, daß sie die nachstehende Formel 83 besitzt: 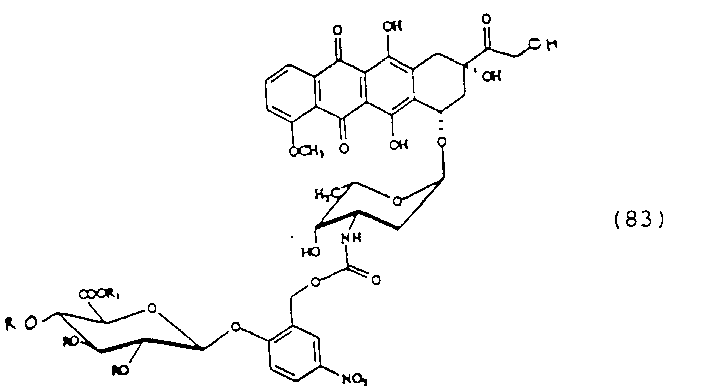 wobei
83a : R = Ac , R₁ = CH₃
83b : R = H , R₁ = CH₃
83c : R = R₁ = H

28. Verfahren zur Herstellung einer Verbindung der Formel I, die insbesondere durch eine Glycosidase abgebaut werden kann, dadurch **gekennzeichnet**, daß man
(1) gegebenenfalls in Gegenwart eines geeigneten Promotors ein Derivat der Formel A, 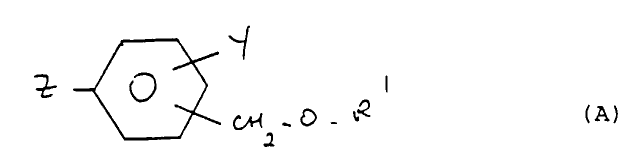 worin
Z eine Hydroxylgruppe, eine O-Trialkylsilylgruppe oder einen Rest 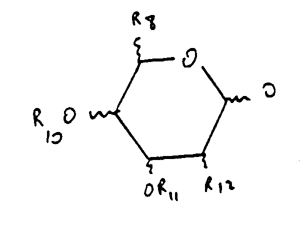 worin R₈, R₁₀, R₁₁ und R₁₂ wie vorstehend definiert sind, bedeutet,
R' ein Wasserstoffatom oder eine der folgenden Gruppen bedeutet, 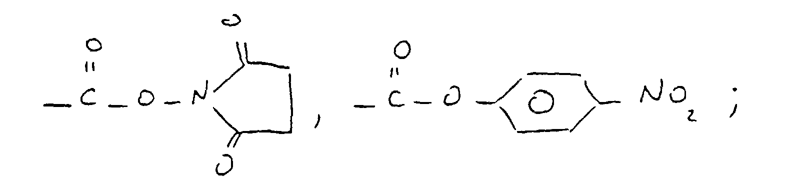 das Benzyl-CH₂ bevorzugt in para- oder ortho-Position der gegebenenfalls modifizierten (glycosyliert oder silyliert) Phenolfunktion steht ,
Y ein Wasserstoffatom oder mindestens eine elektronenziehende Gruppierung, ausgewählt insbesondere aus der Gruppe, umfassend NO₂, ein Halogenatom, eine SO₂X-Gruppierung (wobei X = -CH₃, C₆H₄-CH₃, NH₂, N-(C₁-C₄-Alkyl)₂, NH-C₁-C₄-Alkyl), -CN, Acyl oder COO-Alkyl, und/oder mindestens eine elektronenspendende Gruppierung ausgewählt aus der Gruppe, umfassend Gruppierungen vom Typ O-Alkyl, NHCO-Alkyl, N(Alkyl)CO-Alkyl, S-Alkyl, Alkyl, ist, mit einem Anthracyclin der Formel B 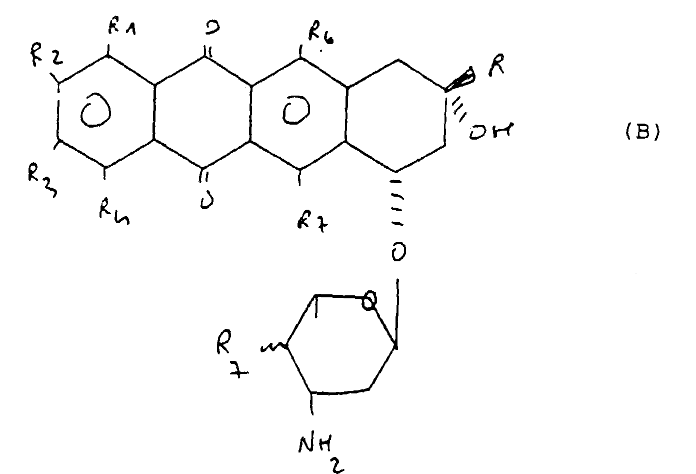 worin R₁, R₂, R₃, R₄, R₅, R₆, R₇ und R wie vorstehend definiert sind, koppelt,
(2) die in den so erhaltenen Verbindungen vorhandenen Schutzgruppen entfernt, insbesondere durch Hydrolyse, Umesterung oder Verseifung, und
(3) gegebenenfalls mit einem Zucker der folgenden Formel 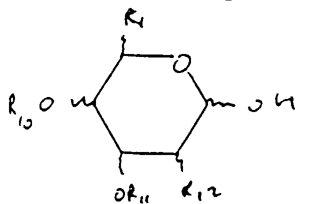 in geeigneter Weise kondensiert,
wenn Z eine Hydroxylgruppe oder eine O-Trialkylsilylgruppe bedeutet,
um eine Anthracyclin-Arzneimittelvorstufe der Formel I zu erhalten, worin alle Reste R₁ bis R₁₂ und R wie vorstehend definiert sind.

29. Verfahren nach Anspruch 28, dadurch **gekennzeichnet**, daß man vor der Stufe (1) das glycosylierte p-Hydroxybenzylderivat erhält durch
(a) Fusion eines Kresols mit einem Zucker oder einem peracetylierten Methylglucuronat,
(b) Benzylbromierung der so erhaltenen Verbindung,
(c) Solvolyse des bromierten Derivats, und
(d) Aktivierung der Hydroxylgruppe durch ein Hydroxysuccinimidylderivat oder Paranitrophenoxycarbonylderivat.

30. Produkte, umfassend eine modifizierte Anthracyclin-Arzneimittelvorstufe nach einem der Ansprüche 1 bis 27 und ein Konjugat aus einem Enzym und einem tumorspezifischen Antikörper der Formel II
Ac-Sp-E (II),
worin
Ac ein Antikörper oder eines seiner Fragmente ist, der eine Spezifität gegenüber einem Tumorantigen besitzt, oder ein Biomolekül ist, das dazu neigt, sich in einem Tumor anzureichern, wie EGF (epidermaler Wachstumsfaktor), α-TGF (transformierender Wachstumsfaktor α), PDGF (Plättchen-abgeleiteter Wachstumsfaktor), IGF I + II (insulinähnlicher Wachstumsfaktor I + II) oder FGF a+b (Fibroblastenwachstumsfaktor a+b),
E eine Glycosidase bedeutet, die nicht immunogen ist oder eine sehr schwache Immunogenität besitzt, bevorzugt eine Säugerglycosidase, wie α- oder β-Glucosidase, α-Galactosidase, α- oder β-Mannosidase, α-Fucosidase, N-Acetyl-α-galactosaminidase, N-Acetyl-β-/N-Acetyl-α-glucosaminidase oder β-Glucuronidase,
Sp (Spacer) eine Gruppe, die ein Sulfid oder Disulfid der Formel III oder IV
X'(S)ₙY' (III)
X'(S)ₙ (IV)
oder einen Polypeptidspacer bedeutet, worin X' oder Y'-CO-R₁₃-(N-Succinimido)- oder -C(=R₁₄) -CH₂-CH₂- bedeutet, wobei R₁₃ eine Gruppieruung -CH₂-CH₂, 1,4-Cyclohexyliden, 1,3- oder 1,4-Phenylen oder Methoxycarbonyl oder 1,4-Chlorphenylen bedeutet, und R₁₄ ein Sauerstoffatom oder eine NH-Gruppe bedeutet, und außerdem
Y' -C(=R₁₄)-CH₂-CH₂ bedeutet, wenn R₁₄ wie vorstehend definiert ist, und
n den Wert 1 oder 2 hat,
zur gleichzeitigen, getrennten oder zeitabgestuften Verwendung bei der zytostatischen Therapie.

31. 4-Brommethylphenyl-2,3,4,6-tetra-O-acetyl-α-D-galactopyranosid der Formel 2 als Zwischenprodukt des Verfahrens nach Anspruch 28.

32. 4-Formylphenyl-2,3,4,6-tetra-O-acetyl-α-D-galactopyranosid der Formel 3 als Zwischenprodukt des Verfahrens nach Anspruch 28.

33. 4-Hydroxymethylphenyl-2,3,4,6-tetra-O-acetyl-α-D-galactopyranosid der Formel 4 als Zwischenprodukt des Verfahrens nach Anspruch 28.

34. 2-Brommethylphenyl-2,3,4,6-tetra-O-acetyl-α-D-galactopyranosid der Formel 9 als Zwischenprodukt des Verfahrens nach Anspruch 28.

35. 2-Formylphenyl-2,3,4,6-tetra-O-acetyl-α-D-galactopyranosid der Formel 10 als Zwischenprodukt des Verfahrens nach Anspruch 28.

36. 2-Hydroxymethylphenyl-2,3,4,6-tetra-O-acetyl-α-D-galactopyranosid der Formel 11 als Zwischenprodukt des Verfahrens nach Anspruch 28.

37. (4-Brommethylphenyl-2,3,4,-tri-O-acetyl-β-D-glucopyranosid)methyluronat der Formel 16 als Zwischenprodukt des Verfahrens nach Anspruch 28.

38. (4-Formylphenyl-2,3,4,-tri-O-acetyl-β-D-glucopyranosid)methyluronat der Formel 17 als Zwischenprodukt des Verfahrens nach Anspruch 28.

39. (4-Hydroxymethylphenyl-2,3,4,-tri-O-acetyl-β-D-glucopyranosid)methyluronat der Formel 18 als Zwischenprodukt des Verfahrens nach Anspruch 28.

40. (2-Brommethylphenyl-2,3,4,-tri-O-acetyl-β-D-glucopyranosid)methyluronat der Formel 23 als Zwischenprodukt des Verfahrens nach Anspruch 28.

41. (2-Hydroxymethylphenyl-2,3,4,-tri-O-acetyl-β-D-glucopyranosid)methyluronat der Formel 24 als Zwischenprodukt des Verfahrens nach Anspruch 28.

42. (2-Formylphenyl-2,3,4,-tri-O-acetyl-β-D-glucopyranosid)methyluronat der Formel 25 als Zwischenprodukt des Verfahrens nach Anspruch 28.

43. 2-tert.-Butyldimethylsilyloxybenzaldehyd der Formel 28 als Zwischenprodukt des Verfahrens nach Anspruch 28.

44. 2-tert.-Butyldimethylsilyloxybenzylalkohol der Formel 29 als Zwischenprodukt des Verfahrens nach Anspruch 28.

45. N-[2-Hydroxybenzyloxycarbonyl]daunorubicin der Formel 32 als Zwischenprodukt des Verfahrens nach Anspruch 28.

46. 4-Formylphenyl-2,3,4,6-tetra-O-acetyl-β-D-glucopyranosid der Formel 33 als Zwischenprodukt des Verfahrens nach Anspruch 28.

47. 4-Hydroxymethylphenyl-2,3,4,6-tetra-O-acetyl-β-D-glucopyranosid der Formel 34 als Zwischenprodukt des Verfahrens nach Anspruch 28.

48. N-[2-Hydroxybenzyloxycarbonyl]daunorubicin der Formel 38 als Zwischenprodukt des Verfahrens nach Anspruch 28.

49. 2-Methyl-4-nitrophenyl-2,3,4,6-tetra-O-acetyl-α-D-galactopyranosid der Formel 42 als Zwischenprodukt des Verfahrens nach Anspruch 28.

50. 2-Brommethyl-4-nitrophenyl-2,3,4,6-tetra-O-acetyl-α-D-galactopyranosid der Formel 43 als Zwischenprodukt des Verfahrens nach Anspruch 28.

51. 2-Dibrommethyl-4-nitrophenyl-2,3,4,6-tetra-O-acetyl-α-D-galactopyranosid der Formel 44 als Zwischenprodukt des Verfahrens nach Anspruch 28.

52. 2-Formyl-4-nitrophenyl-2,3,4,6-tetra-O-acetyl-α-D-galactopyranosid der Formel 45 als Zwischenprodukt des Verfahrens nach Anspruch 28.

53. (2-Hydroxymethyl-4-nitro)phenyl-2,3,4,6-tetra-O-acetyl-α-D-galactopyranosid der Formel 46 als Zwischenprodukt des Verfahrens nach Anspruch 28.

54. (2-Formyl-4-nitrophenyl-2,3,4,-tri-O-acetyl-β-D-glucopyranosid)methyluronat der Formel 51 als Zwischenprodukt des Verfahrens nach Anspruch 28.

55. (2-Hydroxymethyl-4-nitrophenyl-2,3,4,-tri-O-acetyl-β-D-glucopyranosid)methyluronat der Formel 52 als Zwischenprodukt des Verfahrens nach Anspruch 28.

56. 2-Chlor-4-methylphenyl-2,3,4,6-tetra-O-acetyl-α-D-galactopyranosid der Formel 55 als Zwischenprodukt des Verfahrens nach Anspruch 28.

57. 2-Chlor-4-bromethylphenyl-2,3,4,6-tetra-O-acetyl-α-D-galactopyranosid der Formel 56 als Zwischenprodukt des Verfahrens nach Anspruch 28.

58. 2-Chlor-4-hydroxymethylphenyl-2,3,4,6-tetra-O-acetyl-α-D-galactopyranosid der Formel 57 als Zwischenprodukt des Verfahrens nach Anspruch 28.

59. (4-Formyl-2-nitrophenyl-2,3,4,-tri-O-acetyl-β-D-glucopyranosid)methyluronat der Formel 61 als Zwischenprodukt des Verfahrens nach Anspruch 28.

60. (2-Hydroxymethyl-2-nitrophenyl-2,3,4,-tri-O-acetyl-β-D-glucopyranosid)methyluronat der Formel 62 als Zwischenprodukt des Verfahrens nach Anspruch 28.

61. (4-Brommethyl-4-nitro)phenyl-2,3,4,6-tetra-O-acetyl-α-D-galactopyranosid der Formel 73 als Zwischenprodukt des Verfahrens nach Anspruch 28.

62. (4-Hydroxymethyl-2-nitro)phenyl-2,3,4,6-tetra-O-acetyl-α-D-galactopyranosid der Formel 74 als Zwischenprodukt des Verfahrens nach Anspruch 28.

63. 4-Hydroxy-3-chlorbenzaldehyd-2,3,4,-tri-O-acetyl-β-D-methylglucuronid der Formel 76 als Zwischenprodukt des Verfahrens nach Anspruch 28.

64. (4-Hydroxymethyl-2-chlor)phenyl-2,3,4,-tri-O-acetyl-β--D-methylglucuronid der Formel 77 als Zwischenprodukt des Verfahrens nach Anspruch 28.

65. 2,5-Dioxopyrolidin-1-yl-4-(2,3,4,6-tetra-O-acetyl-α-D-galactopyranosyl)benzylcarbonat der Formel 5 als Zwischenprodukt des Verfahrens nach Anspruch 28.

66. 2,5-Dioxopyrolidin-1-yl-2-(2,3,4,6-tetra-O-acetyl-α-D-galactopyranosyl)benzylcarbonat der Formel 12 als Zwischenprodukt des Verfahrens nach Anspruch 28.

67. 2,5-Dioxopyrolidin-1-yl-4-((2,3,4-tri-O-acetyl-β-D-glucopyranosyl)methyluronat)benzylcarbonat der Formel 19 als Zwischenprodukt des Verfahrens nach Anspruch 28.

68. 4-Nitrophenyl-2-((2,3,4-tri-O-acetyl-β-D-glucopyranosyl)methyluronat)benzylcarbonat der Formel 26 als Zwischenprodukt des Verfahrens nach Anspruch 28.

69. 4-Nitrophenyl-2-(tert.-butyldimethylsilyloxy)benzylcarbonat der Formel 30 als Zwischenprodukt des Verfahrens nach Anspruch 28.

70. 2,5-Dioxopyrolidin-1-yl-4-(2,3,4,6-tetra-O-acetyl-β-D-glucopyranosyl)benzylcarbonat der Formel 35 als Zwischenprodukt des Verfahrens nach Anspruch 28.

71. 2,5-Dioxopyrolidin-1-yl-4-dimethylhexylsilyloxybenzylcarbonat der Formel 40 als Zwischenprodukt des Verfahrens nach Anspruch 28.

72. 4-Nitrophenyl-2-(2,3,4,6-tetra-O-acetyl-α-D-galactopyranosyl)-5-nitrobenzylcarbonat der Formel 47 als Zwischenprodukt des Verfahrens nach Anspruch 28.

73. 4-Nitrophenyl-2-((2,3,4-tri-O-acetyl-β-D-glucopyranosyl)methyluronat)-5-nitrobenzylcarbonat der Formel 53 als Zwischenprodukt des Verfahrens nach Anspruch 28.

74. 4-Nitrophenyl-4-methoxy-5-nitro-2-((2,3,4-tri-O-acetyl-β-D-glucopyranosyl)methyluronat)benzylcarbonat der Formel 67 als Zwischenprodukt des Verfahrens nach Anspruch 28.

75. 4-Nitrophenyl-4-(2,3,4-tri-O-acetyl-β-D-glucopyranosyl)methyluronat)-5-nitrobenzylcarbonat der Formel 69 als Zwischenprodukt des Verfahrens nach Anspruch 28.

76. 4-Chlorphenyl-2-((2,3,4-tri-O-acetyl-β-D-glucopyranosyl)methyluronat)-5-nitrobenzylcarbonat der Formel 81 als Zwischenprodukt des Verfahrens nach Anspruch 28.

77. Verwendung der Arzneimittelvorstufen nach einem der Ansprüche 1 bis 27 zur Herstellung eines Arzneimittels zur Behandlung von Krankheiten, an denen aktivierte Makrophagen, aktivierte Granulozyten, aktivierte Thrombozyten oder aktivierte Tumorzellen beteiligt sind.

## Patentansprüche (Patentansprüche für folgende(n) Vertragsstaat(en): ES, GR)

1. Verfahren zur Herstellung von Anthracyclin-Arzneimittelvorstufen (Anthracyclin-Pro-Drugs) der nachstehenden Formel I 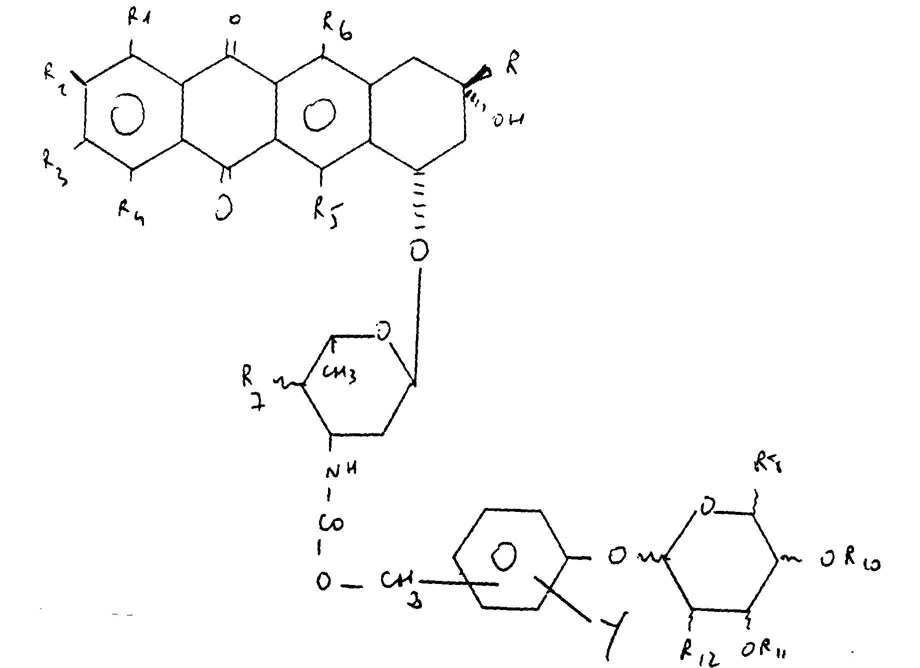 worin
R₁, R₂, R₃, die gleich oder verschieden sein können, ein Wasserstoffatom oder eine Hydroxylgruppe bedeuten,
R₄ ein Wasserstoffatom, eine Hydroxylgruppe oder eine Methoxygruppe bedeutet,
R einen Rest CO-CH₂-R" bedeutet, worin R" ein Wasserstoffatom, ein C₁-C₆-Alkylrest, eine Hydroxylgruppe, ein Alkoxylrest, ein O-Acylrest oder ein Arylrest ist,
R₅ und R₆, die gleich oder verschieden sein können, ein Wasserstoffatom oder eine Hydroxylgruppe bedeuten,
R₇ ein Wasserstoffatom oder eine Hydroxylgruppe bedeutet,
R₈ einen Rest -CH₂-OR₉ oder einen Rest COOR₉ bedeutet, worin R₉ einen C₁-C₃-Alkylrest oder ein Wasserstoffatom bedeutet,
R₁₀ und R₁₁ ein Wasserstoffatom, eine Acylschutzgruppe oder einen Alkylrest bedeuten,
R₁₂ eine Hydroxylgruppe, eine Amingruppe, eine Amidgruppe oder eine O-Acylschutzgruppe bedeutet,
das Benzyl-CH₂ bevorzugt in para- oder ortho-Position des Glycosylsauerstoffs steht,
Y ein Wasserstoffatom oder mindestens eine elektronenziehende Gruppierung, ausgewählt insbesondere aus der Gruppe, umfassend NO₂, ein Halogenatom, eine SO₂X-Gruppierung (wobei X = -CH₃, C₆H₄-CH₃, NH₂, N-(C₁-C₄-Alkyl)₂, NH-C₁-C₄-Alkyl), -CN, Acyl oder COO-Alkyl, und/oder mindestens eine elektronenspendende Gruppierung ausgewählt aus der Gruppe, umfassend Gruppierungen vom Typ O-Alkyl, NHCO-Alkyl, N(Alkyl)CO-Alkyl, S-Alkyl, Alkyl, ist, dadurch **gekennzeichnet**, daß man
(1) gegebenenfalls in Gegenwart eines geeigneten Promotors ein Derivat der Formel A, 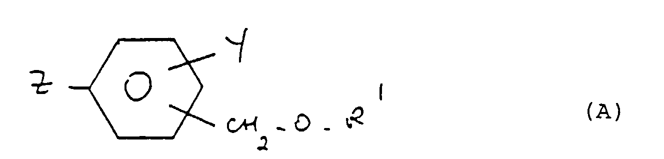 worin
Z eine Hydroxylgruppe, eine O-Trialkylsilylgruppe oder einen Rest 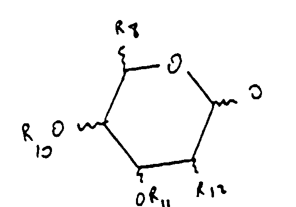 worin R₈, R₁₀, R₁₁ und R₁₂ wie vorstehend definiert sind, bedeutet.
R' ein Wasserstoffatom oder eine der folgenden Gruppen bedeutet, 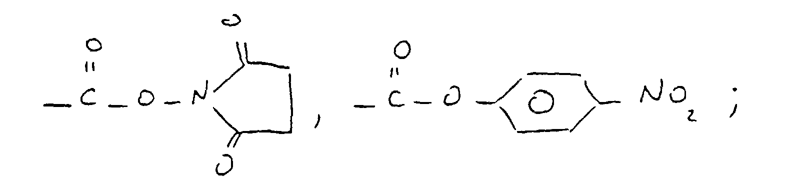 das Benzyl-CH₂ bevorzugt in para- oder ortho-Position der gegebenenfalls modifizierten (glycosyliert oder silyliert) Phenolfunktion steht ,
Y ein Wasserstoffatom oder mindestens eine elektronenziehende Gruppierung, ausgewählt insbesondere aus der Gruppe, umfassend NO₂, ein Halogenatom, eine SO₂X-Gruppierung (wobei X = -CH₃, C₆H₄-CH₃, NH₂, N-(C₁-C₄-Alkyl)₂, NH-C₁-C₄-Alkyl), -CN, Acyl oder COO-Alkyl, und/oder mindestens eine elektronenspendende Gruppierung ausgewählt aus der Gruppe, umfassend Gruppierungen vom Typ O-Alkyl, NHCO-Alkyl, N(Alkyl)CO-Alkyl, S-Alkyl, Alkyl, ist, mit einem Anthracyclin der Formel B 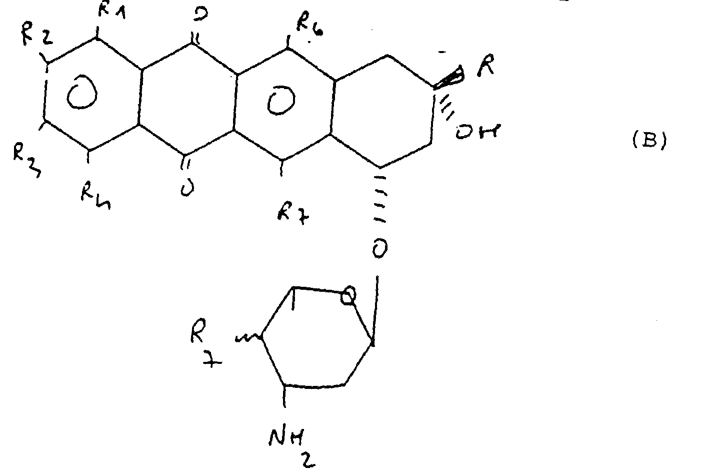 worin R₁, R₂, R₃, R₄, R₅, R₆, R₇ und R wie vorstehend definiert sind, koppelt,
(2) die in den so erhaltenen Verbindungen vorhandenen Schutzgruppen entfernt, insbesondere durch Hydrolyse, Umesterung oder Verseifung, und
(3) gegebenenfalls mit einem Zucker der folgenden Formel 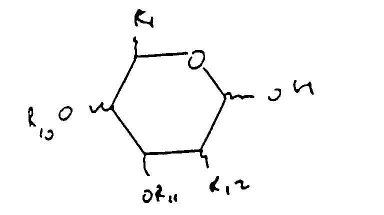 in geeigneter Weise kondensiert,
wenn Z eine Hydroxylgruppe oder eine O-Trialkylsilylgruppe bedeutet,
um eine Anthracyclin-Arzneimittelvorstufe der Formel I zu erhalten, worin alle Reste R₁ bis R₁₂ und R wie vorstehend definiert sind.

2. Verfahren zur Herstellung von Arzneimittelvorstufen nach Anspruch 1, dadurch **gekennzeichnet**, daß wenn Y eine elektronenziehende Gruppierung/Gruppierungen bedeutet, diese sich bevorzugt in ortho- und/oder para-Position des Glycosylsauerstoffs befinden, und wenn Y eine elektronenspendende Gruppierung/Gruppierungen bedeutet, sich diese bevorzugt in meta-Position befinden.

3. Verfahren zur Herstellung von Arzneimittelvorstufen nach Anspruch 1 oder 2, dadurch **gekennzeichnet**, daß die bevorzugten Verbindungen der Formel I insbesondere die folgenden Reste umfassen:
R₁, R₂, R₃ sind Wasserstoffatome,
R₄ ist eine Methoxygruppe,
R₅ und R₆ sind Hydroxylgruppen,
R ist ein Rest -CO-CH₃ oder ein Rest -CO-CH₂OH,
R₇ ist ein Wasserstoffatom oder eine Hydroxylgruppe,
R₈ ist ein Rest -CH₂-OAc, -CH₂OH, -COOMe oder -COOH,
R₁₀ und R₁₁, die gleich oder verschieden sein können, sind Wasserstoffatome oder eine Ac-Gruppe,
R₁₂ ist eine Hydroxylgruppe oder eine OAc-Gruppe,
wobei die Reste R₈, R₁₀, R₁₁ und R₁₂ bevorzugt die folgenden Positionen besitzen: 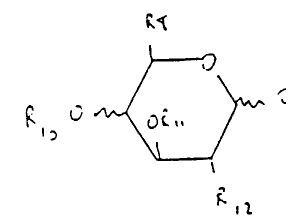
Y ist ein Wasserstoffatom, eine NO₂-Gruppe oder ein Chloratom in para- oder ortho-Position des Glycosylsauerstoffs, oder eine OCH₃-Gruppe in meta-Position des Glycosylsauerstoffs.

4. Verfahren zur Herstellung von Arzneimittelvorstufen nach einem der Ansprüche 1 bis 3, dadurch **gekennzeichnet**, daß die Arzneimittelvorstufe die nachstehende Formel 6 besitzt:

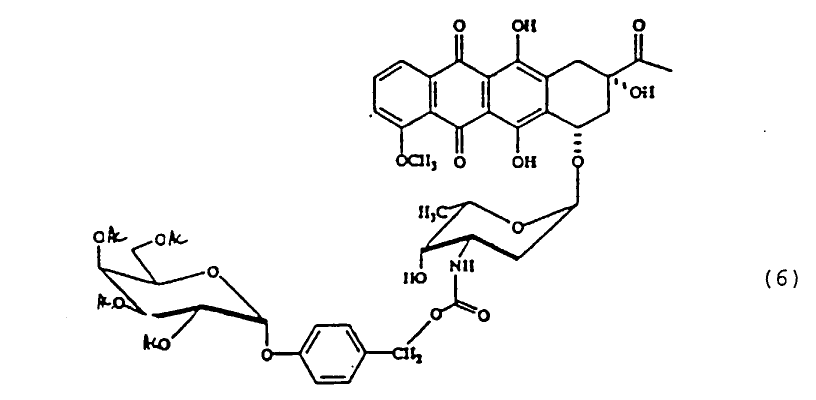

5. Verfahren zur Herstellung einer Arzneimittelvorstufe nach einem der Ansprüche 1 bis 3, dadurch **gekennzeichnet**, daß die Arzneimittelvorstufe die nachstehende Formel 7 besitzt:

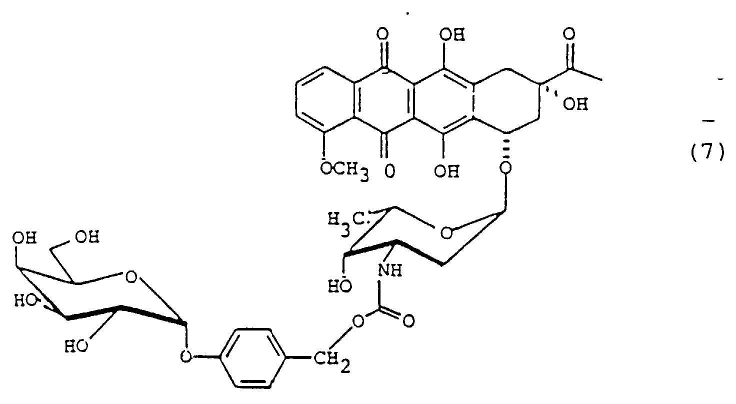

6. Verfahren zur Herstellung einer Arzneimittelvorstufe nach einem der Ansprüche 1 bis 3, dadurch **gekennzeichnet**, daß die Arzneimittelvorstufe die nachstehende Formel 13 besitzt:

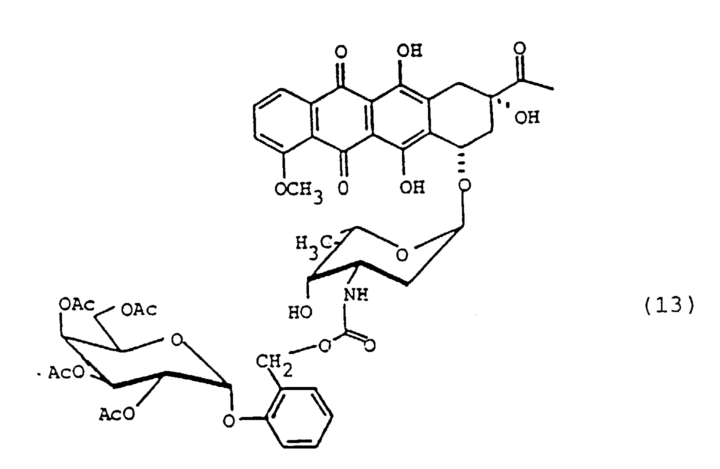

7. Verfahren zur Herstellung einer Arzneimittelvorstufe nach einem der Ansprüche 1 bis 3, dadurch **gekennzeichnet**, daß die Arzneimittelvorstufe- die nachstehende Formel 14 besitzt:

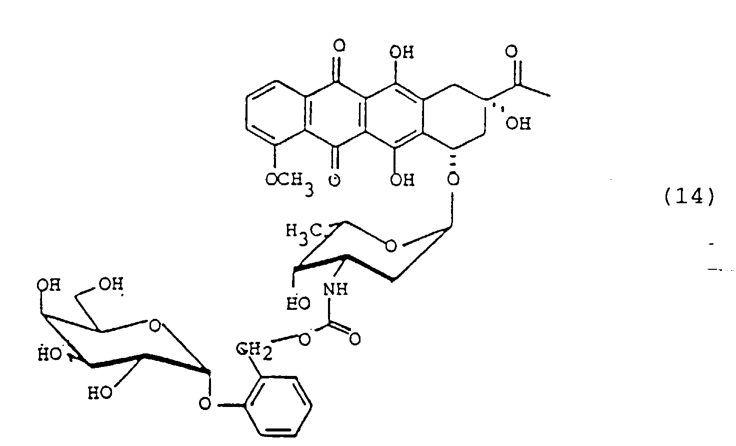

8. Verfahren zur Herstellung einer Arzneimittelvorstufe nach einem der Ansprüche 1 bis 3, dadurch **gekennzeichnet**, daß die Arzneimittelvorstufe die nachstehende Formel 22 besitzt:

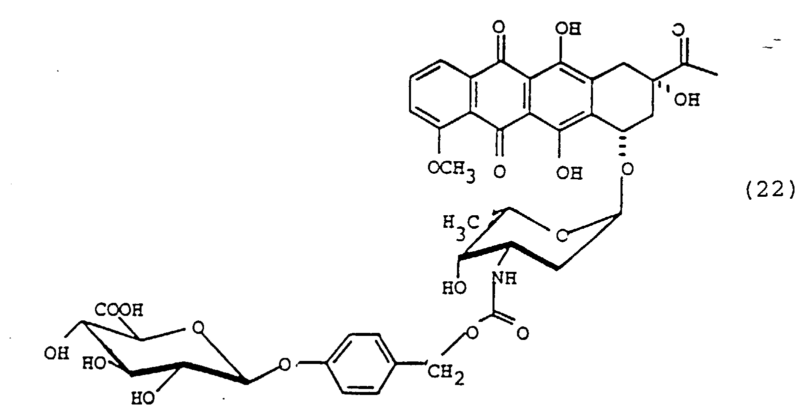

9. Verfahren zur Herstellung einer Arzneimittelvorstufe nach einem der Ansprüche 1 bis 3, dadurch **gekennzeichnet**, daß die Arzneimittelvorstufe die nachstehende Formel 27c besitzt:

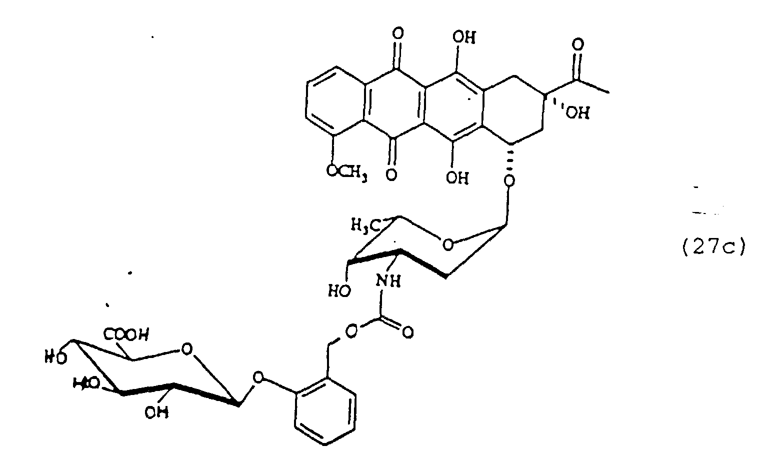

10. Verfahren zur Herstellung einer Arzneimittelvorstufe nach einem der Ansprüche 1 bis 3, dadurch **gekennzeichnet**, daß die Arzneimittelvorstufe die nachstehende Formel 36 besitzt:

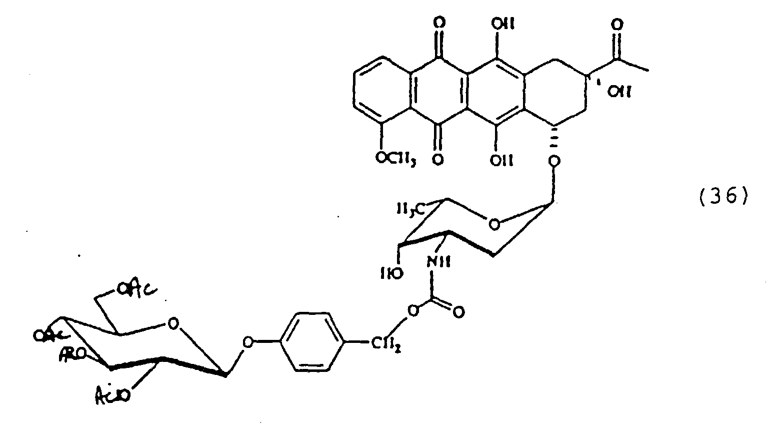

11. Verfahren zur Herstellung einer Arzneimittelvorstufe nach einem der Ansprüche 1 bis 3, dadurch **gekennzeichnet**, daß die Arzneimittelvorstufe die nachstehende Formel 37 besitzt:

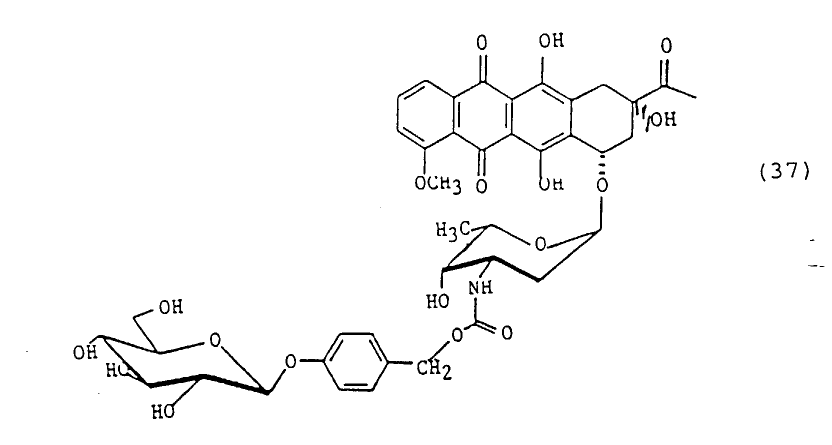

12. Verfahren zur Herstellung einer Arzneimittelvorstufe nach einem der Ansprüche 1 bis 3, dadurch **gekennzeichnet**, daß die Arzneimittelvorstufe die nachstehende Formel 48a besitzt:

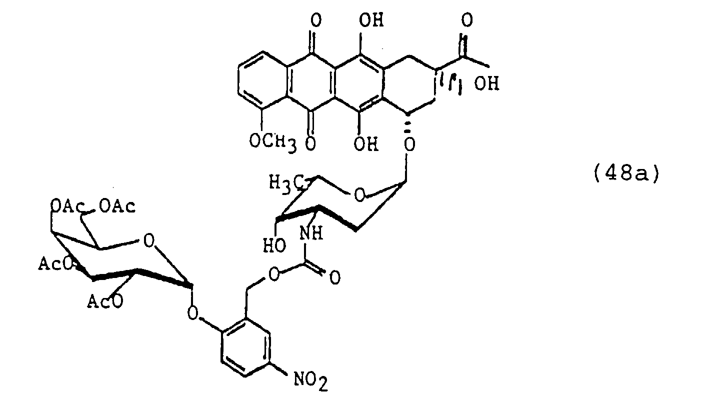

13. Verfahren zur Herstellung einer Arzneimittelvorstufe nach einem der Ansprüche 1 bis 3, dadurch **gekennzeichnet**, daß die Arzneimittelvorstufe die nachstehende Formel 48b besitzt:

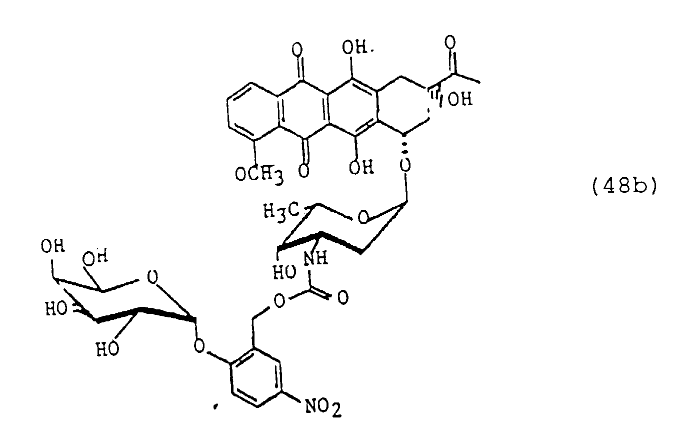

14. Verfahren zur Herstellung einer Arzneimittelvorstufe nach einem der Ansprüche 1 bis 3, dadurch **gekennzeichnet**, daß die Arzneimittelvorstufe die nachstehende Formel 49a besitzt:

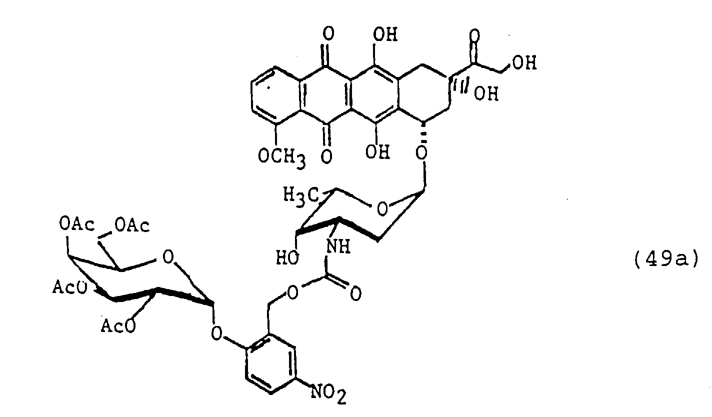

15. Verfahren zur Herstellung einer Arzneimittelvorstufe nach einem der Ansprüche 1 bis 3, dadurch **gekennzeichnet**, daß die Arzneimittelvorstufe die nachstehende Formel 49b besitzt:

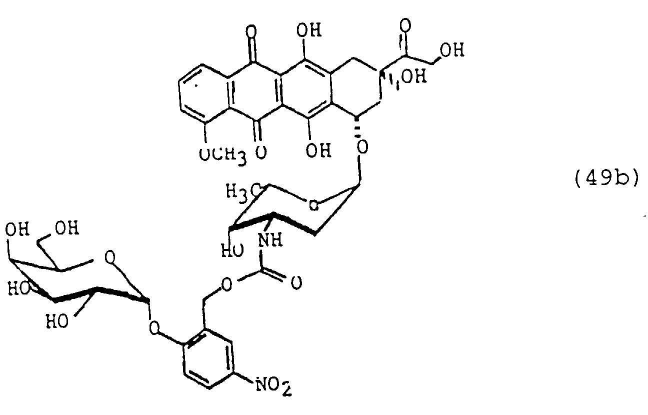

16. Verfahren zur Herstellung einer Arzneimittelvorstufe nach einem der Ansprüche 1 bis 3, dadurch **gekennzeichnet**, daß die Arzneimittelvorstufe die nachstehende Formel 54c besitzt:

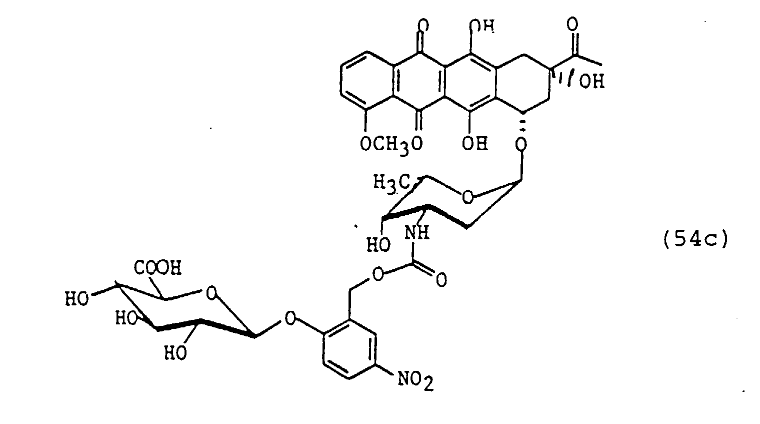

17. Verfahren zur Herstellung einer Arzneimittelvorstufe nach einem der Ansprüche 1 bis 3, dadurch **gekennzeichnet**, daß die Arzneimittelvorstufe die nachstehende Formel 59 besitzt:

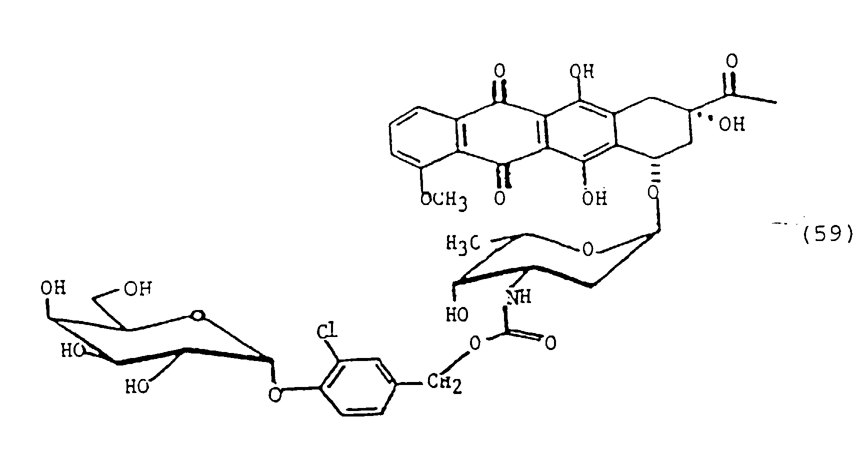

18. Verfahren zur Herstellung einer Arzneimittelvorstufe nach einem der Ansprüche 1 bis 3, dadurch **gekennzeichnet**, daß die Arzneimittelvorstufe die nachstehende Formel 60 besitzt:

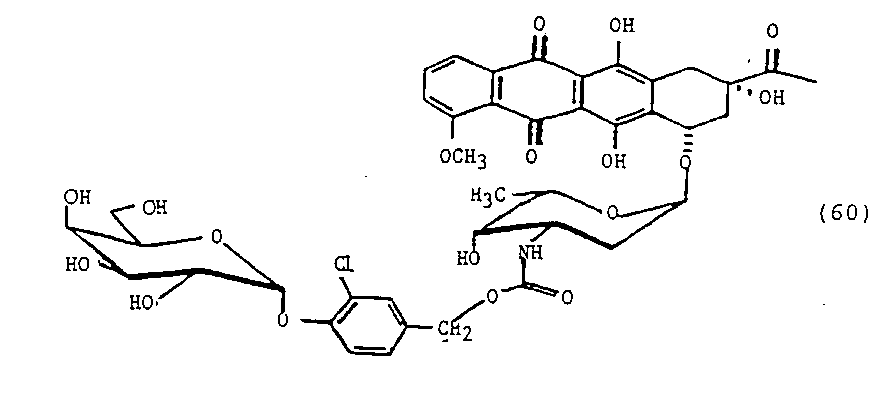

19. Verfahren zur Herstellung einer Arzneimittelvorstufe nach einem der Ansprüche 1 bis 3, dadurch **gekennzeichnet**, daß die Arzneimittelvorstufe die nachstehende Formel 64c besitzt:

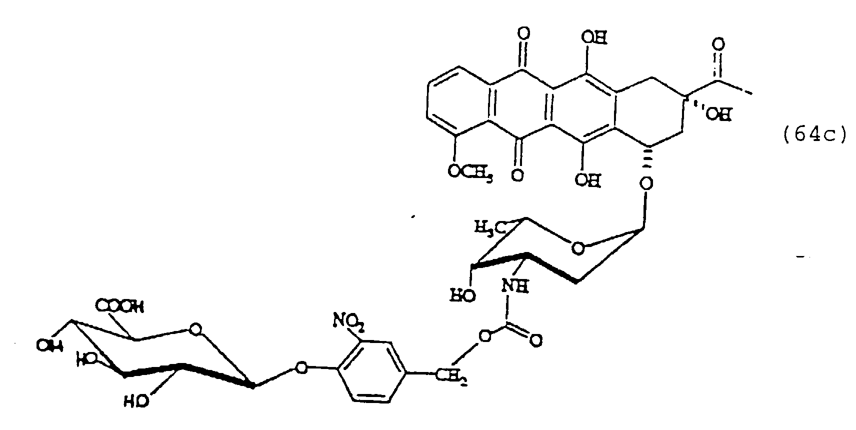

20. Verfahren zur Herstellung einer Arzneimittelvorstufe nach einem der Ansprüche 1 bis 3, dadurch **gekennzeichnet**, daß die Arzneimittelvorstufe die nachstehende Formel 68c besitzt:

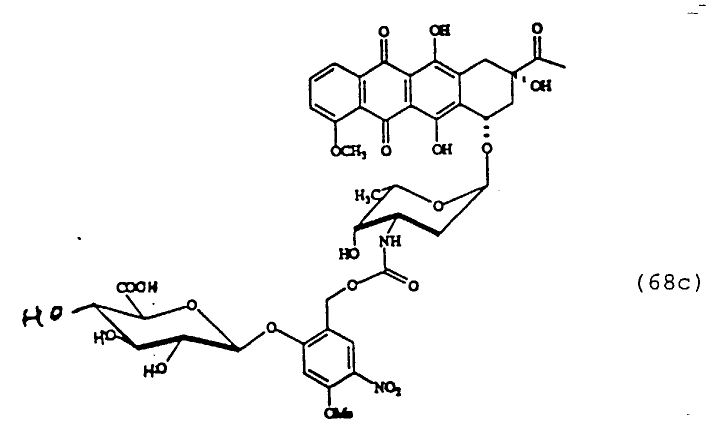

21. Verfahren zur Herstellung einer Arzneimittelvorstufe nach einem der Ansprüche 1 bis 3, dadurch **gekennzeichnet**, daß die Arzneimittelvorstufe die nachstehende Formel 75a besitzt:

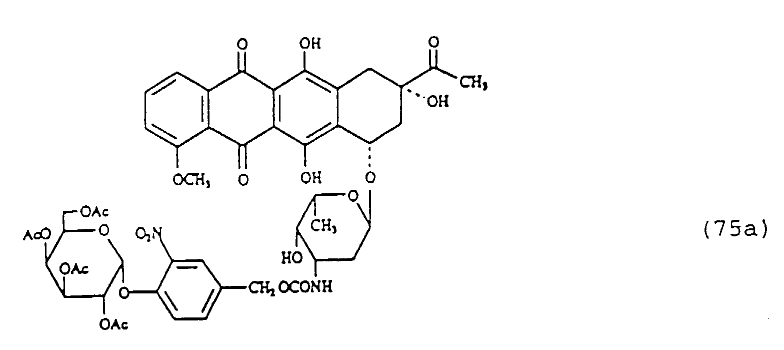

22. Verfahren zur Herstellung einer Arzneimittelvorstufe nach einem der Ansprüche 1 bis 3, dadurch **gekennzeichnet**, daß die Arzneimittelvorstufe die nachstehende Formel 75b besitzt:

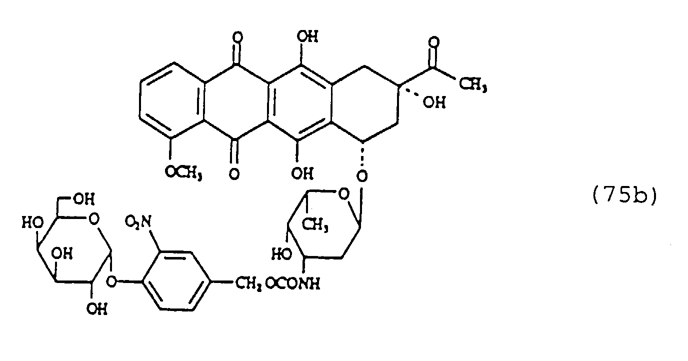

23. Verfahren zur Herstellung einer Arzneimittelvorstufe nach einem der Ansprüche 1 bis 3, dadurch **gekennzeichnet**, daß die Arzneimittelvorstufe die nachstehende Formel 78a besitzt:

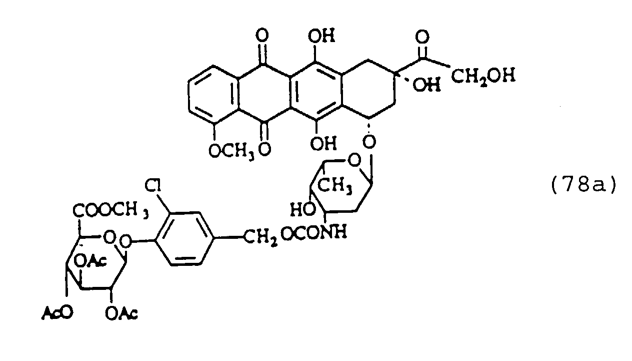

24. Verfahren zur Herstellung einer Arzneimittelvorstufe nach einem der Ansprüche 1 bis 3, dadurch **gekennzeichnet**, daß die Arzneimittelvorstufe die nachstehende Formel 78b besitzt:

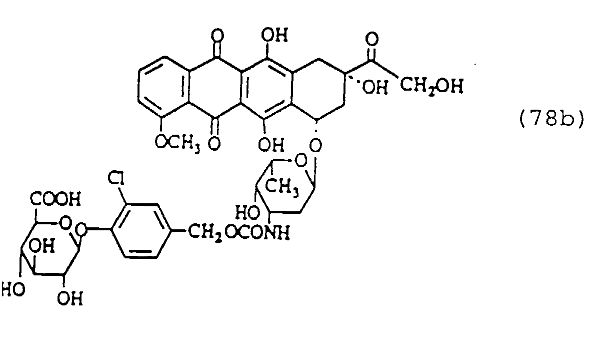

25. Verfahren zur Herstellung einer Arzneimittelvorstufe nach einem der Ansprüche 1 bis 3, dadurch **gekennzeichnet**, daß die Arzneimittelvorstufe die nachstehende Formel 70 besitzt: 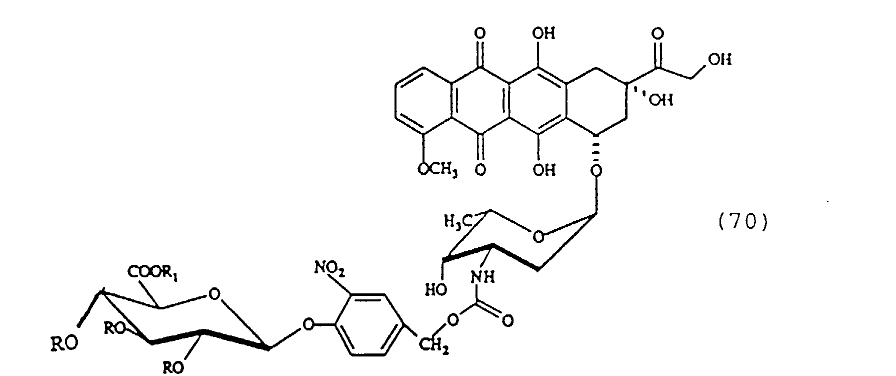 wobei
70a : R=Ac, R₁=CH₃
70b : R=H, R₁=CH₃
70c : R=R₁=H

26. Verfahren zur Herstellung einer Arzneimittelvorstufe nach einem der Ansprüche 1 bis 3, dadurch **gekennzeichnet**, daß die Arzneimittelvorstufe die nachstehende Formel 82 besitzt: 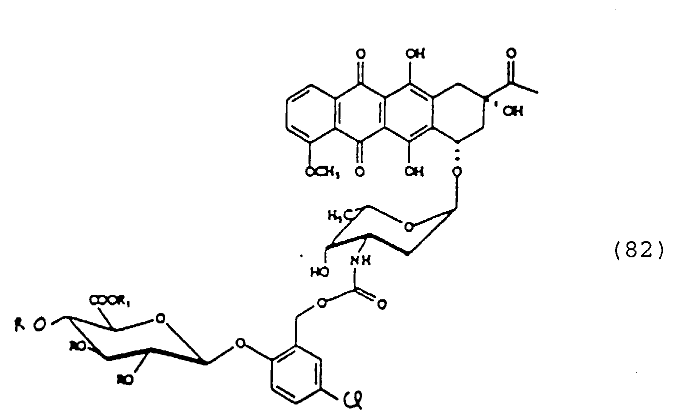 wobei
82a : R = Ac, R₁ = CH₃
82b : R = H , R₁ = CH₃
82c : R = R-1 = H

27. Verfahren zur Herstellung einer Arzneimittelvorstufe nach einem der Ansprüche 1 bis 3, dadurch **gekennzeichnet**, daß die Arzneimittelvorstufe die nachstehende Formel 83 besitzt: 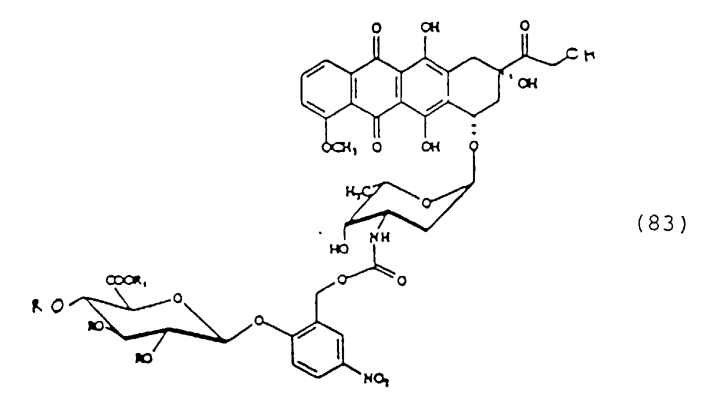 wobei
83a : R = Ac, R₁ = CH₃
83b: R = H , R₁ = CH₃
83c : R = R₁ = H

28. Verfahren nach einem der Ansprüche 1 bis 27, dadurch **gekennzeichnet**, daß man vor der Stufe (1) das glycosylierte p-Hydroxybenzylderivat erhält durch
(a) Fusion eines Kresols mit einem Zucker oder einem peracetylierten Methylglucuronat,
(b) Benzylbromierung der so erhaltenen Verbindung,
(c) Solvolyse des bromierten Derivats, und
(d) Aktivierung der Hydroxylgruppe durch ein Hydroxysuccinimidylderivat oder Paranitrophenoxycarbonylderivat.

29. Verfahren zur Herstellung von Produkten, umfassend eine modifizierte Anthracyclin-Arzneimittelvorstufe nach einem der Ansprüche 1 bis 27, dadurch **gekennzeichnet**, daß sie eine Assoziation einer dieser Arzneimittelvorstufen und eines Konjugats aus einem Enzym und einem tumorspezifischen Antikörper der Formel II
Ac-Sp-E (II),
worin
Ac ein Antikörper oder eines seiner Fragmente ist, der eine Spezifität gegenüber einem Tumorantigen besitzt, oder ein Biomolekül ist, das dazu neigt, sich in einem Tumor anzureichern, wie EGF (epidermaler Wachstumsfaktor), α-TGF (transformierender Wachstumsfaktor α), PDGF (Plättchen-abgeleiteter Wachstumsfaktor), IGF I + II (insulinähnlicher Wachstumsfaktor I + II) oder FGF a+b (Fibroblastenwachstumsfaktor a+b),
E eine Glycosidase bedeutet, die nicht immunogen ist oder eine sehr schwache Immunogenität besitzt, bevorzugt eine Säugerglycosidase, wie α- oder β-Glucosidase, α-Galactosidase, α- oder β-Mannosidase, α-Fucosidase, N-Acetyl-α-galactosaminidase, N-Acetyl-β-/N-Acetyl-α-glucosaminidase oder β-Glucuronidase,
Sp (Spacer) eine Gruppe, die ein Sulfid oder Disulfid der Formel III oder IV
X'(S)ₙY' (III)
X'(S)ₙ (IV)
oder einen Polypeptidspacer bedeutet, worin X' oder Y'-CO-R₁₃-(N-Succinimido)- oder -C(=R₁₄)-CH₂-CH₂- bedeutet, wobei R₁₃ eine Gruppieruung -CH₂-CH₂, 1,4-Cyclohexyliden, 1,3- oder 1,4-Phenylen oder Methoxycarbonyl oder 1,4-Chlorphenylen bedeutet, und R₁₄ ein Sauerstoffatom oder eine NH-Gruppe bedeutet, und außerdem Y' -C(=R₁₄)-CH₂-CH₂ bedeutet, wenn R₁₄ wie vorstehend definiert ist, und n den Wert 1 oder 2 hat,
zur gleichzeitigen, getrennten oder zeitabgestuften Verwendung bei der zytostatischen Therapie umfassen.

30. Verfahren zur Herstellung nach einem der Ansprüche 1 bis 27, dadurch **gekennzeichnet**, daß man als Zwischenprodukt 4-Brommethylphenyl-2,3,4,6-tetra-O-acetyl-α-D-galactopyranosid der Formel 2 einsetzt.

31. Verfahren zur Herstellung nach einem der Ansprüche 1 bis 27, dadurch **gekennzeichnet**, daß man als Zwischenprodukt 4-Formylphenyl-2,3,4,6-tetra-O-acetyl-α-D-galactopyranosid der Formel 3 einsetzt.

32. Verfahren zur Herstellung nach einem der Ansprüche 1 bis 27, dadurch **gekennzeichnet**, daß man als Zwischenprodukt 4-Hydroxymethylphenyl-2,3,4,6-tetra-O-acetyl-α-D-galactopyranosid der Formel 4 einsetzt.

33. Verfahren zur Herstellung nach einem der Ansprüche 1 bis 27, dadurch **gekennzeichnet**, daß man als Zwischenprodukt 2-Brommethylphenyl-2,3,4,6-tetra-O-acetyl-α-D-galactopyranosid der Formel 9 einsetzt.

34. Verfahren zur Herstellung nach einem der Ansprüche 1 bis 27, dadurch **gekennzeichnet**, daß man als Zwischenprodukt 2-Formylphenyl-2,3,4,6-tetra-O-acetyl-α-D-galactopyranosid der Formel 10 einsetzt.

35. Verfahren zur Herstellung nach einem der Ansprüche 1 bis 27, dadurch **gekennzeichnet**, daß man als Zwischenprodukt 2-Hydroxymethylphenyl-2,3,4,6-tetra-O-acetyl-α-D-galactopyranosid der Formel 11 einsetzt.

36. Verfahren zur Herstellung nach einem der Ansprüche 1 bis 27, dadurch **gekennzeichnet**, daß man als Zwischenprodukt (4-Brommethylphenyl-2,3,4,-tri-O-acetyl-β-D-glucopyranosid)methyluronat der Formel 16 einsetzt.

37. Verfahren zur Herstellung nach einem der Ansprüche 1 bis 27, dadurch **gekennzeichnet**, daß man als Zwischenprodukt (4-Formylphenyl-2,3,4,-tri-O-acetyl-β-D-glucopyranosid)methyluronat der Formel 17 einsetzt.

38. Verfahren zur Herstellung nach einem der Ansprüche 1 bis 27, dadurch **gekennzeichnet**, daß man als Zwischenprodukt (4-Hydroxymethylphenyl-2,3,4,-tri-O-acetyl-β-D-glucopyranosid)methyluronat der Formel 18 einsetzt.

39. Verfahren zur Herstellung nach einem der Ansprüche 1 bis 27, dadurch **gekennzeichnet**, daß man als Zwischenprodukt (2-Brommethylphenyl-2,3,4,-tri-O-acetyl-β-D-glucopy-ranosid)methyluronat der Formel 23 einsetzt.

40. Verfahren zur Herstellung nach einem der Ansprüche 1 bis 27, dadurch **gekennzeichnet**, daß man als Zwischenprodukt (2-Hydroxymethylphenyl-2,3,4,-tri-O-acetyl-β-D-glucopyranosid)methyluronat der Formel 24 einsetzt.

41. Verfahren zur Herstellung nach einem der Ansprüche 1 bis 27, dadurch **gekennzeichnet**, daß man als Zwischenprodukt (2-Formylphenyl-2,3,4,-tri-O-acetyl-β-D-glucopyranosid)methyluronat der Formel 25 einsetzt.

42. Verfahren zur Herstellung nach einem der Ansprüche 1 bis 27, dadurch **gekennzeichnet**, daß man als Zwischenprodukt 2-tert.-Butyldimethylsilyloxybenzaldehyd der Formel 28 einsetzt.

43. Verfahren zur Herstellung nach einem der Ansprüche 1 bis 27, dadurch **gekennzeichnet**, daß man als Zwischenprodukt 2-tert.-Butyldimethylsilyloxybenzylalkohol der Formel 29 einsetzt.

44. Verfahren zur Herstellung nach einem der Ansprüche 1 bis 27, dadurch **gekennzeichnet**, daß man als Zwischenprodukt N-[2-Hydroxybenzyloxycarbonyl]daunorubicin der Formel 32 einsetzt.

45. Verfahren zur Herstellung nach einem der Ansprüche 1 bis 27, dadurch **gekennzeichnet**, daß man als Zwischenprodukt 4-Formylphenyl-2,3,4,6-tetra-O-acetyl-β-D-glucopyranosid der Formel 33 einsetzt.

46. Verfahren zur Herstellung nach einem der Ansprüche 1 bis 27, dadurch **gekennzeichnet**, daß man als Zwischenprodukt 4-Hydroxymethylphenyl-2,3,4,6-tetra-O-acetyl-β-D-glucopyranosid der Formel 34 einsetzt.

47. Verfahren zur Herstellung nach einem der Ansprüche 1 bis 27, dadurch **gekennzeichnet**, daß man als Zwischenprodukt N-[2-Hydroxybenzyloxycarbonyl]daunorubicin der Formel 38 einsetzt.

48. Verfahren zur Herstellung nach einem der Ansprüche 1 bis 27, dadurch **gekennzeichnet**, daß man als Zwischenprodukt 2-Methyl-4-nitrophenyl-2,3,4,6-tetra-O-acetyl-α-D-galactopyranosid der Formel 42 einsetzt.

49. Verfahren zur Herstellung nach einem der Ansprüche 1 bis 27, dadurch **gekennzeichnet**, daß man als Zwischenprodukt 2-Brommethyl-4-nitrophenyl-2,3,4,6-tetra-O-acetyl-α-D-galactopyranosid der Formel 43 einsetzt.

50. Verfahren zur Herstellung nach einem der Ansprüche 1 bis 27, dadurch **gekennzeichnet**, daß man als Zwischenprodukt 2-Dibrommethyl-4-nitrophenyl-2,3,4,6-tetra-O-acetyl-α-D-galactopyranosid der Formel 44 einsetzt.

51. Verfahren zur Herstellung nach einem der Ansprüche 1 bis 27, dadurch **gekennzeichnet**, daß man als Zwischenprodukt 2-Formyl-4-nitrophenyl-2,3,4,6-tetra-O-acetyl-α-D-galactopyranosid der Formel 45 einsetzt.

52. Verfahren zur Herstellung nach einem der Ansprüche 1 bis 27, dadurch **gekennzeichnet**, daß man als Zwischenprodukt (2-Hydroxymethyl-4-nitro)phenyl-2,3,-4,6-tetra-O-acetyl-α-D-galactopyranosid der Formel 46-einsetzt.

53. Verfahren zur Herstellung nach einem der Ansprüche 1 bis 27, dadurch **gekennzeichnet**, daß man als Zwischenprodukt (2-Formyl-4-nitrophenyl-2,3,4,-tri-O-acetyl-β-D-glucopyranosid)methyluronat der Formel 51 einsetzt.

54. Verfahren zur Herstellung nach einem der Ansprüche 1 bis 27, dadurch **gekennzeichnet**, daß man als Zwischenprodukt (2-Hydroxymethyl-4-nitrophenyl-2,3,4,-tri-O-acetyl-β-D-glucopyranosid)methyluronat der Formel 52 einsetzt.

55. Verfahren zur Herstellung nach einem der Ansprüche 1 bis 27, dadurch **gekennzeichnet**, daß man als Zwischenprodukt 2-Chlor-4-methylphenyl-2,3,4,6-tetra-O-acetyl-α-D-galactopyranosid der Formel 55 einsetzt.

56. Verfahren zur Herstellung nach einem der Ansprüche 1 bis 27, dadurch **gekennzeichnet**, daß man als Zwischenprodukt 2-Chlor-4-bromethylphenyl-2,3,4,6-tetra-O-acetyl-α-D-galactopyranosid der Formel 56 einsetzt.

57. Verfahren zur Herstellung nach einem der Ansprüche 1 bis 27, dadurch **gekennzeichnet**, daß man als Zwischenprodukt 2-Chlor-4-hydroxymethylphenyl-2,3,4,6-tetra-O-acetyl-α-D-galactopyranosid der Formel 57 einsetzt.

58. Verfahren zur Herstellung nach einem der Ansprüche 1 bis 27, dadurch **gekennzeichnet**, daß man als Zwischenprodukt (4-Formyl-2-nitrophenyl-2,3,4,-tri-O-acetyl-β-D-glucopyranosid)methyluronat der Formel 61 einsetzt.

59. Verfahren zur Herstellung nach einem der Ansprüche 1 bis 27, dadurch **gekennzeichnet**, daß man als Zwischenprodukt (2-Hydroxymethyl-2-nitrophenyl-2,3,4,-tri-O-acetyl-β-D-glucopyranosid)methyluronat der Formel 62 einsetzt.

60. Verfahren zur Herstellung nach einem der Ansprüche 1 bis 27, dadurch **gekennzeichnet**, daß man als Zwischenprodukt (4-Brommethyl-4-nitro)phenyl-2,3,4,6-tetra-O-acetyl-α-D-galactopyranosid der Formel 73 einsetzt.

61. Verfahren zur Herstellung nach einem der Ansprüche 1 bis 27, dadurch **gekennzeichnet**, daß man als Zwischenprodukt (4-Hydroxymethyl-2-nitro)phenyl-2,3,4,6-tetra-O-acetyl-α-D-galactopyranosid der Formel 74 einsetzt.

62. Verfahren zur Herstellung nach einem der Ansprüche 1 bis 27, dadurch **gekennzeichnet**, daß man als Zwischenprodukt 4-Hydroxy-3-chlorbenzaldehyd-2,3,4,-tri-O-acetyl-β-D-methylglucuronid der Formel 76 einsetzt.

63. Verfahren zur Herstellung nach einem der Ansprüche 1 bis 27, dadurch **gekennzeichnet**, daß man als Zwischenprodukt (4-Hydroxymethyl-2-chlor)phenyl-2,3,4,-tri-O-acetyl-β-D-methylglucuronid der Formel 77 einsetzt.

64. Verfahren zur Herstellung nach einem der Ansprüche 1 bis 27, dadurch **gekennzeichnet**, daß man als Zwischenprodukt 2,5-Dioxopyrolidin-1-yl-4-(2,3,4,6-tetra-O-acetyl-α-D-galactopyranosyl)benzylcarbonat der Formel 5 einsetzt.

65. Verfahren zur Herstellung nach einem der Ansprüche 1 bis 27, dadurch **gekennzeichnet**, daß man als Zwischenprodukt 2,5-Dioxopyrolidin-1-yl-2-(2,3,4,6-tetra-O-acetyl-α-D-galactopyranosyl)benzylcarbonat der Formel 12 einsetzt.

66. Verfahren zur Herstellung nach einem der Ansprüche 1 bis 27, dadurch **gekennzeichnet**, daß man als Zwischenprodukt 2,5-Dioxopyrolidin-1-yl-4-((2,3,4-tri-O-acetyl-β-D-glucopyranosyl)methyluronat)benzylcarbonat der Formel 19 einsetzt.

67. Verfahren zur Herstellung nach einem der Ansprüche 1 bis 27, dadurch **gekennzeichnet**, daß man als Zwischenprodukt 4-Nitrophenyl-2-((2,3,4-tri-O-acetyl-β-D-glucopyranosyl)methyluronat)benzylcarbonat der Formel 26 einsetzt.

68. Verfahren zur Herstellung nach einem der Ansprüche 1 bis 27, dadurch **gekennzeichnet**, daß man als Zwischenprodukt 4-Nitrophenyl-2-(tert.-butyldimethylsilyloxy)benzylcarbonat der Formel 30 einsetzt.

69. Verfahren zur Herstellung nach einem der Ansprüche 1 bis 27, dadurch **gekennzeichnet**, daß man als Zwischenprodukt 2,5-Dioxopyrolidin-1-yl-4-(2,3,4,6-tetra-O-acetyl-β-D-glucopyranosyl)benzylcarbonat der Formel 35 einsetzt.

70. Verfahren zur Herstellung nach einem der Ansprüche 1 bis 27, dadurch **gekennzeichnet**, daß man als Zwischenprodukt 2,5-Dioxopyrolidin-1-yl-4-dimethylhexylsilyloxybenzylcarbonat der Formel 40 einsetzt.

71. Verfahren zur Herstellung nach einem der Ansprüche 1 bis 27, dadurch **gekennzeichnet**, daß man als Zwischenprodukt 4-Nitrophenyl-2-(2,3,4,6-tetra-O-acetyl-α-D-galactopyranosyl)-5-nitrobenzylcarbonat der Formel 47 einsetzt.

72. Verfahren zur Herstellung nach einem der Ansprüche 1 bis 27, dadurch **gekennzeichnet**, daß man als Zwischenprodukt 4-Nitrophenyl-2-((2,3,4-tri-O-acetyl-β-D-glucopyranosyl)methyluronat)-5-nitrobenzylcarbonat der Formel 53 einsetzt.

73. Verfahren zur Herstellung nach einem der Ansprüche 1 bis 27, dadurch **gekennzeichnet**, daß man als Zwischenprodukt 4-Nitrophenyl-4-methoxy-5-nitro-2-((2,3,4-tri-O-acetyl-β-D-qlucopyranosyl)methyluronat)benzylcarbonat der Formel 67 einsetzt.

74. Verfahren zur Herstellung nach einem der Ansprüche 1 bis 27, dadurch **gekennzeichnet**, daß man als Zwischenprodukt 4-Nitrophenyl-4-(2,3,4-tri-O-acetyl-β-D-glucopyranosyl)methyluronat)-5-nitrobenzylcarbonat der Formel 69 einsetzt.

75. Verfahren zur Herstellung nach einem der Ansprüche 1 bis 27, dadurch **gekennzeichnet**, daß man als Zwischenprodukt 4-Chlorphenyl-2-((2,3,4-tri-O-acetyl-β-D-glucopyranosyl)methyluronat)-5-nitrobenzylcarbonat der Formel 81 einsetzt.

76. Verfahren zur Herstellung eines Arzneimittels zur Behandlung von Krankheiten, an denen aktivierte Makrophagen, aktivierte Granulozyten, aktivierte Thrombozyten oder aktivierte Tumorzellen beteiligt sind, dadurch **gekennzeichnet**, daß man eine Arzneimittelvorstufe nach einem der Ansprüche 1 bis 27 einsetzt.

## Claims (Claims for the following Contracting State(s): AT, BE, CH, DE, DK, FR, GB, IT, LI, LU, NL, PT, SE)

1. An anthracycline prodrug, characterized in that it has formula I below: 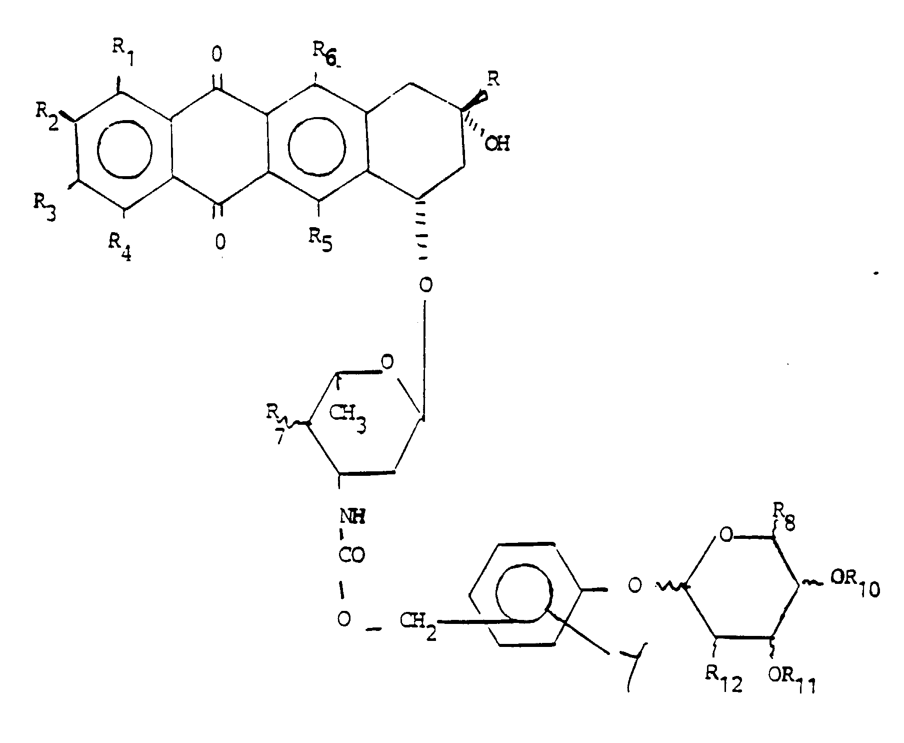 in which
R₁, R₂ and R₃, which can be identical or different, are a hydrogen atom or a hydroxyl group;
R₄ is a hydrogen atom, a hydroxyl group or a methoxy group;
R is a group CO-CH₂-R", in which R" is a hydrogen atom, a C₁-C₆ alkyl group, a hydroxyl group, an alkoxy group, an O-acyl group or an aryl group;
R₅ and R₆, which can be identical or different, are a hydrogen atom or a hydroxyl group;
R₇ is a hydrogen atom or a hydroxyl group;
R₈ is a group -CH₂-OR₉ or a group COOR₉, where R₉ is a C₁-C₃ alkyl or a hydrogen atom;
R₁₀ and R₁₁ are a hydrogen atom, an acyl protecting group or an alkyl group;
R₁₂ is a hydroxyl group, an amine group, an amide group or an O-acyl protecting group;
the benzyl -CH₂ is preferably in the para or ortho position to the glycosyl oxygen; and
Y is a hydrogen atom, or at least one electron-attracting group selected especially from the group comprising the NO₂ group, a halogen atom and a group SO₂X (where X =-CH₃, C₆H₄-CH₃, NH₂, N-(C₁-C₄ alkyl)₂ or NH-C₁-C₄ alkyl), -CN, acyl or COO-alkyl, and/or at least one electron-donating group selected from the group comprising groups of the type O-alkyl, NHCO- alkyl, N(alkyl)CO-alkyl, S-alkyl or alkyl.

2. A prodrug according to Claim 1, characterized in that when Y is one or more electron-attracting groups, these are preferably in the ortho and/or para position to the glycosyl oxygen, and when Y is one or more electron-donating groups, these are preferably in the meta position.

3. A prodrug according to Claim 1 or Claim 2, characterized in that the preferred compounds of formula I contain the following radicals in particular:
R₁, R₂ and R₃ are hydrogen atoms,
R₄ is a methoxy group,
R₅ and R₆ are hydroxyl groups,
R is a -CO-CH₃ group or a -CO-CH₂OH group,
R₇ is a hydrogen atom or a hydroxyl group,
R₈ is a -CH₂-OAc, -CH₂OH, -COOMe or -COOH group,
R₁₀ and R₁₁, which can be identical or different, are a hydrogen atom or an Ac group,
R₁₂ is a hydroxyl group or an OAc group,
said radicals R₈, R₁₀, R₁₁ and R₁₂ preferably being in the following positions: 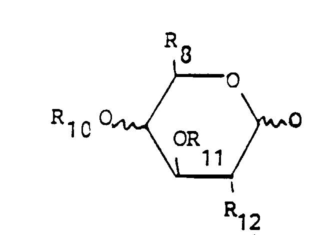 and
Y is a hydrogen atom, an NO₂ group or a chlorine atom in the para or ortho position to the glycosyl oxygen, and/or an OCH₃ group in the meta position to the glycosyl oxygen.

4. A prodrug according to any one of Claims 1 to 3, characterized in that it has formula 6 below:

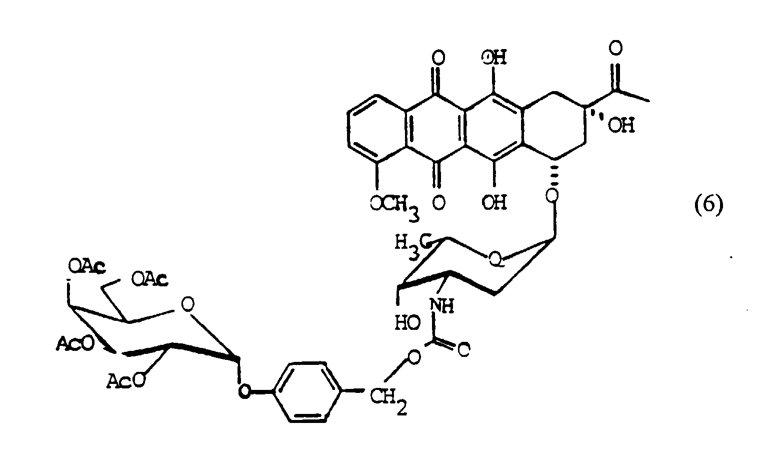

5. A prodrug according to any one of Claims 1 to 3, characterized in that it has formula 7 below:

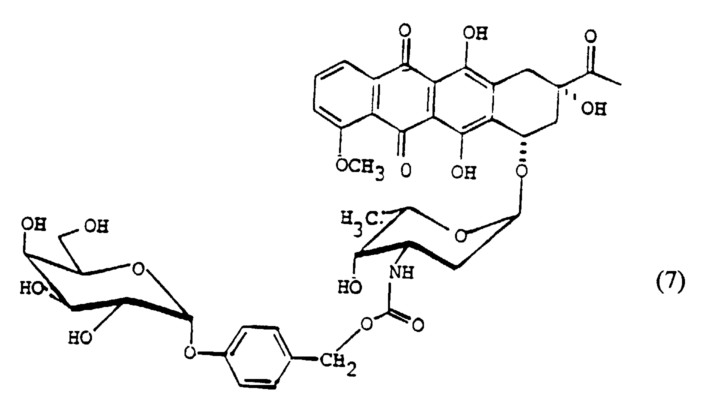

6. A prodrug according to any one of Claims 1 to 3, characterized in that it has formula 13 below:

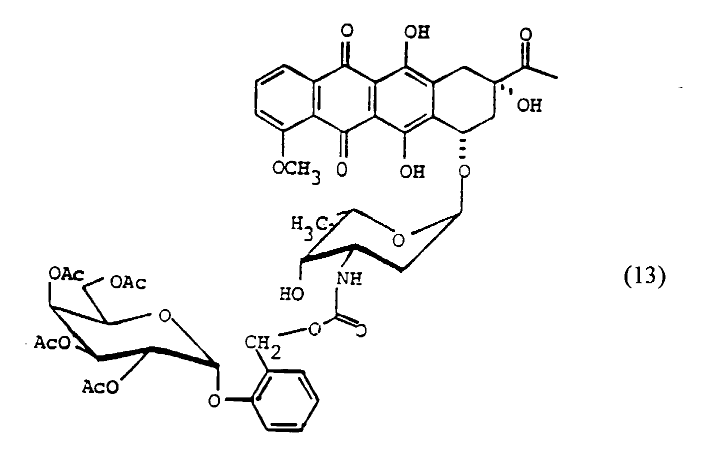

7. A prodrug according to any one of Claims 1 to 3, characterized in that it has formula 14 below:

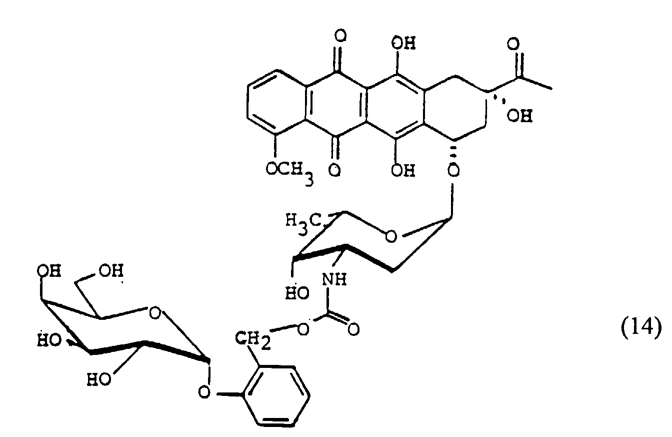

8. A prodrug according to any one of Claims 1 to 3, characterized in that it has formula 22 below:

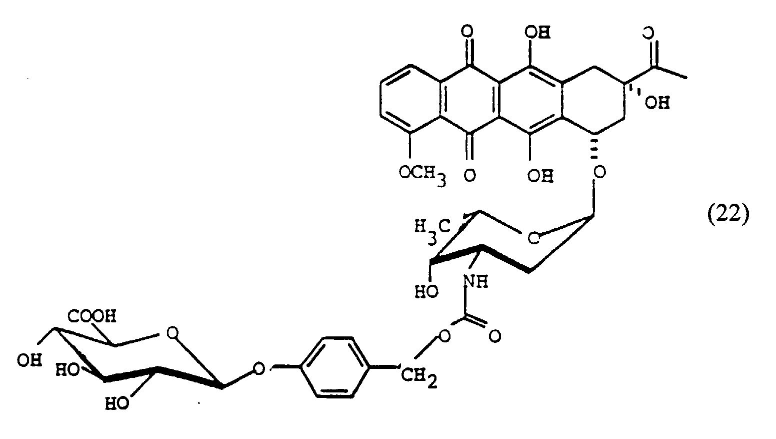

9. A prodrug according to any one of Claims 1 to 3, characterized in that it has formula 27c below:

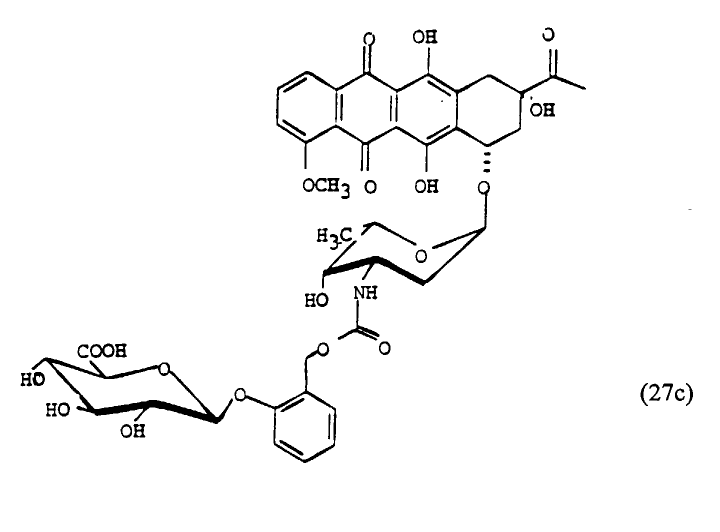

10. A prodrug according to any one of Claims 1 to 3, characterized in that it has formula 36 below:

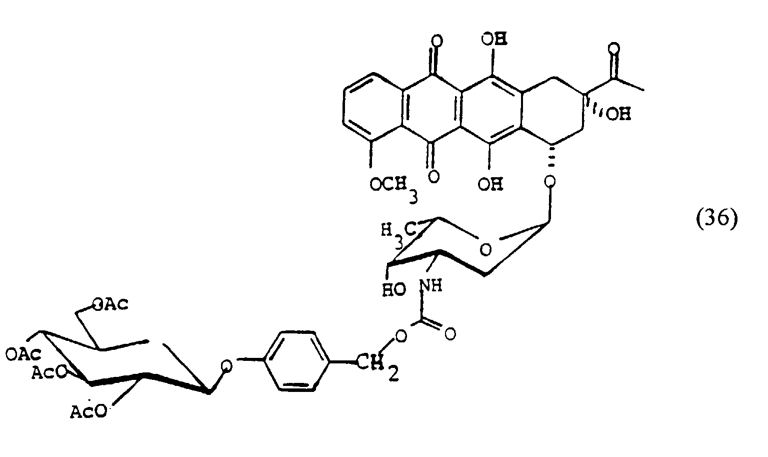

11. A prodrug according to any one of Claims 1 to 3, characterized in that it has formula 37 below:

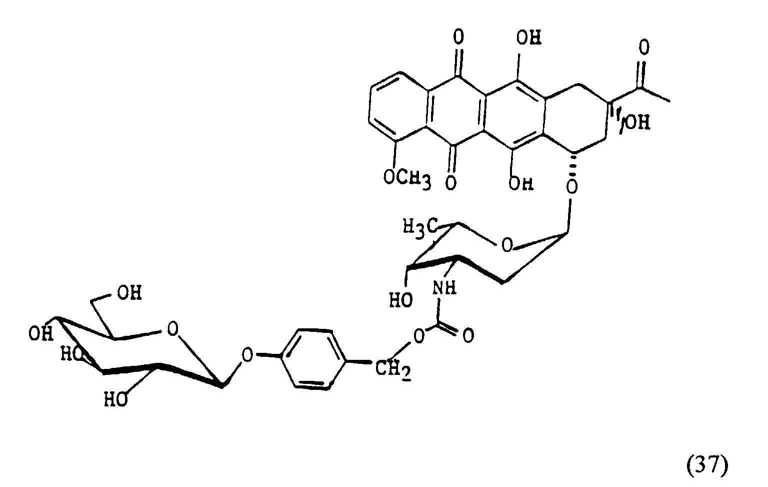

12. A prodrug according to any one of Claims 1 to 3, characterized in that it has formula 48a below:

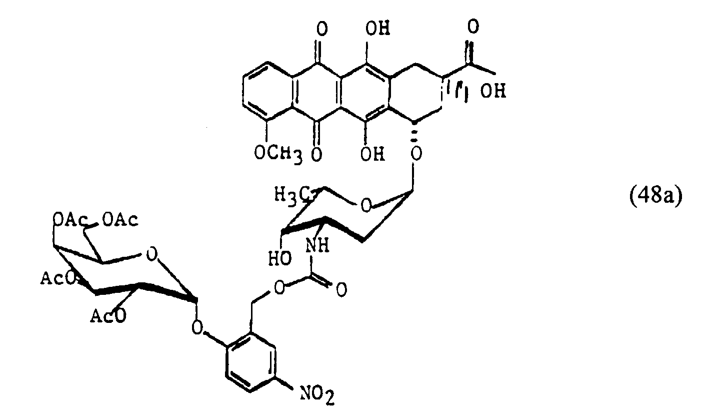

13. A prodrug according to any one of Claims 1 to 3, characterized in that it has formula 48b below:

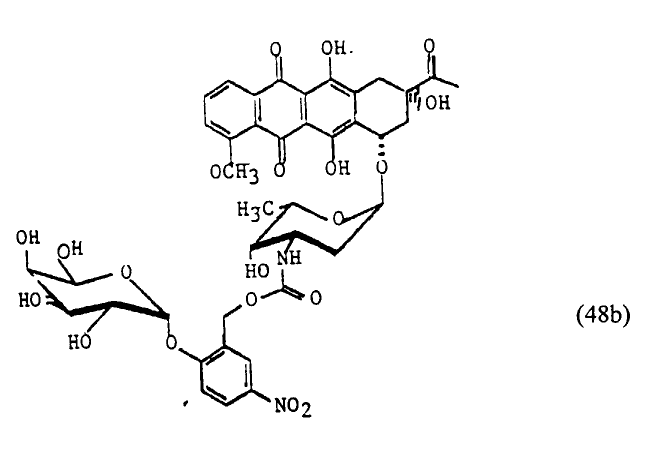

14. A prodrug according to any one of Claims 1 to 3, characterized in that it has formula 49a below:

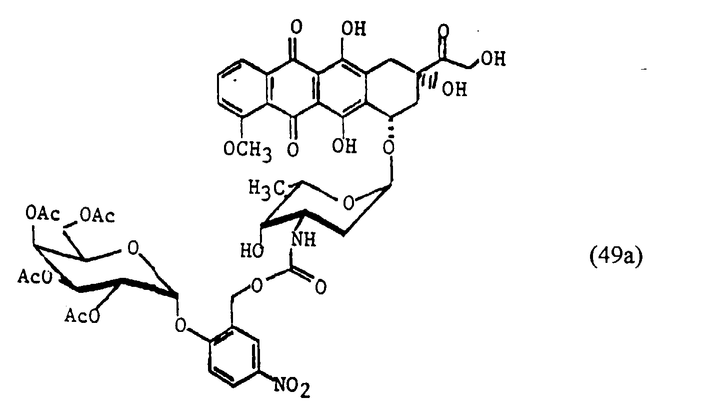

15. A prodrug according to any one of Claims 1 to 3, characterized in that it has formula 49b below:

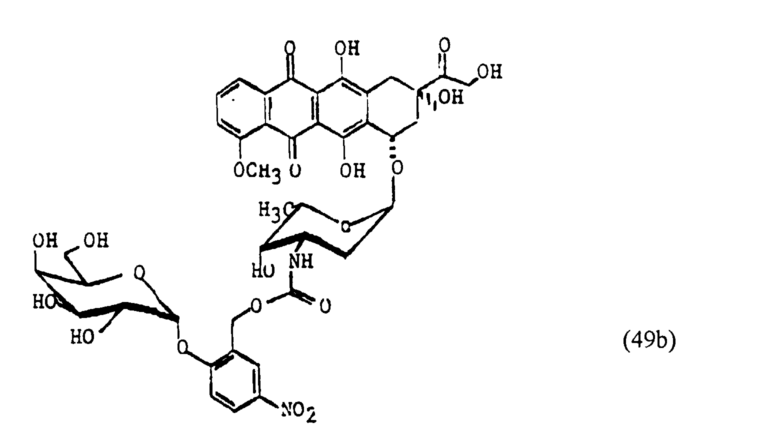

16. A prodrug according to any one of Claims 1 to 3, characterized in that it has formula 54c below:

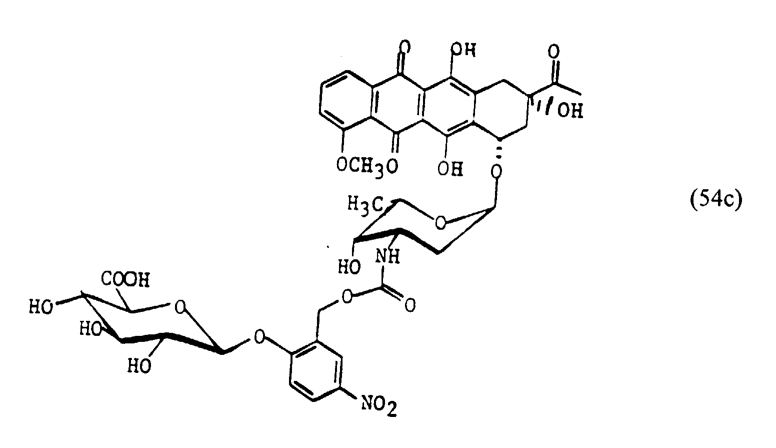

17. A prodrug according to any one of Claims 1 to 3, characterized in that it has formula 59 below:

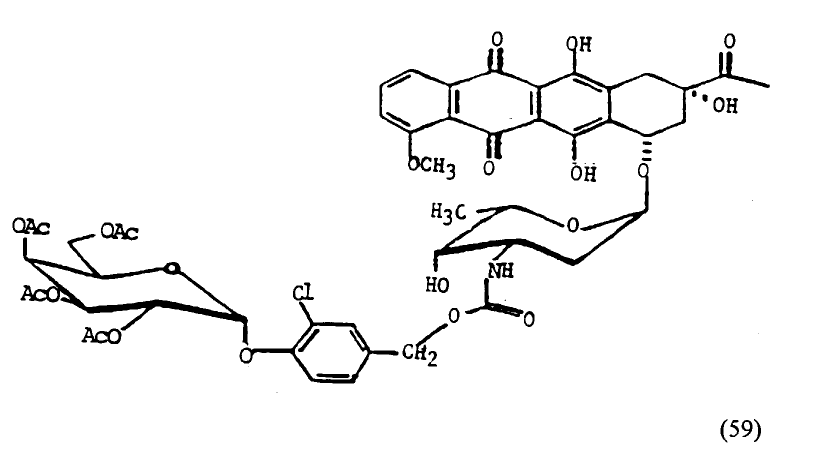

18. A prodrug according to any one of Claims 1 to 3, characterized in that it has formula 60 below:

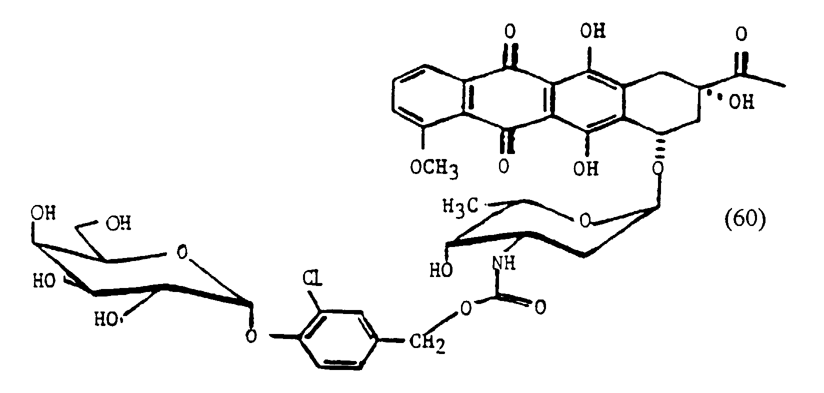

19. A prodrug according to any one of Claims 1 to 3, characterized in that it has formula 64c:

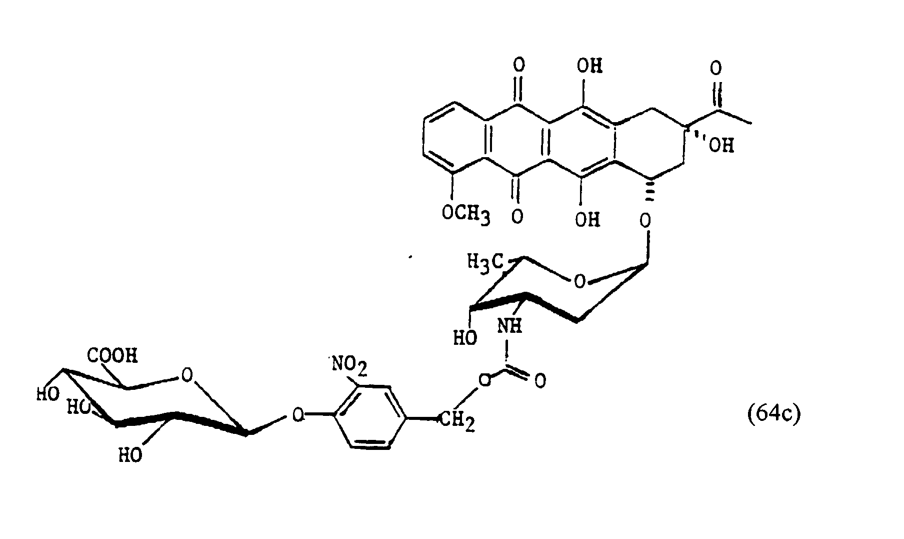

20. A prodrug according to any one of Claims 1 to 3, characterized in that it has formula 68c below:

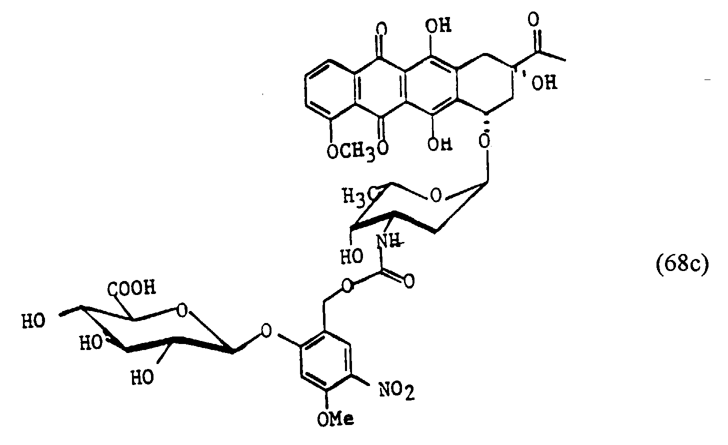

21. A prodrug according to any one of Claims 1 to 3, characterized in that it has formula 75a below:

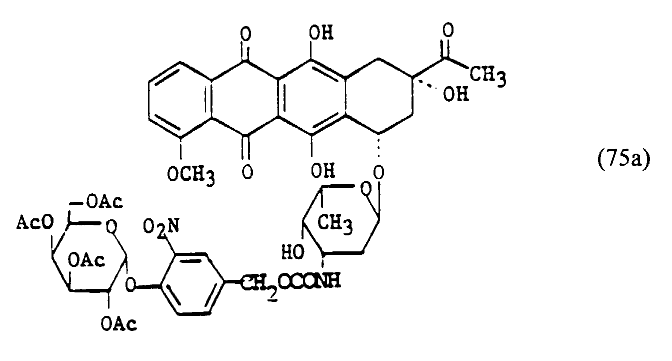

22. A prodrug according to any one of Claims 1 to 3, characterized in that it has formula 75b below:

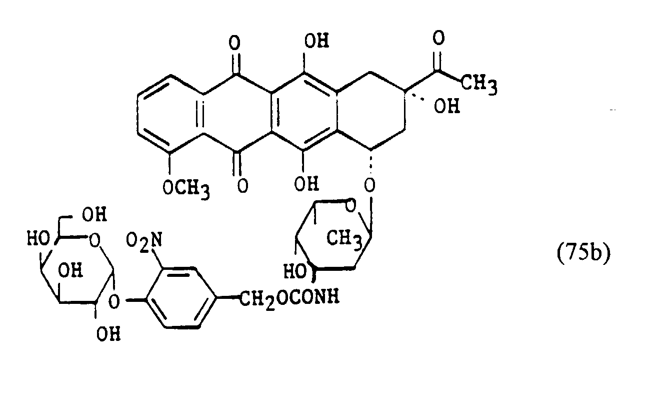

23. A prodrug according to any one of Claims 1 to 3, characterized in that it has formula 78a below:

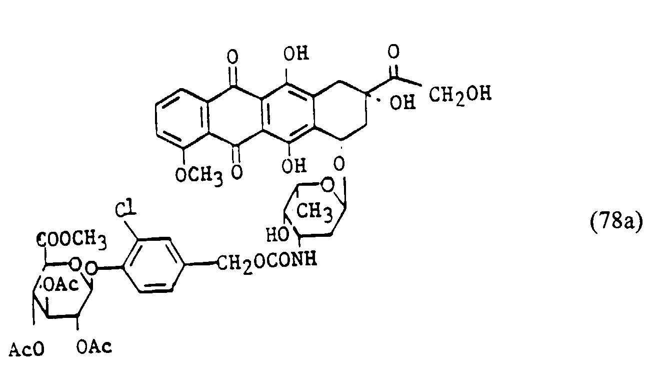

24. A prodrug according to any one of Claims 1 to 3, characterized in that it has formula 78b below:

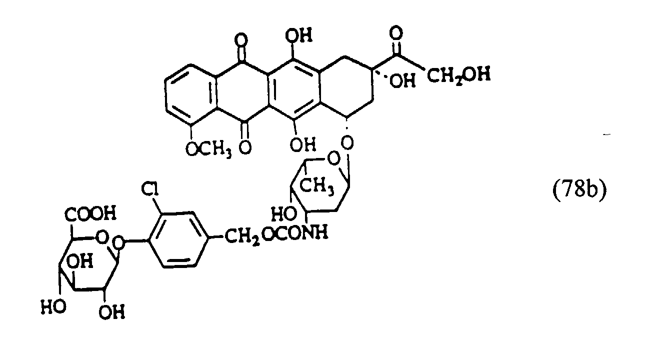

25. A prodrug according to any one of Claims 1 to 3, characterized in that it has formula 70c below: 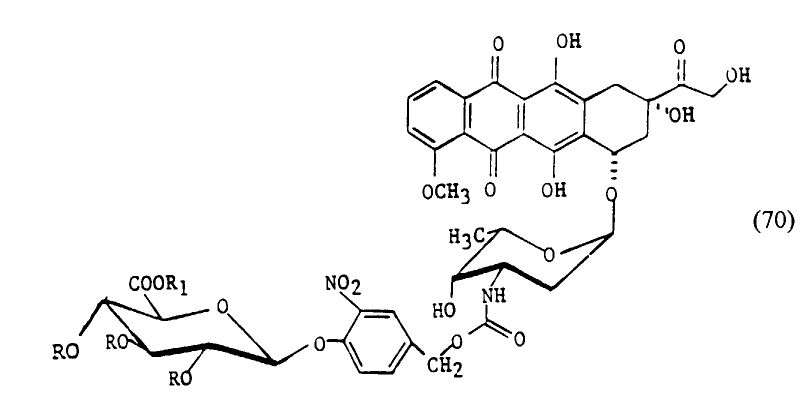 with:
70a: R=Ac R₁=CH₃
70b: R=H R₁=CH₃
70c: R=R₁=H

26. A prodrug according to any one of Claims 1 to 3, characterized in that it has formula 82 below: 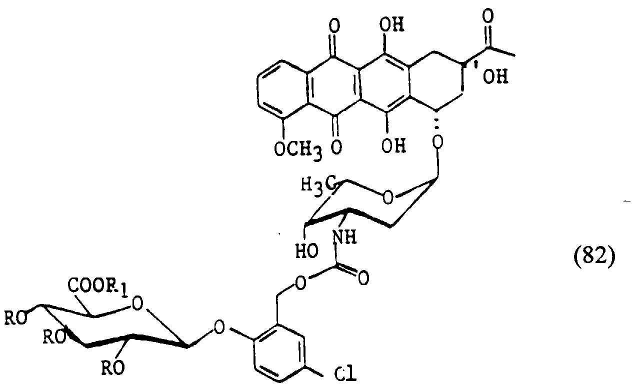 with:
82a : R=Ac, R₁=CH₃
82b : R=H, R₁=CH₃
82c : R=R₁=H

27. A prodrug according to any one of Claims 1 to 3, characterized in that it has formula 83 below: 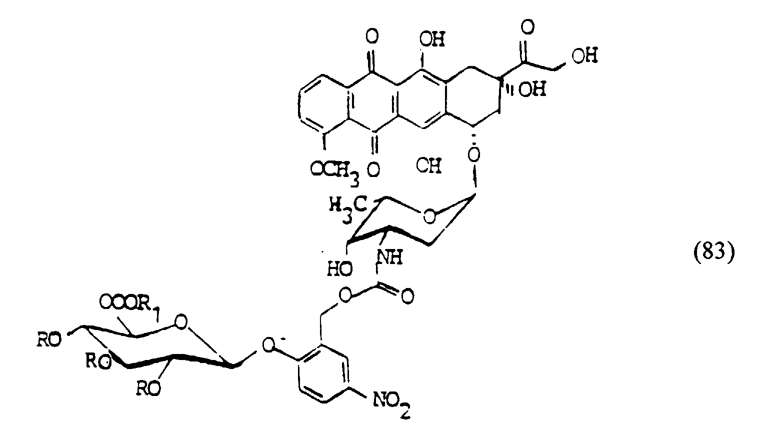 with:
83a : R=Ac, R₁=CH₃
83b : R=H, R₁=CH₃
83c : R=R₁=H

28. A method of preparing a compound of formula I, which in particular can be degraded by a glycosidase, characterized in that it comprises:
(1) the coupling, if appropriate in the presence of a suitable promoter, of a derivative of formula A: 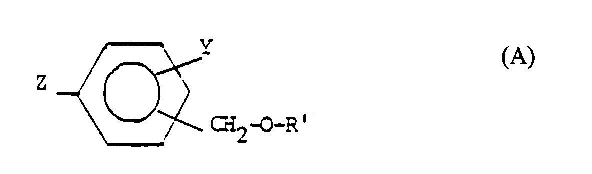 in which
Z is a hydroxyl group, an O-trialkylsilyl group or a group 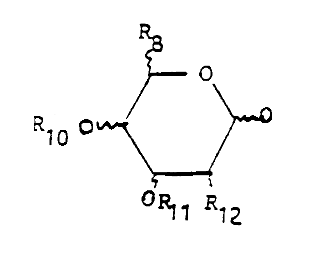 in which R₈, R₁₀, R₁₁ and R₁₂ are as defined above;
R' is one of the following groups: 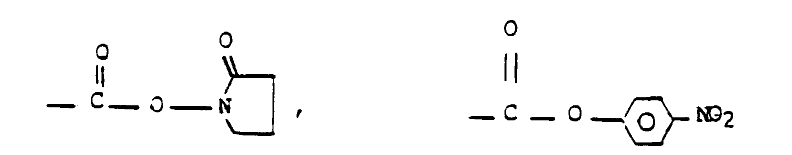 the benzyl -CH₂ is preferably in the para or ortho position to the phenol group, which may be modified (glycosylated or silylated); and
Y is a hydrogen atom, or at least one electron-attracting group selected especially from the group comprising the NO₂ group, a halogen atom and a group SO₂X (where X = -CH₃, C₆H₄-CH₃, NH₂, N-(C₁-C₄ alkyl)₂ or NH-C₁-C₄alkyl), -CN, acyl or COO-alkyl, and/or at least one electron-donating group selected from the group comprising groups of the type O-alkyl, NH-CO-alkyl, N(alkyl)CO-alkyl, S-alkyl or alkyl,
with an anthracycline of formula B: 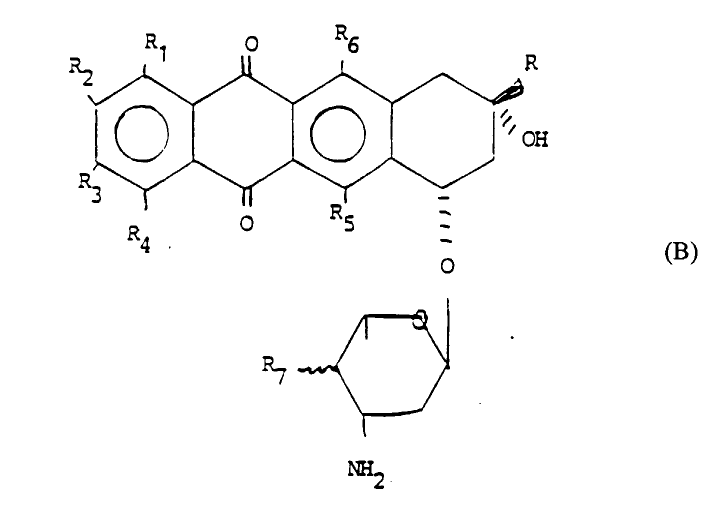 in which R₁, R₂, R₃, R₄, R₅, R₆, R₇ and R are as defined above;
(2) the removal of the protecting groups present in the compounds obtained, especially by hydrolysis, transesterification or saponification; and
(3) if appropriate, suitable condensation with an ose of the following formula: 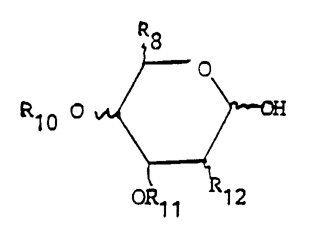 in the case where Z is a hydroxyl group or an O-tri-alkylsilyl group, to give an anthracycline prodrug of formula I, in which all the radicals R₁ to R₁₂ and R are as defined above.

29. A method according to Claim 28, characterized in that, prior to step (1), the glycosylated p-hydroxybenzyl derivative is obtained by:
(a) fusion of a cresol with an ose or a peracetylated methyl glucuronate,
(b) benzyl bromination of the product obtained,
(c) solvolysis of the brominated derivative, and
(d) activation of the hydroxyl group with a hydroxysuccinimidyl or paranitrophenoxycarbonyl derivative.

30. Products comprising a modified anthracycline prodrug according to any one of Claims 1 to 27, and an enzyme/tumour-specific antibody conjugate of formula II:
Ab-Sp-E (II)
in which
Ab is an antibody or one of its fragments, which is specific towards an antigen associated with a tumour, or is a biomolecule which tends to accumulate in a tumour, such as EGF (epidermal growth factor), α-TGF (α transforming growth factor), PDGF (platelet derived growth factor), IGF I+II (insulin growth factor I+II) or FGF a+b (fibroblast growth factor a+b),
E is a glycosidase which is not immunogenic or has a very low immunogenicity, preferably a mammalian glycosidase such as α-or β-glucosidase, α-galactosidase, α- or β-mannosidase, α-fucosidase, N- acetyl-α-galactosaminidase, N-acetyl-β-/N-acetyl-α-glucosaminidase or β-glucuronidase, and
Sp (arm) is a group containing a sulphide or a disulphide, of formula III or IV:
X'(S)ₙY' (III)
X'(S)ₙ (IV)
or a polypeptide arm, in which
X' or Y' is -CO-R₁₃-(N-succinimido)- or -C(=R₁₄)-CH₂-CH₂-, where R₁₃ is a -CH₂-CH₂, 1,4-cyclohexylidene, 1,3- or 1,4-phenylene or methoxycarbonyl- or chloro- 1,4-phenylene group and R₁₄ is an oxygen atom or an NH group,
Y' represents -C(=R₁₄)-CH₂-CH₂ when R₁₄ is as defined above, and
n is 1 or 2,
for use in cytostatic therapy, either simultaneously, separately or spread out over a period of time.

31. 4-Bromomethylphenyl 2,3,4,6-tetra-O-acetyl-α-D-galactopyranoside of formula 2, an intermediate in the method according to Claim 28.

32. 4-Formylphenyl 2,3,4,6-tetra-O-acetyl-α-D-galactopyranoside of formula 3, an intermediate in the method according to Claim 28.

33. 4-Hydroxymethylphenyl 2,3,4,6-tetra-O-acetyl-α-D-galactopyranoside of formula 4, an intermediate in the method according to Claim 28.

34. 2-Bromomethylphenyl 2,3,4,6-tetra-O-acetyl-α-D-galactopyranoside of formula 9, an intermediate in the method according to Claim 28.

35. 2-Formylphenyl 2,3,4,6-tetra-O-acetyl-α-D-galactopyranoside of formula 10, an intermediate in the method according to Claim 28.

36. 2-Hydroxymethylphenyl 2,3,4,6-tetra-O-acetyl-α-D-galactopyranoside of formula 11, an intermediate in the method according to Claim 28.

37. Methyl (4-bromomethylphenyl 2,3,4-tri-O-acetyl-β-D-glucopyranoside)uronate of formula 16, an intermediate in the method according to Claim 28.

38. Methyl (4-formylphenyl 2,3,4-tri-O-acetyl-β-D-glucopyranoside)uronate of formula 17, an intermediate in the method according to Claim 28.

39. Methyl (4-hydroxymethylphenyl 2,3,4-tri-O-acetyl-β-D-glucopyranoside)uronate of formula 18, an intermediate in the method according to Claim 28.

40. Methyl (2-bromomethylphenyl 2,3,4-tri-O-acetyl-β-D-glucopyranoside)uronate of formula 23, an intermediate in the method according to Claim 28.

41. Methyl (2-hydroxymethylphenyl 2,3,4-tri-O-acetyl-β-D-glucopyranoside)uronate of formula 24, an intermediate in the method according to Claim 28.

42. Methyl (2-formylphenyl 2,3,4-tri-O-acetyl-β-D-glucopyranoside)uronate of formula 25, an intermediate in the method according to Claim 28.

43. 2-Tert-butyldimethylsilyloxybenzaldehyde of formula 28, an intermediate in the method according to Claim 28.

44. 2-Tert-butyldimethylsilyloxybenzyl alcohol of formula 29, an intermediate in the method according to Claim 28.

45. N-[2-Hydroxybenzyloxycarbonyl]daunorubicin of formula 32, an intermediate in the method according to Claim 28.

46. 4-Formylphenyl 2,3,4,6-tetra-O-acetyl-β-D-glucopyranoside of formula 33, an intermediate in the method according to Claim 28.

47. 4-Hydroxymethylphenyl 2,3,4,6-tetra-O-acetyl-β-D-glucopyranoside of formula 34, an intermediate in the method according to Claim 28.

48. N-[4-Hydroxybenzyloxycarbonyl]daunorubicin of formula 38, an intermediate in the method according to Claim 28.

49. 2-Methyl-4-nitrophenyl 2,3,4,6-tetra-O-acetyl-α-D- galactopyranoside of formula 42, an intermediate in the method according to Claim 28.

50. 2-Bromomethyl-4-nitrophenyl 2,3,4,6-tetra-O-acetyl-α-D-galactopyranoside of formula 43, an intermediate in the method according to Claim 28.

51. 2-Dibromomethyl-4-nitrophenyl 2,3,4,6-tetra-O-acetyl-α-D-galactopyranoside of formula 44, an intermediate in the method according to Claim 28.

52. 2-Formyl-4-nitrophenyl 2,3,4,6-tetra-O-acetyl-α-D- galactopyranoside of formula 45, an intermediate in the method according to Claim 28.

53. 2-Hydroxymethyl-4-nitrophenyl 2,3,4,6-tetra-O-acetyl-α-D-galactopyranoside of formula 46, an intermediate in the method according to Claim 28.

54. Methyl (2-formyl-4-nitrophenyl 2,3,4-tri-O-acetyl-β-D-glucopyranoside)uronate of formula 51, an intermediate in the method according to Claim 28.

55. Methyl (2-hydroxymethyl-4-nitrophenyl 2,3,4-tri-O- acetyl-β-D-glucopyranoside)uronate of formula 52, an intermediate in the method according to Claim 28.

56. 2-Chloro-4-methylphenyl 2,3,4,6-tetra-O-acetyl-α-D- galactopyranoside of formula 55, an intermediate in the method according to Claim 28.

57. 2-Chloro-4-bromomethylphenyl 2,3,4,6-tetra-O-acetyl-α-D-galactopyranoside of formula 56, an intermediate in the method according to Claim 28.

58. 2-Chloro-4-hydroxymethylphenyl 2,3,4,6-tetra-O-acetyl-α-D-galactopyranoside of formula 57, an intermediate in the method according to Claim 28.

59. Methyl (4-formyl-2-nitrophenyl 2,3,4-tri-O-acetyl-β-D-glucopyranoside)uronate of formula 61, an intermediate in the method according to Claim 28.

60. Methyl (4-hydroxymethyl-2-nitrophenyl 2,3,4-tri-O-acetyl-β-D-glucopyranoside)uronate of formula 62, an intermediate in the method according to Claim 28.

61. (4-bromomethyl 2-nitro)-phenyl 2,3,4,6-tetra-O-acetyl-α-D-galactopyranoside of formula 73, an intermediate in the method according to claim 28.

62. (4-hydroxymethyl 2-nitro)-phenyl 2,3,4,6-tetra-O-acetyl-α-D-galactopyranoside of formula 74, an intermediate in the method according to claim 28.

63. 4-hydroxy-3-chlorobenzaldehyde 2,3,4-tri-O-acetyl-β-D-methylglucuronide of formula 76, an intermediate in the method according to claim 28.

64. (4-hydroxy-methyl-2-chloro)-phenyl 2,3,4-tri-O-acetyl-β-D-methylglucuronide of formula 77, an intermediate in the method according to claim 28.

65. 2,5-Dioxopyrrolidin-1-yl 4-(2,3,4,6-tetra-O-acetyl-α-D-galactopyranosyl)benzyl carbonate of formula 5, an intermediate in the method according to Claim 28.

66. 2,5-Dioxopyrrolidin-1-yl 2-(2,3,4,6-tetra-O-acetyl-α-D-galactopyranosyl)benzyl carbonate of formula 12, an intermediate in the method according to Claim 28.

67. 2,5-Dioxopyrrolidin-1-yl 4-(methyl (2,3,4-tri-O-acetyl-β-D-glucopyranosyl)uronate)benzyl carbonate of formula 19, an intermediate in the method according to Claim 28.

68. 4-Nitrophenyl 2-(methyl (2,3,4-tri-O-acetyl-β-D-glucopyranosyl)uronate)benzyl carbonate of formula 26, an intermediate in the method according to Claim 28.

69. 4-Nitrophenyl 2-(tert-butyldimethylsilyloxy)benzyl carbonate of formula 30, an intermediate in the method according to Claim 28.

70. 2,5-Dioxopyrrolidin-1-yl 4-(2,3,4,6-tetra-O-acetyl-β-D-glucopyranosyl)benzyl carbonate of formula 35, an intermediate in the method according to Claim 28.

71. 2,5-Dioxopyrrolidin-1-yl 4-dimethyl-t-hexylsilyloxybenzyl carbonate of formula 40, an intermediate in the method according to Claim 28.

72. 4-Nitrophenyl 2-(2,3,4,6-tetra-O-acetyl-α-D-galactopyranosyl)-5-nitrobenzyl carbonate of formula 47, an intermediate in the method according to Claim 28.

73. 4-Nitrophenyl 2-(methyl (2,3,4-tri-O-acetyl-β-D-glucopyranosyl)uronate)-5-nitrobenzyl carbonate of formula 53, an intermediate in the method according to Claim 28.

74. 4-Nitrophenyl 4-methoxy-5-nitro-2-(methyl (2,3,4-tri-O-acetyl-β-D-glucopyranosyl)uronate)benzyl carbonate of formula 67, an intermediate in the method according to Claim 28.

75. 4-Nitro-phenyl 4-(methyl (2,3,4-tri-O-acetyl-β-glucopyranosyl) uronate)-5-nitro-benzyl carbonate of formula 69, an intermediate in the method according to claim 28.

76. 4-chlorophenyl 2-(methyl (2,3,4-tri-O-acetyl-β-D-glucopyranosyl) uronate)-5-nitrobenzyl carbonate of formula 81, an intermediate in the method according to claim 28.

77. Application of the prodrugs according to claims 1 to 27 to the preparation of drugs for the treatment of diseases, which involve activated macrophages, granulocytes, thrombocytes or human tumoural cells.

## Claims (Claims for the following Contracting State(s): ES, GR)

1. A method of preparing anthracycline prodrugs having the following formula I: 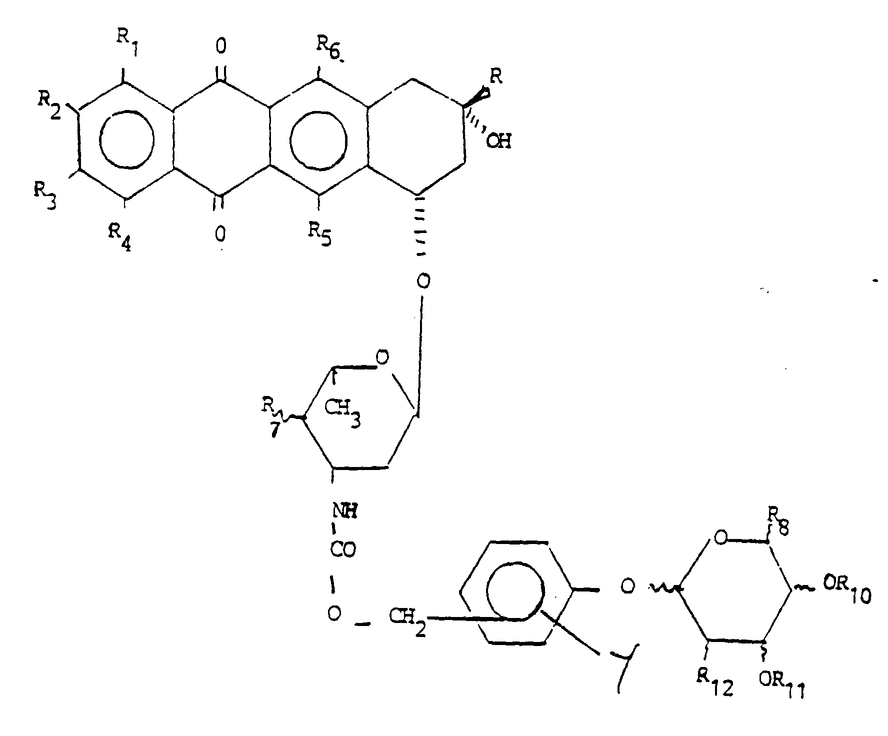 in which
R₁, R₂ and R₃, which can be identical or different, are a hydrogen atom or a hydroxyl group;
R₄ is a hydrogen atom, a hydroxyl group or a methoxy group;
R is a group CO-CH₂-R", in which R" is a hydrogen atom, a C₁-C₆ alkyl group, a hydroxyl group, an alkoxy group, an O-acyl group or an aryl group;
R₅ and R₆, which can be identical or different, are a hydrogen atom or a hydroxyl group;
R₇ is a hydrogen atom or a hydroxyl group;
R₈ is a group -CH₂-OR₉ or a group COOR₉, where R₉ is a C₁-C₃ alkyl or a hydrogen atom;
R₁₀ and R₁₁ are a hydrogen atom, an acyl protecting group or an alkyl group;
R₁₂ is a hydroxyl group, an amine group, an amide group or an O-acyl protecting group;
the benzyl -CH₂ is preferably in the para or ortho position to the glycosyl oxygen; and Y is a hydrogen atom, or at least one electron-attracting group selected especially from the group comprising the NO₂ group, a halogen atom and a group SO₂X (where X =-CH₃, C₆H₄-CH₃, NH₂, N-(C₁-C₄ alkyl)₂ or NH-C₁-C₄ alkyl), -CN, acyl or COO-alkyl, and/or at least one electron-donating group selected from the group comprising groups of the type O-alkyl, NHCO- alkyl, N(alkyl)CO-alkyl, S-alkyl or alkyl.
which can be degraded by a glycosidase, characterized in that it comprises:
(1) the coupling, if appropriate in the presence of a suitable promoter, of a derivative of formula A: 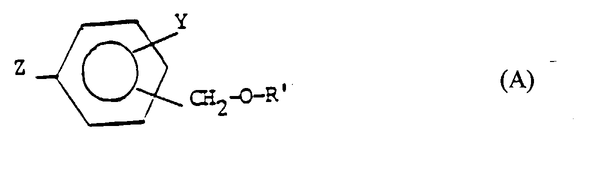 in which
Z is a hydroxyl group, an O-trialkylsilyl group or a group 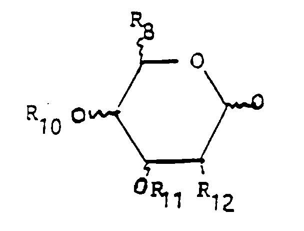 in which R₈, R₁₀, R₁₁ and R₁₂ are as defined above;
R' is one of the following groups: 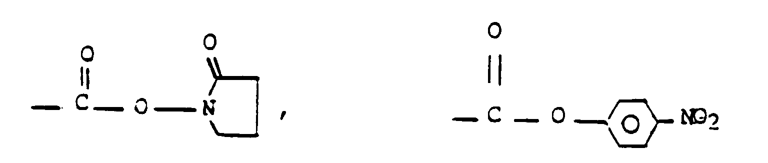 the benzyl -CH₂ is preferably in the para or ortho position to the phenol group, which may be modified (glycosylated or silylated); and
Y is a hydrogen atom, or at least one electron-attracting group selected especially from the group comprising the NO₂ group, a halogen atom and a group SO₂X (where X = -CH₃, C₆H₄-CH₃, NH₂, N-(C₁-C₄ alkyl)₂ or NH-C₁-C₄alkyl), -CN, acyl or COO-alkyl, and/or at least one electron-donating group selected from the group comprising groups of the type O-alkyl, NH-CO-alkyl, N(alkyl)CO-alkyl, S-alkyl or alkyl,
with an anthracycline of formula B: 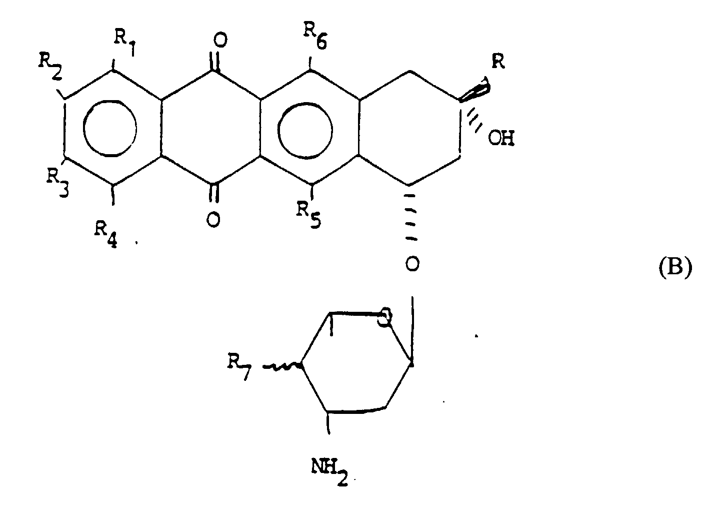 in which R₁, R₂, R₃, R₄, R₅, R₆, R₇ and R are as defined above;
(2) the removal of the protecting groups present in the compounds obtained, especially by hydrolysis, transesterification or saponification; and
(3) if appropriate, suitable condensation with an ose of the following formula: 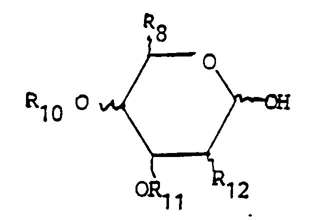 in the case where Z is a hydroxyl group or an O-tri-alkylsilyl group, to give an anthracycline prodrug of formula I, in which all the radicals R₁ to R₁₂ and R are as defined above.

2. Method of preparing a prodrug according to Claim 1, characterized in that when Y is one or more electron-attracting groups, these are preferably in the ortho and/or para position to the glycosyl oxygen, and when Y is one or more electron-donating groups, these are preferably in the meta position.

3. Method of preparing a prodrug according to Claim 1 or Claim 2, characterized in that the preferred compounds of formula I contain the following radicals in particular:
R₁, R₂ and R₃ are hydrogen atoms,
R₄ is a methoxy group,
R₅ and R₆ are hydroxyl groups,
R is a -CO-CH₃ group or a -CO-CH₂OH group,
R₇ is a hydrogen atom or a hydroxyl group,
R₈ is a -CH₂-OAc, -CH₂OH, -COOMe or -COOH group,
R₁₀ and R₁₁, which can be identical or different, are a hydrogen atom or an Ac group,
R₁₂ is a hydroxyl group or an OAc group,
said radicals R₈, R₁₀, R₁₁ and R₁₂ preferably being in the following positions: 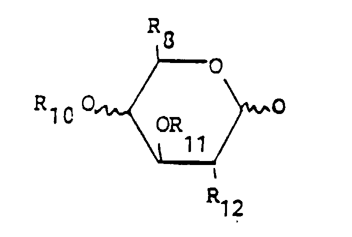 and
Y is a hydrogen atom, an NO₂ group or a chlorine atom in the para or ortho position to the glycosyl oxygen, and/or an OCH₃ group in the meta position to the glycosyl oxygen.

4. Method of preparing a prodrug according to any one of Claims 1 to 3, characterized in that it has formula 6 below:

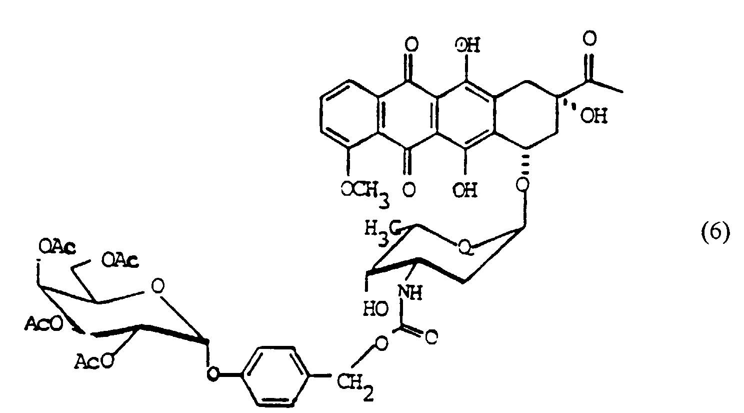

5. Method of preparing a prodrug according to any one of Claims 1 to 3, characterized in that it has formula 7 below:

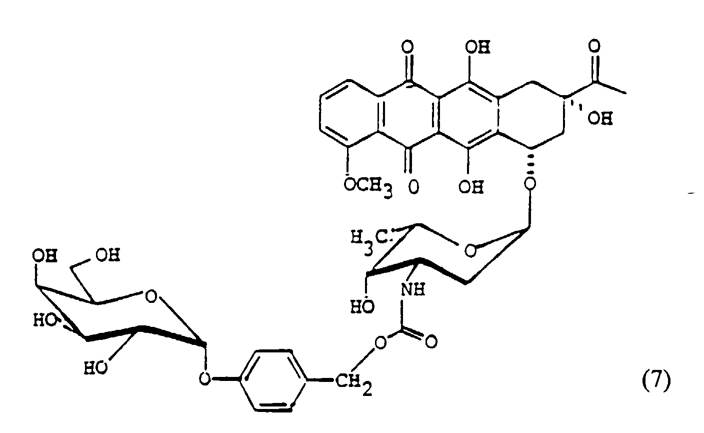

6. Method of preparing a prodrug according to any one of Claims 1 to 3, characterized in that it has formula 13 below:

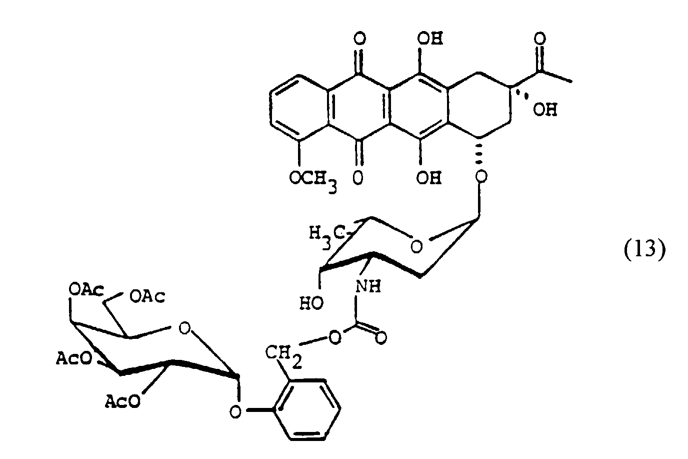

7. Method of preparing a prodrug according to any one of Claims 1 to 3, characterized in that it has formula 14 below:

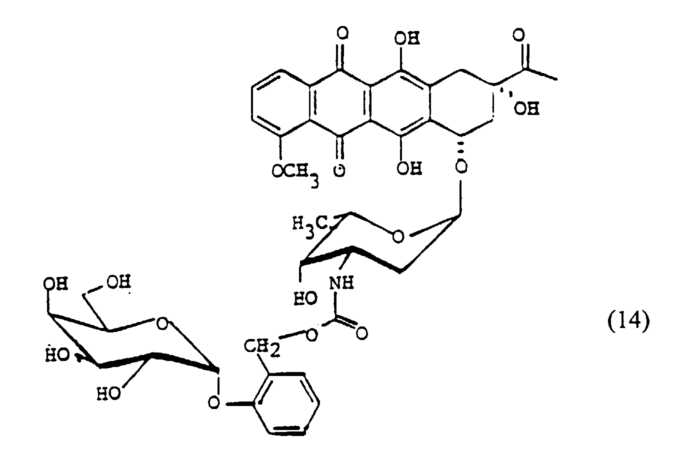

8. Method of preparing a prodrug according to any one of Claims 1 to 3, characterized in that it has formula 22 below:

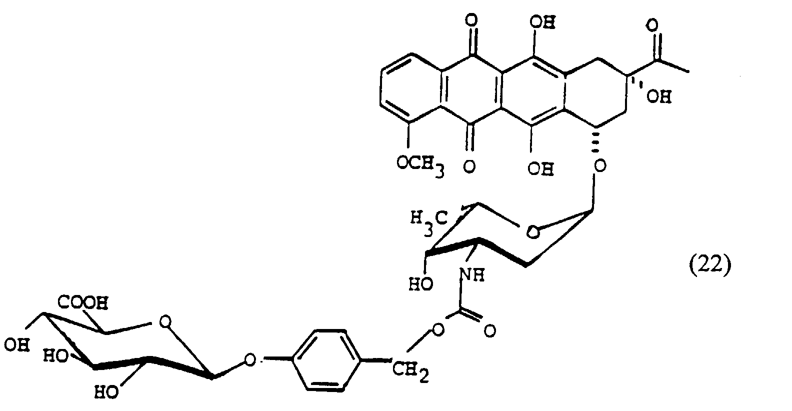

9. Method of preparing a prodrug according to any one of Claims 1 to 3, characterized in that it has formula 27c below:

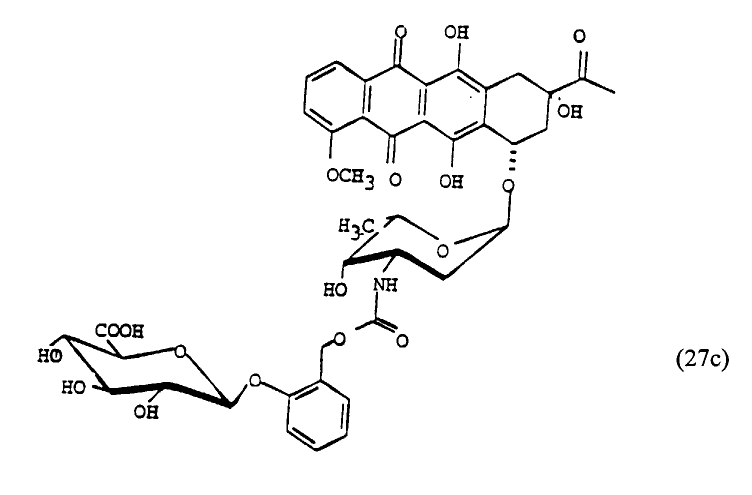

10. Method of preparing a prodrug according to any one of Claims 1 to 3, characterized in that it has formula 36 below:

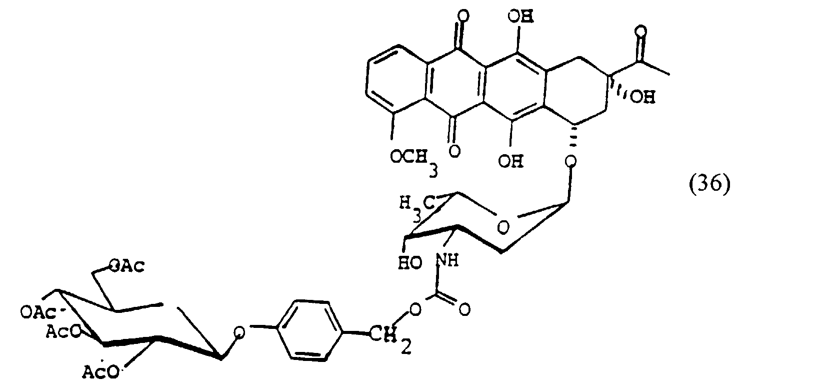

11. Method of preparing a prodrug according to any one of Claims 1 to 3, characterized in that it has formula 37 below:

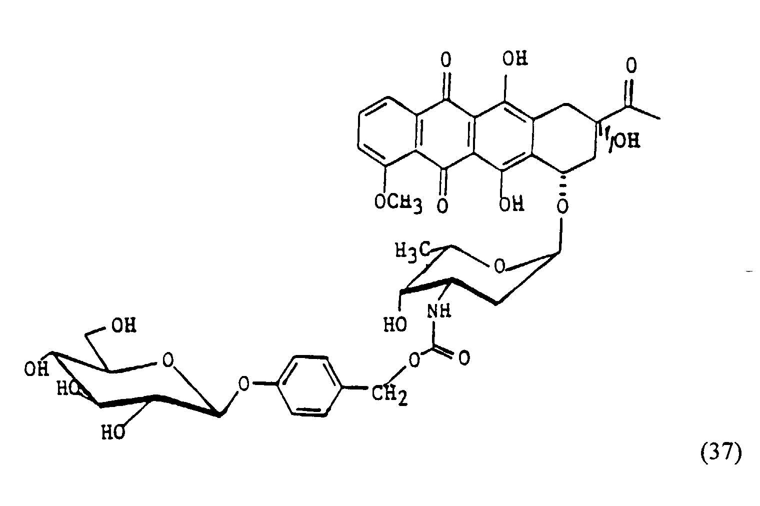

12. Method of preparing a prodrug according to any one of Claims 1 to 3, characterized in that it has formula 48a below:

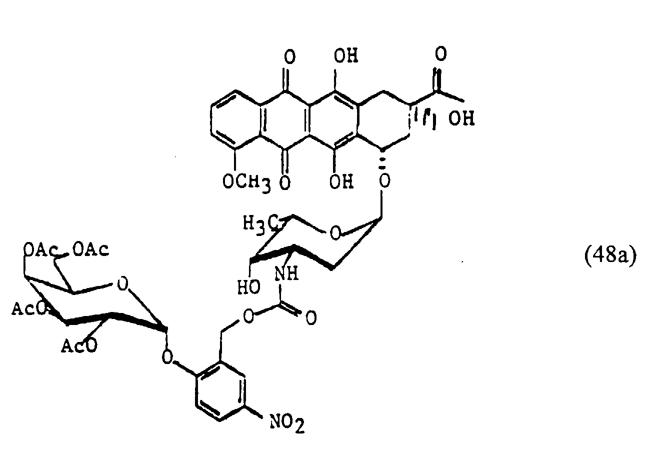

13. Method of preparing a prodrug according to any one of Claims 1 to 3, characterized in that it has formula 48b below:

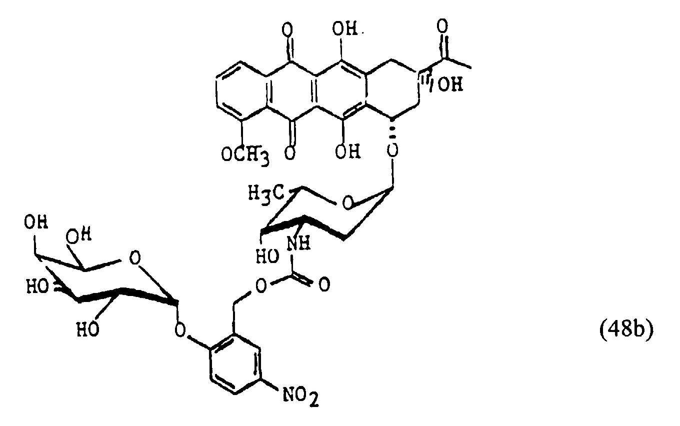

14. Method of preparing a prodrug according to any one of Claims 1 to 3, characterized in that it has formula 49a below:

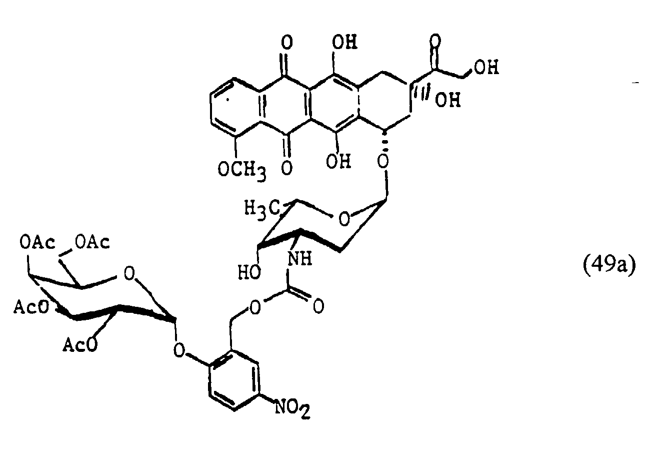

15. Method of preparing a prodrug according to any one of Claims 1 to 3, characterized in that it has formula 49b below:

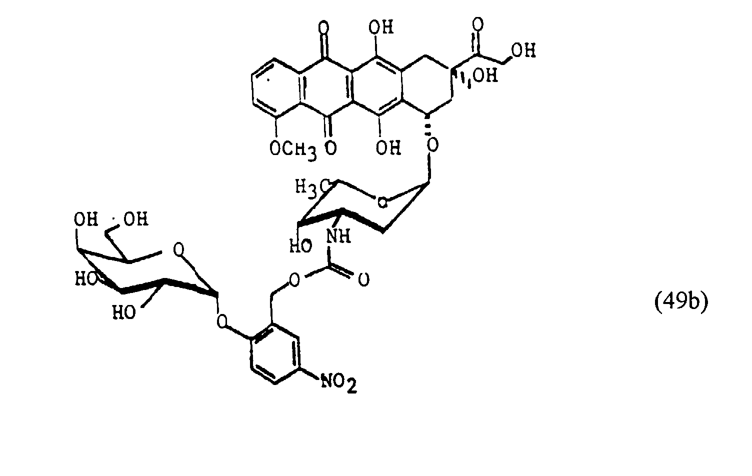

16. Method of preparing a prodrug according to any one of Claims 1 to 3, characterized in that it has formula 54c below:

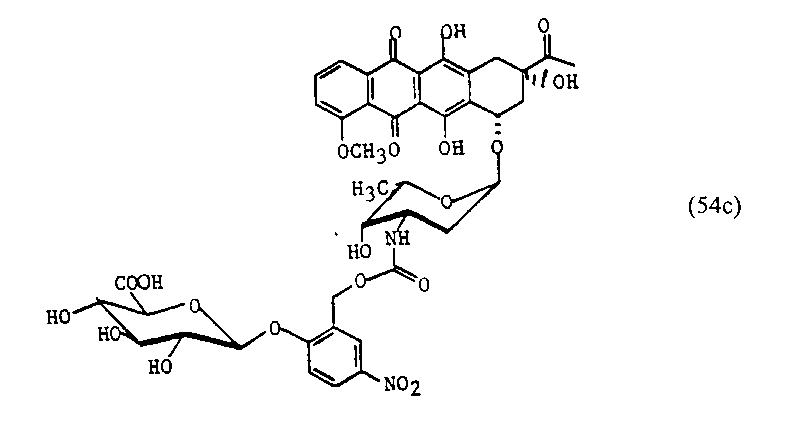

17. Method of preparing a prodrug according to any one of Claims 1 to 3, characterized in that it has formula 59 below:

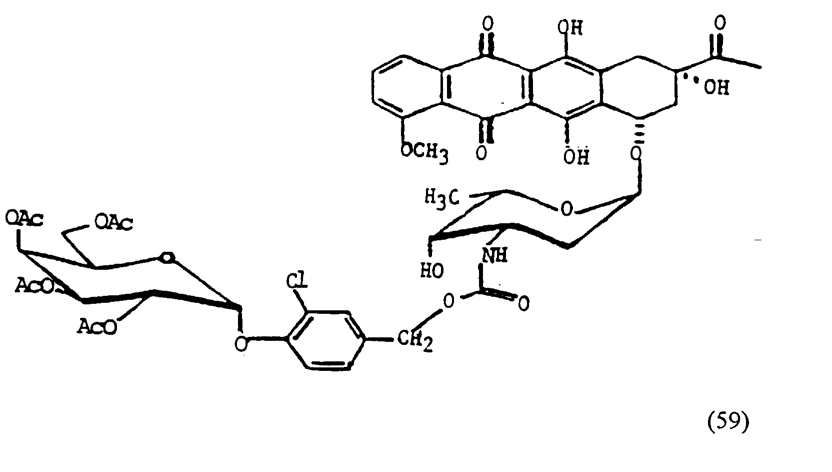

18. Method of preparing a prodrug according to any one of Claims 1 to 3, characterized in that it has formula 60 below:

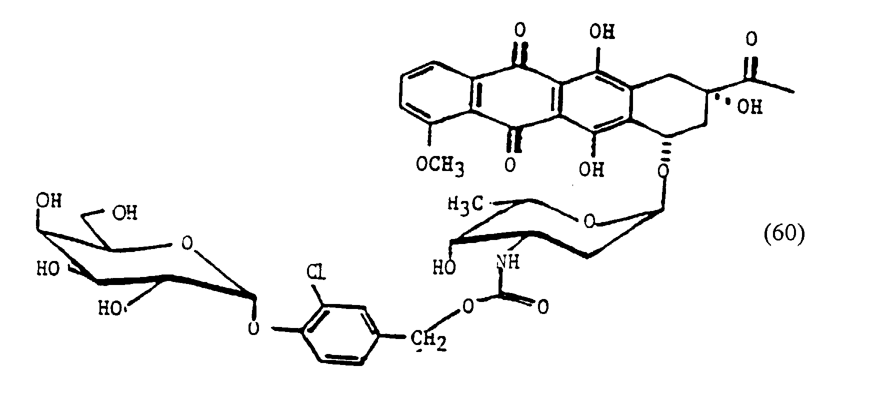

19. Method of preparing a prodrug according to any one of Claims 1 to 3, characterized in that it has formula 64c:

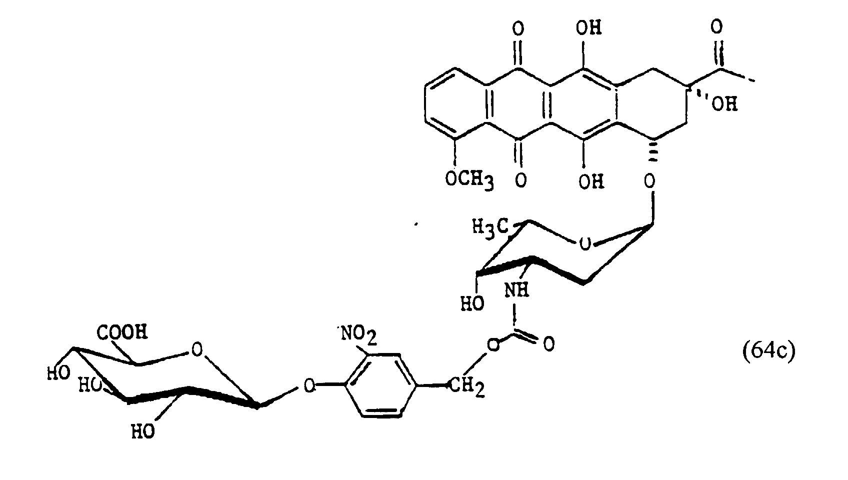

20. Method of preparing a prodrug according to any one of Claims 1 to 3, characterized in that it has formula 68c below:

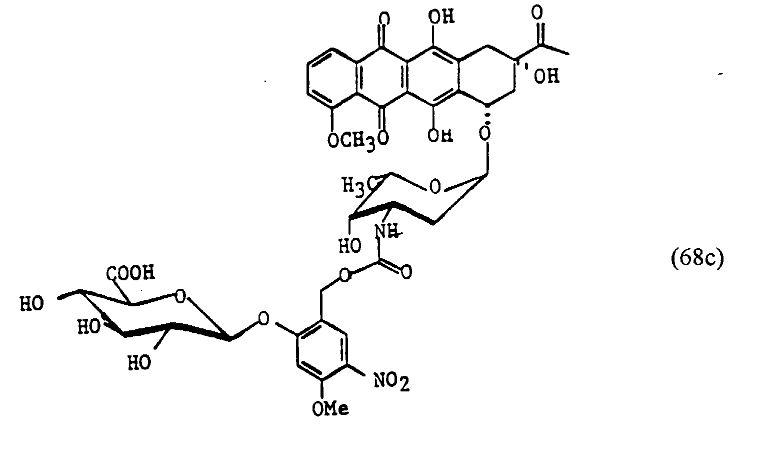

21. Method of preparing a prodrug according to any one of Claims 1 to 3, characterized in that it has formula 75a below:

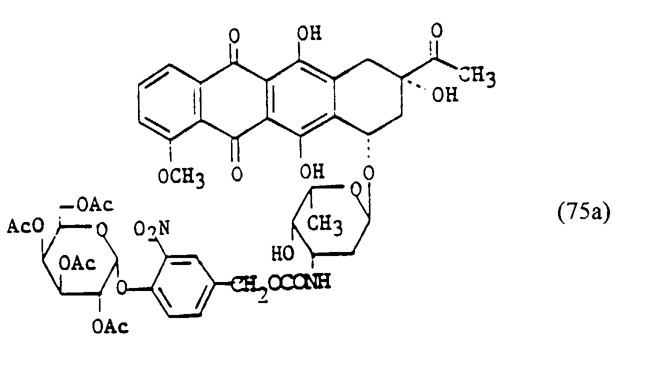

22. Method of preparing a prodrug according to any one of Claims 1 to 3, characterized in that it has formula 75b below:

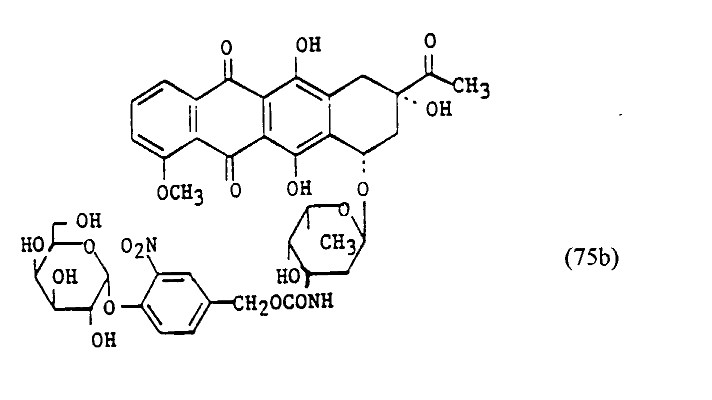

23. Method of preparing a prodrug according to any one of Claims 1 to 3, characterized in that it has formula 78a below:

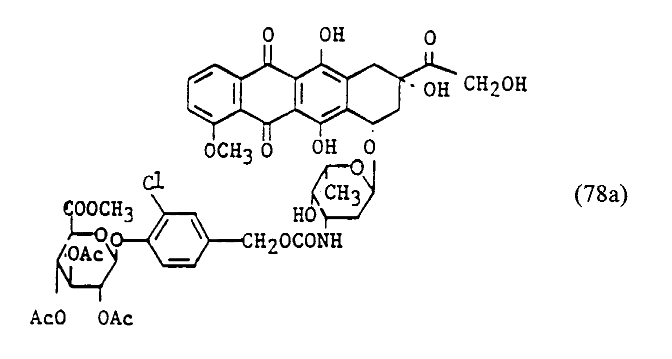

24. Method of preparing a prodrug according to any one of Claims 1 to 3, characterized in that it has formula 78b below:

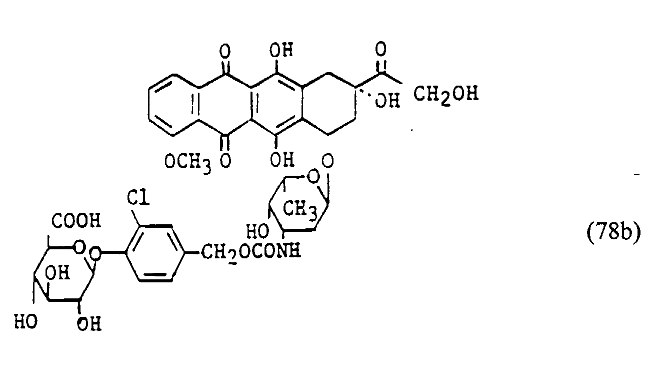

25. Method of preparing a prodrug according to any one of Claims 1 to 3, characterized in that it has formula 70c below: 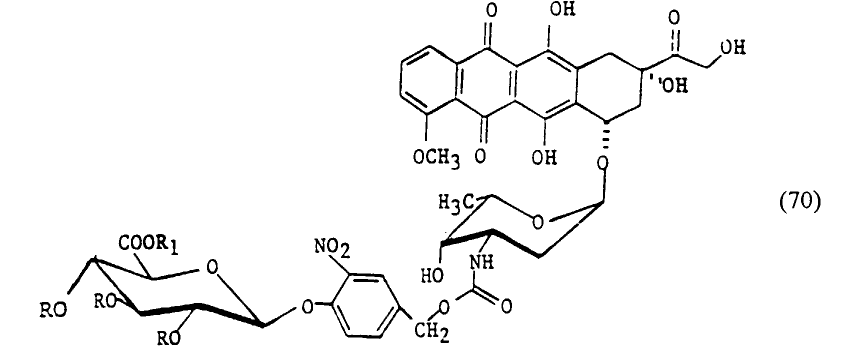 with:
70a: R=Ac R₁=CH₃
70b: R=H R₁=CH₃
70c: R=R₁=H

26. Method of preparing a prodrug according to any one of Claims 1 to 3, characterized in that it has formula 82 below: 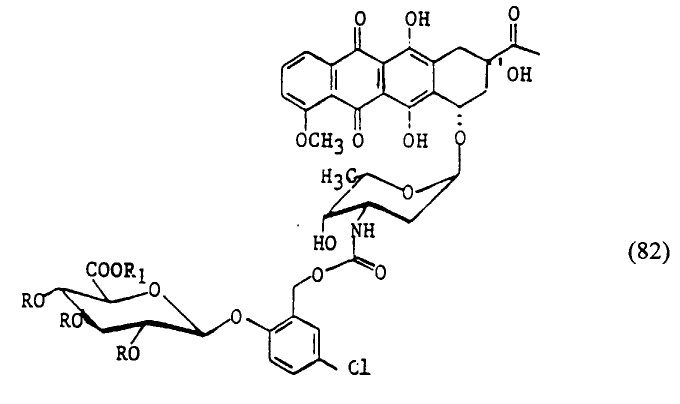 with:
82a : R=Ac, R₁=CH₃
82b : R=H, R₁=CH₃
82c : R=R₁=H

27. Method of preparing a prodrug according to any one of Claims 1 to 3, characterized in that it has formula 83 below: 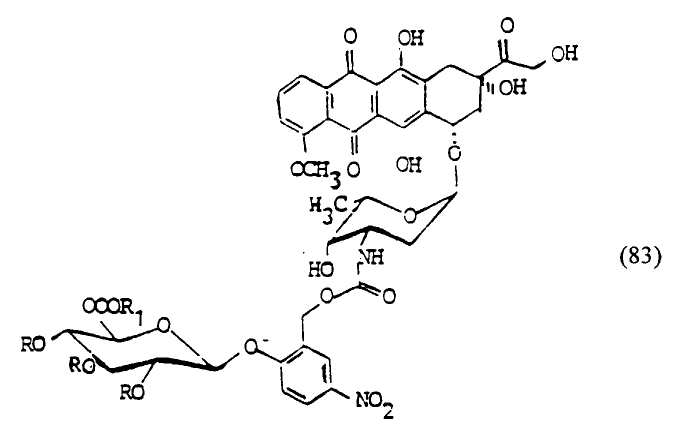 with:
83a : R=Ac, R₁=CH₃
83b : R=H, R₁=CH₃
83c : R=R₁=H

28. A method according to any one of claim 1 to 27, characterized in that, prior to step (1), the glycosylated p-hydroxybenzyl derivative is obtained by:
(a) fusion of a cresol with an ose or a peracetylated methyl glucuronate,
(b) benzyl bromination of the product obtained,
(c) solvolysis of the brominated derivative, and
(d) activation of the hydroxyl group with a hydroxysuccinimidyl or paranitrophenoxycarbonyl deriva tive.

29. Method of preparing products comprising a modified anthracycline prodrug according to any one of Claims 1 to 27, characterized in that it comprises the association of one of said prodrug with an enzyme/tumour-specific antibody conjugate of formula II:
Ab-Sp-E (II)
in which
Ab is an antibody or one of its fragments, which is specific towards an antigen associated with a tumour, or is a biomolecule which tends to accumulate in a tumour, such as EGF (epidermal growth factor), α-TGF (α transforming growth factor), PDGF (platelet derived growth factor), IGF I+II (insulin growth factor I+II) or FGF a+b (fibroblast growth factor a+b),
E is a glycosidase which is not immunogenic or has a very low immunogenicity, preferably a mammalian glycosidase such as α-or β-glucosidase, α-galactosidase, α- or β-mannosidase, α-fucosidase, N- acetyl-α-galactosaminidase, N-acetyl-β-/N-acetyl-α-glucosaminidase or β-glucuronidase, and
Sp (arm) is a group containing a sulphide or a disulphide, of formula III or IV:
X'(S)ₙY' (III)
X'(S)ₙ (IV)
or a polypeptide arm, in which
X' or Y' is -CO-R₁₃-(N-succinimido)- or -C(=R₁₄)-CH₂-CH₂-, where R₁₃ is a -CH₂-CH₂, 1,4-cyclohexylidene, 1,3- or 1,4-phenylene or methoxycarbonyl- or chloro- 1,4-phenylene group and R₁₄ is an oxygen atom or an NH group,
Y' represents -C(=R₁₄)-CH₂-CH₂ when R₁₄ is as defined above, and
n is 1 or 2,
for use in cytostatic therapy, either simultaneously, separately or spread out over a period of time.

30. Method of preparing according to any one of claims 1 to 27, characterized in that it carries out, as an intermediate product, 4-Bromomethylphenyl 2,3,4,6-tetra-O-acetyl-α-D-galactopyranoside of formula 2.

31. Method of preparing according to any one of claims 1 to 27, characterized in that it carries out, as an intermediate product, 4-Formylphenyl 2,3,4,6-tetra-O-acetyl-α-D-galactopyranoside of formula 3.

32. Method of preparing according to any one of claims 1 to 27, characterized in that it carries out, as an intermediate product, 4-Hydroxymethylphenyl 2,3,4,6-tetra-O-acetyl-α-D-galactopyranoside of formula 4.

33. Method of preparing according to any one of claims 1 to 27, characterized in that it carries out, as an intermediate product, 2-Bromomethylphenyl 2,3,4,6-tetra-O-acetyl-α-D-galactopyranoside of formula 9.

34. Method of preparing according to any one of claims 1 to 27, characterized in that it carries out, as an intermediate product, 2-Formylphenyl 2,3,4,6-tetra-O-acetyl-α-D-galactopyranoside of formula 10.

35. Method of preparing according to any one of claims 1 to 27, characterized in that it carries out, as an intermediate product, 2-Hydroxymethylphenyl 2,3,4,6-tetra-O-acetyl-α-D-galactopyranoside of formula 11.

36. Method of preparing according to any one of claims 1 to 27, characterized in that it carries out, as an intermediate product, Methyl (4-bromomethylphenyl 2,3,4-tri-O-acetyl-β-D-glucopyranoside)uronate of formula 16.

37. Method of preparing according to any one of claims 1 to 27, characterized in that it carries out, as an intermediate product, Methyl (4-formylphenyl 2,3,4-tri-O-acetyl-β-D-glucopyranoside)uronate of formula 17.

38. Method of preparing according to any one of claims 1 to 27, characterized in that it carries out, as an intermediate product, Methyl (4-hydroxymethylphenyl 2,3,4-tri-O-acetyl-β-D-glucopyranoside)uronate of formula 18.

39. Method of preparing according to any one of claims 1 to 27, characterized in that it carries out, as an intermediate product, Methyl (2-bromomethylphenyl 2,3,4-tri-O-acetyl-β-D-glucopyranoside)uronate of formula 23.

40. Method of preparing according to any one of claims 1 to 27, characterized in that it carries out, as an intermediate product, Methyl (2-hydroxymethylphenyl 2,3,4-tri-O-acetyl-β-D-glucopyranoside)uronate of formula 24.

41. Method of preparing according to any one of claims 1 to 27, characterized in that it carries out, as an intermediate product, Methyl (2-formylphenyl 2,3,4-tri-O-acetyl-β-D-glucopyranoside)uronate of formula 25.

42. Method of preparing according to any one of claims 1 to 27, characterized in that it carries out, as an intermediate product, 2-Tert-butyldimethylsilyloxybenzaldehyde of formula 28.

43. Method of preparing according to any one of claims 1 to 27, characterized in that it carries out, as an intermediate product, 2-Tert-butyldimethylsilyloxybenzyl alcohol of formula 29.

44. Method of preparing according to any one of claims 1 to 27, characterized in that it carries out, as an intermediate product, N-[2-Hydroxybenzyloxycarbonyl]daunorubicin of formula 32.

45. Method of preparing according to any one of claims 1 to 27, characterized in that it carries out, as an intermediate product, 4-Formylphenyl 2,3,4,6-tetra-O-acetyl-β-D-glucopyranoside of formula 33.

46. Method of preparing according to any one of claims 1 to 27, characterized in that it carries out, as an intermediate product, 4-Hydroxymethylphenyl 2,3,4,6-tetra-O-acetyl-β-D-glucopyranoside of formula 34.

47. Method of preparing according to any one of claims 1 to 27, characterized in that it carries out, as an intermediate product, N-[4-Hydroxybenzyloxycarbonyl]daunorubicin of formula 38.

48. Method of preparing according to any one of claims 1 to 27, characterized in that it carries out, as an intermediate product, 2-Methyl-4-nitrophenyl 2,3,4,6-tetra-O-acetyl-α-D- galactopyranoside of formula 42.

49. Method of preparing according to any one of claims 1 to 27, characterized in that it carries out, as an intermediate product, 2-Bromomethyl-4-nitrophenyl 2,3,4,6-tetra-O-acetyl-α-D-galactopyranoside of formula 43.

50. Method of preparing according to any one of claims 1 to 27, characterized in that it carries out, as an intermediate product, 2-Dibromomethyl-4-nitrophenyl 2,3,4,6-tetra-O-acetyl-α-D-galactopyranoside of formula 44.

51. Method of preparing according to any one of claims 1 to 27, characterized in that it carries out, as an intermediate product, 2-Formyl-4-nitrophenyl 2,3,4,6-tetra-O-acetyl-α-D- galactopyranoside of formula 45.

52. Method of preparing according to any one of claims 1 to 27, characterized in that it carries out, as an intermediate product, 2-Hydroxymethyl-4-nitrophenyl 2,3,4,6-tetra-O-acetyl-α-D-galactopyranoside of formula 46.

53. Method of preparing according to any one of claims 1 to 27, characterized in that it carries out, as an intermediate product, Methyl (2-formyl-4-nitrophenyl 2,3,4-tri-O-acetyl-β-D-glucopyranoside)uronate of formula 51.

54. Method of preparing according to any one of claims 1 to 27, characterized in that it carries out, as an intermediate product, Methyl (2-hydroxymethyl-4-nitrophenyl 2,3,4-tri-O- acetyl-β-D-glucopyranoside)uronate of formula 52.

55. Method of preparing according to any one of claims 1 to 27, characterized in that it carries out, as an intermediate product, 2-Chloro-4-methylphenyl 2,3,4,6-tetra-O-acetyl-α-D- galactopyranoside of formula 55.

56. Method of preparing according to any one of claims 1 to 27, characterized in that it carries out, as an intermediate product, 2-Chloro-4-bromomethylphenyl 2,3,4,6-tetra-O-acetyl-α-D-galactopyranoside of formula 56.

57. Method of preparing according to any one of claims 1 to 27, characterized in that it carries out, as an intermediate product, 2-Chloro-4-hydroxymethylphenyl 2,3,4,6-tetra-O-acetyl-α-D-galactopyranoside of formula 57.

58. Method of preparing according to any one of claims 1 to 27, characterized in that it carries out, as an intermediate product, Methyl (4-formyl-2-nitrophenyl 2,3,4-tri-O-acetyl-β-D-glucopyranoside)uronate of formula 61.

59. Method of preparing according to any one of claims 1 to 27, characterized in that it carries out, as an intermediate product, Methyl (4-hydroxymethyl-2-nitrophenyl 2,3,4-tri-O-acetyl-β-D-glucopyranoside)uronate of formula 62.

60. Method of preparing according to any one of claims 1 to 27, characterized in that it carries out, as an intermediate product, (4-bromomethyl 2-nitro)-phenyl 2,3,4,6-tetra-O-acetyl-α-D-galactopyranoside of formula 73.

61. Method of preparing according to any one of claims 1 to 27, characterized in that it carries out, as an intermediate product, (4-hydroxymethyl 2-nitro)-phenyl 2,3,4,6-tetra-O-acetyl-α-D-galactopyranoside of formula 74.

62. Method of preparing according to any one of claims 1 to 27, characterized in that it carries out, as an intermediate product, 4-hydroxy-3-chlorobenzaldehyde 2,3,4-tri-O-acetyl-β-D-methylglucuronide of formula 76.

63. Method of preparing according to any one of claims 1 to 27, characterized in that it carries out, as an intermediate product, (4-hydroxy-methyl-2-chloro)-phenyl 2,3,4-tri-O-acetyl-β-D-methylglucuronide of formula 77.

64. Method of preparing according to any one of claims 1 to 27, characterized in that it carries out, as an intermediate product, 2,5-Dioxopyrrolidin-1-yl 4-(2,3,4,6-tetra-O-acetyl-α-D-galactopyranosyl)benzyl carbonate of formula 5.

65. Method of preparing according to any one of claims 1 to 27, characterized in that it carries out, as an intermediate product, 2,5-Dioxopyrrolidin-1-yl 2-(2,3,4,6-tetra-O-acetyl-α-D-galactopyranosyl)benzyl carbonate of formula 12.

66. Method of preparing according to any one of claims 1 to 27, characterized in that it carries out, as an intermediate product, 2,5-Dioxopyrrolidin-1-yl 4-(methyl (2,3,4-tri-O-acetyl-β-D-glucopyranosyl)uronate)benzyl carbonate of formula 19.

67. Method of preparing according to any one of claims 1 to 27, characterized in that it carries out, as an intermediate product, 4-Nitrophenyl 2-(methyl (2,3,4-tri-O-acetyl-β-D-glucopyranosyl)uronate)benzyl carbonate of formula 26.

68. Method of preparing according to any one of claims 1 to 27, characterized in that it carries out, as an intermediate product, 4-Nitrophenyl 2-(tert-butyldimethylsilyloxy)benzyl carbonate of formula 30.

69. Method of preparing according to any one of claims 1 to 27, characterized in that it carries out, as an intermediate product, 2,5-Dioxopyrrolidin-1-yl 4-(2,3,4,6-tetra-O-acetyl-β-D-glucopyranosyl)benzyl carbonate of formula 35.

70. Method of preparing according to any one of claims 1 to 27, characterized in that it carries out, as an intermediate product, 2,5-Dioxopyrrolidin-1-yl 4-dimethyl-t-hexylsilyloxybenzyl carbonate of formula 40.

71. Method of preparing according to any one of claims 1 to 27, characterized in that it carries out, as an intermediate product, 4-Nitrophenyl 2-(2,3,4,6-tetra-O-acetyl-α-D-galactopyranosyl)-5-nitrobenzyl carbonate of formula 47.

72. Method of preparing according to any one of claims 1 to 27, characterized in that it carries out, as an intermediate product, 4-Nitrophenyl 2-(methyl (2,3,4-tri-O-acetyl-β-D-glucopyranosyl)uronate)-5-nitrobenzyl carbonate of formula 53.

73. Method of preparing according to any one of claims 1 to 27, characterized in that it carries out, as an intermediate product, 4-Nitrophenyl 4-methoxy-5-nitro-2-(methyl (2,3,4-tri-O-acetyl-β-D-glucopyranosyl)uronate)benzyl carbonate of formula 67.

74. Method of preparing according to any one of claims 1 to 27, characterized in that it carries out, as an intermediate product, 4-Nitro-phenyl 4-(methyl (2,3,4-tri-O-acetyl-β-glucopyranosyl) uronate)-5-nitro-benzyl carbonate of formula 69.

75. Method of preparing according to any one of claims 1 to 27, characterized in that it carries out, as an intermediate product, 4-chlorophenyl 2-(methyl (2,3,4-tri-O-acetyl-β-D-glucopyranosyl) uronate)-5-nitrobenzyl carbonate of formula 81.

76. Method of preparing a drug for the treatment of diseases, which involve activated macrophages, granulocytes, thrombocytes or human tumoural cells, characterized in that it carries out a prodrug as defined in claims 1 to 27.
